# EUROPEAN PATENT SPECIFICATION

(11) **EP 2 494 051 B1**
(45) Date of publication and mention of the grant of the patent: **09.03.2016**
(21) Application number: 10827209.7
(22) Date of filing: 29.10.2010
(51) Int. Cl.: C12N 15/29, C07K 14/415, C12N 15/79

(54) **MODIFIED OIL ENCAPSULATING PROTEINS AND USES THEREOF**
MODIFIZIERTE PROTEINE MIT ÖLEINKAPSELUNG UND ANWENDUNGEN DAVON
PROTÉINES D'ENCAPSULATION D'HUILE MODIFIÉE ET UTILISATIONS DE CELLES-CI

(30) Priority: 30.10.2009 US 256689 P
(43) Date of publication of application: 05.09.2012
(73) Proprietor: Agresearch Limited, Hamilton (NZ)
(72) Inventor: ROBERTS, Nicholas, John, Feilding 4777 (NZ); SCOTT, Richard, WIlliam, Palmerston North 4410 (NZ); WINICHAYAKUL, Somrutai, Palmerston North 4414 (NZ); ROLDAN, Marissa, Palmerston North 4414 (NZ)
(74) Representative: Kremer, Simon Mark
(86) International application number: PCT/NZ2010/000218
(87) International publication number: WO 2011/053169

(56) References cited:
- WO-A1-2004/076673
- WO-A1-2008/130248
- WO-A2-2007/045019
- TING-HANG LIU ET AL: "Stability of Artificial Oil Bodies Constituted with Recombinant Caleosins", JOURNAL OF AGRICULTURAL AND FOOD CHEMISTRY, vol. 57, no. 6, 25 March 2009 (2009-03-25) , pages 2308-2313, XP055056030, ISSN: 0021-8561, DOI: 10.1021/jf803566w
- PURKRTOVA ET AL: "Structural properties of caleosin: A MS and CD study", ARCHIVES OF BIOCHEMISTRY AND BIOPHYSICS, ACADEMIC PRESS, US, vol. 464, no. 2, 8 August 2007 (2007-08-08), pages 335-343, XP022191352, ISSN: 0003-9861, DOI: 10.1016/J.ABB.2007.04.041
- NAOT D ET AL: "Induction of a gene encoding an oleosin homologue in cultured citrus cells exposed to salt stress", GENE, ELSEVIER, AMSTERDAM, NL, vol. 161, no. 2, 19 August 1995 (1995-08-19), pages 171-173, XP004042077, ISSN: 0378-1119, DOI: 10.1016/0378-1119(95)00224-T
- PURKRTOVA Z ET AL: "Structure and function of seed lipid body-associated proteins", COMPTES RENDUS - BIOLOGIES, ELSEVIER, PARIS, FR, vol. 331, no. 10, 1 October 2008 (2008-10-01), pages 746-754, XP025474042, ISSN: 1631-0691, DOI: 10.1016/J.CRVI.2008.07.016 [retrieved on 2008-09-04]
- SCOTT, R. W. ET AL.: 'Elevation of oil body integrity and emulsion stability by polyoleosins, multiple oleosin units joined in tandem head-to-tail fusions' PLANT BIOTECHNOLOGY JOURNAL vol. 8, no. 8, 2010, pages 912 - 927, XP008156324
- FRANDSEN, G. I. ET AL.: 'Oil bodies and their associated proteins, oleosin and caleosin' PHYSIOLOGICA PLANTARUM vol. 112, no. 3, 2001, pages 301 - 307, XP008156318
- CHEN, M. C. M. ET AL.: 'Constitution of Stable Artificial Oil Bodies with Triacylglycerol, Phospholipid, and Caleosin' JOURNAL OF AGRICULTURAL AND FOOD CHEMISTRY vol. 52, no. 12, 16 June 2004, pages 3982 - 3987, XP008156321

### SEQUENCE LISTING

<110> AGRESEARCH LIMITED
<120> MODIFIED OIL ENCAPSULATING PROTEINS AND USES THEREOF
<130> 631138
<150> 61/256,689 <151> 2009-10-30
<160> 107
<170> Patent In version 3.5
<210> 1
   <211> 579
   <212> DNA
   <213> Artificial Sequence
<220>
   <223> Artificial
<400> 1
   atgaaagaaa ccgctgctgc taaattcgaa cgccagcaca tggacagccc agatctgggt      60
   accctggtgc cacgcggttc catggctgag cattatggtc aacaacagca gaccagggcg     120
   cctcacctgc agctgcagcc gcgcgcccag cgggtagtga aggcggccac cgccgtgaca     180
   gccggcggct cgcttctcgt cctctctggc ctcactttag ccggaactgt tattgcgctc     240
   accatcgcca ctccgctgct tgtgatcttt agccccgttc tggtgccggc ggtcataacc     300
   attttcttgc tgggtgcggg ttttctggca tccggaggct tcggcgtggc ggcgctgagt     360
   gtgctgtcgt ggatttacag atatctgaca gggaaacacc cgccgggggc ggatcagctg     420
   gaatcggcaa agacgaagct ggcgagcaag gcgcgagaga tgaaggatag ggcagagcag     480
   ttctcgcagc agcctgttcc atggctgata tcggatccga attcgagctc cgtcgacaag     540
   cttgcggccg cactcgagca ccaccaccac caccactga                            579
<210> 2
   <211> 531
   <212> DNA
   <213> Artificial sequence
<220>
   <223> Artificial
<400> 2
   atgaaagaaa ccgctgctgc taaattcgaa cgccagcaca tggacagccc agatctgggt 60
   accctggtgc cacgcggttc catggcttgt cattatggtc aacaacagca gaccagggcg 120
   cctcacctgc agctgcagcc gcgcgcccag cgggtagtga aggcggccac cgccgtgaca 180
   gccggcggat cccttctcgt cctctctggc ctcactttag ccggaactgt tattgcgctc 240
   accatcgcca ctccgctgct tgtgatcttt agccccgttc tggtgccggc ggtcataacc 300
   attttcttgc tgggtgcggg ttttctggca tccggaggct tcggcgtggc ggcgctgagt 360
   gtgctgtcgt ggatttacag atatctgaca gggaaacacc cgccgggggc ggatcagctg 420
   gaatcggcaa agacgaagct ggcgagcaag gcgcgagaga tgaaggatag ggcagagcag 480
   ttctcgtgcc agcctgttcc gtggctcgag caccaccacc accaccactg a 531
<210> 3
   <211> 531
   <212> DNA
   <213> Artificial Sequence
<220>
   <223> Artificial
<400> 3
   atgaaagaaa ccgctgctgc taaattcgaa cgccagcaca tggacagccc agatctgggt 60
   accctggtgc cacgcggttc catggcttgt cattatggtc aacaacagca gaccagggcg 120
   cctcacctgc agctgcagcc gcgcgcccag cgggtagtga aggcggccac cgccgtgaca 180
   gccggcggat cccttctcgt cctctctggc ctcactttag ccggaactgt tattgcgctc 240
   accatcgcca ctccgctgct tgtgatcttt agccccgttc tggtgccggc ggtcataacc 300
   attttcttgc tgggtgcggg ttttctggca tccggaggct tcggcgtggc ggcgctgagt 360
   gtgctgtcgt ggatttacag atatctgaca gggaaacacc cgccgggggc ggattgcctg 420
   gaatcggcaa agacgaagct ggcgagctgt gcgcgagaga tgaaggatag ggcagagcag 480
   ttctcgtgcc agcctgttcc gtggctcgag caccaccacc accaccactg a 531
<210> 4
   <211> 531
   <212> DNA
   <213> Artificial Sequence
<220>
   <223> Artificial
<400> 4
   atgaaagaaa ccgctgctgc taaattcgaa cgccagcaca tggacagccc agatctgggt     60
   accctggtgc cacgcggttc catggcttgt cattatggtc aacaacagca gacctgcgcg    120
   cctcacctgc agctgcagcc gcgcgcctgt cgggtagtga aggcggccac cgccgtgaca    180
   gcgggcggat cccttctcgt cctctctggc ctcactttag ccggtaccgt tattgcgctc    240
   accatcgcca ctccgctgct tgtgatcttt agccccgttc tggttccggc ggtcataacc    300
   attttcttgc tgggtgcggg ttttctggca tccggaggct tcggcgtggc ggcgctgagt    360
   gtgctgtcgt ggatttacag atatctgaca gggaaacacc cgccgggggc ggatcagctg    420
   gaatcggcaa agacgaagct ggcgagcaag gcgcgagaga tgaaggatag ggcagagcag    480
   ttctcgtgcc agcctgttcc gtggctcgag caccaccacc accaccactg a             531
<210> 5
   <211> 531
   <212> DNA
   <213> Artificial Sequence
<220>
   <223> Artificial
<400> 5
   atgaaagaaa ccgctgctgc taaattcgaa cgccagcaca tggacagccc agatctgggt     60
   accctggtgc cacgcggttc catggcttgt cattatggtc aacaacagca gacctgcgcg    120
   cctcacctgc agctgcagcc gcgcgcctgt cgggtagtga aggcggccac cgccgtgaca    180
   gcgggcggat cccttctcgt cctctctggc ctcactttag ccggtaccgt tattgcgctc    240
   accatcgcca ctccgctgct tgtgatcttt agccccgttc tggttccggc ggtcataacc    300
   attttcttgc tgggtgcggg ttttctggca tccggaggct tcggcgtggc ggcgctgagt    360
   gtgctgtcgt ggatttacag atatctgaca gggaaacacc cgccgggggc ggattgcctg    420
   gaatcggcaa agacgaagct ggcgagctgt gcgcgagaga tgaaggatag ggcagagcag    480
   ttctcgtgcc agcctgttcc gtggctcgag caccaccacc accaccactg a             531
<210> 6
   <211> 193
   <212> PRT
   <213> Artificial Sequence
<220>
   <223> Artificial
<400> 6
   Met Lys Glu Thr Ala Ala Ala Lys Phe Glu Arg Gln His Met Asp Ser
   1 5 10 15
   Pro Asp Leu Gly Thr Leu Val Pro Arg Gly Ser Met Ala Glu His Tyr
   20 25 30
   Gly Gln Gln Gln Gln Thr Arg Ala Pro His Leu Gln Leu Gln Pro Arg
   35 40 45
   Ala Gln Arg Val Val Lys Ala Ala Thr Ala Val Thr Ala Gly Gly Ser
   50 55 60
   Leu Leu Val Leu Ser Gly Leu Thr Leu Ala Gly Thr Val Ile Ala Leu
   65 70 75 80
   Thr Ile Ala Thr Pro Leu Leu Val Ile Phe Ser Pro Val Leu Val Pro
   85 90 95
   Ala Val Ile Thr Ile Phe Leu Leu Gly Ala Gly Phe Leu Ala Ser Gly
   100 105 110
   Gly Phe Gly Val Ala Ala Leu Ser Val Leu Ser Trp Ile Tyr Arg Tyr
   115 120 125
   Leu Thr Gly Lys His Pro Pro Gly Ala Asp Gln Leu Glu Ser Ala Lys
   130 135 140
   Thr Lys Leu Ala Ser Lys Ala Arg Glu Met Lys Asp Arg Ala Glu Gln
   145 150 155 160
   Phe Ser Gln Gln Pro Val Pro Trp Leu Ile Ser Asp Pro Asn Ser Ser
   165 170 175
   Ser Val Asp Lys Leu Ala Ala Ala Leu Glu His His His His His His
   180 185 190
   His
<210> 7
   <211> 176
   <212> PRT
   <213> Artificial Sequence
<220>
   <223> Artificial
<400> 7
   Met Lys Glu Thr Ala Ala Ala Lys Phe Glu Arg Gln His Met Asp Ser
   1 5 10 15
   Pro Asp Leu Gly Thr Leu Val Pro Arg Gly Ser Met Ala Cys His Tyr
   20 25 30
   Gly Gln Gln Gln Gln Thr Arg Ala Pro His Leu Gln Leu Gln Pro Arg
   35 40 45
   Ala Gln Arg Val Val Lys Ala Ala Thr Ala Val Thr Ala Gly Gly Ser
   50 55 60
   Leu Leu Val Leu Ser Gly Leu Thr Leu Ala Gly Thr Val Ile Ala Leu
   65 70 75 80
   Thr Ile Ala Thr Pro Leu Leu Val Ile Phe Ser Pro Val Leu Val Pro
   85 90 95
   Ala Val Ile Thr Ile Phe Leu Leu Gly Ala Gly Phe Leu Ala Ser Gly
   100 105 110
   Gly Phe Gly Val Ala Ala Leu Ser Val Leu Ser Trp Ile Tyr Arg Tyr
   115 120 125
   Leu Thr Gly Lys His Pro Pro Gly Ala Asp Gln Leu Glu Ser Ala Lys
   130 135 140
   Thr Lys Leu Ala Ser Lys Ala Arg Glu Met Lys Asp Arg Ala Glu Gln
   145 150 155 160
   Phe Ser Cys Gln Pro Val Pro Trp Leu Glu His His His His His His
   165 170 175
<210> 8
   <211> 176
   <212> PRT
   <213> Artificial Sequence
<220>
   <223> Artificial
<400> 8
   Met Lys Glu Thr Ala Ala Ala Lys Phe Glu Arg Gln His Met Asp Ser
   1 5 10 15
   Pro Asp Leu Gly Thr Leu Val Pro Arg Gly Ser Met Ala Cys His Tyr
   20 25 30
   Gly Gln Gln Gln Gln Thr Arg Ala Pro His Leu Gln Leu Gln Pro Arg
   35 40 45
   Ala Gln Arg Val Val Lys Ala Ala Thr Ala Val Thr Ala Gly Gly Ser
   50 55 60
   Leu Leu Val Leu Ser Gly Leu Thr Leu Ala Gly Thr Val Ile Ala Leu
   65 70 75 80
   Thr Ile Ala Thr Pro Leu Leu Val Ile Phe Ser Pro Val Leu Val Pro
   85 90 95
   Ala Val Ile Thr Ile Phe Leu Leu Gly Ala Gly Phe Leu Ala Ser Gly
   100 105 110
   Gly Phe Gly Val Ala Ala Leu Ser Val Leu Ser Trp Ile Tyr Arg Tyr
   115 120 125
   Leu Thr Gly Lys His Pro Pro Gly Ala Asp Cys Leu Glu Ser Ala Lys
   130 135 140
   Thr Lys Leu Ala Ser Cys Ala Arg Glu Met Lys Asp Arg Ala Glu Gln
   145 150 155 160
   Phe Ser Cys Gln Pro Val Pro Trp Leu Glu His His His His His His
   165 170 175
<210> 9
   <211> 176
   <212> PRT
   <213> Artificial Sequence
<220>
   <223> Artificial
<400> 9
   Met Lys Glu Thr Ala Ala Ala Lys Phe Glu Arg Gln His Met Asp Ser
   1 5 10 15
   Pro Asp Leu Gly Thr Leu Val Pro Arg Gly Ser Met Ala Cys His Tyr
   20 25 30
   Gly Gln Gln Gln Gln Thr Cys Ala Pro His Leu Gln Leu Gln Pro Arg
   35 40 45
   Ala Cys Arg Val Val Lys Ala Ala Thr Ala Val Thr Ala Gly Gly Ser
   50 55 60
   Leu Leu Val Leu Ser Gly Leu Thr Leu Ala Gly Thr Val Ile Ala Leu
   65 70 75 80
   Thr Ile Ala Thr Pro Leu Leu Val Ile Phe Ser Pro Val Leu Val Pro
   85 90 95
   Ala Val Ile Thr Ile Phe Leu Leu Gly Ala Gly Phe Leu Ala Ser Gly
   100 105 110
   Gly Phe Gly Val Ala Ala Leu Ser Val Leu Ser Trp Ile Tyr Arg Tyr
   115 120 125
   Leu Thr Gly Lys His Pro Pro Gly Ala Asp Gln Leu Glu Ser Ala Lys
   130 135 140
   Thr Lys Leu Ala Ser Lys Ala Arg Glu Met Lys Asp Arg Ala Glu Gln
   145 150 155 160
   Phe Ser Cys Gln Pro Val Pro Trp Leu Glu His His His His His His
   165 170 175
<210> 10
   <211> 176
   <212> PRT
   <213> Artificial Sequence
<220>
   <223> Artificial
<400> 10
   Met Lys Glu Thr Ala Ala Ala Lys Phe Glu Arg Gln His Met Asp Ser
   1               5                   10                  15
   Pro Asp Leu Gly Thr Leu Val Pro Arg Gly Ser Met Ala Cys His Tyr
               20                  25                  30
   Gly Gln Gln Gln Gln Thr Cys Ala Pro His Leu Gln Leu Gln Pro Arg
           35                  40                  45
   Ala Cys Arg Val Val Lys Ala Ala Thr Ala Val Thr Ala Gly Gly Ser
       50                  55                  60
   Leu Leu Val Leu Ser Gly Leu Thr Leu Ala Gly Thr Val Ile Ala Leu
   65                  70                  75                  80
   Thr Ile Ala Thr Pro Leu Leu Val Ile Phe Ser Pro Val Leu Val Pro
                   85                  90                  95
   Ala Val Ile Thr Ile Phe Leu Leu Gly Ala Gly Phe Leu Ala Ser Gly
               100                 105                 110
   Gly Phe Gly Val Ala Ala Leu Ser Val Leu Ser Trp Ile Tyr Arg Tyr
           115                 120                 125
   Leu Thr Gly Lys His Pro Pro Gly Ala Asp Cys Leu Glu Ser Ala Lys
       130                 135                 140
   Thr Lys Leu Ala Ser Cys Ala Arg Glu Met Lys Asp Arg Ala Glu Gln
   145                 150                 155                 160
   Phe Ser Cys Gln Pro Val Pro Trp Leu Glu His His His His His His
                   165                 170                 175
<210> 11
   <211> 766
   <212> DNA
   <213> Artificial Sequence
<220>
   <223> Artificial
<400> 11
   ttgctccctt aaaaaaaacc atggctgagc attatggtca acaacagcag accagggcgc      60
   ctcacctgca gctgcagccg cgcgcccagc gggtagtgaa ggcggccacc gccgtgacag     120
   taaatttctg tgttccttat tctctcaaaa tcttcgattt tgttttcgtt cgatcccaat     180
   ttcgtatatg ttctttggtt tagattctgt taatcttaga tcgaagacga ttttctgggt     240
   ttgatcgtta gatatcatct taattctcga ttagggtttc atagatatca tccgatttgt     300
   tcaaataatt tgagttttgt cgaataatta ctcttcgatt tgtgatttct atctagatct     360
   ggtgttagtt tctagtttgt gcgatcgaat ttgtcgatta atctgagttt ttctgattaa     420
   caggccggcg gctcgcttct cgtcctctct ggcctcactt tagccggaac tgttattgcg     480
   ctcaccatcg ccactccgct gcttgtgatc tttagccccg ttctggtgcc ggcggtcata     540
   accattttct tgctgggtgc gggttttctg gcatccggag gcttcggcgt ggcggcgctg     600
   agtgtgctgt cgtggattta cagatatctg acagggaaac acccgccggg ggcggatcag     660
   ctggaatcgg caaagacgaa gctggcgagc aaggcgcgag agatgaagga tagggcagag     720
   cagttctcgc agcagcctgt tgcggggtct caaacttctt aatgaa                    766
<210> 12
   <211> 766
   <212> DNA
   <213> Artificial Sequence
<220>
   <223> Artificial
<400> 12
   ttgctccctt aaaaaaaacc atggcttgtc attatggtca acaacagcag accagggcgc      60
   ctcacctgca gctgcagccg cgcgcccagc gggtagtgaa ggcggccacc gccgtgacag     120
   taaatttctg tgttccttat tctctcaaaa tcttcgattt tgttttcgtt cgatcccaat     180
   ttcgtatatg ttctttggtt tagattctgt taatcttaga tcgaagacga ttttctgggt     240
   ttgatcgtta gatatcatct taattctcga ttagggtttc atagatatca tccgatttgt     300
   tcaaataatt tgagttttgt cgaataatta ctcttcgatt tgtgatttct atctagatct     360
   ggtgttagtt tctagtttgt gcgatcgaat ttgtcgatta atctgagttt ttctgattaa     420
   caggccggcg gctcgcttct cgtcctctct ggcctcactt tagccggaac tgttattgcg     480
   ctcaccatcg ccactccgct gcttgtgatc tttagccccg ttctggtgcc ggcggtcata     540
   accattttct tgctgggtgc gggttttctg gcatccggag gcttcggcgt ggcggcgctg     600
   agtgtgctgt cgtggattta cagatatctg acagggaaac acccgccggg ggcggatcag     660
   ctggaatcgg caaagacgaa gctggcgagc aaggcgcgag agatgaagga tagggcagag     720
   cagttctcgt gtcagcctgt tgcggggtct caaacttctt aatgaa                    766
<210> 13
   <211> 766
   <212> DNA
   <213> Artificial Sequence
<220>
   <223> Artificial
<400> 13
   ttgctccctt aaaaaaaacc atggcttgtc attatggtca acaacagcag accagggcgc      60
   ctcacctgca gctgcagccg cgcgcccagc gggtagtgaa ggcggccacc gccgtgacag     120
   taaatttctg tgttccttat tctctcaaaa tcttcgattt tgttttcgtt cgatcccaat     180
   ttcgtatatg ttctttggtt tagattctgt taatcttaga tcgaagacga ttttctgggt     240
   ttgatcgtta gatatcatct taattctcga ttagggtttc atagatatca tccgatttgt     300
   tcaaataatt tgagttttgt cgaataatta ctcttcgatt tgtgatttct atctagatct     360
   ggtgttagtt tctagtttgt gcgatcgaat ttgtcgatta atctgagttt ttctgattaa     420
   caggccggcg gctcgcttct cgtcctctct ggcctcactt tagccggaac tgttattgcg     480
   ctcaccatcg ccactccgct gcttgtgatc tttagccccg ttctggtgcc ggcggtcata     540
   accattttct tgctgggtgc gggttttctg gcatccggag gcttcggcgt ggcggcgctg     600
   agtgtgctgt cgtggattta cagatatctg acagggaaac acccgccggg ggcggattgt     660
   ctggaatcgg caaagacgaa gctggcgagc tgtgcgcgag agatgaagga tagggcagag     720
   cagttctcgt gtcagcctgt tgcggggtct caaacttctt aatgaa                    766
<210> 14
   <211> 766
   <212> DNA
   <213> Artificial Sequence
<220>
   <223> Artificial
<400> 14
   ttgctccctt aaaaaaaacc atggcttgtc attatggtca acaacagcag acctgtgcgc    60
   ctcacctgca gctgcagccg cgcgcctgtc gggtagtgaa ggcggccacc gccgtgacag   120
   taaatttctg tgttccttat tctctcaaaa tcttcgattt tgttttcgtt cgatcccaat   180
   ttcgtatatg ttctttggtt tagattctgt taatcttaga tcgaagacga ttttctgggt   240
   ttgatcgtta gatatcatct taattctcga ttagggtttc atagatatca tccgatttgt   300
   tcaaataatt tgagttttgt cgaataatta ctcttcgatt tgtgatttct atctagatct   360
   ggtgttagtt tctagtttgt gcgatcgaat ttgtcgatta atctgagttt ttctgattaa   420
   caggccggcg gctcgcttct cgtcctctct ggcctcactt tagccggaac tgttattgcg   480
   ctcaccatcg ccactccgct gcttgtgatc tttagccccg ttctggtgcc ggcggtcata   540
   accattttct tgctgggtgc gggttttctg gcatccggag gcttcggcgt ggcggcgctg   600
   agtgtgctgt cgtggattta cagatatctg acagggaaac acccgccggg ggcggatcag   660
   ctggaatcgg caaagacgaa gctggcgagc aaggcgcgag agatgaagga tagggcagag   720
   cagttctcgt gtcagcctgt tgcggggtct caaacttctt aatgaa                  766
<210> 15
   <211> 766
   <212> DNA
   <213> Artificial Sequence
<220>
   <223> Artificial
<400> 15
   ttgctccctt aaaaaaaacc atggcttgtc attatggtca acaacagcag acctgtgcgc    60
   ctcacctgca gctgcagccg cgcgcctgtc gggtagtgaa ggcggccacc gccgtgacag   120
   taaatttctg tgttccttat tctctcaaaa tcttcgattt tgttttcgtt cgatcccaat   180
   ttcgtatatg ttctttggtt tagattctgt taatcttaga tcgaagacga ttttctgggt   240
   ttgatcgtta gatatcatct taattctcga ttagggtttc atagatatca tccgatttgt   300
   tcaaataatt tgagttttgt cgaataatta ctcttcgatt tgtgatttct atctagatct   360
   ggtgttagtt tctagtttgt gcgatcgaat ttgtcgatta atctgagttt ttctgattaa   420
   caggccggcg gctcgcttct cgtcctctct ggcctcactt tagccggaac tgttattgcg   480
   ctcaccatcg ccactccgct gcttgtgatc tttagccccg ttctggtgcc ggcggtcata   540
   accattttct tgctgggtgc gggttttctg gcatccggag gcttcggcgt ggcggcgctg   600
   agtgtgctgt cgtggattta cagatatctg acagggaaac acccgccggg ggcggattgt   660
   ctggaatcgg caaagacgaa gctggcgagc tgtgcgcgag agatgaagga tagggcagag   720
   cagttctcgt gtcagcctgt tgcggggtct caaacttctt aatgaa                  766
<210> 16
   <211> 145
   <212> PRT
   <213> Artificial Sequence
<220>
   <223> Artificial
<400> 16
   Met Ala Glu His Tyr Gly Gln Gln Gln Gln Thr Arg Ala Pro His Leu
   1               5                   10                  15
   Gln Leu Gln Pro Arg Ala Gln Arg Val Val Lys Ala Ala Thr Ala Val
               20                  25                  30
   Thr Ala Gly Gly Ser Leu Leu Val Leu Ser Gly Leu Thr Leu Ala Gly
           35                  40                  45
   Thr Val Ile Ala Leu Thr Ile Ala Thr Pro Leu Leu Val Ile Phe Ser
       50                  55                  60
   Pro Val Leu Val Pro Ala Val Ile Thr Ile Phe Leu Leu Gly Ala Gly
   65                  70                  75                  80
   Phe Leu Ala Ser Gly Gly Phe Gly Val Ala Ala Leu Ser Val Leu Ser
                   85                  90                  95
   Trp Ile Tyr Arg Tyr Leu Thr Gly Lys His Pro Pro Gly Ala Asp Gln
               100                 105                 110
   Leu Glu Ser Ala Lys Thr Lys Leu Ala Ser Lys Ala Arg Glu Met Lys
           115                 120                 125
   Asp Arg Ala Glu Gln Phe Ser Gln Gln Pro Val Ala Gly Ser Gln Thr
       130                 135                 140
   Ser
   145
<210> 17
   <211> 145
   <212> PRT
   <213> Artificial Sequence
<220>
   <223> Artificial
<400> 17
   Met Ala Cys His Tyr Gly Gln Gln Gln Gln Thr Arg Ala Pro His Leu
   1               5                   10                  15
   Gln Leu Gln Pro Arg Ala Gln Arg Val Val Lys Ala Ala Thr Ala Val
                   20                  25                  30
   Thr Ala Gly Gly Ser Leu Leu Val Leu Ser Gly Leu Thr Leu Ala Gly
           35                  40                  45
   Thr Val Ile Ala Leu Thr Ile Ala Thr Pro Leu Leu Val Ile Phe Ser
       50                  55                  60
   Pro Val Leu Val Pro Ala Val Ile Thr Ile Phe Leu Leu Gly Ala Gly
   65                  70                  75                  80
   Phe Leu Ala Ser Gly Gly Phe Gly Val Ala Ala Leu Ser Val Leu Ser
                   85                  90                  95
   Trp Ile Tyr Arg Tyr Leu Thr Gly Lys His Pro Pro Gly Ala Asp Gln
               100                 105                 110
   Leu Glu Ser Ala Lys Thr Lys Leu Ala Ser Lys Ala Arg Glu Met Lys
           115                 120                 125
   Asp Arg Ala Glu Gln Phe Ser Cys Gln Pro Val Ala Gly Ser Gln Thr
       130                 135                 140
   Ser
   145
<210> 18
   <211> 145
   <212> PRT
   <213> Artificial Sequence
<220>
   <223> Artificial
<400> 18
   Met Ala Cys His Tyr Gly Gln Gln Gln Gln Thr Arg Ala Pro His Leu
   1 5 10 15
   Gln Leu Gln Pro Arg Ala Gln Arg Val Val Lys Ala Ala Thr Ala Val
   20 25 30
   Thr Ala Gly Gly Ser Leu Leu Val Leu Ser Gly Leu Thr Leu Ala Gly
   35 40 45
   Thr Val Ile Ala Leu Thr Ile Ala Thr Pro Leu Leu Val Ile Phe Ser
   50 55 60
   Pro Val Leu Val Pro Ala Val Ile Thr Ile Phe Leu Leu Gly Ala Gly
   65 70 75 80
   Phe Leu Ala Ser Gly Gly Phe Gly Val Ala Ala Leu Ser Val Leu Ser
   85 90 95
   Trp Ile Tyr Arg Tyr Leu Thr Gly Lys His Pro Pro Gly Ala Asp Cys
   100 105 110
   Leu Glu Ser Ala Lys Thr Lys Leu Ala Ser Cys Ala Arg Glu Met Lys
   115 120 125
   Asp Arg Ala Glu Gln Phe Ser Cys Gln Pro Val Ala Gly Ser Gln Thr
   130 135 140
   Ser
   145
<210> 19
   <211> 145
   <212> PRT
   <213> Artificial Sequence
<220>
   <223> Artificial
<400> 19
   Met Ala Cys His Tyr Gly Gln Gln Gln Gln Thr Cys Ala Pro His Leu
   1               5                   10                  15
   Gln Leu Gln Pro Arg Ala Cys Arg Val Val Lys Ala Ala Thr Ala Val
               20                  25                  30
   Thr Ala Gly Gly Ser Leu Leu Val Leu Ser Gly Leu Thr Leu Ala Gly
           35                  40                  45
   Thr Val Ile Ala Leu Thr Ile Ala Thr Pro Leu Leu Val Ile Phe Ser
       50                  55                  60
   Pro Val Leu Val Pro Ala Val Ile Thr Ile Phe Leu Leu Gly Ala Gly
   65                  70                  75                  80
   Phe Leu Ala Ser Gly Gly Phe Gly Val Ala Ala Leu Ser Val Leu Ser
                   85                  90                  95
   Trp Ile Tyr Arg Tyr Leu Thr Gly Lys His Pro Pro Gly Ala Asp Gln
               100                 105                 110
   Leu Glu Ser Ala Lys Thr Lys Leu Ala Ser Lys Ala Arg Glu Met Lys
           115                 120                 125
   Asp Arg Ala Glu Gln Phe Ser Cys Gln Pro Val Ala Gly Ser Gln Thr
       130                 135                 140
   Ser
   145
<210> 20
   <211> 145
   <212> PRT
   <213> Artificial Sequence
<220>
   <223> Artificial
<400> 20
   Met Ala Cys His Tyr Gly Gln Gln Gln Gln Thr Cys Ala Pro His Leu
   1 5 10 15
   Gln Leu Gln Pro Arg Ala Cys Arg Val Val Lys Ala Ala Thr Ala Val
   20 25 30
   Thr Ala Gly Gly Ser Leu Leu Val Leu Ser Gly Leu Thr Leu Ala Gly
   35 40 45
   Thr Val Ile Ala Leu Thr Ile Ala Thr Pro Leu Leu Val Ile Phe Ser
   50 55 60
   Pro Val Leu Val Pro Ala Val Ile Thr Ile Phe Leu Leu Gly Ala Gly
   65 70 75 80
   Phe Leu Ala Ser Gly Gly Phe Gly Val Ala Ala Leu Ser Val Leu Ser
   85 90 95
   Trp Ile Tyr Arg Tyr Leu Thr Gly Lys His Pro Pro Gly Ala Asp Cys
   100 105 110
   Leu Glu Ser Ala Lys Thr Lys Leu Ala Ser Cys Ala Arg Glu Met Lys
   115 120 125
   Asp Arg Ala Glu Gln Phe Ser Cys Gln Pro Val Ala Gly Ser Gln Thr
   130 135 140
   Ser
   145
<210> 21
   <211> 810
   <212> DNA
   <213> Artificial Sequence
<220>
   <223> Artificial
<400> 21
   ggtaccgcgg ccgcttgctc ccttaaaaaa aaccatggca tgtcattacg gatgtggaca 60
   acagcaacag acttgtgcac ctcatctttg tcagcttcaa cctagagcat gtagagttgt 120
   gaaggctgct actgctgtta ctgtaaattt ctgtgttcct tattctctca aaatcttcga 180
   ttttgttttc gttcgatccc aatttcgtat atgttctttg gtttagattc tgttaatctt 240
   agatcgaaga cgattttctg ggtttgatcg ttagatatca tcttaattct cgattagggt 300
   ttcatagata tcatccgatt tgttcaaata atttgagttt tgtcgaataa ttactcttcg 360
   atttgtgatt tctatctaga tctggtgtta gtttctagtt tgtgcgatcg aatttgtcga 420
   ttaatctgag tttttctgat taacaggctg gaggatctct tcttgttctc tctggactta 480
   ctctcgctgg aactgttatc gctctcacta tcgctacacc tcttctcgtt atcttctctc 540
   ctgttctcgt tcctgctgtg atcactatct tccttctcgg agctggattt cttgcttctg 600
   gtggatttgg agttgctgct ctctctgttc tctcttggat ctacagatac ctcactggat 660
   gtaaacatcc tccaggtgct gattgtcttg agtctgcttg taagactaag ctcgcttctt 720
   gtgctagaga gatgaaggat tgtagagcag agcaattctc ttgtcagcct gttgctggat 780
   ctcagacttc ttaatgaaca tatggagctc 810
<210> 22
   <211> 831
   <212> DNA
   <213> Artificial Sequence
<220>
   <223> Artificial
<400> 22
   ggtaccgcgg ccgcttgctc ccttaaaaaa aaccatggca tgtcattacg gaatggcatg 60
   tcattacgga tgtggacaac agcaacagac ttgtgcacct catctttgtc agcttcaacc 120
   tagagcatgt agagttgtga aggctgctac tgctgttact gtaaatttct gtgttcctta 180
   ttctctcaaa atcttcgatt ttgttttcgt tcgatcccaa tttcgtatat gttctttggt 240
   ttagattctg ttaatcttag atcgaagacg attttctggg tttgatcgtt agatatcatc 300
   ttaattctcg attagggttt catagatatc atccgatttg ttcaaataat ttgagttttg 360
   tcgaataatt actcttcgat ttgtgatttc tatctagatc tggtgttagt ttctagtttg 420
   tgcgatcgaa tttgtcgatt aatctgagtt tttctgatta acaggctgga ggatctcttc 480
   ttgttctctc tggacttact ctcgctggaa ctgttatcgc tctcactatc gctacacctc 540
   ttctcgttat cttctctcct gttctcgttc ctgctgtgat cactatcttc cttctcggag 600
   ctggatttct tgcttctggt ggatttggag ttgctgctct ctctgttctc tcttggatct 660
   acagatacct cactggatgt aaacatcctc caggtgctga ttgtcttgag tctgcttgta 720
   agactaagct cgcttcttgt gctagagaga tgaaggattg tagagcagag caattctctt 780
   gtcagcctgt tgctggatgt tctcagactt cttaatgaac atatggagct c 831
<210> 23
   <211> 151
   <212> PRT
   <213> Artificial Sequence
<220>
   <223> Artificial
<400> 23
   Met Ala Cys His Tyr Gly Cys Gly Gln Gln Gln Gln Thr Cys Ala Pro
   1 5 10 15
   His Leu Cys Gln Leu Gln Pro Arg Ala Cys Arg Val Val Lys Ala Ala
   20 25 30
   Thr Ala Val Thr Ala Gly Gly Ser Leu Leu Val Leu Ser Gly Leu Thr
   35 40 45
   Leu Ala Gly Thr Val Ile Ala Leu Thr Ile Ala Thr Pro Leu Leu Val
   50 55 60
   Ile Phe Ser Pro Val Leu Val Pro Ala Val Ile Thr Ile Phe Leu Leu
   65 70 75 80
   Gly Ala Gly Phe Leu Ala Ser Gly Gly Phe Gly Val Ala Ala Leu Ser
   85 90 95
   Val Leu Ser Trp Ile Tyr Arg Tyr Leu Thr Gly Cys Lys His Pro Pro
   100 105 110
   Gly Ala Asp Cys Leu Glu Ser Ala Cys Lys Thr Lys Leu Ala Ser Cys
   115 120 125
   Ala Arg Glu Met Lys Asp Cys Arg Ala Glu Gln Phe Ser Cys Gln Pro
   130 135 140
   Val Ala Gly Ser Gln Thr Ser
   145 150
<210> 24
   <211> 158
   <212> PRT
   <213> Artificial sequence
<220>
   <223> Artificial
<400> 24
   Met Ala Cys His Tyr Gly Met Ala Cys His Tyr Gly Cys Gly Gln Gln
   1               5                   10                  15
   Gln Gln Thr Cys Ala Pro His Leu Cys Gln Leu Gln Pro Arg Ala Cys
               20                  25                  30
   Arg Val Val Lys Ala Ala Thr Ala Val Thr Ala Gly Gly Ser Leu Leu
           35                  40                  45
   Val Leu Ser Gly Leu Thr Leu Ala Gly Thr Val Ile Ala Leu Thr Ile
       50                  55                  60
   Ala Thr Pro Leu Leu Val Ile Phe Ser Pro Val Leu Val Pro Ala Val
   65                  70                  75                  80
   Ile Thr Ile Phe Leu Leu Gly Ala Gly Phe Leu Ala Ser Gly Gly Phe
                   85                  90                  95
   Gly Val Ala Ala Leu Ser Val Leu Ser Trp Ile Tyr Arg Tyr Leu Thr
               100                 105                 110
   Gly Cys Lys His Pro Pro Gly Ala Asp Cys Leu Glu Ser Ala Cys Lys
           115                 120                 125
   Thr Lys Leu Ala Ser Cys Ala Arg Glu Met Lys Asp Cys Arg Ala Glu
       130                 135                 140
   Gln Phe Ser Cys Gln Pro Val Ala Gly Cys Ser Gln Thr Ser
   145                 150                 155
<210> 25
   <211> 4104
   <212> DNA
   <213> Artificial Sequence
<220>
   <223> Artificial
<400> 25
   ggtaccacaa gtttgtacaa aaaagcaggc tgcggccgca aaattttcat atggtcctgc     60
   tttaatgaga tatgcgagac gcctatgatc gcatgatatt tgctttcaat tctgttgtgc    120
   acgttgtaaa aaacctgagc atgtgtagct cagatcctta ccgccggttt cggttcattc    180
   taatgaatat atcacccgtt actatcgtat ttttatgaat aatattctcc gttcaattta    240
   ctgattgtac cctactactt atatgtacaa tattaaaatg aaaacaatat attgtgctga    300
   ataggtttat agcgacatct atgatagagc gccacaataa caaacaattg cgttttatta    360
   ttacaaatcc aattttaaaa aaagcggcag aaccggtcaa acctaaaaga ctgattacat    420
   aaatcttatt caaatttcaa aaggccccag gggctagtat ctacgacaca ccgagcggcg    480
   aactaataac gttcactgaa gggaactccg gttccccgcc ggcgcgcatg ggtgagattc    540
   cttgaagttg agtattggcc gtccgctcta ccgaaagtta cgggcaccat tcaacccggt    600
   ccagcacggc ggccgggtaa ccgacttgct gccccgagaa ttatgcagca tttttttggt    660
   gtatgtgggc cccaaatgaa gtgcaggtca aaccttgaca gtgacgacaa atcgttgggc    720
   gggtccaggg cgaattttgc gacaacatgt cgaggctcag caggacctgc aggcatgcaa    780
   gctagcttac tagtgatgca tattctatag tgtcacctaa atcttcgacg aattaattcc    840
   aatcccacaa aaatctgagc ttaacagcac agttgctcct ctcagagcag aatcgggtat    900
   tcaacaccct catatcaact actacgttgt gtataacggt ccacatgccg gtatatacga    960
   tgactggggt tgtacaaagg cggcaacaaa cggcgttccc ggagttgcac acaagaaatt   1020
   tgccactatt acagaggcaa gagcagcagc tgacgcgtac acaacaagtc agcaaacaga   1080
   caggttgaac ttcatcccca aaggagaagc tcaactcaag cccaagagct ttgctaaggc   1140
   cctaacaagc ccaccaaagc aaaaagccca ctggctcacg ctaggaacca aaaggcccag      1200
   cagtgatcca gccccaaaag agatctcctt tgccccggag attacaatgg acgatttcct      1260
   ctatctttac gatctaggaa ggaagttcga aggtgaaggt gacgacacta tgttcaccac      1320
   tgataatgag aaggttagcc tcttcaattt cagaaagaat gctgacccac agatggttag      1380
   agaggcctac gcagcaggtc tcatcaagac gatctacccg agtaacaatc tccaggagat      1440
   caaatacctt cccaagaagg ttaaagatgc agtcaaaaga ttcaggacta attgcatcaa      1500
   gaacacagag aaagacatat ttctcaagat cagaagtact attccagtat ggacgattca      1560
   aggcttgctt cataaaccaa ggcaagtaat agagattgga gtctctaaaa aggtagttcc      1620
   tactgaatct aaggccatgc atggagtcta agattcaaat cgaggatcta acagaactcg      1680
   ccgtgaagac tggcgaacag ttcatacaga gtcttttacg actcaatgac aagaagaaaa      1740
   tcttcgtcaa catggtggag cacgacactc tggtctactc caaaaatgtc aaagatacag      1800
   tctcagaaga ccaaagggct attgagactt ttcaacaaag gataatttcg ggaaacctcc      1860
   tcggattcca ttgcccagct atctgtcact tcatcgaaag gacagtagaa aaggaaggtg      1920
   gctcctacaa atgccatcat tgcgataaag gaaaggctat cattcaagat ctctctgccg      1980
   acagtggtcc caaagatgga cccccaccca cgaggagcat cgtggaaaaa gaagacgttc      2040
   caaccacgtc ttcaaagcaa gtggattgat gtgacatctc cactgacgta agggatgacg      2100
   cacaatccca ctatccttcg caagaccctt cctctatata aggaagttca tttcatttgg      2160
   agaggacacg ggatccttgc tccgttaaaa aaaaccatgg ctatcctcga ttctgctggt      2220
   gttactactg tgactgagaa tggtggtgga gagttcgttg atctcgatag actcagaaga      2280
   agaaagtcta gatctgtaaa tttctgtgtt ccttattctc tcaaaatctt cgattttgtt      2340
   ttcgttcgat cccaatttcg tatatgttct ttggtttaga ttctgttaat cttagatcga      2400
   agacgatttt ctgggtttga tcgttagata tcatcttaat tctcgattag ggtttcatag      2460
   atatcatccg atttgttcaa ataatttgag ttttgtcgaa taattactct tcgatttgtg      2520
   atttctatct agatctggtg ttagtttcta gtttgtgcga tcgaatttgt cgattaatct      2580
   gagtttttct gattaacagg attcttctaa cggacttctc ctctctggat ctgataacaa      2640
   ctctccttct gatgatgttg gtgctcctgc tgatgtgaga gatagaatcg attctgtggt      2700
   gaacgatgat gctcaaggta ctgctaacct cgctggtgat aataacggtg gaggtgataa      2760
   caatggtgga ggaagaggtg gaggtgaagg tagaggaaac gctgatgcta ctttcactta      2820
   cagaccatct gtgcctgctc atagaagagc tagagagtct cctctctctt ctgatgctat      2880
   cttcaagcag tctcacgctg gacttttcaa cctctgtgtg gtggttctta tcgctgtgaa      2940
   ctctagactc atcatcgaga acctcatgaa gtacggatgg ctcatcagaa ctgatttctg      3000
   gttctcttct agatctctca gagattggcc tcttttcatg tgctgcatct cactctcaat      3060
   cttccctctc gctgctttta ctgttgagaa gctcgtgctc cagaagtata tcgctgaacc      3120
   tgtggtgatc ttcctccaca tcatcatcac tatgactgag gttctctacc ctgtttacgt      3180
   gactctcaga tgcgattctg ctttcctctc tggtgttact cttatgctcc tcacttgcat 3240
   tgtgtggctt aagctcgtgt cttacgctca cacttcttac gatatcagat ctctcgctaa 3300
   cgctgctgat aaggctaacc ctgaagtgtc ttactacgtg tctctcaagt ctctcgctta 3360
   cttcatggtt gctcctacac tttgttacca gccatcttac cctagatctg cttgcattag 3420
   aaagggatgg gtggcaagac aattcgctaa gttggtgatc ttcactggat tcatgggatt 3480
   catcatcgag cagtacatca accctattgt gagaaactct aagcaccctc tcaagggtga 3540
   tcttctctac gctatcgaga gagttcttaa gctctctgtg cctaaccttt atgtgtggct 3600
   ctgcatgttc tactgtttct tccacctctg gcttaacatc cttgctgagt tgctttgctt 3660
   cggagataga gagttctaca aggattggtg gaacgctaag tctgttggag attattggag 3720
   aatgtggaac atgcctgtgc ataagtggat ggtgcgtcac atctacttcc cttgcctcag 3780
   atctaagatc cctaagactc tcgctatcat tatcgctttc ctcgtgtctg ctgttttcca 3840
   tgagttgtgt atcgctgttc cttgcagact tttcaagctt tgggctttcc tcggaatcat 3900
   gttccaggtt ccactcgtgt tcatcactaa ctacctccaa gagagattcg gatctactgt 3960
   tggaaacatg attttctggt tcattttctg catcttcgga cagcctatgt gcgttctcct 4020
   ctactaccac gatctcatga acagaaaggg atctatgtct taatgaagga tccacccagc 4080
   tttcttgtac aaagtggtga gctc 4104
<210> 26
   <211> 18784
   <212> DNA
   <213> Artificial Sequence
<220>
   <223> Artificial
<400> 26
   tcgacatcgt caacgttcac ttctaaagaa atagcgccac tcagcttcct cagcggcttt      60
   atccagcgat ttcctattat gtcggcatag ttctcaagat cgacagcctg tcacggttaa     120
   gcgagaaatg aataagaagg ctgataattc ggatctctgc gaggagatga tatttgatca     180
   caggcagcaa cgctctgtca tcgttacaat caacatgcta ccctccgcga gatcatccgt     240
   gtttcaaacc cggcagctta gttgccgttc ttccgaatag catcgggtaa catgagcaaa     300
   gtctgccgcc ttacaacggc tctcccgctg acgccgtccc ggactgatgg gctgcctgta     360
   tcgagtggtg attttgtgcc gagctgccgg tcggggagct gttggctggc tggtggcagg     420
   atatattgtg gtgtaaacaa attgacgctt agacaactta ataacacatt gcggacgttt     480
   ttaatgtact gaattcgccg ctcggtgtgt cgtagatact agcccctggg gcacttttga     540
   aatttgaata agatttatgt aatcagtctt ttaggtttga ccggttctgc cgcttttttt     600
   aaaattggat ttgtaataat aaaacgcaat tgtttgttat tgtggcgctc tatcatagat     660
   gtcgctataa acctattcag cacaatatat tgttttcatt ttaatattgt acatataagt     720
   agtagggtac aatcagtaaa ttgaacggag aatattattc ataaaaatac gatagtaacg     780
   ggtgatatat tcattagaat gaaccgaaac cggcggtaag gatctgagct acacatgctc 840
   aggtttttta caacgtgcac aacagaattg aaagcaaata tcatgcgatc ataggcgtct 900
   cgcatatctc attaaacaag tgaagatttg attcaaactc cattgagagc cctgactatg 960
   cattcggttt gacccttcca ggttgagaga cgatagcccc ctaccttaat taaggggccc 1020
   cccctcgagg tcgacggtat cgataagctt gatatcgaat tcctgcagcc agggggatcc 1080
   cccgggtcat cagatctcgg tgacgggcag gaccggacgg ggcggtaccg gcaggctgaa 1140
   gtccagctgc cagaaaccca cgtcatgcca gttcccgtgc ttgaagccgg ccgcccgcag 1200
   catgccgcgg ggggcatatc cgagcgcctc gtgcatgcgc acgctcgggt cgttgggcag 1260
   cccgatgaca gcgaccacgc tcttgaagcc ctgtgcctcc agggacttca gcaggtgggt 1320
   gtagagcgtg gagcccagtc ccgtccgctg gtggcggggg gagacgtaca cggtcgactc 1380
   ggccgtccag tcgtaggcgt tgcgtgcctt ccaggggccc gcgtaggcga tgccggcgac 1440
   ctcgccgtcc acctcggcga cgagccaggg atagcgctcc cgcagacgga cgaggtcgtc 1500
   cgtccactcc tgcggttcct gcggctcggt acggaagttg accgtgcttg tctcgatgta 1560
   gtggttgacg atggtgcaga ccgccggcat gtccgcctcg gtggcacggc ggatgtcggc 1620
   cgggcgtcgt tctgggctca tggtagatcc cctcgagaga gatagatttg tagagagaga 1680
   ctggtgattt cagcgtgtcc tctccaaatg aaatgaactt ccttatatag aggaaggtct 1740
   tgcgaaggat agtgggattg tgcgtcatcc cttacgtcag tggagatatc acatcaatcc 1800
   acttgctttg aagacgtggt tggaacgtct tctttttcca cgatgctcct cgtgggtggg 1860
   ggtccatctt tgggaccact gtcggcagag gcatcttgaa cgatagcctt tcctttatcg 1920
   caatgatggc atttgtaggt gccaccttcc ttttctactg tccttttgat gaagtgacag 1980
   atagctgggc aatggaatcc gaggaggttt cccgatatta ccctttgttg aaaagtctca 2040
   atagcccttt ggtcttctga gactgtatct ttgatattct tggagtagac gagagtgtcg 2100
   tgctccacca tgttatcaca tcaatccact tgctttgaag acgtggttgg aacgtcttct 2160
   ttttccacga tgctcctcgt gggtgggggt ccatctttgg gaccactgtc ggcagaggca 2220
   tcttgaacga tagcctttcc tttatcgcaa tgatggcatt tgtaggtgcc accttccttt 2280
   tctactgtcc ttttgatgaa gtgacagata gctgggcaat ggaatccgag gaggtttccc 2340
   gatattaccc tttgttgaaa agtctcaata gccctttggt cttctgagac tgtatctttg 2400
   atattcttgg agtagacgag agtgtcgtgc tccaccatgt tgctgatagt gaccttaggc 2460
   gacttttgaa cgcgcaataa tggtttctga cgtatgtgct tagctcatta aactccagaa 2520
   acccattaac gcttacaatt tccattcgcc attcaggctg cgcaactgtt gggaagggcg 2580
   atcggtgcgg gcctcttcgc tattacgcca gctggcgaaa gggggatgtg ctgcaaggcg 2640
   attaagttgg gtaacgccag ggttttccca gtcacgacgt tgtaaaacga cggccagtga 2700
   attgtaatac gactcactat agggcgaatt gggcccgacg tcgcatgctc ccggccgcca 2760
   tggccgcggg atatcactag tgcggccgct cgacgaatta attccaatcc cacaaaaatc 2820
   tgagcttaac agcacagttg ctcctctcag agcagaatcg ggtattcaac accctcatat 2880
   caactactac gttgtgtata acggtccaca tgccggtata tacgatgact ggggttgtac 2940
   aaaggcggca acaaacggcg ttcccggagt tgcacacaag aaatttgcca ctattacaga 3000
   ggcaagagca gcagctgacg cgtacacaac aagtcagcaa acagacaggt tgaacttcat 3060
   ccccaaagga gaagctcaac tcaagcccaa gagctttgct aaggccctaa caagcccacc 3120
   aaagcaaaaa gcccactggc tcacgctagg aaccaaaagg cccagcagtg atccagcccc 3180
   aaaagagatc tcctttgccc cggagattac aatggacgat ttcctctatc tttacgatct 3240
   aggaaggaag ttcgaaggtg aaggtgacga cactatgttc accactgata atgagaaggt 3300
   tagcctcttc aatttcagaa agaatgctga cccacagatg gttagagagg cctacgcagc 3360
   aggtctcatc aagacgatct acccgagtaa caatctccag gagatcaaat accttcccaa 3420
   gaaggttaaa gatgcagtca aaagattcag gactaattgc atcaagaaca cagagaaaga 3480
   catatttctc aagatcagaa gtactattcc agtatggacg attcaaggct tgcttcataa 3540
   accaaggcaa gtaatagaga ttggagtctc taaaaaggta gttcctactg aatctaaggc 3600
   catgcatgga gtctaagatt caaatcgagg atctaacaga actcgccgtg aagactggcg 3660
   aacagttcat acagagtctt ttacgactca atgacaagaa gaaaatcttc gtcaacatgg 3720
   tggagcacga cactctggtc tactccaaaa atgtcaaaga tacagtctca gaagaccaaa 3780
   gggctattga gacttttcaa caaaggataa tttcgggaaa cctcctcgga ttccattgcc 3840
   cagctatctg tcacttcatc gaaaggacag tagaaaagga aggtggctcc tacaaatgcc 3900
   atcattgcga taaaggaaag gctatcattc aagatctctc tgccgacagt ggtcccaaag 3960
   atggaccccc acccacgagg agcatcgtgg aaaaagaaga cgttccaacc acgtcttcaa 4020
   agcaagtgga ttgatgtgac atctccactg acgtaaggga tgacgcacaa tcccactatc 4080
   cttcgcaaga cccttcctct atataaggaa gttcatttca tttggagagg acacgctcga 4140
   ggaattcggt accccatcac aagtttgtac aaaaaagcag gctgcggccg cttgctccct 4200
   taaaaaaaac catggcagag cattacggac aacagcaaca gactagagca cctcatcttc 4260
   agcttcaacc tagagcacag agagttgtga aggctgctac tgctgttact gtaaatttct 4320
   gtgttcctta ttctctcaaa atcttcgatt ttgttttcgt tcgatcccaa tttcgtatat 4380
   gttctttggt ttagattctg ttaatcttag atcgaagacg attttctggg tttgatcgtt 4440
   agatatcatc ttaattctcg attagggttt catagatatc atccgatttg ttcaaataat 4500
   ttgagttttg tcgaataatt actcttcgat ttgtgatttc tatctagatc tggtgttagt 4560
   ttctagtttg tgcgatcgaa tttgtcgatt aatctgagtt tttctgatta acaggctgga 4620
   ggatctcttc ttgttctctc tggacttact ctcgctggaa ctgttatcgc tctcactatc 4680
   gctacacctc ttctcgttat cttctctcct gttctcgttc ctgctgtgat cactatcttc 4740
   cttctcggag ctggatttct tgcttctggt ggatttggag ttgctgctct ctctgttctc 4800
   tcttggatct acagatacct cactggaaaa catcctccag gtgctgatca acttgagtct 4860
   gctaagacta agctcgcttc taaggctaga gagatgaagg atagagcaga gcaattctct 4920
   caacagcctg ttgctggatc tcagacttct taatgaacat atggtcctgc tttaatgaga 4980
   tatgcgagac gcctatgatc gcatgatatt tgctttcaat tctgttgtgc acgttgtaaa 5040
   aaacctgagc atgtgtagct cagatcctta ccgccggttt cggttcattc taatgaatat 5100
   atcacccgtt actatcgtat ttttatgaat aatattctcc gttcaattta ctgattgtac 5160
   cctactactt atatgtacaa tattaaaatg aaaacaatat attgtgctga ataggtttat 5220
   agcgacatct atgatagagc gccacaataa caaacaattg cgttttatta ttacaaatcc 5280
   aattttaaaa aaagcggcag aaccggtcaa acctaaaaga ctgattacat aaatcttatt 5340
   caaatttcaa aaggccccag gggctagtat ctacgacaca ccgagcggcg aactaataac 5400
   gttcactgaa gggaactccg gttccccgcc ggcgcgcatg ggtgagattc cttgaagttg 5460
   agtattggcc gtccgctcta ccgaaagtta cgggcaccat tcaacccggt ccagcacggc 5520
   ggccgggtaa ccgacttgct gccccgagaa ttatgcagca tttttttggt gtatgtgggc 5580
   cccaaatgaa gtgcaggtca aaccttgaca gtgacgacaa atcgttgggc gggtccaggg 5640
   cgaattttgc gacaacatgt cgaggctcag caggacctgc aggcatgcaa gctagcttac 5700
   tagtgatgca tattctatag tgtcacctaa atcttcgacg aattaattcc aatcccacaa 5760
   aaatctgagc ttaacagcac agttgctcct ctcagagcag aatcgggtat tcaacaccct 5820
   catatcaact actacgttgt gtataacggt ccacatgccg gtatatacga tgactggggt 5880
   tgtacaaagg cggcaacaaa cggcgttccc ggagttgcac acaagaaatt tgccactatt 5940
   acagaggcaa gagcagcagc tgacgcgtac acaacaagtc agcaaacaga caggttgaac 6000
   ttcatcccca aaggagaagc tcaactcaag cccaagagct ttgctaaggc cctaacaagc 6060
   ccaccaaagc aaaaagccca ctggctcacg ctaggaacca aaaggcccag cagtgatcca 6120
   gccccaaaag agatctcctt tgccccggag attacaatgg acgatttcct ctatctttac 6180
   gatctaggaa ggaagttcga aggtgaaggt gacgacacta tgttcaccac tgataatgag 6240
   aaggttagcc tcttcaattt cagaaagaat gctgacccac agatggttag agaggcctac 6300
   gcagcaggtc tcatcaagac gatctacccg agtaacaatc tccaggagat caaatacctt 6360
   cccaagaagg ttaaagatgc agtcaaaaga ttcaggacta attgcatcaa gaacacagag 6420
   aaagacatat ttctcaagat cagaagtact attccagtat ggacgattca aggcttgctt 6480
   cataaaccaa ggcaagtaat agagattgga gtctctaaaa aggtagttcc tactgaatct 6540
   aaggccatgc atggagtcta agattcaaat cgaggatcta acagaactcg ccgtgaagac 6600
   tggcgaacag ttcatacaga gtcttttacg actcaatgac aagaagaaaa tcttcgtcaa 6660
   catggtggag cacgacactc tggtctactc caaaaatgtc aaagatacag tctcagaaga 6720
   ccaaagggct attgagactt ttcaacaaag gataatttcg ggaaacctcc tcggattcca 6780
   ttgcccagct atctgtcact tcatcgaaag gacagtagaa aaggaaggtg gctcctacaa 6840
   atgccatcat tgcgataaag gaaaggctat cattcaagat ctctctgccg acagtggtcc 6900
   caaagatgga cccccaccca cgaggagcat cgtggaaaaa gaagacgttc caaccacgtc 6960
   ttcaaagcaa gtggattgat gtgacatctc cactgacgta agggatgacg cacaatccca 7020
   ctatccttcg caagaccctt cctctatata aggaagttca tttcatttgg agaggacacg 7080
   ggatccttgc tccgttaaaa aaaaccatgg ctatcctcga ttctgctggt gttactactg 7140
   tgactgagaa tggtggtgga gagttcgttg atctcgatag actcagaaga agaaagtcta 7200
   gatctgtaaa tttctgtgtt ccttattctc tcaaaatctt cgattttgtt ttcgttcgat 7260
   cccaatttcg tatatgttct ttggtttaga ttctgttaat cttagatcga agacgatttt 7320
   ctgggtttga tcgttagata tcatcttaat tctcgattag ggtttcatag atatcatccg 7380
   atttgttcaa ataatttgag ttttgtcgaa taattactct tcgatttgtg atttctatct 7440
   agatctggtg ttagtttcta gtttgtgcga tcgaatttgt cgattaatct gagtttttct 7500
   gattaacagg attcttctaa cggacttctc ctctctggat ctgataacaa ctctccttct 7560
   gatgatgttg gtgctcctgc tgatgtgaga gatagaatcg attctgtggt gaacgatgat 7620
   gctcaaggta ctgctaacct cgctggtgat aataacggtg gaggtgataa caatggtgga 7680
   ggaagaggtg gaggtgaagg tagaggaaac gctgatgcta ctttcactta cagaccatct 7740
   gtgcctgctc atagaagagc tagagagtct cctctctctt ctgatgctat cttcaagcag 7800
   tctcacgctg gacttttcaa cctctgtgtg gtggttctta tcgctgtgaa ctctagactc 7860
   atcatcgaga acctcatgaa gtacggatgg ctcatcagaa ctgatttctg gttctcttct 7920
   agatctctca gagattggcc tcttttcatg tgctgcatct cactctcaat cttccctctc 7980
   gctgctttta ctgttgagaa gctcgtgctc cagaagtata tcgctgaacc tgtggtgatc 8040
   ttcctccaca tcatcatcac tatgactgag gttctctacc ctgtttacgt gactctcaga 8100
   tgcgattctg ctttcctctc tggtgttact cttatgctcc tcacttgcat tgtgtggctt 8160
   aagctcgtgt cttacgctca cacttcttac gatatcagat ctctcgctaa cgctgctgat 8220
   aaggctaacc ctgaagtgtc ttactacgtg tctctcaagt ctctcgctta cttcatggtt 8280
   gctcctacac tttgttacca gccatcttac cctagatctg cttgcattag aaagggatgg 8340
   gtggcaagac aattcgctaa gttggtgatc ttcactggat tcatgggatt catcatcgag 8400
   cagtacatca accctattgt gagaaactct aagcaccctc tcaagggtga tcttctctac 8460
   gctatcgaga gagttcttaa gctctctgtg cctaaccttt atgtgtggct ctgcatgttc 8520
   tactgtttct tccacctctg gcttaacatc cttgctgagt tgctttgctt cggagataga 8580
   gagttctaca aggattggtg gaacgctaag tctgttggag attattggag aatgtggaac 8640
   atgcctgtgc ataagtggat ggtgcgtcac atctacttcc cttgcctcag atctaagatc 8700
   cctaagactc tcgctatcat tatcgctttc ctcgtgtctg ctgttttcca tgagttgtgt 8760
   atcgctgttc cttgcagact tttcaagctt tgggctttcc tcggaatcat gttccaggtt 8820
   ccactcgtgt tcatcactaa ctacctccaa gagagattcg gatctactgt tggaaacatg 8880
   attttctggt tcattttctg catcttcgga cagcctatgt gcgttctcct ctactaccac 8940
   gatctcatga acagaaaggg atctatgtct taatgaagga tccacccagc tttcttgtac 9000
   aaagtggtga tgggttcgaa atcgataagc ttggatcctc tagagtcctg ctttaatgag 9060
   atatgcgaga cgcctatgat cgcatgatat ttgctttcaa ttctgttgtg cacgttgtaa 9120
   aaaacctgag catgtgtagc tcagatcctt accgccggtt tcggttcatt ctaatgaata 9180
   tatcacccgt tactatcgta tttttatgaa taatattctc cgttcaattt actgattgta 9240
   ccctactact tatatgtaca atattaaaat gaaaacaata tattgtgctg aataggttta 9300
   tagcgacatc tatgatagag cgccacaata acaaacaatt gcgttttatt attacaaatc 9360
   caattttaaa aaaagcggca gaaccggtca aacctaaaag actgattaca taaatcttat 9420
   tcaaatttca aaaggcccca ggggctagta tctacgacac accgagcggc gaactaataa 9480
   cgttcactga agggaactcc ggttccccgc cggcgcgcat gggtgagatt ccttgaagtt 9540
   gagtattggc cgtccgctct accgaaagtt acgggcacca ttcaacccgg tccagcacgg 9600
   cggccgggta accgacttgc tgccccgaga attatgcagc atttttttgg tgtatgtggg 9660
   ccccaaatga agtgcaggtc aaaccttgac agtgacgaca aatcgttggg cgggtccagg 9720
   gcgaattttg cgacaacatg tcgaggctca gcaggacctg caggcatgca agctagctta 9780
   ctagtgatgc atattctata gtgtcaccta aatctgcggc cgcctgcagg tcgatatggg 9840
   agagctccca acgcgttgga tgcatagctt gagtattcta tagtgtcacc taaatagctt 9900
   ggcgtaatca tggtcatagc tgtttcctgt gtgaaattgt tatccgctca caattccaca 9960
   caacatacga gccggaagca taaagtgtaa agcctggggt gcctaatgag tgagctaact 10020
   cacattaatt gcgttgcgct cactgcccgc tttccagtcg ggaaacctgt cgtgccagct 10080
   gcattaatga atcggccaac gcgcggggag aggcggtttg cgtattgggg ctgagtggct 10140
   ccttcaacgt tgcggttctg tcagttccaa acgtaaaacg gcttgtcccg cgtcatcggc 10200
   gggggtcata acgtgactcc cttaattctc cgctcatgat cagattgtcg tttcccgcct 10260
   tcagtttaaa ctatcagtgt ttgacaggat atattggcgg gtaaacctaa gagaaaagag 10320
   cgtttattag aataatcgga tatttaaaag ggcgtgaaaa ggtttatccg ttcgtccatt 10380
   tgtatgtgca tgccaaccac agggttcccc tcgggagtgc ttggcattcc gtgcgataat 10440
   gacttctgtt caaccaccca aacgtcggaa agcctgacga cggagcagca ttccaaaaag 10500
   atcccttggc tcgtctgggt cggctagaag gtcgagtggg ctgctgtggc ttgatccctc 10560
   aacgcggtcg cggacgtagc gcagcgccga aaaatcctcg atcgcaaatc cgacgctgtc 10620
   gaaaatcgtg atctgcttgt cgctctttcg gccgacgtcc tggccagtca tcacgcgcca 10680
   aagttccgtc acaggatgat ctggcgcgag ttgctggatc tcgccttcaa tccgggtctg 10740
   tggcgggaac tccacgaaaa tatccgaacg cagcaagatg tcgacggatc ttttccgctg 10800
   cataaccctg cttcggggtc attatagcga ttttttcggt atatccatcc tttttcgcac 10860
   gatatacagg attttgccaa agggttcgtg tagactttcc ttggtgtatc caacggcgtc 10920
   agccgggcag gataggtgaa gtaggcccac ccgcgagcgg gtgttccttc ttcactgtcc 10980
   cttattcgca cctggcggtg ctcaacggga atcctgctct gcgaggctgg ccggctaccg 11040
   ccggcgtaac agatgagggc aagcggatgg ctgatgaaac caagccaacc aggggtgatg 11100
   ctgccaactt actgatttag tgtatgatgg tgtttttgag gtgctccagt ggcttctgtt 11160
   tctatcagct gtccctcctg ttcagctact gacggggtgg tgcgtaacgg caaaagcacc 11220
   gccggacatc agcgctatct ctgctctcac tgccgtaaaa catggcaact gcagttcact 11280
   tacaccgctt ctcaacccgg tacgcaccag aaaatcattg atatggccat gaatggcgtt 11340
   ggatgccggg caacagcccg cattatgggc gttggcctca acacgatttt acgtcactta 11400
   aaaaactcag gccgcagtcg gtaacctcgc gcatacagcc gggcagtgac gtcatcgtct 11460
   gcgcggaaat ggacgaacag tggggctatg tcggggctaa atcgcgccag cgctggctgt 11520
   tttacgcgta tgacagtctc cggaagacgg ttgttgcgca cgtattcggt gaacgcacta 11580
   tggcgacgct ggggcgtctt atgagcctgc tgtcaccctt tgacgtggtg atatggatga 11640
   cggatggctg gccgctgtat gaatcccgcc tgaagggaaa gctgcacgta atcagcaagc 11700
   gatatacgca gcgaattgag cggcataacc tgaatctgag gcagcacctg gcacggctgg 11760
   gacggaagtc gctgtcgttc tcaaaatcgg tggagctgca tgacaaagtc atcgggcatt 11820
   atctgaacat aaaacactat caataagttg gagtcattac ccaaccagga agggcagccc 11880
   acctatcaag gtgtactgcc ttccagacga acgaagagcg attgaggaaa aggcggcggc 11940
   ggccggcatg agcctgtcgg cctacctgct ggccgtcggc cagggctaca aaatcacggg 12000
   cgtcgtggac tatgagcacg tccgcgagct ggcccgcatc aatggcgacc tgggccgcct 12060
   gggcggcctg ctgaaactct ggctcaccga cgacccgcgc acggcgcggt tcggtgatgc 12120
   cacgatcctc gccctgctgg cgaagatcga agagaagcag gacgagcttg gcaaggtcat 12180
   gatgggcgtg gtccgcccga gggcagagcc atgacttttt tagccgctaa aacggccggg 12240
   gggtgcgcgt gattgccaag cacgtcccca tgcgctccat caagaagagc gacttcgcgg 12300
   agctggtatt cgtgcagggc aagattcgga ataccaagta cgagaaggac ggccagacgg 12360
   tctacgggac cgacttcatt gccgataagg tggattatct ggacaccaag gcaccaggcg 12420
   ggtcaaatca ggaataaggg cacattgccc cggcgtgagt cggggcaatc ccgcaaggag 12480
   ggtgaatgaa tcggacgttt gaccggaagg catacaggca agaactgatc gacgcggggt 12540
   tttccgccga ggatgccgaa accatcgcaa gccgcaccgt catgcgtgcg ccccgcgaaa 12600
   ccttccagtc cgtcggctcg atggtccagc aagctacggc caagatcgag cgcgacagcg 12660
   tgcaactggc tccccctgcc ctgcccgcgc catcggccgc cgtggagcgt tcgcgtcgtc 12720
   tcgaacagga ggcggcaggt ttggcgaagt cgatgaccat cgacacgcga ggaactatga 12780
   cgaccaagaa gcgaaaaacc gccggcgagg acctggcaaa acaggtcagc gaggccaagc 12840
   aggccgcgtt gctgaaacac acgaagcagc agatcaagga aatgcagctt tccttgttcg 12900
   atattgcgcc gtggccggac acgatgcgag cgatgccaaa cgacacggcc cgctctgccc 12960
   tgttcaccac gcgcaacaag aaaatcccgc gcgaggcgct gcaaaacaag gtcattttcc 13020
   acgtcaacaa ggacgtgaag atcacctaca ccggcgtcga gctgcgggcc gacgatgacg 13080
   aactggtgtg gcagcaggtg ttggagtacg cgaagcgcac ccctatcggc gagccgatca 13140
   ccttcacgtt ctacgagctt tgccaggacc tgggctggtc gatcaatggc cggtattaca 13200
   cgaaggccga ggaatgcctg tcgcgcctac aggcgacggc gatgggcttc acgtccgacc 13260
   gcgttgggca cctggaatcg gtgtcgctgc tgcaccgctt ccgcgtcctg gaccgtggca 13320
   agaaaacgtc ccgttgccag gtcctgatcg acgaggaaat cgtcgtgctg tttgctggcg 13380
   accactacac gaaattcata tgggagaagt accgcaagct gtcgccgacg gcccgacgga 13440
   tgttcgacta tttcagctcg caccgggagc cgtacccgct caagctggaa accttccgcc 13500
   tcatgtgcgg atcggattcc acccgcgtga agaagtggcg cgagcaggtc ggcgaagcct 13560
   gcgaagagtt gcgaggcagc ggcctggtgg aacacgcctg ggtcaatgat gacctggtgc 13620
   attgcaaacg ctagggcctt gtggggtcag ttccggctgg gggttcagca gccagcgctt 13680
   tactggcatt tcaggaacaa gcgggcactg ctcgacgcac ttgcttcgct cagtatcgct 13740
   cgggacgcac ggcgcgctct acgaactgcc gataaacaga ggattaaaat tgacaattgt 13800
   gattaaggct cagattcgac ggcttggagc ggccgacgtg caggatttcc gcgagatccg 13860
   attgtcggcc ctgaagaaag ctccagagat gttcgggtcc gtttacgagc acgaggagaa 13920
   aaagcccatg gaggcgttcg ctgaacggtt gcgagatgcc gtggcattcg gcgcctacat 13980
   cgacggcgag atcattgggc tgtcggtctt caaacaggag gacggcccca aggacgctca 14040
   caaggcgcat ctgtccggcg ttttcgtgga gcccgaacag cgaggccgag ggtcgccggg 14100
   tatgctgctg cgggcgttgc cggcgggttt attgctcgtg atgatcgtcc gacagattcc 14160
   aacgggaatc tggtggatgc gcatcttcat cctcggcgca cttaatattt cgctattctg 14220
   gagcttgttg tttatttcgg tctaccgcct gccgggcggg gtcgcggcga cggtaggcgc 14280
   tgtgcagccg ctgatggtcg tgttcatctc tgccgctctg ctaggtagcc cgatacgatt 14340
   gatggcggtc ctgggggcta tttgcggaac tgcgggcgtg gcgctgttgg tgttgacacc 14400
   aaacgcagcg ctagatcctg tcggcgtcgc agcgggcctg gcgggggcgg tttccatggc 14460
   gttcggaacc gtgctgaccc gcaagtggca acctcccgtg cctctgctca cctttaccgc 14520
   ctggcaactg gcggccggag gacttctgct cgttccagta gctttagtgt ttgatccgcc 14580
   aatcccgatg cctacaggaa ccaatgttct cggcctggcg tggctcggcc tgatcggagc 14640
   gggtttaacc tacttccttt ggttccgggg gatctcgcga ctcgaaccta cagttgtttc 14700
   cttactgggc tttctcagcc gggatggcgc taagaagcta ttgccgccga tcttcatatg 14760
   cggtgtgaaa taccgcacag atgcgtaagg agaaaatacc gcatcaggcg ctcttccgct 14820
   tcctcgctca ctgactcgct gcgctcggtc gttcggctgc ggcgagcggt atcagctcac 14880
   tcaaaggcgg taatacggtt atccacagaa tcaggggata acgcaggaaa gaacatgtga 14940
   gcaaaaggcc agcaaaaggc caggaaccgt aaaaaggccg cgttgctggc gtttttccat 15000
   aggctccgcc cccctgacga gcatcacaaa aatcgacgct caagtcagag gtggcgaaac 15060
   ccgacaggac tataaagata ccaggcgttt ccccctggaa gctccctcgt gcgctctcct 15120
   gttccgaccc tgccgcttac cggatacctg tccgcctttc tcccttcggg aagcgtggcg 15180
   ctttctcaat gctcacgctg taggtatctc agttcggtgt aggtcgttcg ctccaagctg 15240
   ggctgtgtgc acgaaccccc cgttcagccc gaccgctgcg ccttatccgg taactatcgt 15300
   cttgagtcca acccggtaag acacgactta tcgccactgg cagcagccac tggtaacagg 15360
   attagcagag cgaggtatgt aggcggtgct acagagttct tgaagtggtg gcctaactac 15420
   ggctacacta gaaggacagt atttggtatc tgcgctctgc tgaagccagt taccttcgga 15480
   aaaagagttg gtagctcttg atccggcaaa caaaccaccg ctggtagcgg tggttttttt 15540
   gtttgcaagc agcagattac gcgcagaaaa aaaggatatc aagaagatcc tttgatcttt 15600
   tctacggggt ctgacgctca gtggaacgaa aactcacgtt aagggatttt ggtcatgaga 15660
   ttatcaaaaa ggatcttcac ctagatcctt ttaaattaaa aatgaagttt taaatcaatc 15720
   taaagtatat atgagtaaac ttggtctgac agttaccaat gcttaatcag tgaggcacct 15780
   atctcagcga tctgtctatt tcgttcatcc atagttgcct gactccccgt cgtgtagata 15840
   actacgatac gggagggctt accatctggc cccagtgctg caatgatacc gcgagaccca 15900
   cgctcaccgg ctccagattt atcagcaata aaccagccag ccggaagggc cgagcgcaga 15960
   agtggtcctg caactttatc cgcctccatc cagtctatta aacaagtggc agcaacggat 16020
   tcgcaaacct gtcacgcctt ttgtgccaaa agccgcgcca ggtttgcgat ccgctgtgcc 16080
   aggcgttagg cgtcatatga agatttcggt gatccctgag caggtggcgg aaacattgga 16140
   tgctgagaac catttcattg ttcgtgaagt gttcgatgtg cacctatccg accaaggctt 16200
   tgaactatct accagaagtg tgagccccta ccggaaggat tacatctcgg atgatgactc 16260
   tgatgaagac tctgcttgct atggcgcatt catcgaccaa gagcttgtcg ggaagattga 16320
   actcaactca acatggaacg atctagcctc tatcgaacac attgttgtgt cgcacacgca 16380
   ccgaggcaaa ggagtcgcgc acagtctcat cgaatttgcg aaaaagtggg cactaagcag 16440
   acagctcctt ggcatacgat tagagacaca aacgaacaat gtacctgcct gcaatttgta 16500
   cgcaaaatgt ggctttactc tcggcggcat tgacctgttc acgtataaaa ctagacctca 16560
   agtctcgaac gaaacagcga tgtactggta ctggttctcg ggagcacagg atgacgccta 16620
   acaattcatt caagccgaca ccgcttcgcg gcgcggctta attcaggagt taaacatcat 16680
   gagggaagcg gtgatcgccg aagtatcgac tcaactatca gaggtagttg gcgtcatcga 16740
   gcgccatctc gaaccgacgt tgctggccgt acatttgtac ggctccgcag tggatggcgg 16800
   cctgaagcca cacagtgata ttgatttgct ggttacggtg accgtaaggc ttgatgaaac 16860
   aacgcggcga gctttgatca acgacctttt ggaaacttcg gcttcccctg gagagagcga 16920
   gattctccgc gctgtagaag tcaccattgt tgtgcacgac gacatcattc cgtggcgtta 16980
   tccagctaag cgcgaactgc aatttggaga atggcagcgc aatgacattc ttgcaggtat 17040
   cttcgagcca gccacgatcg acattgatct ggctatcttg ctgacaaaag caagagaaca 17100
   tagcgttgcc ttggtaggtc cagcggcgga ggaactcttt gatccggttc ctgaacagga 17160
   tctatttgag gcgctaaatg aaaccttaac gctatggaac tcgccgcccg actgggctgg 17220
   cgatgagcga aatgtagtgc ttacgttgtc ccgcatttgg tacagcgcag taaccggcaa 17280
   aatcgcgccg aaggatgtcg ctgccgactg ggcaatggag cgcctgccgg cccagtatca 17340
   gcccgtcata cttgaagcta ggcaggctta tcttggacaa gaagatcgct tggcctcgcg 17400
   cgcagatcag ttggaagaat ttgttcacta cgtgaaaggc gagatcacca aggtagtcgg 17460
   caaataatgt ctaacaattc gttcaagccg acgccgcttc gcggcgcggc ttaactcaag 17520
   cgttagagag ctggggaaga ctatgcgcga tctgttgaag gtggttctaa gcctcgtact 17580
   tgcgatggca tcggggcagg cacttgctga cctgccaatt gttttagtgg atgaagctcg 17640
   tcttccctat gactactccc catccaacta cgacatttct ccaagcaact acgacaactc 17700
   cataagcaat tacgacaata gtccatcaaa ttacgacaac tctgagagca actacgataa 17760
   tagttcatcc aattacgaca atagtcgcaa cggaaatcgt aggcttatat atagcgcaaa 17820
   tgggtctcgc actttcgccg gctactacgt cattgccaac aatgggacaa cgaacttctt 17880
   ttccacatct ggcaaaagga tgttctacac cccaaaaggg gggcgcggcg tctatggcgg 17940
   caaagatggg agcttctgcg gggcattggt cgtcataaat ggccaatttt cgcttgccct 18000
   gacagataac ggcctgaaga tcatgtatct aagcaactag cctgctctct aataaaatgt 18060
   taggagcttg gctgccattt ttggggtgag gccgttcgcg gccgaggggc gcagcccctg 18120
   gggggatggg aggcccgcgt tagcgggccg ggagggttcg agaagggggg gcacccccct 18180
   tcggcgtgcg cggtcacgcg ccagggcgca gccctggtta aaaacaaggt ttataaatat 18240
   tggtttaaaa gcaggttaaa agacaggtta gcggtggccg aaaaacgggc ggaaaccctt 18300
   gcaaatgctg gattttctgc ctgtggacag cccctcaaat gtcaataggt gcgcccctca 18360
   tctgtcagca ctctgcccct caagtgtcaa ggatcgcgcc cctcatctgt cagtagtcgc 18420
   gcccctcaag tgtcaatacc gcagggcact tatccccagg cttgtccaca tcatctgtgg 18480
   gaaactcgcg taaaatcagg cgttttcgcc gatttgcgag gctggccagc tccacgtcgc 18540
   cggccgaaat cgagcctgcc cctcatctgt caacgccgcg ccgggtgagt cggcccctca 18600
   agtgtcaacg tccgcccctc atctgtcagt gagggccaag ttttccgcga ggtatccaca 18660
   acgccggcgg ccggccgcgg tgtctcgcac acggcttcga cggcgtttct ggcgcgtttg 18720
   cagggccata gacggccgcc agcccagcgg cgagggcaac cagcccggtg agcgtcggaa 18780
   aggg 18784
<210> 27
   <211> 18784
   <212> DNA
   <213> Artificial Sequence
<220>
   <223> Artificial
<400> 27
   tcgacatcgt caacgttcac ttctaaagaa atagcgccac tcagcttcct cagcggcttt 60
   atccagcgat ttcctattat gtcggcatag ttctcaagat cgacagcctg tcacggttaa 120
   gcgagaaatg aataagaagg ctgataattc ggatctctgc gaggagatga tatttgatca 180
   caggcagcaa cgctctgtca tcgttacaat caacatgcta ccctccgcga gatcatccgt 240
   gtttcaaacc cggcagctta gttgccgttc ttccgaatag catcgggtaa catgagcaaa 300
   gtctgccgcc ttacaacggc tctcccgctg acgccgtccc ggactgatgg gctgcctgta 360
   tcgagtggtg attttgtgcc gagctgccgg tcggggagct gttggctggc tggtggcagg 420
   atatattgtg gtgtaaacaa attgacgctt agacaactta ataacacatt gcggacgttt 480
   ttaatgtact gaattcgccg ctcggtgtgt cgtagatact agcccctggg gcacttttga 540
   aatttgaata agatttatgt aatcagtctt ttaggtttga ccggttctgc cgcttttttt 600
   aaaattggat ttgtaataat aaaacgcaat tgtttgttat tgtggcgctc tatcatagat 660
   gtcgctataa acctattcag cacaatatat tgttttcatt ttaatattgt acatataagt 720
   agtagggtac aatcagtaaa ttgaacggag aatattattc ataaaaatac gatagtaacg 780
   ggtgatatat tcattagaat gaaccgaaac cggcggtaag gatctgagct acacatgctc 840
   aggtttttta caacgtgcac aacagaattg aaagcaaata tcatgcgatc ataggcgtct 900
   cgcatatctc attaaacaag tgaagatttg attcaaactc cattgagagc cctgactatg 960
   cattcggttt gacccttcca ggttgagaga cgatagcccc ctaccttaat taaggggccc 1020
   cccctcgagg tcgacggtat cgataagctt gatatcgaat tcctgcagcc agggggatcc 1080
   cccgggtcat cagatctcgg tgacgggcag gaccggacgg ggcggtaccg gcaggctgaa 1140
   gtccagctgc cagaaaccca cgtcatgcca gttcccgtgc ttgaagccgg ccgcccgcag 1200
   catgccgcgg ggggcatatc cgagcgcctc gtgcatgcgc acgctcgggt cgttgggcag 1260
   cccgatgaca gcgaccacgc tcttgaagcc ctgtgcctcc agggacttca gcaggtgggt 1320
   gtagagcgtg gagcccagtc ccgtccgctg gtggcggggg gagacgtaca cggtcgactc 1380
   ggccgtccag tcgtaggcgt tgcgtgcctt ccaggggccc gcgtaggcga tgccggcgac 1440
   ctcgccgtcc acctcggcga cgagccaggg atagcgctcc cgcagacgga cgaggtcgtc 1500
   cgtccactcc tgcggttcct gcggctcggt acggaagttg accgtgcttg tctcgatgta 1560
   gtggttgacg atggtgcaga ccgccggcat gtccgcctcg gtggcacggc ggatgtcggc 1620
   cgggcgtcgt tctgggctca tggtagatcc cctcgagaga gatagatttg tagagagaga 1680
   ctggtgattt cagcgtgtcc tctccaaatg aaatgaactt ccttatatag aggaaggtct 1740
   tgcgaaggat agtgggattg tgcgtcatcc cttacgtcag tggagatatc acatcaatcc 1800
   acttgctttg aagacgtggt tggaacgtct tctttttcca cgatgctcct cgtgggtggg 1860
   ggtccatctt tgggaccact gtcggcagag gcatcttgaa cgatagcctt tcctttatcg 1920
   caatgatggc atttgtaggt gccaccttcc ttttctactg tccttttgat gaagtgacag 1980
   atagctgggc aatggaatcc gaggaggttt cccgatatta ccctttgttg aaaagtctca 2040
   atagcccttt ggtcttctga gactgtatct ttgatattct tggagtagac gagagtgtcg     2100
   tgctccacca tgttatcaca tcaatccact tgctttgaag acgtggttgg aacgtcttct     2160
   ttttccacga tgctcctcgt gggtgggggt ccatctttgg gaccactgtc ggcagaggca     2220
   tcttgaacga tagcctttcc tttatcgcaa tgatggcatt tgtaggtgcc accttccttt     2280
   tctactgtcc ttttgatgaa gtgacagata gctgggcaat ggaatccgag gaggtttccc     2340
   gatattaccc tttgttgaaa agtctcaata gccctttggt cttctgagac tgtatctttg     2400
   atattcttgg agtagacgag agtgtcgtgc tccaccatgt tgctgatagt gaccttaggc     2460
   gacttttgaa cgcgcaataa tggtttctga cgtatgtgct tagctcatta aactccagaa     2520
   acccattaac gcttacaatt tccattcgcc attcaggctg cgcaactgtt gggaagggcg     2580
   atcggtgcgg gcctcttcgc tattacgcca gctggcgaaa gggggatgtg ctgcaaggcg     2640
   attaagttgg gtaacgccag ggttttccca gtcacgacgt tgtaaaacga cggccagtga     2700
   attgtaatac gactcactat agggcgaatt gggcccgacg tcgcatgctc ccggccgcca     2760
   tggccgcggg atatcactag tgcggccgct cgacgaatta attccaatcc cacaaaaatc     2820
   tgagcttaac agcacagttg ctcctctcag agcagaatcg ggtattcaac accctcatat     2880
   caactactac gttgtgtata acggtccaca tgccggtata tacgatgact ggggttgtac     2940
   aaaggcggca acaaacggcg ttcccggagt tgcacacaag aaatttgcca ctattacaga     3000
   ggcaagagca gcagctgacg cgtacacaac aagtcagcaa acagacaggt tgaacttcat     3060
   ccccaaagga gaagctcaac tcaagcccaa gagctttgct aaggccctaa caagcccacc     3120
   aaagcaaaaa gcccactggc tcacgctagg aaccaaaagg cccagcagtg atccagcccc     3180
   aaaagagatc tcctttgccc cggagattac aatggacgat ttcctctatc tttacgatct     3240
   aggaaggaag ttcgaaggtg aaggtgacga cactatgttc accactgata atgagaaggt     3300
   tagcctcttc aatttcagaa agaatgctga cccacagatg gttagagagg cctacgcagc     3360
   aggtctcatc aagacgatct acccgagtaa caatctccag gagatcaaat accttcccaa     3420
   gaaggttaaa gatgcagtca aaagattcag gactaattgc atcaagaaca cagagaaaga     3480
   catatttctc aagatcagaa gtactattcc agtatggacg attcaaggct tgcttcataa     3540
   accaaggcaa gtaatagaga ttggagtctc taaaaaggta gttcctactg aatctaaggc     3600
   catgcatgga gtctaagatt caaatcgagg atctaacaga actcgccgtg aagactggcg     3660
   aacagttcat acagagtctt ttacgactca atgacaagaa gaaaatcttc gtcaacatgg     3720
   tggagcacga cactctggtc tactccaaaa atgtcaaaga tacagtctca gaagaccaaa     3780
   gggctattga gacttttcaa caaaggataa tttcgggaaa cctcctcgga ttccattgcc     3840
   cagctatctg tcacttcatc gaaaggacag tagaaaagga aggtggctcc tacaaatgcc     3900
   atcattgcga taaaggaaag gctatcattc aagatctctc tgccgacagt ggtcccaaag     3960
   atggaccccc acccacgagg agcatcgtgg aaaaagaaga cgttccaacc acgtcttcaa     4020
   agcaagtgga ttgatgtgac atctccactg acgtaaggga tgacgcacaa tcccactatc     4080
   cttcgcaaga cccttcctct atataaggaa gttcatttca tttggagagg acacgctcga    4140
   ggaattcggt accccatcac aagtttgtac aaaaaagcag gctgcggccg cttgctccct    4200
   taaaaaaaac catggcatgt cattacggac aacagcaaca gactagagca cctcatcttc    4260
   agcttcaacc tagagcacag agagttgtga aggctgctac tgctgttact gtaaatttct    4320
   gtgttcctta ttctctcaaa atcttcgatt ttgttttcgt tcgatcccaa tttcgtatat    4380
   gttctttggt ttagattctg ttaatcttag atcgaagacg attttctggg tttgatcgtt    4440
   agatatcatc ttaattctcg attagggttt catagatatc atccgatttg ttcaaataat    4500
   ttgagttttg tcgaataatt actcttcgat ttgtgatttc tatctagatc tggtgttagt    4560
   ttctagtttg tgcgatcgaa tttgtcgatt aatctgagtt tttctgatta acaggctgga    4620
   ggatctcttc ttgttctctc tggacttact ctcgctggaa ctgttatcgc tctcactatc    4680
   gctacacctc ttctcgttat cttctctcct gttctcgttc ctgctgtgat cactatcttc    4740
   cttctcggag ctggatttct tgcttctggt ggatttggag ttgctgctct ctctgttctc    4800
   tcttggatct acagatacct cactggaaaa catcctccag gtgctgatca acttgagtct    4860
   gctaagacta agctcgcttc taaggctaga gagatgaagg atagagcaga gcaattctct    4920
   tgtcagcctg ttgctggatc tcagacttct taatgaacat atggtcctgc tttaatgaga    4980
   tatgcgagac gcctatgatc gcatgatatt tgctttcaat tctgttgtgc acgttgtaaa    5040
   aaacctgagc atgtgtagct cagatcctta ccgccggttt cggttcattc taatgaatat    5100
   atcacccgtt actatcgtat ttttatgaat aatattctcc gttcaattta ctgattgtac    5160
   cctactactt atatgtacaa tattaaaatg aaaacaatat attgtgctga ataggtttat    5220
   agcgacatct atgatagagc gccacaataa caaacaattg cgttttatta ttacaaatcc    5280
   aattttaaaa aaagcggcag aaccggtcaa acctaaaaga ctgattacat aaatcttatt    5340
   caaatttcaa aaggccccag gggctagtat ctacgacaca ccgagcggcg aactaataac    5400
   gttcactgaa gggaactccg gttccccgcc ggcgcgcatg ggtgagattc cttgaagttg    5460
   agtattggcc gtccgctcta ccgaaagtta cgggcaccat tcaacccggt ccagcacggc    5520
   ggccgggtaa ccgacttgct gccccgagaa ttatgcagca tttttttggt gtatgtgggc    5580
   cccaaatgaa gtgcaggtca aaccttgaca gtgacgacaa atcgttgggc gggtccaggg    5640
   cgaattttgc gacaacatgt cgaggctcag caggacctgc aggcatgcaa gctagcttac    5700
   tagtgatgca tattctatag tgtcacctaa atcttcgacg aattaattcc aatcccacaa    5760
   aaatctgagc ttaacagcac agttgctcct ctcagagcag aatcgggtat tcaacaccct    5820
   catatcaact actacgttgt gtataacggt ccacatgccg gtatatacga tgactggggt    5880
   tgtacaaagg cggcaacaaa cggcgttccc ggagttgcac acaagaaatt tgccactatt    5940
   acagaggcaa gagcagcagc tgacgcgtac acaacaagtc agcaaacaga caggttgaac    6000
   ttcatcccca aaggagaagc tcaactcaag cccaagagct ttgctaaggc cctaacaagc    6060
   ccaccaaagc aaaaagccca ctggctcacg ctaggaacca aaaggcccag cagtgatcca    6120
   gccccaaaag agatctcctt tgccccggag attacaatgg acgatttcct ctatctttac 6180
   gatctaggaa ggaagttcga aggtgaaggt gacgacacta tgttcaccac tgataatgag 6240
   aaggttagcc tcttcaattt cagaaagaat gctgacccac agatggttag agaggcctac 6300
   gcagcaggtc tcatcaagac gatctacccg agtaacaatc tccaggagat caaatacctt 6360
   cccaagaagg ttaaagatgc agtcaaaaga ttcaggacta attgcatcaa gaacacagag 6420
   aaagacatat ttctcaagat cagaagtact attccagtat ggacgattca aggcttgctt 6480
   cataaaccaa ggcaagtaat agagattgga gtctctaaaa aggtagttcc tactgaatct 6540
   aaggccatgc atggagtcta agattcaaat cgaggatcta acagaactcg ccgtgaagac 6600
   tggcgaacag ttcatacaga gtcttttacg actcaatgac aagaagaaaa tcttcgtcaa 6660
   catggtggag cacgacactc tggtctactc caaaaatgtc aaagatacag tctcagaaga 6720
   ccaaagggct attgagactt ttcaacaaag gataatttcg ggaaacctcc tcggattcca 6780
   ttgcccagct atctgtcact tcatcgaaag gacagtagaa aaggaaggtg gctcctacaa 6840
   atgccatcat tgcgataaag gaaaggctat cattcaagat ctctctgccg acagtggtcc 6900
   caaagatgga cccccaccca cgaggagcat cgtggaaaaa gaagacgttc caaccacgtc 6960
   ttcaaagcaa gtggattgat gtgacatctc cactgacgta agggatgacg cacaatccca 7020
   ctatccttcg caagaccctt cctctatata aggaagttca tttcatttgg agaggacacg 7080
   ggatccttgc tccgttaaaa aaaaccatgg ctatcctcga ttctgctggt gttactactg 7140
   tgactgagaa tggtggtgga gagttcgttg atctcgatag actcagaaga agaaagtcta 7200
   gatctgtaaa tttctgtgtt ccttattctc tcaaaatctt cgattttgtt ttcgttcgat 7260
   cccaatttcg tatatgttct ttggtttaga ttctgttaat cttagatcga agacgatttt 7320
   ctgggtttga tcgttagata tcatcttaat tctcgattag ggtttcatag atatcatccg 7380
   atttgttcaa ataatttgag ttttgtcgaa taattactct tcgatttgtg atttctatct 7440
   agatctggtg ttagtttcta gtttgtgcga tcgaatttgt cgattaatct gagtttttct 7500
   gattaacagg attcttctaa cggacttctc ctctctggat ctgataacaa ctctccttct 7560
   gatgatgttg gtgctcctgc tgatgtgaga gatagaatcg attctgtggt gaacgatgat 7620
   gctcaaggta ctgctaacct cgctggtgat aataacggtg gaggtgataa caatggtgga 7680
   ggaagaggtg gaggtgaagg tagaggaaac gctgatgcta ctttcactta cagaccatct 7740
   gtgcctgctc atagaagagc tagagagtct cctctctctt ctgatgctat cttcaagcag 7800
   tctcacgctg gacttttcaa cctctgtgtg gtggttctta tcgctgtgaa ctctagactc 7860
   atcatcgaga acctcatgaa gtacggatgg ctcatcagaa ctgatttctg gttctcttct 7920
   agatctctca gagattggcc tcttttcatg tgctgcatct cactctcaat cttccctctc 7980
   gctgctttta ctgttgagaa gctcgtgctc cagaagtata tcgctgaacc tgtggtgatc 8040
   ttcctccaca tcatcatcac tatgactgag gttctctacc ctgtttacgt gactctcaga 8100
   tgcgattctg ctttcctctc tggtgttact cttatgctcc tcacttgcat tgtgtggctt 8160
   aagctcgtgt cttacgctca cacttcttac gatatcagat ctctcgctaa cgctgctgat 8220
   aaggctaacc ctgaagtgtc ttactacgtg tctctcaagt ctctcgctta cttcatggtt 8280
   gctcctacac tttgttacca gccatcttac cctagatctg cttgcattag aaagggatgg 8340
   gtggcaagac aattcgctaa gttggtgatc ttcactggat tcatgggatt catcatcgag 8400
   cagtacatca accctattgt gagaaactct aagcaccctc tcaagggtga tcttctctac 8460
   gctatcgaga gagttcttaa gctctctgtg cctaaccttt atgtgtggct ctgcatgttc 8520
   tactgtttct tccacctctg gcttaacatc cttgctgagt tgctttgctt cggagataga 8580
   gagttctaca aggattggtg gaacgctaag tctgttggag attattggag aatgtggaac 8640
   atgcctgtgc ataagtggat ggtgcgtcac atctacttcc cttgcctcag atctaagatc 8700
   cctaagactc tcgctatcat tatcgctttc ctcgtgtctg ctgttttcca tgagttgtgt 8760
   atcgctgttc cttgcagact tttcaagctt tgggctttcc tcggaatcat gttccaggtt 8820
   ccactcgtgt tcatcactaa ctacctccaa gagagattcg gatctactgt tggaaacatg 8880
   attttctggt tcattttctg catcttcgga cagcctatgt gcgttctcct ctactaccac 8940
   gatctcatga acagaaaggg atctatgtct taatgaagga tccacccagc tttcttgtac 9000
   aaagtggtga tgggttcgaa atcgataagc ttggatcctc tagagtcctg ctttaatgag 9060
   atatgcgaga cgcctatgat cgcatgatat ttgctttcaa ttctgttgtg cacgttgtaa 9120
   aaaacctgag catgtgtagc tcagatcctt accgccggtt tcggttcatt ctaatgaata 9180
   tatcacccgt tactatcgta tttttatgaa taatattctc cgttcaattt actgattgta 9240
   ccctactact tatatgtaca atattaaaat gaaaacaata tattgtgctg aataggttta 9300
   tagcgacatc tatgatagag cgccacaata acaaacaatt gcgttttatt attacaaatc 9360
   caattttaaa aaaagcggca gaaccggtca aacctaaaag actgattaca taaatcttat 9420
   tcaaatttca aaaggcccca ggggctagta tctacgacac accgagcggc gaactaataa 9480
   cgttcactga agggaactcc ggttccccgc cggcgcgcat gggtgagatt ccttgaagtt 9540
   gagtattggc cgtccgctct accgaaagtt acgggcacca ttcaacccgg tccagcacgg 9600
   cggccgggta accgacttgc tgccccgaga attatgcagc atttttttgg tgtatgtggg 9660
   ccccaaatga agtgcaggtc aaaccttgac agtgacgaca aatcgttggg cgggtccagg 9720
   gcgaattttg cgacaacatg tcgaggctca gcaggacctg caggcatgca agctagctta 9780
   ctagtgatgc atattctata gtgtcaccta aatctgcggc cgcctgcagg tcgatatggg 9840
   agagctccca acgcgttgga tgcatagctt gagtattcta tagtgtcacc taaatagctt 9900
   ggcgtaatca tggtcatagc tgtttcctgt gtgaaattgt tatccgctca caattccaca 9960
   caacatacga gccggaagca taaagtgtaa agcctggggt gcctaatgag tgagctaact 10020
   cacattaatt gcgttgcgct cactgcccgc tttccagtcg ggaaacctgt cgtgccagct 10080
   gcattaatga atcggccaac gcgcggggag aggcggtttg cgtattgggg ctgagtggct 10140
   ccttcaacgt tgcggttctg tcagttccaa acgtaaaacg gcttgtcccg cgtcatcggc 10200
   gggggtcata acgtgactcc cttaattctc cgctcatgat cagattgtcg tttcccgcct 10260
   tcagtttaaa ctatcagtgt ttgacaggat atattggcgg gtaaacctaa gagaaaagag 10320
   cgtttattag aataatcgga tatttaaaag ggcgtgaaaa ggtttatccg ttcgtccatt 10380
   tgtatgtgca tgccaaccac agggttcccc tcgggagtgc ttggcattcc gtgcgataat 10440
   gacttctgtt caaccaccca aacgtcggaa agcctgacga cggagcagca ttccaaaaag 10500
   atcccttggc tcgtctgggt cggctagaag gtcgagtggg ctgctgtggc ttgatccctc 10560
   aacgcggtcg cggacgtagc gcagcgccga aaaatcctcg atcgcaaatc cgacgctgtc 10620
   gaaaatcgtg atctgcttgt cgctctttcg gccgacgtcc tggccagtca tcacgcgcca 10680
   aagttccgtc acaggatgat ctggcgcgag ttgctggatc tcgccttcaa tccgggtctg 10740
   tggcgggaac tccacgaaaa tatccgaacg cagcaagatg tcgacggatc ttttccgctg 10800
   cataaccctg cttcggggtc attatagcga ttttttcggt atatccatcc tttttcgcac 10860
   gatatacagg attttgccaa agggttcgtg tagactttcc ttggtgtatc caacggcgtc 10920
   agccgggcag gataggtgaa gtaggcccac ccgcgagcgg gtgttccttc ttcactgtcc 10980
   cttattcgca cctggcggtg ctcaacggga atcctgctct gcgaggctgg ccggctaccg 11040
   ccggcgtaac agatgagggc aagcggatgg ctgatgaaac caagccaacc aggggtgatg 11100
   ctgccaactt actgatttag tgtatgatgg tgtttttgag gtgctccagt ggcttctgtt 11160
   tctatcagct gtccctcctg ttcagctact gacggggtgg tgcgtaacgg caaaagcacc 11220
   gccggacatc agcgctatct ctgctctcac tgccgtaaaa catggcaact gcagttcact 11280
   tacaccgctt ctcaacccgg tacgcaccag aaaatcattg atatggccat gaatggcgtt 11340
   ggatgccggg caacagcccg cattatgggc gttggcctca acacgatttt acgtcactta 11400
   aaaaactcag gccgcagtcg gtaacctcgc gcatacagcc gggcagtgac gtcatcgtct 11460
   gcgcggaaat ggacgaacag tggggctatg tcggggctaa atcgcgccag cgctggctgt 11520
   tttacgcgta tgacagtctc cggaagacgg ttgttgcgca cgtattcggt gaacgcacta 11580
   tggcgacgct ggggcgtctt atgagcctgc tgtcaccctt tgacgtggtg atatggatga 11640
   cggatggctg gccgctgtat gaatcccgcc tgaagggaaa gctgcacgta atcagcaagc 11700
   gatatacgca gcgaattgag cggcataacc tgaatctgag gcagcacctg gcacggctgg 11760
   gacggaagtc gctgtcgttc tcaaaatcgg tggagctgca tgacaaagtc atcgggcatt 11820
   atctgaacat aaaacactat caataagttg gagtcattac ccaaccagga agggcagccc 11880
   acctatcaag gtgtactgcc ttccagacga acgaagagcg attgaggaaa aggcggcggc 11940
   ggccggcatg agcctgtcgg cctacctgct ggccgtcggc cagggctaca aaatcacggg 12000
   cgtcgtggac tatgagcacg tccgcgagct ggcccgcatc aatggcgacc tgggccgcct 12060
   gggcggcctg ctgaaactct ggctcaccga cgacccgcgc acggcgcggt tcggtgatgc 12120
   cacgatcctc gccctgctgg cgaagatcga agagaagcag gacgagcttg gcaaggtcat 12180
   gatgggcgtg gtccgcccga gggcagagcc atgacttttt tagccgctaa aacggccggg 12240
   gggtgcgcgt gattgccaag cacgtcccca tgcgctccat caagaagagc gacttcgcgg 12300
   agctggtatt cgtgcagggc aagattcgga ataccaagta cgagaaggac ggccagacgg 12360
   tctacgggac cgacttcatt gccgataagg tggattatct ggacaccaag gcaccaggcg 12420
   ggtcaaatca ggaataaggg cacattgccc cggcgtgagt cggggcaatc ccgcaaggag 12480
   ggtgaatgaa tcggacgttt gaccggaagg catacaggca agaactgatc gacgcggggt 12540
   tttccgccga ggatgccgaa accatcgcaa gccgcaccgt catgcgtgcg ccccgcgaaa 12600
   ccttccagtc cgtcggctcg atggtccagc aagctacggc caagatcgag cgcgacagcg 12660
   tgcaactggc tccccctgcc ctgcccgcgc catcggccgc cgtggagcgt tcgcgtcgtc 12720
   tcgaacagga ggcggcaggt ttggcgaagt cgatgaccat cgacacgcga ggaactatga 12780
   cgaccaagaa gcgaaaaacc gccggcgagg acctggcaaa acaggtcagc gaggccaagc 12840
   aggccgcgtt gctgaaacac acgaagcagc agatcaagga aatgcagctt tccttgttcg 12900
   atattgcgcc gtggccggac acgatgcgag cgatgccaaa cgacacggcc cgctctgccc 12960
   tgttcaccac gcgcaacaag aaaatcccgc gcgaggcgct gcaaaacaag gtcattttcc 13020
   acgtcaacaa ggacgtgaag atcacctaca ccggcgtcga gctgcgggcc gacgatgacg 13080
   aactggtgtg gcagcaggtg ttggagtacg cgaagcgcac ccctatcggc gagccgatca 13140
   ccttcacgtt ctacgagctt tgccaggacc tgggctggtc gatcaatggc cggtattaca 13200
   cgaaggccga ggaatgcctg tcgcgcctac aggcgacggc gatgggcttc acgtccgacc 13260
   gcgttgggca cctggaatcg gtgtcgctgc tgcaccgctt ccgcgtcctg gaccgtggca 13320
   agaaaacgtc ccgttgccag gtcctgatcg acgaggaaat cgtcgtgctg tttgctggcg 13380
   accactacac gaaattcata tgggagaagt accgcaagct gtcgccgacg gcccgacgga 13440
   tgttcgacta tttcagctcg caccgggagc cgtacccgct caagctggaa accttccgcc 13500
   tcatgtgcgg atcggattcc acccgcgtga agaagtggcg cgagcaggtc ggcgaagcct 13560
   gcgaagagtt gcgaggcagc ggcctggtgg aacacgcctg ggtcaatgat gacctggtgc 13620
   attgcaaacg ctagggcctt gtggggtcag ttccggctgg gggttcagca gccagcgctt 13680
   tactggcatt tcaggaacaa gcgggcactg ctcgacgcac ttgcttcgct cagtatcgct 13740
   cgggacgcac ggcgcgctct acgaactgcc gataaacaga ggattaaaat tgacaattgt 13800
   gattaaggct cagattcgac ggcttggagc ggccgacgtg caggatttcc gcgagatccg 13860
   attgtcggcc ctgaagaaag ctccagagat gttcgggtcc gtttacgagc acgaggagaa 13920
   aaagcccatg gaggcgttcg ctgaacggtt gcgagatgcc gtggcattcg gcgcctacat 13980
   cgacggcgag atcattgggc tgtcggtctt caaacaggag gacggcccca aggacgctca 14040
   caaggcgcat ctgtccggcg ttttcgtgga gcccgaacag cgaggccgag ggtcgccggg 14100
   tatgctgctg cgggcgttgc cggcgggttt attgctcgtg atgatcgtcc gacagattcc 14160
   aacgggaatc tggtggatgc gcatcttcat cctcggcgca cttaatattt cgctattctg 14220
   gagcttgttg tttatttcgg tctaccgcct gccgggcggg gtcgcggcga cggtaggcgc 14280
   tgtgcagccg ctgatggtcg tgttcatctc tgccgctctg ctaggtagcc cgatacgatt 14340
   gatggcggtc ctgggggcta tttgcggaac tgcgggcgtg gcgctgttgg tgttgacacc 14400
   aaacgcagcg ctagatcctg tcggcgtcgc agcgggcctg gcgggggcgg tttccatggc 14460
   gttcggaacc gtgctgaccc gcaagtggca acctcccgtg cctctgctca cctttaccgc 14520
   ctggcaactg gcggccggag gacttctgct cgttccagta gctttagtgt ttgatccgcc 14580
   aatcccgatg cctacaggaa ccaatgttct cggcctggcg tggctcggcc tgatcggagc 14640
   gggtttaacc tacttccttt ggttccgggg gatctcgcga ctcgaaccta cagttgtttc 14700
   cttactgggc tttctcagcc gggatggcgc taagaagcta ttgccgccga tcttcatatg 14760
   cggtgtgaaa taccgcacag atgcgtaagg agaaaatacc gcatcaggcg ctcttccgct 14820
   tcctcgctca ctgactcgct gcgctcggtc gttcggctgc ggcgagcggt atcagctcac 14880
   tcaaaggcgg taatacggtt atccacagaa tcaggggata acgcaggaaa gaacatgtga 14940
   gcaaaaggcc agcaaaaggc caggaaccgt aaaaaggccg cgttgctggc gtttttccat 15000
   aggctccgcc cccctgacga gcatcacaaa aatcgacgct caagtcagag gtggcgaaac 15060
   ccgacaggac tataaagata ccaggcgttt ccccctggaa gctccctcgt gcgctctcct 15120
   gttccgaccc tgccgcttac cggatacctg tccgcctttc tcccttcggg aagcgtggcg 15180
   ctttctcaat gctcacgctg taggtatctc agttcggtgt aggtcgttcg ctccaagctg 15240
   ggctgtgtgc acgaaccccc cgttcagccc gaccgctgcg ccttatccgg taactatcgt 15300
   cttgagtcca acccggtaag acacgactta tcgccactgg cagcagccac tggtaacagg 15360
   attagcagag cgaggtatgt aggcggtgct acagagttct tgaagtggtg gcctaactac 15420
   ggctacacta gaaggacagt atttggtatc tgcgctctgc tgaagccagt taccttcgga 15480
   aaaagagttg gtagctcttg atccggcaaa caaaccaccg ctggtagcgg tggttttttt 15540
   gtttgcaagc agcagattac gcgcagaaaa aaaggatatc aagaagatcc tttgatcttt 15600
   tctacggggt ctgacgctca gtggaacgaa aactcacgtt aagggatttt ggtcatgaga 15660
   ttatcaaaaa ggatcttcac ctagatcctt ttaaattaaa aatgaagttt taaatcaatc 15720
   taaagtatat atgagtaaac ttggtctgac agttaccaat gcttaatcag tgaggcacct 15780
   atctcagcga tctgtctatt tcgttcatcc atagttgcct gactccccgt cgtgtagata 15840
   actacgatac gggagggctt accatctggc cccagtgctg caatgatacc gcgagaccca 15900
   cgctcaccgg ctccagattt atcagcaata aaccagccag ccggaagggc cgagcgcaga 15960
   agtggtcctg caactttatc cgcctccatc cagtctatta aacaagtggc agcaacggat 16020
   tcgcaaacct gtcacgcctt ttgtgccaaa agccgcgcca ggtttgcgat ccgctgtgcc 16080
   aggcgttagg cgtcatatga agatttcggt gatccctgag caggtggcgg aaacattgga 16140
   tgctgagaac catttcattg ttcgtgaagt gttcgatgtg cacctatccg accaaggctt 16200
   tgaactatct accagaagtg tgagccccta ccggaaggat tacatctcgg atgatgactc 16260
   tgatgaagac tctgcttgct atggcgcatt catcgaccaa gagcttgtcg ggaagattga 16320
   actcaactca acatggaacg atctagcctc tatcgaacac attgttgtgt cgcacacgca 16380
   ccgaggcaaa ggagtcgcgc acagtctcat cgaatttgcg aaaaagtggg cactaagcag 16440
   acagctcctt ggcatacgat tagagacaca aacgaacaat gtacctgcct gcaatttgta 16500
   cgcaaaatgt ggctttactc tcggcggcat tgacctgttc acgtataaaa ctagacctca 16560
   agtctcgaac gaaacagcga tgtactggta ctggttctcg ggagcacagg atgacgccta 16620
   acaattcatt caagccgaca ccgcttcgcg gcgcggctta attcaggagt taaacatcat 16680
   gagggaagcg gtgatcgccg aagtatcgac tcaactatca gaggtagttg gcgtcatcga 16740
   gcgccatctc gaaccgacgt tgctggccgt acatttgtac ggctccgcag tggatggcgg 16800
   cctgaagcca cacagtgata ttgatttgct ggttacggtg accgtaaggc ttgatgaaac 16860
   aacgcggcga gctttgatca acgacctttt ggaaacttcg gcttcccctg gagagagcga 16920
   gattctccgc gctgtagaag tcaccattgt tgtgcacgac gacatcattc cgtggcgtta 16980
   tccagctaag cgcgaactgc aatttggaga atggcagcgc aatgacattc ttgcaggtat 17040
   cttcgagcca gccacgatcg acattgatct ggctatcttg ctgacaaaag caagagaaca 17100
   tagcgttgcc ttggtaggtc cagcggcgga ggaactcttt gatccggttc ctgaacagga 17160
   tctatttgag gcgctaaatg aaaccttaac gctatggaac tcgccgcccg actgggctgg 17220
   cgatgagcga aatgtagtgc ttacgttgtc ccgcatttgg tacagcgcag taaccggcaa 17280
   aatcgcgccg aaggatgtcg ctgccgactg ggcaatggag cgcctgccgg cccagtatca 17340
   gcccgtcata cttgaagcta ggcaggctta tcttggacaa gaagatcgct tggcctcgcg 17400
   cgcagatcag ttggaagaat ttgttcacta cgtgaaaggc gagatcacca aggtagtcgg 17460
   caaataatgt ctaacaattc gttcaagccg acgccgcttc gcggcgcggc ttaactcaag 17520
   cgttagagag ctggggaaga ctatgcgcga tctgttgaag gtggttctaa gcctcgtact 17580
   tgcgatggca tcggggcagg cacttgctga cctgccaatt gttttagtgg atgaagctcg 17640
   tcttccctat gactactccc catccaacta cgacatttct ccaagcaact acgacaactc 17700
   cataagcaat tacgacaata gtccatcaaa ttacgacaac tctgagagca actacgataa 17760
   tagttcatcc aattacgaca atagtcgcaa cggaaatcgt aggcttatat atagcgcaaa 17820
   tgggtctcgc actttcgccg gctactacgt cattgccaac aatgggacaa cgaacttctt 17880
   ttccacatct ggcaaaagga tgttctacac cccaaaaggg gggcgcggcg tctatggcgg 17940
   caaagatggg agcttctgcg gggcattggt cgtcataaat ggccaatttt cgcttgccct 18000
   gacagataac ggcctgaaga tcatgtatct aagcaactag cctgctctct aataaaatgt 18060
   taggagcttg gctgccattt ttggggtgag gccgttcgcg gccgaggggc gcagcccctg 18120
   gggggatggg aggcccgcgt tagcgggccg ggagggttcg agaagggggg gcacccccct 18180
   tcggcgtgcg cggtcacgcg ccagggcgca gccctggtta aaaacaaggt ttataaatat 18240
   tggtttaaaa gcaggttaaa agacaggtta gcggtggccg aaaaacgggc ggaaaccctt 18300
   gcaaatgctg gattttctgc ctgtggacag cccctcaaat gtcaataggt gcgcccctca 18360
   tctgtcagca ctctgcccct caagtgtcaa ggatcgcgcc cctcatctgt cagtagtcgc 18420
   gcccctcaag tgtcaatacc gcagggcact tatccccagg cttgtccaca tcatctgtgg 18480
   gaaactcgcg taaaatcagg cgttttcgcc gatttgcgag gctggccagc tccacgtcgc 18540
   cggccgaaat cgagcctgcc cctcatctgt caacgccgcg ccgggtgagt cggcccctca 18600
   agtgtcaacg tccgcccctc atctgtcagt gagggccaag ttttccgcga ggtatccaca 18660
   acgccggcgg ccggccgcgg tgtctcgcac acggcttcga cggcgtttct ggcgcgtttg 18720
   cagggccata gacggccgcc agcccagcgg cgagggcaac cagcccggtg agcgtcggaa 18780
   aggg 18784
<210> 28
   <211> 18784
   <212> DNA
   <213> Artificial Sequence
<220>
   <223> Artificial
<400> 28
   tcgacatcgt caacgttcac ttctaaagaa atagcgccac tcagcttcct cagcggcttt      60
   atccagcgat ttcctattat gtcggcatag ttctcaagat cgacagcctg tcacggttaa     120
   gcgagaaatg aataagaagg ctgataattc ggatctctgc gaggagatga tatttgatca     180
   caggcagcaa cgctctgtca tcgttacaat caacatgcta ccctccgcga gatcatccgt     240
   gtttcaaacc cggcagctta gttgccgttc ttccgaatag catcgggtaa catgagcaaa     300
   gtctgccgcc ttacaacggc tctcccgctg acgccgtccc ggactgatgg gctgcctgta     360
   tcgagtggtg attttgtgcc gagctgccgg tcggggagct gttggctggc tggtggcagg     420
   atatattgtg gtgtaaacaa attgacgctt agacaactta ataacacatt gcggacgttt     480
   ttaatgtact gaattcgccg ctcggtgtgt cgtagatact agcccctggg gcacttttga     540
   aatttgaata agatttatgt aatcagtctt ttaggtttga ccggttctgc cgcttttttt     600
   aaaattggat ttgtaataat aaaacgcaat tgtttgttat tgtggcgctc tatcatagat     660
   gtcgctataa acctattcag cacaatatat tgttttcatt ttaatattgt acatataagt     720
   agtagggtac aatcagtaaa ttgaacggag aatattattc ataaaaatac gatagtaacg     780
   ggtgatatat tcattagaat gaaccgaaac cggcggtaag gatctgagct acacatgctc     840
   aggtttttta caacgtgcac aacagaattg aaagcaaata tcatgcgatc ataggcgtct     900
   cgcatatctc attaaacaag tgaagatttg attcaaactc cattgagagc cctgactatg     960
   cattcggttt gacccttcca ggttgagaga cgatagcccc ctaccttaat taaggggccc    1020
   cccctcgagg tcgacggtat cgataagctt gatatcgaat tcctgcagcc agggggatcc    1080
   cccgggtcat cagatctcgg tgacgggcag gaccggacgg ggcggtaccg gcaggctgaa    1140
   gtccagctgc cagaaaccca cgtcatgcca gttcccgtgc ttgaagccgg ccgcccgcag    1200
   catgccgcgg ggggcatatc cgagcgcctc gtgcatgcgc acgctcgggt cgttgggcag    1260
   cccgatgaca gcgaccacgc tcttgaagcc ctgtgcctcc agggacttca gcaggtgggt 1320
   gtagagcgtg gagcccagtc ccgtccgctg gtggcggggg gagacgtaca cggtcgactc 1380
   ggccgtccag tcgtaggcgt tgcgtgcctt ccaggggccc gcgtaggcga tgccggcgac 1440
   ctcgccgtcc acctcggcga cgagccaggg atagcgctcc cgcagacgga cgaggtcgtc 1500
   cgtccactcc tgcggttcct gcggctcggt acggaagttg accgtgcttg tctcgatgta 1560
   gtggttgacg atggtgcaga ccgccggcat gtccgcctcg gtggcacggc ggatgtcggc 1620
   cgggcgtcgt tctgggctca tggtagatcc cctcgagaga gatagatttg tagagagaga 1680
   ctggtgattt cagcgtgtcc tctccaaatg aaatgaactt ccttatatag aggaaggtct 1740
   tgcgaaggat agtgggattg tgcgtcatcc cttacgtcag tggagatatc acatcaatcc 1800
   acttgctttg aagacgtggt tggaacgtct tctttttcca cgatgctcct cgtgggtggg 1860
   ggtccatctt tgggaccact gtcggcagag gcatcttgaa cgatagcctt tcctttatcg 1920
   caatgatggc atttgtaggt gccaccttcc ttttctactg tccttttgat gaagtgacag 1980
   atagctgggc aatggaatcc gaggaggttt cccgatatta ccctttgttg aaaagtctca 2040
   atagcccttt ggtcttctga gactgtatct ttgatattct tggagtagac gagagtgtcg 2100
   tgctccacca tgttatcaca tcaatccact tgctttgaag acgtggttgg aacgtcttct 2160
   ttttccacga tgctcctcgt gggtgggggt ccatctttgg gaccactgtc ggcagaggca 2220
   tcttgaacga tagcctttcc tttatcgcaa tgatggcatt tgtaggtgcc accttccttt 2280
   tctactgtcc ttttgatgaa gtgacagata gctgggcaat ggaatccgag gaggtttccc 2340
   gatattaccc tttgttgaaa agtctcaata gccctttggt cttctgagac tgtatctttg 2400
   atattcttgg agtagacgag agtgtcgtgc tccaccatgt tgctgatagt gaccttaggc 2460
   gacttttgaa cgcgcaataa tggtttctga cgtatgtgct tagctcatta aactccagaa 2520
   acccattaac gcttacaatt tccattcgcc attcaggctg cgcaactgtt gggaagggcg 2580
   atcggtgcgg gcctcttcgc tattacgcca gctggcgaaa gggggatgtg ctgcaaggcg 2640
   attaagttgg gtaacgccag ggttttccca gtcacgacgt tgtaaaacga cggccagtga 2700
   attgtaatac gactcactat agggcgaatt gggcccgacg tcgcatgctc ccggccgcca 2760
   tggccgcggg atatcactag tgcggccgct cgacgaatta attccaatcc cacaaaaatc 2820
   tgagcttaac agcacagttg ctcctctcag agcagaatcg ggtattcaac accctcatat 2880
   caactactac gttgtgtata acggtccaca tgccggtata tacgatgact ggggttgtac 2940
   aaaggcggca acaaacggcg ttcccggagt tgcacacaag aaatttgcca ctattacaga 3000
   ggcaagagca gcagctgacg cgtacacaac aagtcagcaa acagacaggt tgaacttcat 3060
   ccccaaagga gaagctcaac tcaagcccaa gagctttgct aaggccctaa caagcccacc 3120
   aaagcaaaaa gcccactggc tcacgctagg aaccaaaagg cccagcagtg atccagcccc 3180
   aaaagagatc tcctttgccc cggagattac aatggacgat ttcctctatc tttacgatct 3240
   aggaaggaag ttcgaaggtg aaggtgacga cactatgttc accactgata atgagaaggt 3300
   tagcctcttc aatttcagaa agaatgctga cccacagatg gttagagagg cctacgcagc      3360
   aggtctcatc aagacgatct acccgagtaa caatctccag gagatcaaat accttcccaa      3420
   gaaggttaaa gatgcagtca aaagattcag gactaattgc atcaagaaca cagagaaaga      3480
   catatttctc aagatcagaa gtactattcc agtatggacg attcaaggct tgcttcataa      3540
   accaaggcaa gtaatagaga ttggagtctc taaaaaggta gttcctactg aatctaaggc      3600
   catgcatgga gtctaagatt caaatcgagg atctaacaga actcgccgtg aagactggcg      3660
   aacagttcat acagagtctt ttacgactca atgacaagaa gaaaatcttc gtcaacatgg      3720
   tggagcacga cactctggtc tactccaaaa atgtcaaaga tacagtctca gaagaccaaa      3780
   gggctattga gacttttcaa caaaggataa tttcgggaaa cctcctcgga ttccattgcc      3840
   cagctatctg tcacttcatc gaaaggacag tagaaaagga aggtggctcc tacaaatgcc      3900
   atcattgcga taaaggaaag gctatcattc aagatctctc tgccgacagt ggtcccaaag      3960
   atggaccccc acccacgagg agcatcgtgg aaaaagaaga cgttccaacc acgtcttcaa      4020
   agcaagtgga ttgatgtgac atctccactg acgtaaggga tgacgcacaa tcccactatc      4080
   cttcgcaaga cccttcctct atataaggaa gttcatttca tttggagagg acacgctcga      4140
   ggaattcggt accccatcac aagtttgtac aaaaaagcag gctgcggccg cttgctccct      4200
   taaaaaaaac catggcatgt cattacggac aacagcaaca gactagagca cctcatcttc      4260
   agcttcaacc tagagcacag agagttgtga aggctgctac tgctgttact gtaaatttct      4320
   gtgttcctta ttctctcaaa atcttcgatt ttgttttcgt tcgatcccaa tttcgtatat      4380
   gttctttggt ttagattctg ttaatcttag atcgaagacg attttctggg tttgatcgtt      4440
   agatatcatc ttaattctcg attagggttt catagatatc atccgatttg ttcaaataat      4500
   ttgagttttg tcgaataatt actcttcgat ttgtgatttc tatctagatc tggtgttagt      4560
   ttctagtttg tgcgatcgaa tttgtcgatt aatctgagtt tttctgatta acaggctgga      4620
   ggatctcttc ttgttctctc tggacttact ctcgctggaa ctgttatcgc tctcactatc      4680
   gctacacctc ttctcgttat cttctctcct gttctcgttc ctgctgtgat cactatcttc      4740
   cttctcggag ctggatttct tgcttctggt ggatttggag ttgctgctct ctctgttctc      4800
   tcttggatct acagatacct cactggaaaa catcctccag gtgctgattg tcttgagtct      4860
   gctaagacta agctcgcttc ttgtgctaga gagatgaagg atagagcaga gcaattctct      4920
   tgtcagcctg ttgctggatc tcagacttct taatgaacat atggtcctgc tttaatgaga      4980
   tatgcgagac gcctatgatc gcatgatatt tgctttcaat tctgttgtgc acgttgtaaa      5040
   aaacctgagc atgtgtagct cagatcctta ccgccggttt cggttcattc taatgaatat      5100
   atcacccgtt actatcgtat ttttatgaat aatattctcc gttcaattta ctgattgtac      5160
   cctactactt atatgtacaa tattaaaatg aaaacaatat attgtgctga ataggtttat      5220
   agcgacatct atgatagagc gccacaataa caaacaattg cgttttatta ttacaaatcc      5280
   aattttaaaa aaagcggcag aaccggtcaa acctaaaaga ctgattacat aaatcttatt      5340
   caaatttcaa aaggccccag gggctagtat ctacgacaca ccgagcggcg aactaataac 5400
   gttcactgaa gggaactccg gttccccgcc ggcgcgcatg ggtgagattc cttgaagttg 5460
   agtattggcc gtccgctcta ccgaaagtta cgggcaccat tcaacccggt ccagcacggc 5520
   ggccgggtaa ccgacttgct gccccgagaa ttatgcagca tttttttggt gtatgtgggc 5580
   cccaaatgaa gtgcaggtca aaccttgaca gtgacgacaa atcgttgggc gggtccaggg 5640
   cgaattttgc gacaacatgt cgaggctcag caggacctgc aggcatgcaa gctagcttac 5700
   tagtgatgca tattctatag tgtcacctaa atcttcgacg aattaattcc aatcccacaa 5760
   aaatctgagc ttaacagcac agttgctcct ctcagagcag aatcgggtat tcaacaccct 5820
   catatcaact actacgttgt gtataacggt ccacatgccg gtatatacga tgactggggt 5880
   tgtacaaagg cggcaacaaa cggcgttccc ggagttgcac acaagaaatt tgccactatt 5940
   acagaggcaa gagcagcagc tgacgcgtac acaacaagtc agcaaacaga caggttgaac 6000
   ttcatcccca aaggagaagc tcaactcaag cccaagagct ttgctaaggc cctaacaagc 6060
   ccaccaaagc aaaaagccca ctggctcacg ctaggaacca aaaggcccag cagtgatcca 6120
   gccccaaaag agatctcctt tgccccggag attacaatgg acgatttcct ctatctttac 6180
   gatctaggaa ggaagttcga aggtgaaggt gacgacacta tgttcaccac tgataatgag 6240
   aaggttagcc tcttcaattt cagaaagaat gctgacccac agatggttag agaggcctac 6300
   gcagcaggtc tcatcaagac gatctacccg agtaacaatc tccaggagat caaatacctt 6360
   cccaagaagg ttaaagatgc agtcaaaaga ttcaggacta attgcatcaa gaacacagag 6420
   aaagacatat ttctcaagat cagaagtact attccagtat ggacgattca aggcttgctt 6480
   cataaaccaa ggcaagtaat agagattgga gtctctaaaa aggtagttcc tactgaatct 6540
   aaggccatgc atggagtcta agattcaaat cgaggatcta acagaactcg ccgtgaagac 6600
   tggcgaacag ttcatacaga gtcttttacg actcaatgac aagaagaaaa tcttcgtcaa 6660
   catggtggag cacgacactc tggtctactc caaaaatgtc aaagatacag tctcagaaga 6720
   ccaaagggct attgagactt ttcaacaaag gataatttcg ggaaacctcc tcggattcca 6780
   ttgcccagct atctgtcact tcatcgaaag gacagtagaa aaggaaggtg gctcctacaa 6840
   atgccatcat tgcgataaag gaaaggctat cattcaagat ctctctgccg acagtggtcc 6900
   caaagatgga cccccaccca cgaggagcat cgtggaaaaa gaagacgttc caaccacgtc 6960
   ttcaaagcaa gtggattgat gtgacatctc cactgacgta agggatgacg cacaatccca 7020
   ctatccttcg caagaccctt cctctatata aggaagttca tttcatttgg agaggacacg 7080
   ggatccttgc tccgttaaaa aaaaccatgg ctatcctcga ttctgctggt gttactactg 7140
   tgactgagaa tggtggtgga gagttcgttg atctcgatag actcagaaga agaaagtcta 7200
   gatctgtaaa tttctgtgtt ccttattctc tcaaaatctt cgattttgtt ttcgttcgat 7260
   cccaatttcg tatatgttct ttggtttaga ttctgttaat cttagatcga agacgatttt 7320
   ctgggtttga tcgttagata tcatcttaat tctcgattag ggtttcatag atatcatccg 7380
   atttgttcaa ataatttgag ttttgtcgaa taattactct tcgatttgtg atttctatct 7440
   agatctggtg ttagtttcta gtttgtgcga tcgaatttgt cgattaatct gagtttttct 7500
   gattaacagg attcttctaa cggacttctc ctctctggat ctgataacaa ctctccttct 7560
   gatgatgttg gtgctcctgc tgatgtgaga gatagaatcg attctgtggt gaacgatgat 7620
   gctcaaggta ctgctaacct cgctggtgat aataacggtg gaggtgataa caatggtgga 7680
   ggaagaggtg gaggtgaagg tagaggaaac gctgatgcta ctttcactta cagaccatct 7740
   gtgcctgctc atagaagagc tagagagtct cctctctctt ctgatgctat cttcaagcag 7800
   tctcacgctg gacttttcaa cctctgtgtg gtggttctta tcgctgtgaa ctctagactc 7860
   atcatcgaga acctcatgaa gtacggatgg ctcatcagaa ctgatttctg gttctcttct 7920
   agatctctca gagattggcc tcttttcatg tgctgcatct cactctcaat cttccctctc 7980
   gctgctttta ctgttgagaa gctcgtgctc cagaagtata tcgctgaacc tgtggtgatc 8040
   ttcctccaca tcatcatcac tatgactgag gttctctacc ctgtttacgt gactctcaga 8100
   tgcgattctg ctttcctctc tggtgttact cttatgctcc tcacttgcat tgtgtggctt 8160
   aagctcgtgt cttacgctca cacttcttac gatatcagat ctctcgctaa cgctgctgat 8220
   aaggctaacc ctgaagtgtc ttactacgtg tctctcaagt ctctcgctta cttcatggtt 8280
   gctcctacac tttgttacca gccatcttac cctagatctg cttgcattag aaagggatgg 8340
   gtggcaagac aattcgctaa gttggtgatc ttcactggat tcatgggatt catcatcgag 8400
   cagtacatca accctattgt gagaaactct aagcaccctc tcaagggtga tcttctctac 8460
   gctatcgaga gagttcttaa gctctctgtg cctaaccttt atgtgtggct ctgcatgttc 8520
   tactgtttct tccacctctg gcttaacatc cttgctgagt tgctttgctt cggagataga 8580
   gagttctaca aggattggtg gaacgctaag tctgttggag attattggag aatgtggaac 8640
   atgcctgtgc ataagtggat ggtgcgtcac atctacttcc cttgcctcag atctaagatc 8700
   cctaagactc tcgctatcat tatcgctttc ctcgtgtctg ctgttttcca tgagttgtgt 8760
   atcgctgttc cttgcagact tttcaagctt tgggctttcc tcggaatcat gttccaggtt 8820
   ccactcgtgt tcatcactaa ctacctccaa gagagattcg gatctactgt tggaaacatg 8880
   attttctggt tcattttctg catcttcgga cagcctatgt gcgttctcct ctactaccac 8940
   gatctcatga acagaaaggg atctatgtct taatgaagga tccacccagc tttcttgtac 9000
   aaagtggtga tgggttcgaa atcgataagc ttggatcctc tagagtcctg ctttaatgag 9060
   atatgcgaga cgcctatgat cgcatgatat ttgctttcaa ttctgttgtg cacgttgtaa 9120
   aaaacctgag catgtgtagc tcagatcctt accgccggtt tcggttcatt ctaatgaata 9180
   tatcacccgt tactatcgta tttttatgaa taatattctc cgttcaattt actgattgta 9240
   ccctactact tatatgtaca atattaaaat gaaaacaata tattgtgctg aataggttta 9300
   tagcgacatc tatgatagag cgccacaata acaaacaatt gcgttttatt attacaaatc 9360
   caattttaaa aaaagcggca gaaccggtca aacctaaaag actgattaca taaatcttat 9420
   tcaaatttca aaaggcccca ggggctagta tctacgacac accgagcggc gaactaataa 9480
   cgttcactga agggaactcc ggttccccgc cggcgcgcat gggtgagatt ccttgaagtt 9540
   gagtattggc cgtccgctct accgaaagtt acgggcacca ttcaacccgg tccagcacgg 9600
   cggccgggta accgacttgc tgccccgaga attatgcagc atttttttgg tgtatgtggg 9660
   ccccaaatga agtgcaggtc aaaccttgac agtgacgaca aatcgttggg cgggtccagg 9720
   gcgaattttg cgacaacatg tcgaggctca gcaggacctg caggcatgca agctagctta 9780
   ctagtgatgc atattctata gtgtcaccta aatctgcggc cgcctgcagg tcgatatggg 9840
   agagctccca acgcgttgga tgcatagctt gagtattcta tagtgtcacc taaatagctt 9900
   ggcgtaatca tggtcatagc tgtttcctgt gtgaaattgt tatccgctca caattccaca 9960
   caacatacga gccggaagca taaagtgtaa agcctggggt gcctaatgag tgagctaact 10020
   cacattaatt gcgttgcgct cactgcccgc tttccagtcg ggaaacctgt cgtgccagct 10080
   gcattaatga atcggccaac gcgcggggag aggcggtttg cgtattgggg ctgagtggct 10140
   ccttcaacgt tgcggttctg tcagttccaa acgtaaaacg gcttgtcccg cgtcatcggc 10200
   gggggtcata acgtgactcc cttaattctc cgctcatgat cagattgtcg tttcccgcct 10260
   tcagtttaaa ctatcagtgt ttgacaggat atattggcgg gtaaacctaa gagaaaagag 10320
   cgtttattag aataatcgga tatttaaaag ggcgtgaaaa ggtttatccg ttcgtccatt 10380
   tgtatgtgca tgccaaccac agggttcccc tcgggagtgc ttggcattcc gtgcgataat 10440
   gacttctgtt caaccaccca aacgtcggaa agcctgacga cggagcagca ttccaaaaag 10500
   atcccttggc tcgtctgggt cggctagaag gtcgagtggg ctgctgtggc ttgatccctc 10560
   aacgcggtcg cggacgtagc gcagcgccga aaaatcctcg atcgcaaatc cgacgctgtc 10620
   gaaaatcgtg atctgcttgt cgctctttcg gccgacgtcc tggccagtca tcacgcgcca 10680
   aagttccgtc acaggatgat ctggcgcgag ttgctggatc tcgccttcaa tccgggtctg 10740
   tggcgggaac tccacgaaaa tatccgaacg cagcaagatg tcgacggatc ttttccgctg 10800
   cataaccctg cttcggggtc attatagcga ttttttcggt atatccatcc tttttcgcac 10860
   gatatacagg attttgccaa agggttcgtg tagactttcc ttggtgtatc caacggcgtc 10920
   agccgggcag gataggtgaa gtaggcccac ccgcgagcgg gtgttccttc ttcactgtcc 10980
   cttattcgca cctggcggtg ctcaacggga atcctgctct gcgaggctgg ccggctaccg 11040
   ccggcgtaac agatgagggc aagcggatgg ctgatgaaac caagccaacc aggggtgatg 11100
   ctgccaactt actgatttag tgtatgatgg tgtttttgag gtgctccagt ggcttctgtt 11160
   tctatcagct gtccctcctg ttcagctact gacggggtgg tgcgtaacgg caaaagcacc 11220
   gccggacatc agcgctatct ctgctctcac tgccgtaaaa catggcaact gcagttcact 11280
   tacaccgctt ctcaacccgg tacgcaccag aaaatcattg atatggccat gaatggcgtt 11340
   ggatgccggg caacagcccg cattatgggc gttggcctca acacgatttt acgtcactta 11400
   aaaaactcag gccgcagtcg gtaacctcgc gcatacagcc gggcagtgac gtcatcgtct 11460
   gcgcggaaat ggacgaacag tggggctatg tcggggctaa atcgcgccag cgctggctgt 11520
   tttacgcgta tgacagtctc cggaagacgg ttgttgcgca cgtattcggt gaacgcacta 11580
   tggcgacgct ggggcgtctt atgagcctgc tgtcaccctt tgacgtggtg atatggatga 11640
   cggatggctg gccgctgtat gaatcccgcc tgaagggaaa gctgcacgta atcagcaagc 11700
   gatatacgca gcgaattgag cggcataacc tgaatctgag gcagcacctg gcacggctgg 11760
   gacggaagtc gctgtcgttc tcaaaatcgg tggagctgca tgacaaagtc atcgggcatt 11820
   atctgaacat aaaacactat caataagttg gagtcattac ccaaccagga agggcagccc 11880
   acctatcaag gtgtactgcc ttccagacga acgaagagcg attgaggaaa aggcggcggc 11940
   ggccggcatg agcctgtcgg cctacctgct ggccgtcggc cagggctaca aaatcacggg 12000
   cgtcgtggac tatgagcacg tccgcgagct ggcccgcatc aatggcgacc tgggccgcct 12060
   gggcggcctg ctgaaactct ggctcaccga cgacccgcgc acggcgcggt tcggtgatgc 12120
   cacgatcctc gccctgctgg cgaagatcga agagaagcag gacgagcttg gcaaggtcat 12180
   gatgggcgtg gtccgcccga gggcagagcc atgacttttt tagccgctaa aacggccggg 12240
   gggtgcgcgt gattgccaag cacgtcccca tgcgctccat caagaagagc gacttcgcgg 12300
   agctggtatt cgtgcagggc aagattcgga ataccaagta cgagaaggac ggccagacgg 12360
   tctacgggac cgacttcatt gccgataagg tggattatct ggacaccaag gcaccaggcg 12420
   ggtcaaatca ggaataaggg cacattgccc cggcgtgagt cggggcaatc ccgcaaggag 12480
   ggtgaatgaa tcggacgttt gaccggaagg catacaggca agaactgatc gacgcggggt 12540
   tttccgccga ggatgccgaa accatcgcaa gccgcaccgt catgcgtgcg ccccgcgaaa 12600
   ccttccagtc cgtcggctcg atggtccagc aagctacggc caagatcgag cgcgacagcg 12660
   tgcaactggc tccccctgcc ctgcccgcgc catcggccgc cgtggagcgt tcgcgtcgtc 12720
   tcgaacagga ggcggcaggt ttggcgaagt cgatgaccat cgacacgcga ggaactatga 12780
   cgaccaagaa gcgaaaaacc gccggcgagg acctggcaaa acaggtcagc gaggccaagc 12840
   aggccgcgtt gctgaaacac acgaagcagc agatcaagga aatgcagctt tccttgttcg 12900
   atattgcgcc gtggccggac acgatgcgag cgatgccaaa cgacacggcc cgctctgccc 12960
   tgttcaccac gcgcaacaag aaaatcccgc gcgaggcgct gcaaaacaag gtcattttcc 13020
   acgtcaacaa ggacgtgaag atcacctaca ccggcgtcga gctgcgggcc gacgatgacg 13080
   aactggtgtg gcagcaggtg ttggagtacg cgaagcgcac ccctatcggc gagccgatca 13140
   ccttcacgtt ctacgagctt tgccaggacc tgggctggtc gatcaatggc cggtattaca 13200
   cgaaggccga ggaatgcctg tcgcgcctac aggcgacggc gatgggcttc acgtccgacc 13260
   gcgttgggca cctggaatcg gtgtcgctgc tgcaccgctt ccgcgtcctg gaccgtggca 13320
   agaaaacgtc ccgttgccag gtcctgatcg acgaggaaat cgtcgtgctg tttgctggcg 13380
   accactacac gaaattcata tgggagaagt accgcaagct gtcgccgacg gcccgacgga 13440
   tgttcgacta tttcagctcg caccgggagc cgtacccgct caagctggaa accttccgcc 13500
   tcatgtgcgg atcggattcc acccgcgtga agaagtggcg cgagcaggtc ggcgaagcct 13560
   gcgaagagtt gcgaggcagc ggcctggtgg aacacgcctg ggtcaatgat gacctggtgc 13620
   attgcaaacg ctagggcctt gtggggtcag ttccggctgg gggttcagca gccagcgctt 13680
   tactggcatt tcaggaacaa gcgggcactg ctcgacgcac ttgcttcgct cagtatcgct 13740
   cgggacgcac ggcgcgctct acgaactgcc gataaacaga ggattaaaat tgacaattgt 13800
   gattaaggct cagattcgac ggcttggagc ggccgacgtg caggatttcc gcgagatccg 13860
   attgtcggcc ctgaagaaag ctccagagat gttcgggtcc gtttacgagc acgaggagaa 13920
   aaagcccatg gaggcgttcg ctgaacggtt gcgagatgcc gtggcattcg gcgcctacat 13980
   cgacggcgag atcattgggc tgtcggtctt caaacaggag gacggcccca aggacgctca 14040
   caaggcgcat ctgtccggcg ttttcgtgga gcccgaacag cgaggccgag gggtcgccgg 14100
   tatgctgctg cgggcgttgc cggcgggttt attgctcgtg atgatcgtcc gacagattcc 14160
   aacgggaatc tggtggatgc gcatcttcat cctcggcgca cttaatattt cgctattctg 14220
   gagcttgttg tttatttcgg tctaccgcct gccgggcggg gtcgcggcga cggtaggcgc 14280
   tgtgcagccg ctgatggtcg tgttcatctc tgccgctctg ctaggtagcc cgatacgatt 14340
   gatggcggtc ctgggggcta tttgcggaac tgcgggcgtg gcgctgttgg tgttgacacc 14400
   aaacgcagcg ctagatcctg tcggcgtcgc agcgggcctg gcgggggcgg tttccatggc 14460
   gttcggaacc gtgctgaccc gcaagtggca acctcccgtg cctctgctca cctttaccgc 14520
   ctggcaactg gcggccggag gacttctgct cgttccagta gctttagtgt ttgatccgcc 14580
   aatcccgatg cctacaggaa ccaatgttct cggcctggcg tggctcggcc tgatcggagc 14640
   gggtttaacc tacttccttt ggttccgggg gatctcgcga ctcgaaccta cagttgtttc 14700
   cttactgggc tttctcagcc gggatggcgc taagaagcta ttgccgccga tcttcatatg 14760
   cggtgtgaaa taccgcacag atgcgtaagg agaaaatacc gcatcaggcg ctcttccgct 14820
   tcctcgctca ctgactcgct gcgctcggtc gttcggctgc ggcgagcggt atcagctcac 14880
   tcaaaggcgg taatacggtt atccacagaa tcaggggata acgcaggaaa gaacatgtga 14940
   gcaaaaggcc agcaaaaggc caggaaccgt aaaaaggccg cgttgctggc gtttttccat 15000
   aggctccgcc cccctgacga gcatcacaaa aatcgacgct caagtcagag gtggcgaaac 15060
   ccgacaggac tataaagata ccaggcgttt ccccctggaa gctccctcgt gcgctctcct 15120
   gttccgaccc tgccgcttac cggatacctg tccgcctttc tcccttcggg aagcgtggcg 15180
   ctttctcaat gctcacgctg taggtatctc agttcggtgt aggtcgttcg ctccaagctg 15240
   ggctgtgtgc acgaaccccc cgttcagccc gaccgctgcg ccttatccgg taactatcgt 15300
   cttgagtcca acccggtaag acacgactta tcgccactgg cagcagccac tggtaacagg 15360
   attagcagag cgaggtatgt aggcggtgct acagagttct tgaagtggtg gcctaactac 15420
   ggctacacta gaaggacagt atttggtatc tgcgctctgc tgaagccagt taccttcgga 15480
   aaaagagttg gtagctcttg atccggcaaa caaaccaccg ctggtagcgg tggttttttt 15540
   gtttgcaagc agcagattac gcgcagaaaa aaaggatatc aagaagatcc tttgatcttt 15600
   tctacggggt ctgacgctca gtggaacgaa aactcacgtt aagggatttt ggtcatgaga 15660
   ttatcaaaaa ggatcttcac ctagatcctt ttaaattaaa aatgaagttt taaatcaatc 15720
   taaagtatat atgagtaaac ttggtctgac agttaccaat gcttaatcag tgaggcacct 15780
   atctcagcga tctgtctatt tcgttcatcc atagttgcct gactccccgt cgtgtagata 15840
   actacgatac gggagggctt accatctggc cccagtgctg caatgatacc gcgagaccca 15900
   cgctcaccgg ctccagattt atcagcaata aaccagccag ccggaagggc cgagcgcaga 15960
   agtggtcctg caactttatc cgcctccatc cagtctatta aacaagtggc agcaacggat 16020
   tcgcaaacct gtcacgcctt ttgtgccaaa agccgcgcca ggtttgcgat ccgctgtgcc 16080
   aggcgttagg cgtcatatga agatttcggt gatccctgag caggtggcgg aaacattgga 16140
   tgctgagaac catttcattg ttcgtgaagt gttcgatgtg cacctatccg accaaggctt 16200
   tgaactatct accagaagtg tgagccccta ccggaaggat tacatctcgg atgatgactc 16260
   tgatgaagac tctgcttgct atggcgcatt catcgaccaa gagcttgtcg ggaagattga 16320
   actcaactca acatggaacg atctagcctc tatcgaacac attgttgtgt cgcacacgca 16380
   ccgaggcaaa ggagtcgcgc acagtctcat cgaatttgcg aaaaagtggg cactaagcag 16440
   acagctcctt ggcatacgat tagagacaca aacgaacaat gtacctgcct gcaatttgta 16500
   cgcaaaatgt ggctttactc tcggcggcat tgacctgttc acgtataaaa ctagacctca 16560
   agtctcgaac gaaacagcga tgtactggta ctggttctcg ggagcacagg atgacgccta 16620
   acaattcatt caagccgaca ccgcttcgcg gcgcggctta attcaggagt taaacatcat 16680
   gagggaagcg gtgatcgccg aagtatcgac tcaactatca gaggtagttg gcgtcatcga 16740
   gcgccatctc gaaccgacgt tgctggccgt acatttgtac ggctccgcag tggatggcgg 16800
   cctgaagcca cacagtgata ttgatttgct ggttacggtg accgtaaggc ttgatgaaac 16860
   aacgcggcga gctttgatca acgacctttt ggaaacttcg gcttcccctg gagagagcga 16920
   gattctccgc gctgtagaag tcaccattgt tgtgcacgac gacatcattc cgtggcgtta 16980
   tccagctaag cgcgaactgc aatttggaga atggcagcgc aatgacattc ttgcaggtat 17040
   cttcgagcca gccacgatcg acattgatct ggctatcttg ctgacaaaag caagagaaca 17100
   tagcgttgcc ttggtaggtc cagcggcgga ggaactcttt gatccggttc ctgaacagga 17160
   tctatttgag gcgctaaatg aaaccttaac gctatggaac tcgccgcccg actgggctgg 17220
   cgatgagcga aatgtagtgc ttacgttgtc ccgcatttgg tacagcgcag taaccggcaa 17280
   aatcgcgccg aaggatgtcg ctgccgactg ggcaatggag cgcctgccgg cccagtatca 17340
   gcccgtcata cttgaagcta ggcaggctta tcttggacaa gaagatcgct tggcctcgcg 17400
   cgcagatcag ttggaagaat ttgttcacta cgtgaaaggc gagatcacca aggtagtcgg 17460
   caaataatgt ctaacaattc gttcaagccg acgccgcttc gcggcgcggc ttaactcaag 17520
   cgttagagag ctggggaaga ctatgcgcga tctgttgaag gtggttctaa gcctcgtact 17580
   tgcgatggca tcggggcagg cacttgctga cctgccaatt gttttagtgg atgaagctcg 17640
   tcttccctat gactactccc catccaacta cgacatttct ccaagcaact acgacaactc 17700
   cataagcaat tacgacaata gtccatcaaa ttacgacaac tctgagagca actacgataa 17760
   tagttcatcc aattacgaca atagtcgcaa cggaaatcgt aggcttatat atagcgcaaa 17820
   tgggtctcgc actttcgccg gctactacgt cattgccaac aatgggacaa cgaacttctt 17880
   ttccacatct ggcaaaagga tgttctacac cccaaaaggg gggcgcggcg tctatggcgg 17940
   caaagatggg agcttctgcg gggcattggt cgtcataaat ggccaatttt cgcttgccct 18000
   gacagataac ggcctgaaga tcatgtatct aagcaactag cctgctctct aataaaatgt 18060
   taggagcttg gctgccattt ttggggtgag gccgttcgcg gccgaggggc gcagcccctg 18120
   gggggatggg aggcccgcgt tagcgggccg ggagggttcg agaagggggg gcacccccct 18180
   tcggcgtgcg cggtcacgcg ccagggcgca gccctggtta aaaacaaggt ttataaatat 18240
   tggtttaaaa gcaggttaaa agacaggtta gcggtggccg aaaaacgggc ggaaaccctt 18300
   gcaaatgctg gattttctgc ctgtggacag cccctcaaat gtcaataggt gcgcccctca 18360
   tctgtcagca ctctgcccct caagtgtcaa ggatcgcgcc cctcatctgt cagtagtcgc 18420
   gcccctcaag tgtcaatacc gcagggcact tatccccagg cttgtccaca tcatctgtgg 18480
   gaaactcgcg taaaatcagg cgttttcgcc gatttgcgag gctggccagc tccacgtcgc 18540
   cggccgaaat cgagcctgcc cctcatctgt caacgccgcg ccgggtgagt cggcccctca 18600
   agtgtcaacg tccgcccctc atctgtcagt gagggccaag ttttccgcga ggtatccaca 18660
   acgccggcgg ccggccgcgg tgtctcgcac acggcttcga cggcgtttct ggcgcgtttg 18720
   cagggccata gacggccgcc agcccagcgg cgagggcaac cagcccggtg agcgtcggaa 18780
   aggg 18784
<210> 29
   <211> 18784
   <212> DNA
   <213> Artificial Sequence
<220>
   <223> Artificial
<400> 29
   gtcgacatcg tcaacgttca cttctaaaga aatagcgcca ctcagcttcc tcagcggctt 60
   tatccagcga tttcctatta tgtcggcata gttctcaaga tcgacagcct gtcacggtta 120
   agcgagaaat gaataagaag gctgataatt cggatctctg cgaggagatg atatttgatc 180
   acaggcagca acgctctgtc atcgttacaa tcaacatgct accctccgcg agatcatccg 240
   tgtttcaaac ccggcagctt agttgccgtt cttccgaata gcatcgggta acatgagcaa 300
   agtctgccgc cttacaacgg ctctcccgct gacgccgtcc cggactgatg ggctgcctgt 360
   atcgagtggt gattttgtgc cgagctgccg gtcggggagc tgttggctgg ctggtggcag 420
   gatatattgt ggtgtaaaca aattgacgct tagacaactt aataacacat tgcggacgtt 480
   tttaatgtac tgaattcgcc gctcggtgtg tcgtagatac tagcccctgg ggcacttttg 540
   aaatttgaat aagatttatg taatcagtct tttaggtttg accggttctg ccgctttttt 600
   taaaattgga tttgtaataa taaaacgcaa ttgtttgtta ttgtggcgct ctatcataga 660
   tgtcgctata aacctattca gcacaatata ttgttttcat tttaatattg tacatataag 720
   tagtagggta caatcagtaa attgaacgga gaatattatt cataaaaata cgatagtaac 780
   gggtgatata ttcattagaa tgaaccgaaa ccggcggtaa ggatctgagc tacacatgct 840
   caggtttttt acaacgtgca caacagaatt gaaagcaaat atcatgcgat cataggcgtc 900
   tcgcatatct cattaaacaa gtgaagattt gattcaaact ccattgagag ccctgactat 960
   gcattcggtt tgacccttcc aggttgagag acgatagccc cctaccttaa ttaaggggcc 1020
   ccccctcgag gtcgacggta tcgataagct tgatatcgaa ttcctgcagc cagggggatc 1080
   ccccgggtca tcagatctcg gtgacgggca ggaccggacg gggcggtacc ggcaggctga 1140
   agtccagctg ccagaaaccc acgtcatgcc agttcccgtg cttgaagccg gccgcccgca 1200
   gcatgccgcg gggggcatat ccgagcgcct cgtgcatgcg cacgctcggg tcgttgggca 1260
   gcccgatgac agcgaccacg ctcttgaagc cctgtgcctc cagggacttc agcaggtggg 1320
   tgtagagcgt ggagcccagt cccgtccgct ggtggcgggg ggagacgtac acggtcgact 1380
   cggccgtcca gtcgtaggcg ttgcgtgcct tccaggggcc cgcgtaggcg atgccggcga 1440
   cctcgccgtc cacctcggcg acgagccagg gatagcgctc ccgcagacgg acgaggtcgt 1500
   ccgtccactc ctgcggttcc tgcggctcgg tacggaagtt gaccgtgctt gtctcgatgt 1560
   agtggttgac gatggtgcag accgccggca tgtccgcctc ggtggcacgg cggatgtcgg 1620
   ccgggcgtcg ttctgggctc atggtagatc ccctcgagag agatagattt gtagagagag 1680
   actggtgatt tcagcgtgtc ctctccaaat gaaatgaact tccttatata gaggaaggtc 1740
   ttgcgaagga tagtgggatt gtgcgtcatc ccttacgtca gtggagatat cacatcaatc 1800
   cacttgcttt gaagacgtgg ttggaacgtc ttctttttcc acgatgctcc tcgtgggtgg 1860
   gggtccatct ttgggaccac tgtcggcaga ggcatcttga acgatagcct ttcctttatc 1920
   gcaatgatgg catttgtagg tgccaccttc cttttctact gtccttttga tgaagtgaca 1980
   gatagctggg caatggaatc cgaggaggtt tcccgatatt accctttgtt gaaaagtctc 2040
   aatagccctt tggtcttctg agactgtatc tttgatattc ttggagtaga cgagagtgtc 2100
   gtgctccacc atgttatcac atcaatccac ttgctttgaa gacgtggttg gaacgtcttc 2160
   tttttccacg atgctcctcg tgggtggggg tccatctttg ggaccactgt cggcagaggc 2220
   atcttgaacg atagcctttc ctttatcgca atgatggcat ttgtaggtgc caccttcctt 2280
   ttctactgtc cttttgatga agtgacagat agctgggcaa tggaatccga ggaggtttcc 2340
   cgatattacc ctttgttgaa aagtctcaat agccctttgg tcttctgaga ctgtatcttt 2400
   gatattcttg gagtagacga gagtgtcgtg ctccaccatg ttgctgatag tgaccttagg 2460
   cgacttttga acgcgcaata atggtttctg acgtatgtgc ttagctcatt aaactccaga 2520
   aacccattaa cgcttacaat ttccattcgc cattcaggct gcgcaactgt tgggaagggc 2580
   gatcggtgcg ggcctcttcg ctattacgcc agctggcgaa agggggatgt gctgcaaggc 2640
   gattaagttg ggtaacgcca gggttttccc agtcacgacg ttgtaaaacg acggccagtg 2700
   aattgtaata cgactcacta tagggcgaat tgggcccgac gtcgcatgct cccggccgcc 2760
   atggccgcgg gatatcacta gtgcggccgc tcgacgaatt aattccaatc ccacaaaaat 2820
   ctgagcttaa cagcacagtt gctcctctca gagcagaatc gggtattcaa caccctcata 2880
   tcaactacta cgttgtgtat aacggtccac atgccggtat atacgatgac tggggttgta 2940
   caaaggcggc aacaaacggc gttcccggag ttgcacacaa gaaatttgcc actattacag 3000
   aggcaagagc agcagctgac gcgtacacaa caagtcagca aacagacagg ttgaacttca 3060
   tccccaaagg agaagctcaa ctcaagccca agagctttgc taaggcccta acaagcccac 3120
   caaagcaaaa agcccactgg ctcacgctag gaaccaaaag gcccagcagt gatccagccc 3180
   caaaagagat ctcctttgcc ccggagatta caatggacga tttcctctat ctttacgatc 3240
   taggaaggaa gttcgaaggt gaaggtgacg acactatgtt caccactgat aatgagaagg 3300
   ttagcctctt caatttcaga aagaatgctg acccacagat ggttagagag gcctacgcag 3360
   caggtctcat caagacgatc tacccgagta acaatctcca ggagatcaaa taccttccca 3420
   agaaggttaa agatgcagtc aaaagattca ggactaattg catcaagaac acagagaaag 3480
   acatatttct caagatcaga agtactattc cagtatggac gattcaaggc ttgcttcata 3540
   aaccaaggca agtaatagag attggagtct ctaaaaaggt agttcctact gaatctaagg 3600
   ccatgcatgg agtctaagat tcaaatcgag gatctaacag aactcgccgt gaagactggc 3660
   gaacagttca tacagagtct tttacgactc aatgacaaga agaaaatctt cgtcaacatg 3720
   gtggagcacg acactctggt ctactccaaa aatgtcaaag atacagtctc agaagaccaa 3780
   agggctattg agacttttca acaaaggata atttcgggaa acctcctcgg attccattgc 3840
   ccagctatct gtcacttcat cgaaaggaca gtagaaaagg aaggtggctc ctacaaatgc 3900
   catcattgcg ataaaggaaa ggctatcatt caagatctct ctgccgacag tggtcccaaa 3960
   gatggacccc cacccacgag gagcatcgtg gaaaaagaag acgttccaac cacgtcttca 4020
   aagcaagtgg attgatgtga catctccact gacgtaaggg atgacgcaca atcccactat 4080
   ccttcgcaag acccttcctc tatataagga agttcatttc atttggagag gacacgctcg 4140
   aggaattcgg taccccatca caagtttgta caaaaaagca ggctgcggcc gcttgctccc 4200
   ttaaaaaaaa ccatggcatg tcattacgga caacagcaac agacttgtgc acctcatctt 4260
   cagcttcaac ctagagcatg tagagttgtg aaggctgcta ctgctgttac tgtaaatttc 4320
   tgtgttcctt attctctcaa aatcttcgat tttgttttcg ttcgatccca atttcgtata 4380
   tgttctttgg tttagattct gttaatctta gatcgaagac gattttctgg gtttgatcgt 4440
   tagatatcat cttaattctc gattagggtt tcatagatat catccgattt gttcaaataa 4500
   tttgagtttt gtcgaataat tactcttcga tttgtgattt ctatctagat ctggtgttag 4560
   tttctagttt gtgcgatcga atttgtcgat taatctgagt ttttctgatt aacaggctgg     4620
   aggatctctt cttgttctct ctggacttac tctcgctgga actgttatcg ctctcactat     4680
   cgctacacct cttctcgtta tcttctctcc tgttctcgtt cctgctgtga tcactatctt     4740
   ccttctcgga gctggatttc ttgcttctgg tggatttgga gttgctgctc tctctgttct     4800
   ctcttggatc tacagatacc tcactggaaa acatcctcca ggtgctgatc aacttgagtc     4860
   tgctaagact aagctcgctt ctaaggctag agagatgaag gatagagcag agcaattctc     4920
   ttgtcagcct gttgctggat ctcagacttc ttaatgaaca tatggtcctg ctttaatgag     4980
   atatgcgaga cgcctatgat cgcatgatat ttgctttcaa ttctgttgtg cacgttgtaa     5040
   aaaacctgag catgtgtagc tcagatcctt accgccggtt tcggttcatt ctaatgaata     5100
   tatcacccgt tactatcgta tttttatgaa taatattctc cgttcaattt actgattgta     5160
   ccctactact tatatgtaca atattaaaat gaaaacaata tattgtgctg aataggttta     5220
   tagcgacatc tatgatagag cgccacaata acaaacaatt gcgttttatt attacaaatc     5280
   caattttaaa aaaagcggca gaaccggtca aacctaaaag actgattaca taaatcttat     5340
   tcaaatttca aaaggcccca ggggctagta tctacgacac accgagcggc gaactaataa     5400
   cgttcactga agggaactcc ggttccccgc cggcgcgcat gggtgagatt ccttgaagtt     5460
   gagtattggc cgtccgctct accgaaagtt acgggcacca ttcaacccgg tccagcacgg     5520
   cggccgggta accgacttgc tgccccgaga attatgcagc atttttttgg tgtatgtggg     5580
   ccccaaatga agtgcaggtc aaaccttgac agtgacgaca aatcgttggg cgggtccagg     5640
   gcgaattttg cgacaacatg tcgaggctca gcaggacctg caggcatgca agctagctta     5700
   ctagtgatgc atattctata gtgtcaccta aatcttcgac gaattaattc caatcccaca     5760
   aaaatctgag cttaacagca cagttgctcc tctcagagca gaatcgggta ttcaacaccc     5820
   tcatatcaac tactacgttg tgtataacgg tccacatgcc ggtatatacg atgactgggg     5880
   ttgtacaaag gcggcaacaa acggcgttcc cggagttgca cacaagaaat ttgccactat     5940
   tacagaggca agagcagcag ctgacgcgta cacaacaagt cagcaaacag acaggttgaa     6000
   cttcatcccc aaaggagaag ctcaactcaa gcccaagagc tttgctaagg ccctaacaag     6060
   cccaccaaag caaaaagccc actggctcac gctaggaacc aaaaggccca gcagtgatcc     6120
   agccccaaaa gagatctcct ttgccccgga gattacaatg gacgatttcc tctatcttta     6180
   cgatctagga aggaagttcg aaggtgaagg tgacgacact atgttcacca ctgataatga     6240
   gaaggttagc ctcttcaatt tcagaaagaa tgctgaccca cagatggtta gagaggccta     6300
   cgcagcaggt ctcatcaaga cgatctaccc gagtaacaat ctccaggaga tcaaatacct     6360
   tcccaagaag gttaaagatg cagtcaaaag attcaggact aattgcatca agaacacaga     6420
   gaaagacata tttctcaaga tcagaagtac tattccagta tggacgattc aaggcttgct     6480
   tcataaacca aggcaagtaa tagagattgg agtctctaaa aaggtagttc ctactgaatc     6540
   taaggccatg catggagtct aagattcaaa tcgaggatct aacagaactc gccgtgaaga     6600
   ctggcgaaca gttcatacag agtcttttac gactcaatga caagaagaaa atcttcgtca 6660
   acatggtgga gcacgacact ctggtctact ccaaaaatgt caaagataca gtctcagaag 6720
   accaaagggc tattgagact tttcaacaaa ggataatttc gggaaacctc ctcggattcc 6780
   attgcccagc tatctgtcac ttcatcgaaa ggacagtaga aaaggaaggt ggctcctaca 6840
   aatgccatca ttgcgataaa ggaaaggcta tcattcaaga tctctctgcc gacagtggtc 6900
   ccaaagatgg acccccaccc acgaggagca tcgtggaaaa agaagacgtt ccaaccacgt 6960
   cttcaaagca agtggattga tgtgacatct ccactgacgt aagggatgac gcacaatccc 7020
   actatccttc gcaagaccct tcctctatat aaggaagttc atttcatttg gagaggacac 7080
   gggatccttg ctccgttaaa aaaaaccatg gctatcctcg attctgctgg tgttactact 7140
   gtgactgaga atggtggtgg agagttcgtt gatctcgata gactcagaag aagaaagtct 7200
   agatctgtaa atttctgtgt tccttattct ctcaaaatct tcgattttgt tttcgttcga 7260
   tcccaatttc gtatatgttc tttggtttag attctgttaa tcttagatcg aagacgattt 7320
   tctgggtttg atcgttagat atcatcttaa ttctcgatta gggtttcata gatatcatcc 7380
   gatttgttca aataatttga gttttgtcga ataattactc ttcgatttgt gatttctatc 7440
   tagatctggt gttagtttct agtttgtgcg atcgaatttg tcgattaatc tgagtttttc 7500
   tgattaacag gattcttcta acggacttct cctctctgga tctgataaca actctccttc 7560
   tgatgatgtt ggtgctcctg ctgatgtgag agatagaatc gattctgtgg tgaacgatga 7620
   tgctcaaggt actgctaacc tcgctggtga taataacggt ggaggtgata acaatggtgg 7680
   aggaagaggt ggaggtgaag gtagaggaaa cgctgatgct actttcactt acagaccatc 7740
   tgtgcctgct catagaagag ctagagagtc tcctctctct tctgatgcta tcttcaagca 7800
   gtctcacgct ggacttttca acctctgtgt ggtggttctt atcgctgtga actctagact 7860
   catcatcgag aacctcatga agtacggatg gctcatcaga actgatttct ggttctcttc 7920
   tagatctctc agagattggc ctcttttcat gtgctgcatc tcactctcaa tcttccctct 7980
   cgctgctttt actgttgaga agctcgtgct ccagaagtat atcgctgaac ctgtggtgat 8040
   cttcctccac atcatcatca ctatgactga ggttctctac cctgtttacg tgactctcag 8100
   atgcgattct gctttcctct ctggtgttac tcttatgctc ctcacttgca ttgtgtggct 8160
   taagctcgtg tcttacgctc acacttctta cgatatcaga tctctcgcta acgctgctga 8220
   taaggctaac cctgaagtgt cttactacgt gtctctcaag tctctcgctt acttcatggt 8280
   tgctcctaca ctttgttacc agccatctta ccctagatct gcttgcatta gaaagggatg 8340
   ggtggcaaga caattcgcta agttggtgat cttcactgga ttcatgggat tcatcatcga 8400
   gcagtacatc aaccctattg tgagaaactc taagcaccct ctcaagggtg atcttctcta 8460
   cgctatcgag agagttctta agctctctgt gcctaacctt tatgtgtggc tctgcatgtt 8520
   ctactgtttc ttccacctct ggcttaacat ccttgctgag ttgctttgct tcggagatag 8580
   agagttctac aaggattggt ggaacgctaa gtctgttgga gattattgga gaatgtggaa 8640
   catgcctgtg cataagtgga tggtgcgtca catctacttc ccttgcctca gatctaagat 8700
   ccctaagact ctcgctatca ttatcgcttt cctcgtgtct gctgttttcc atgagttgtg 8760
   tatcgctgtt ccttgcagac ttttcaagct ttgggctttc ctcggaatca tgttccaggt 8820
   tccactcgtg ttcatcacta actacctcca agagagattc ggatctactg ttggaaacat 8880
   gattttctgg ttcattttct gcatcttcgg acagcctatg tgcgttctcc tctactacca 8940
   cgatctcatg aacagaaagg gatctatgtc ttaatgaagg atccacccag ctttcttgta 9000
   caaagtggtg atgggttcga aatcgataag cttggatcct ctagagtcct gctttaatga 9060
   gatatgcgag acgcctatga tcgcatgata tttgctttca attctgttgt gcacgttgta 9120
   aaaaacctga gcatgtgtag ctcagatcct taccgccggt ttcggttcat tctaatgaat 9180
   atatcacccg ttactatcgt atttttatga ataatattct ccgttcaatt tactgattgt 9240
   accctactac ttatatgtac aatattaaaa tgaaaacaat atattgtgct gaataggttt 9300
   atagcgacat ctatgataga gcgccacaat aacaaacaat tgcgttttat tattacaaat 9360
   ccaattttaa aaaaagcggc agaaccggtc aaacctaaaa gactgattac ataaatctta 9420
   ttcaaatttc aaaaggcccc aggggctagt atctacgaca caccgagcgg cgaactaata 9480
   acgttcactg aagggaactc cggttccccg ccggcgcgca tgggtgagat tccttgaagt 9540
   tgagtattgg ccgtccgctc taccgaaagt tacgggcacc attcaacccg gtccagcacg 9600
   gcggccgggt aaccgacttg ctgccccgag aattatgcag catttttttg gtgtatgtgg 9660
   gccccaaatg aagtgcaggt caaaccttga cagtgacgac aaatcgttgg gcgggtccag 9720
   ggcgaatttt gcgacaacat gtcgaggctc agcaggacct gcaggcatgc aagctagctt 9780
   actagtgatg catattctat agtgtcacct aaatctgcgg ccgcctgcag gtcgatatgg 9840
   gagagctccc aacgcgttgg atgcatagct tgagtattct atagtgtcac ctaaatagct 9900
   tggcgtaatc atggtcatag ctgtttcctg tgtgaaattg ttatccgctc acaattccac 9960
   acaacatacg agccggaagc ataaagtgta aagcctgggg tgcctaatga gtgagctaac 10020
   tcacattaat tgcgttgcgc tcactgcccg ctttccagtc gggaaacctg tcgtgccagc 10080
   tgcattaatg aatcggccaa cgcgcgggga gaggcggttt gcgtattggg gctgagtggc 10140
   tccttcaacg ttgcggttct gtcagttcca aacgtaaaac ggcttgtccc gcgtcatcgg 10200
   cgggggtcat aacgtgactc ccttaattct ccgctcatga tcagattgtc gtttcccgcc 10260
   ttcagtttaa actatcagtg tttgacagga tatattggcg ggtaaaccta agagaaaaga 10320
   gcgtttatta gaataatcgg atatttaaaa gggcgtgaaa aggtttatcc gttcgtccat 10380
   ttgtatgtgc atgccaacca cagggttccc ctcgggagtg cttggcattc cgtgcgataa 10440
   tgacttctgt tcaaccaccc aaacgtcgga aagcctgacg acggagcagc attccaaaaa 10500
   gatcccttgg ctcgtctggg tcggctagaa ggtcgagtgg gctgctgtgg cttgatccct 10560
   caacgcggtc gcggacgtag cgcagcgccg aaaaatcctc gatcgcaaat ccgacgctgt 10620
   cgaaaatcgt gatctgcttg tcgctctttc ggccgacgtc ctggccagtc atcacgcgcc 10680
   aaagttccgt cacaggatga tctggcgcga gttgctggat ctcgccttca atccgggtct 10740
   gtggcgggaa ctccacgaaa atatccgaac gcagcaagat gtcgacggat cttttccgct 10800
   gcataaccct gcttcggggt cattatagcg attttttcgg tatatccatc ctttttcgca 10860
   cgatatacag gattttgcca aagggttcgt gtagactttc cttggtgtat ccaacggcgt 10920
   cagccgggca ggataggtga agtaggccca cccgcgagcg ggtgttcctt cttcactgtc 10980
   ccttattcgc acctggcggt gctcaacggg aatcctgctc tgcgaggctg gccggctacc 11040
   gccggcgtaa cagatgaggg caagcggatg gctgatgaaa ccaagccaac caggggtgat 11100
   gctgccaact tactgattta gtgtatgatg gtgtttttga ggtgctccag tggcttctgt 11160
   ttctatcagc tgtccctcct gttcagctac tgacggggtg gtgcgtaacg gcaaaagcac 11220
   cgccggacat cagcgctatc tctgctctca ctgccgtaaa acatggcaac tgcagttcac 11280
   ttacaccgct tctcaacccg gtacgcacca gaaaatcatt gatatggcca tgaatggcgt 11340
   tggatgccgg gcaacagccc gcattatggg cgttggcctc aacacgattt tacgtcactt 11400
   aaaaaactca ggccgcagtc ggtaacctcg cgcatacagc cgggcagtga cgtcatcgtc 11460
   tgcgcggaaa tggacgaaca gtggggctat gtcggggcta aatcgcgcca gcgctggctg 11520
   ttttacgcgt atgacagtct ccggaagacg gttgttgcgc acgtattcgg tgaacgcact 11580
   atggcgacgc tggggcgtct tatgagcctg ctgtcaccct ttgacgtggt gatatggatg 11640
   acggatggct ggccgctgta tgaatcccgc ctgaagggaa agctgcacgt aatcagcaag 11700
   cgatatacgc agcgaattga gcggcataac ctgaatctga ggcagcacct ggcacggctg 11760
   ggacggaagt cgctgtcgtt ctcaaaatcg gtggagctgc atgacaaagt catcgggcat 11820
   tatctgaaca taaaacacta tcaataagtt ggagtcatta cccaaccagg aagggcagcc 11880
   cacctatcaa ggtgtactgc cttccagacg aacgaagagc gattgaggaa aaggcggcgg 11940
   cggccggcat gagcctgtcg gcctacctgc tggccgtcgg ccagggctac aaaatcacgg 12000
   gcgtcgtgga ctatgagcac gtccgcgagc tggcccgcat caatggcgac ctgggccgcc 12060
   tgggcggcct gctgaaactc tggctcaccg acgacccgcg cacggcgcgg ttcggtgatg 12120
   ccacgatcct cgccctgctg gcgaagatcg aagagaagca ggacgagctt ggcaaggtca 12180
   tgatgggcgt ggtccgcccg agggcagagc catgactttt ttagccgcta aaacggccgg 12240
   ggggtgcgcg tgattgccaa gcacgtcccc atgcgctcca tcaagaagag cgacttcgcg 12300
   gagctggtat tcgtgcaggg caagattcgg aataccaagt acgagaagga cggccagacg 12360
   gtctacggga ccgacttcat tgccgataag gtggattatc tggacaccaa ggcaccaggc 12420
   gggtcaaatc aggaataagg gcacattgcc ccggcgtgag tcggggcaat cccgcaagga 12480
   gggtgaatga atcggacgtt tgaccggaag gcatacaggc aagaactgat cgacgcgggg 12540
   ttttccgccg aggatgccga aaccatcgca agccgcaccg tcatgcgtgc gccccgcgaa 12600
   accttccagt ccgtcggctc gatggtccag caagctacgg ccaagatcga gcgcgacagc 12660
   gtgcaactgg ctccccctgc cctgcccgcg ccatcggccg ccgtggagcg ttcgcgtcgt 12720
   ctcgaacagg aggcggcagg tttggcgaag tcgatgacca tcgacacgcg aggaactatg 12780
   acgaccaaga agcgaaaaac cgccggcgag gacctggcaa aacaggtcag cgaggccaag 12840
   caggccgcgt tgctgaaaca cacgaagcag cagatcaagg aaatgcagct ttccttgttc 12900
   gatattgcgc cgtggccgga cacgatgcga gcgatgccaa acgacacggc ccgctctgcc 12960
   ctgttcacca cgcgcaacaa gaaaatcccg cgcgaggcgc tgcaaaacaa ggtcattttc 13020
   cacgtcaaca aggacgtgaa gatcacctac accggcgtcg agctgcgggc cgacgatgac 13080
   gaactggtgt ggcagcaggt gttggagtac gcgaagcgca cccctatcgg cgagccgatc 13140
   accttcacgt tctacgagct ttgccaggac ctgggctggt cgatcaatgg ccggtattac 13200
   acgaaggccg aggaatgcct gtcgcgccta caggcgacgg cgatgggctt cacgtccgac 13260
   cgcgttgggc acctggaatc ggtgtcgctg ctgcaccgct tccgcgtcct ggaccgtggc 13320
   aagaaaacgt cccgttgcca ggtcctgatc gacgaggaaa tcgtcgtgct gtttgctggc 13380
   gaccactaca cgaaattcat atgggagaag taccgcaagc tgtcgccgac ggcccgacgg 13440
   atgttcgact atttcagctc gcaccgggag ccgtacccgc tcaagctgga aaccttccgc 13500
   ctcatgtgcg gatcggattc cacccgcgtg aagaagtggc gcgagcaggt cggcgaagcc 13560
   tgcgaagagt tgcgaggcag cggcctggtg gaacacgcct gggtcaatga tgacctggtg 13620
   cattgcaaac gctagggcct tgtggggtca gttccggctg ggggttcagc agccagcgct 13680
   ttactggcat ttcaggaaca agcgggcact gctcgacgca cttgcttcgc tcagtatcgc 13740
   tcgggacgca cggcgcgctc tacgaactgc cgataaacag aggattaaaa ttgacaattg 13800
   tgattaaggc tcagattcga cggcttggag cggccgacgt gcaggatttc cgcgagatcc 13860
   gattgtcggc cctgaagaaa gctccagaga tgttcgggtc cgtttacgag cacgaggaga 13920
   aaaagcccat ggaggcgttc gctgaacggt tgcgagatgc cgtggcattc ggcgcctaca 13980
   tcgacggcga gatcattggg ctgtcggtct tcaaacagga ggacggcccc aaggacgctc 14040
   acaaggcgca tctgtccggc gttttcgtgg agcccgaaca gcgaggccga ggggtcgccg 14100
   gtatgctgct gcgggcgttg ccggcgggtt tattgctcgt gatgatcgtc cgacagattc 14160
   caacgggaat ctggtggatg cgcatcttca tcctcggcgc acttaatatt tcgctattct 14220
   ggagcttgtt gtttatttcg gtctaccgcc tgccgggcgg ggtcgcggcg acggtaggcg 14280
   ctgtgcagcc gctgatggtc gtgttcatct ctgccgctct gctaggtagc ccgatacgat 14340
   tgatggcggt cctgggggct atttgcggaa ctgcgggcgt ggcgctgttg gtgttgacac 14400
   caaacgcagc gctagatcct gtcggcgtcg cagcgggcct ggcgggggcg gtttccatgg 14460
   cgttcggaac cgtgctgacc cgcaagtggc aacctcccgt gcctctgctc acctttaccg 14520
   cctggcaact ggcggccgga ggacttctgc tcgttccagt agctttagtg tttgatccgc 14580
   caatcccgat gcctacagga accaatgttc tcggcctggc gtggctcggc ctgatcggag 14640
   cgggtttaac ctacttcctt tggttccggg ggatctcgcg actcgaacct acagttgttt 14700
   ccttactggg ctttctcagc cgggatggcg ctaagaagct attgccgccg atcttcatat 14760
   gcggtgtgaa ataccgcaca gatgcgtaag gagaaaatac cgcatcaggc gctcttccgc 14820
   ttcctcgctc actgactcgc tgcgctcggt cgttcggctg cggcgagcgg tatcagctca 14880
   ctcaaaggcg gtaatacggt tatccacaga atcaggggat aacgcaggaa agaacatgtg 14940
   agcaaaaggc cagcaaaagg ccaggaaccg taaaaaggcc gcgttgctgg cgtttttcca 15000
   taggctccgc ccccctgacg agcatcacaa aaatcgacgc tcaagtcaga ggtggcgaaa 15060
   cccgacagga ctataaagat accaggcgtt tccccctgga agctccctcg tgcgctctcc 15120
   tgttccgacc ctgccgctta ccggatacct gtccgccttt ctcccttcgg gaagcgtggc 15180
   gctttctcaa tgctcacgct gtaggtatct cagttcggtg taggtcgttc gctccaagct 15240
   gggctgtgtg cacgaacccc ccgttcagcc cgaccgctgc gccttatccg gtaactatcg 15300
   tcttgagtcc aacccggtaa gacacgactt atcgccactg gcagcagcca ctggtaacag 15360
   gattagcaga gcgaggtatg taggcggtgc tacagagttc ttgaagtggt ggcctaacta 15420
   cggctacact agaaggacag tatttggtat ctgcgctctg ctgaagccag ttaccttcgg 15480
   aaaaagagtt ggtagctctt gatccggcaa acaaaccacc gctggtagcg gtggtttttt 15540
   tgtttgcaag cagcagatta cgcgcagaaa aaaaggatat caagaagatc ctttgatctt 15600
   ttctacgggg tctgacgctc agtggaacga aaactcacgt taagggattt tggtcatgag 15660
   attatcaaaa aggatcttca cctagatcct tttaaattaa aaatgaagtt ttaaatcaat 15720
   ctaaagtata tatgagtaaa cttggtctga cagttaccaa tgcttaatca gtgaggcacc 15780
   tatctcagcg atctgtctat ttcgttcatc catagttgcc tgactccccg tcgtgtagat 15840
   aactacgata cgggagggct taccatctgg ccccagtgct gcaatgatac cgcgagaccc 15900
   acgctcaccg gctccagatt tatcagcaat aaaccagcca gccggaaggg ccgagcgcag 15960
   aagtggtcct gcaactttat ccgcctccat ccagtctatt aaacaagtgg cagcaacgga 16020
   ttcgcaaacc tgtcacgcct tttgtgccaa aagccgcgcc aggtttgcga tccgctgtgc 16080
   caggcgttag gcgtcatatg aagatttcgg tgatccctga gcaggtggcg gaaacattgg 16140
   atgctgagaa ccatttcatt gttcgtgaag tgttcgatgt gcacctatcc gaccaaggct 16200
   ttgaactatc taccagaagt gtgagcccct accggaagga ttacatctcg gatgatgact 16260
   ctgatgaaga ctctgcttgc tatggcgcat tcatcgacca agagcttgtc gggaagattg 16320
   aactcaactc aacatggaac gatctagcct ctatcgaaca cattgttgtg tcgcacacgc 16380
   accgaggcaa aggagtcgcg cacagtctca tcgaatttgc gaaaaagtgg gcactaagca 16440
   gacagctcct tggcatacga ttagagacac aaacgaacaa tgtacctgcc tgcaatttgt 16500
   acgcaaaatg tggctttact ctcggcggca ttgacctgtt cacgtataaa actagacctc 16560
   aagtctcgaa cgaaacagcg atgtactggt actggttctc gggagcacag gatgacgcct 16620
   aacaattcat tcaagccgac accgcttcgc ggcgcggctt aattcaggag ttaaacatca 16680
   tgagggaagc ggtgatcgcc gaagtatcga ctcaactatc agaggtagtt ggcgtcatcg 16740
   agcgccatct cgaaccgacg ttgctggccg tacatttgta cggctccgca gtggatggcg 16800
   gcctgaagcc acacagtgat attgatttgc tggttacggt gaccgtaagg cttgatgaaa 16860
   caacgcggcg agctttgatc aacgaccttt tggaaacttc ggcttcccct ggagagagcg 16920
   agattctccg cgctgtagaa gtcaccattg ttgtgcacga cgacatcatt ccgtggcgtt 16980
   atccagctaa gcgcgaactg caatttggag aatggcagcg caatgacatt cttgcaggta 17040
   tcttcgagcc agccacgatc gacattgatc tggctatctt gctgacaaaa gcaagagaac 17100
   atagcgttgc cttggtaggt ccagcggcgg aggaactctt tgatccggtt cctgaacagg 17160
   atctatttga ggcgctaaat gaaaccttaa cgctatggaa ctcgccgccc gactgggctg 17220
   gcgatgagcg aaatgtagtg cttacgttgt cccgcatttg gtacagcgca gtaaccggca 17280
   aaatcgcgcc gaaggatgtc gctgccgact gggcaatgga gcgcctgccg gcccagtatc 17340
   agcccgtcat acttgaagct aggcaggctt atcttggaca agaagatcgc ttggcctcgc 17400
   gcgcagatca gttggaagaa tttgttcact acgtgaaagg cgagatcacc aaggtagtcg 17460
   gcaaataatg tctaacaatt cgttcaagcc gacgccgctt cgcggcgcgg cttaactcaa 17520
   gcgttagaga gctggggaag actatgcgcg atctgttgaa ggtggttcta agcctcgtac 17580
   ttgcgatggc atcggggcag gcacttgctg acctgccaat tgttttagtg gatgaagctc 17640
   gtcttcccta tgactactcc ccatccaact acgacatttc tccaagcaac tacgacaact 17700
   ccataagcaa ttacgacaat agtccatcaa attacgacaa ctctgagagc aactacgata 17760
   atagttcatc caattacgac aatagtcgca acggaaatcg taggcttata tatagcgcaa 17820
   atgggtctcg cactttcgcc ggctactacg tcattgccaa caatgggaca acgaacttct 17880
   tttccacatc tggcaaaagg atgttctaca ccccaaaagg ggggcgcggc gtctatggcg 17940
   gcaaagatgg gagcttctgc ggggcattgg tcgtcataaa tggccaattt tcgcttgccc 18000
   tgacagataa cggcctgaag atcatgtatc taagcaacta gcctgctctc taataaaatg 18060
   ttaggagctt ggctgccatt tttggggtga ggccgttcgc ggccgagggg cgcagcccct 18120
   ggggggatgg gaggcccgcg ttagcgggcc gggagggttc gagaaggggg ggcacccccc 18180
   ttcggcgtgc gcggtcacgc gccagggcgc agccctggtt aaaaacaagg tttataaata 18240
   ttggtttaaa agcaggttaa aagacaggtt agcggtggcc gaaaaacggg cggaaaccct 18300
   tgcaaatgct ggattttctg cctgtggaca gcccctcaaa tgtcaatagg tgcgcccctc 18360
   atctgtcagc actctgcccc tcaagtgtca aggatcgcgc ccctcatctg tcagtagtcg 18420
   cgcccctcaa gtgtcaatac cgcagggcac ttatccccag gcttgtccac atcatctgtg 18480
   ggaaactcgc gtaaaatcag gcgttttcgc cgatttgcga ggctggccag ctccacgtcg 18540
   ccggccgaaa tcgagcctgc ccctcatctg tcaacgccgc gccgggtgag tcggcccctc 18600
   aagtgtcaac gtccgcccct catctgtcag tgagggccaa gttttccgcg aggtatccac 18660
   aacgccggcg gccggccgcg gtgtctcgca cacggcttcg acggcgtttc tggcgcgttt 18720
   gcagggccat agacggccgc cagcccagcg gcgagggcaa ccagcccggt gagcgtcgga 18780
   aagg 18784
<210> 30
   <211> 18784
   <212> DNA
   <213> Artificial Sequence
<220>
   <223> Artificial
<400> 30
   tcgacatcgt caacgttcac ttctaaagaa atagcgccac tcagcttcct cagcggcttt      60
   atccagcgat ttcctattat gtcggcatag ttctcaagat cgacagcctg tcacggttaa     120
   gcgagaaatg aataagaagg ctgataattc ggatctctgc gaggagatga tatttgatca     180
   caggcagcaa cgctctgtca tcgttacaat caacatgcta ccctccgcga gatcatccgt     240
   gtttcaaacc cggcagctta gttgccgttc ttccgaatag catcgggtaa catgagcaaa     300
   gtctgccgcc ttacaacggc tctcccgctg acgccgtccc ggactgatgg gctgcctgta     360
   tcgagtggtg attttgtgcc gagctgccgg tcggggagct gttggctggc tggtggcagg     420
   atatattgtg gtgtaaacaa attgacgctt agacaactta ataacacatt gcggacgttt     480
   ttaatgtact gaattcgccg ctcggtgtgt cgtagatact agcccctggg gcacttttga     540
   aatttgaata agatttatgt aatcagtctt ttaggtttga ccggttctgc cgcttttttt     600
   aaaattggat ttgtaataat aaaacgcaat tgtttgttat tgtggcgctc tatcatagat     660
   gtcgctataa acctattcag cacaatatat tgttttcatt ttaatattgt acatataagt     720
   agtagggtac aatcagtaaa ttgaacggag aatattattc ataaaaatac gatagtaacg     780
   ggtgatatat tcattagaat gaaccgaaac cggcggtaag gatctgagct acacatgctc     840
   aggtttttta caacgtgcac aacagaattg aaagcaaata tcatgcgatc ataggcgtct     900
   cgcatatctc attaaacaag tgaagatttg attcaaactc cattgagagc cctgactatg     960
   cattcggttt gacccttcca ggttgagaga cgatagcccc ctaccttaat taaggggccc    1020
   cccctcgagg tcgacggtat cgataagctt gatatcgaat tcctgcagcc agggggatcc    1080
   cccgggtcat cagatctcgg tgacgggcag gaccggacgg ggcggtaccg gcaggctgaa    1140
   gtccagctgc cagaaaccca cgtcatgcca gttcccgtgc ttgaagccgg ccgcccgcag    1200
   catgccgcgg ggggcatatc cgagcgcctc gtgcatgcgc acgctcgggt cgttgggcag    1260
   cccgatgaca gcgaccacgc tcttgaagcc ctgtgcctcc agggacttca gcaggtgggt    1320
   gtagagcgtg gagcccagtc ccgtccgctg gtggcggggg gagacgtaca cggtcgactc    1380
   ggccgtccag tcgtaggcgt tgcgtgcctt ccaggggccc gcgtaggcga tgccggcgac    1440
   ctcgccgtcc acctcggcga cgagccaggg atagcgctcc cgcagacgga cgaggtcgtc    1500
   cgtccactcc tgcggttcct gcggctcggt acggaagttg accgtgcttg tctcgatgta    1560
   gtggttgacg atggtgcaga ccgccggcat gtccgcctcg gtggcacggc ggatgtcggc    1620
   cgggcgtcgt tctgggctca tggtagatcc cctcgagaga gatagatttg tagagagaga    1680
   ctggtgattt cagcgtgtcc tctccaaatg aaatgaactt ccttatatag aggaaggtct    1740
   tgcgaaggat agtgggattg tgcgtcatcc cttacgtcag tggagatatc acatcaatcc 1800
   acttgctttg aagacgtggt tggaacgtct tctttttcca cgatgctcct cgtgggtggg 1860
   ggtccatctt tgggaccact gtcggcagag gcatcttgaa cgatagcctt tcctttatcg 1920
   caatgatggc atttgtaggt gccaccttcc ttttctactg tccttttgat gaagtgacag 1980
   atagctgggc aatggaatcc gaggaggttt cccgatatta ccctttgttg aaaagtctca 2040
   atagcccttt ggtcttctga gactgtatct ttgatattct tggagtagac gagagtgtcg 2100
   tgctccacca tgttatcaca tcaatccact tgctttgaag acgtggttgg aacgtcttct 2160
   ttttccacga tgctcctcgt gggtgggggt ccatctttgg gaccactgtc ggcagaggca 2220
   tcttgaacga tagcctttcc tttatcgcaa tgatggcatt tgtaggtgcc accttccttt 2280
   tctactgtcc ttttgatgaa gtgacagata gctgggcaat ggaatccgag gaggtttccc 2340
   gatattaccc tttgttgaaa agtctcaata gccctttggt cttctgagac tgtatctttg 2400
   atattcttgg agtagacgag agtgtcgtgc tccaccatgt tgctgatagt gaccttaggc 2460
   gacttttgaa cgcgcaataa tggtttctga cgtatgtgct tagctcatta aactccagaa 2520
   acccattaac gcttacaatt tccattcgcc attcaggctg cgcaactgtt gggaagggcg 2580
   atcggtgcgg gcctcttcgc tattacgcca gctggcgaaa gggggatgtg ctgcaaggcg 2640
   attaagttgg gtaacgccag ggttttccca gtcacgacgt tgtaaaacga cggccagtga 2700
   attgtaatac gactcactat agggcgaatt gggcccgacg tcgcatgctc ccggccgcca 2760
   tggccgcggg atatcactag tgcggccgct cgacgaatta attccaatcc cacaaaaatc 2820
   tgagcttaac agcacagttg ctcctctcag agcagaatcg ggtattcaac accctcatat 2880
   caactactac gttgtgtata acggtccaca tgccggtata tacgatgact ggggttgtac 2940
   aaaggcggca acaaacggcg ttcccggagt tgcacacaag aaatttgcca ctattacaga 3000
   ggcaagagca gcagctgacg cgtacacaac aagtcagcaa acagacaggt tgaacttcat 3060
   ccccaaagga gaagctcaac tcaagcccaa gagctttgct aaggccctaa caagcccacc 3120
   aaagcaaaaa gcccactggc tcacgctagg aaccaaaagg cccagcagtg atccagcccc 3180
   aaaagagatc tcctttgccc cggagattac aatggacgat ttcctctatc tttacgatct 3240
   aggaaggaag ttcgaaggtg aaggtgacga cactatgttc accactgata atgagaaggt 3300
   tagcctcttc aatttcagaa agaatgctga cccacagatg gttagagagg cctacgcagc 3360
   aggtctcatc aagacgatct acccgagtaa caatctccag gagatcaaat accttcccaa 3420
   gaaggttaaa gatgcagtca aaagattcag gactaattgc atcaagaaca cagagaaaga 3480
   catatttctc aagatcagaa gtactattcc agtatggacg attcaaggct tgcttcataa 3540
   accaaggcaa gtaatagaga ttggagtctc taaaaaggta gttcctactg aatctaaggc 3600
   catgcatgga gtctaagatt caaatcgagg atctaacaga actcgccgtg aagactggcg 3660
   aacagttcat acagagtctt ttacgactca atgacaagaa gaaaatcttc gtcaacatgg 3720
   tggagcacga cactctggtc tactccaaaa atgtcaaaga tacagtctca gaagaccaaa 3780
   gggctattga gacttttcaa caaaggataa tttcgggaaa cctcctcgga ttccattgcc 3840
   cagctatctg tcacttcatc gaaaggacag tagaaaagga aggtggctcc tacaaatgcc 3900
   atcattgcga taaaggaaag gctatcattc aagatctctc tgccgacagt ggtcccaaag 3960
   atggaccccc acccacgagg agcatcgtgg aaaaagaaga cgttccaacc acgtcttcaa 4020
   agcaagtgga ttgatgtgac atctccactg acgtaaggga tgacgcacaa tcccactatc 4080
   cttcgcaaga cccttcctct atataaggaa gttcatttca tttggagagg acacgctcga 4140
   ggaattcggt accccatcac aagtttgtac aaaaaagcag gctgcggccg cttgctccct 4200
   taaaaaaaac catggcatgt cattacggac aacagcaaca gacttgtgca cctcatcttc 4260
   agcttcaacc tagagcatgt agagttgtga aggctgctac tgctgttact gtaaatttct 4320
   gtgttcctta ttctctcaaa atcttcgatt ttgttttcgt tcgatcccaa tttcgtatat 4380
   gttctttggt ttagattctg ttaatcttag atcgaagacg attttctggg tttgatcgtt 4440
   agatatcatc ttaattctcg attagggttt catagatatc atccgatttg ttcaaataat 4500
   ttgagttttg tcgaataatt actcttcgat ttgtgatttc tatctagatc tggtgttagt 4560
   ttctagtttg tgcgatcgaa tttgtcgatt aatctgagtt tttctgatta acaggctgga 4620
   ggatctcttc ttgttctctc tggacttact ctcgctggaa ctgttatcgc tctcactatc 4680
   gctacacctc ttctcgttat cttctctcct gttctcgttc ctgctgtgat cactatcttc 4740
   cttctcggag ctggatttct tgcttctggt ggatttggag ttgctgctct ctctgttctc 4800
   tcttggatct acagatacct cactggaaaa catcctccag gtgctgattg tcttgagtct 4860
   gctaagacta agctcgcttc ttgtgctaga gagatgaagg atagagcaga gcaattctct 4920
   tgtcagcctg ttgctggatc tcagacttct taatgaacat atggtcctgc tttaatgaga 4980
   tatgcgagac gcctatgatc gcatgatatt tgctttcaat tctgttgtgc acgttgtaaa 5040
   aaacctgagc atgtgtagct cagatcctta ccgccggttt cggttcattc taatgaatat 5100
   atcacccgtt actatcgtat ttttatgaat aatattctcc gttcaattta ctgattgtac 5160
   cctactactt atatgtacaa tattaaaatg aaaacaatat attgtgctga ataggtttat 5220
   agcgacatct atgatagagc gccacaataa caaacaattg cgttttatta ttacaaatcc 5280
   aattttaaaa aaagcggcag aaccggtcaa acctaaaaga ctgattacat aaatcttatt 5340
   caaatttcaa aaggccccag gggctagtat ctacgacaca ccgagcggcg aactaataac 5400
   gttcactgaa gggaactccg gttccccgcc ggcgcgcatg ggtgagattc cttgaagttg 5460
   agtattggcc gtccgctcta ccgaaagtta cgggcaccat tcaacccggt ccagcacggc 5520
   ggccgggtaa ccgacttgct gccccgagaa ttatgcagca tttttttggt gtatgtgggc 5580
   cccaaatgaa gtgcaggtca aaccttgaca gtgacgacaa atcgttgggc gggtccaggg 5640
   cgaattttgc gacaacatgt cgaggctcag caggacctgc aggcatgcaa gctagcttac 5700
   tagtgatgca tattctatag tgtcacctaa atcttcgacg aattaattcc aatcccacaa 5760
   aaatctgagc ttaacagcac agttgctcct ctcagagcag aatcgggtat tcaacaccct 5820
   catatcaact actacgttgt gtataacggt ccacatgccg gtatatacga tgactggggt 5880
   tgtacaaagg cggcaacaaa cggcgttccc ggagttgcac acaagaaatt tgccactatt 5940
   acagaggcaa gagcagcagc tgacgcgtac acaacaagtc agcaaacaga caggttgaac 6000
   ttcatcccca aaggagaagc tcaactcaag cccaagagct ttgctaaggc cctaacaagc 6060
   ccaccaaagc aaaaagccca ctggctcacg ctaggaacca aaaggcccag cagtgatcca 6120
   gccccaaaag agatctcctt tgccccggag attacaatgg acgatttcct ctatctttac 6180
   gatctaggaa ggaagttcga aggtgaaggt gacgacacta tgttcaccac tgataatgag 6240
   aaggttagcc tcttcaattt cagaaagaat gctgacccac agatggttag agaggcctac 6300
   gcagcaggtc tcatcaagac gatctacccg agtaacaatc tccaggagat caaatacctt 6360
   cccaagaagg ttaaagatgc agtcaaaaga ttcaggacta attgcatcaa gaacacagag 6420
   aaagacatat ttctcaagat cagaagtact attccagtat ggacgattca aggcttgctt 6480
   cataaaccaa ggcaagtaat agagattgga gtctctaaaa aggtagttcc tactgaatct 6540
   aaggccatgc atggagtcta agattcaaat cgaggatcta acagaactcg ccgtgaagac 6600
   tggcgaacag ttcatacaga gtcttttacg actcaatgac aagaagaaaa tcttcgtcaa 6660
   catggtggag cacgacactc tggtctactc caaaaatgtc aaagatacag tctcagaaga 6720
   ccaaagggct attgagactt ttcaacaaag gataatttcg ggaaacctcc tcggattcca 6780
   ttgcccagct atctgtcact tcatcgaaag gacagtagaa aaggaaggtg gctcctacaa 6840
   atgccatcat tgcgataaag gaaaggctat cattcaagat ctctctgccg acagtggtcc 6900
   caaagatgga cccccaccca cgaggagcat cgtggaaaaa gaagacgttc caaccacgtc 6960
   ttcaaagcaa gtggattgat gtgacatctc cactgacgta agggatgacg cacaatccca 7020
   ctatccttcg caagaccctt cctctatata aggaagttca tttcatttgg agaggacacg 7080
   ggatccttgc tccgttaaaa aaaaccatgg ctatcctcga ttctgctggt gttactactg 7140
   tgactgagaa tggtggtgga gagttcgttg atctcgatag actcagaaga agaaagtcta 7200
   gatctgtaaa tttctgtgtt ccttattctc tcaaaatctt cgattttgtt ttcgttcgat 7260
   cccaatttcg tatatgttct ttggtttaga ttctgttaat cttagatcga agacgatttt 7320
   ctgggtttga tcgttagata tcatcttaat tctcgattag ggtttcatag atatcatccg 7380
   atttgttcaa ataatttgag ttttgtcgaa taattactct tcgatttgtg atttctatct 7440
   agatctggtg ttagtttcta gtttgtgcga tcgaatttgt cgattaatct gagtttttct 7500
   gattaacagg attcttctaa cggacttctc ctctctggat ctgataacaa ctctccttct 7560
   gatgatgttg gtgctcctgc tgatgtgaga gatagaatcg attctgtggt gaacgatgat 7620
   gctcaaggta ctgctaacct cgctggtgat aataacggtg gaggtgataa caatggtgga 7680
   ggaagaggtg gaggtgaagg tagaggaaac gctgatgcta ctttcactta cagaccatct 7740
   gtgcctgctc atagaagagc tagagagtct cctctctctt ctgatgctat cttcaagcag 7800
   tctcacgctg gacttttcaa cctctgtgtg gtggttctta tcgctgtgaa ctctagactc 7860
   atcatcgaga acctcatgaa gtacggatgg ctcatcagaa ctgatttctg gttctcttct 7920
   agatctctca gagattggcc tcttttcatg tgctgcatct cactctcaat cttccctctc 7980
   gctgctttta ctgttgagaa gctcgtgctc cagaagtata tcgctgaacc tgtggtgatc 8040
   ttcctccaca tcatcatcac tatgactgag gttctctacc ctgtttacgt gactctcaga 8100
   tgcgattctg ctttcctctc tggtgttact cttatgctcc tcacttgcat tgtgtggctt 8160
   aagctcgtgt cttacgctca cacttcttac gatatcagat ctctcgctaa cgctgctgat 8220
   aaggctaacc ctgaagtgtc ttactacgtg tctctcaagt ctctcgctta cttcatggtt 8280
   gctcctacac tttgttacca gccatcttac cctagatctg cttgcattag aaagggatgg 8340
   gtggcaagac aattcgctaa gttggtgatc ttcactggat tcatgggatt catcatcgag 8400
   cagtacatca accctattgt gagaaactct aagcaccctc tcaagggtga tcttctctac 8460
   gctatcgaga gagttcttaa gctctctgtg cctaaccttt atgtgtggct ctgcatgttc 8520
   tactgtttct tccacctctg gcttaacatc cttgctgagt tgctttgctt cggagataga 8580
   gagttctaca aggattggtg gaacgctaag tctgttggag attattggag aatgtggaac 8640
   atgcctgtgc ataagtggat ggtgcgtcac atctacttcc cttgcctcag atctaagatc 8700
   cctaagactc tcgctatcat tatcgctttc ctcgtgtctg ctgttttcca tgagttgtgt 8760
   atcgctgttc cttgcagact tttcaagctt tgggctttcc tcggaatcat gttccaggtt 8820
   ccactcgtgt tcatcactaa ctacctccaa gagagattcg gatctactgt tggaaacatg 8880
   attttctggt tcattttctg catcttcgga cagcctatgt gcgttctcct ctactaccac 8940
   gatctcatga acagaaaggg atctatgtct taatgaagga tccacccagc tttcttgtac 9000
   aaagtggtga tgggttcgaa atcgataagc ttggatcctc tagagtcctg ctttaatgag 9060
   atatgcgaga cgcctatgat cgcatgatat ttgctttcaa ttctgttgtg cacgttgtaa 9120
   aaaacctgag catgtgtagc tcagatcctt accgccggtt tcggttcatt ctaatgaata 9180
   tatcacccgt tactatcgta tttttatgaa taatattctc cgttcaattt actgattgta 9240
   ccctactact tatatgtaca atattaaaat gaaaacaata tattgtgctg aataggttta 9300
   tagcgacatc tatgatagag cgccacaata acaaacaatt gcgttttatt attacaaatc 9360
   caattttaaa aaaagcggca gaaccggtca aacctaaaag actgattaca taaatcttat 9420
   tcaaatttca aaaggcccca ggggctagta tctacgacac accgagcggc gaactaataa 9480
   cgttcactga agggaactcc ggttccccgc cggcgcgcat gggtgagatt ccttgaagtt 9540
   gagtattggc cgtccgctct accgaaagtt acgggcacca ttcaacccgg tccagcacgg 9600
   cggccgggta accgacttgc tgccccgaga attatgcagc atttttttgg tgtatgtggg 9660
   ccccaaatga agtgcaggtc aaaccttgac agtgacgaca aatcgttggg cgggtccagg 9720
   gcgaattttg cgacaacatg tcgaggctca gcaggacctg caggcatgca agctagctta 9780
   ctagtgatgc atattctata gtgtcaccta aatctgcggc cgcctgcagg tcgatatggg 9840
   agagctccca acgcgttgga tgcatagctt gagtattcta tagtgtcacc taaatagctt 9900
   ggcgtaatca tggtcatagc tgtttcctgt gtgaaattgt tatccgctca caattccaca 9960
   caacatacga gccggaagca taaagtgtaa agcctggggt gcctaatgag tgagctaact 10020
   cacattaatt gcgttgcgct cactgcccgc tttccagtcg ggaaacctgt cgtgccagct 10080
   gcattaatga atcggccaac gcgcggggag aggcggtttg cgtattgggg ctgagtggct 10140
   ccttcaacgt tgcggttctg tcagttccaa acgtaaaacg gcttgtcccg cgtcatcggc 10200
   gggggtcata acgtgactcc cttaattctc cgctcatgat cagattgtcg tttcccgcct 10260
   tcagtttaaa ctatcagtgt ttgacaggat atattggcgg gtaaacctaa gagaaaagag 10320
   cgtttattag aataatcgga tatttaaaag ggcgtgaaaa ggtttatccg ttcgtccatt 10380
   tgtatgtgca tgccaaccac agggttcccc tcgggagtgc ttggcattcc gtgcgataat 10440
   gacttctgtt caaccaccca aacgtcggaa agcctgacga cggagcagca ttccaaaaag 10500
   atcccttggc tcgtctgggt cggctagaag gtcgagtggg ctgctgtggc ttgatccctc 10560
   aacgcggtcg cggacgtagc gcagcgccga aaaatcctcg atcgcaaatc cgacgctgtc 10620
   gaaaatcgtg atctgcttgt cgctctttcg gccgacgtcc tggccagtca tcacgcgcca 10680
   aagttccgtc acaggatgat ctggcgcgag ttgctggatc tcgccttcaa tccgggtctg 10740
   tggcgggaac tccacgaaaa tatccgaacg cagcaagatg tcgacggatc ttttccgctg 10800
   cataaccctg cttcggggtc attatagcga ttttttcggt atatccatcc tttttcgcac 10860
   gatatacagg attttgccaa agggttcgtg tagactttcc ttggtgtatc caacggcgtc 10920
   agccgggcag gataggtgaa gtaggcccac ccgcgagcgg gtgttccttc ttcactgtcc 10980
   cttattcgca cctggcggtg ctcaacggga atcctgctct gcgaggctgg ccggctaccg 11040
   ccggcgtaac agatgagggc aagcggatgg ctgatgaaac caagccaacc aggggtgatg 11100
   ctgccaactt actgatttag tgtatgatgg tgtttttgag gtgctccagt ggcttctgtt 11160
   tctatcagct gtccctcctg ttcagctact gacggggtgg tgcgtaacgg caaaagcacc 11220
   gccggacatc agcgctatct ctgctctcac tgccgtaaaa catggcaact gcagttcact 11280
   tacaccgctt ctcaacccgg tacgcaccag aaaatcattg atatggccat gaatggcgtt 11340
   ggatgccggg caacagcccg cattatgggc gttggcctca acacgatttt acgtcactta 11400
   aaaaactcag gccgcagtcg gtaacctcgc gcatacagcc gggcagtgac gtcatcgtct 11460
   gcgcggaaat ggacgaacag tggggctatg tcggggctaa atcgcgccag cgctggctgt 11520
   tttacgcgta tgacagtctc cggaagacgg ttgttgcgca cgtattcggt gaacgcacta 11580
   tggcgacgct ggggcgtctt atgagcctgc tgtcaccctt tgacgtggtg atatggatga 11640
   cggatggctg gccgctgtat gaatcccgcc tgaagggaaa gctgcacgta atcagcaagc 11700
   gatatacgca gcgaattgag cggcataacc tgaatctgag gcagcacctg gcacggctgg 11760
   gacggaagtc gctgtcgttc tcaaaatcgg tggagctgca tgacaaagtc atcgggcatt 11820
   atctgaacat aaaacactat caataagttg gagtcattac ccaaccagga agggcagccc 11880
   acctatcaag gtgtactgcc ttccagacga acgaagagcg attgaggaaa aggcggcggc 11940
   ggccggcatg agcctgtcgg cctacctgct ggccgtcggc cagggctaca aaatcacggg 12000
   cgtcgtggac tatgagcacg tccgcgagct ggcccgcatc aatggcgacc tgggccgcct 12060
   gggcggcctg ctgaaactct ggctcaccga cgacccgcgc acggcgcggt tcggtgatgc 12120
   cacgatcctc gccctgctgg cgaagatcga agagaagcag gacgagcttg gcaaggtcat 12180
   gatgggcgtg gtccgcccga gggcagagcc atgacttttt tagccgctaa aacggccggg 12240
   gggtgcgcgt gattgccaag cacgtcccca tgcgctccat caagaagagc gacttcgcgg 12300
   agctggtatt cgtgcagggc aagattcgga ataccaagta cgagaaggac ggccagacgg 12360
   tctacgggac cgacttcatt gccgataagg tggattatct ggacaccaag gcaccaggcg 12420
   ggtcaaatca ggaataaggg cacattgccc cggcgtgagt cggggcaatc ccgcaaggag 12480
   ggtgaatgaa tcggacgttt gaccggaagg catacaggca agaactgatc gacgcggggt 12540
   tttccgccga ggatgccgaa accatcgcaa gccgcaccgt catgcgtgcg ccccgcgaaa 12600
   ccttccagtc cgtcggctcg atggtccagc aagctacggc caagatcgag cgcgacagcg 12660
   tgcaactggc tccccctgcc ctgcccgcgc catcggccgc cgtggagcgt tcgcgtcgtc 12720
   tcgaacagga ggcggcaggt ttggcgaagt cgatgaccat cgacacgcga ggaactatga 12780
   cgaccaagaa gcgaaaaacc gccggcgagg acctggcaaa acaggtcagc gaggccaagc 12840
   aggccgcgtt gctgaaacac acgaagcagc agatcaagga aatgcagctt tccttgttcg 12900
   atattgcgcc gtggccggac acgatgcgag cgatgccaaa cgacacggcc cgctctgccc 12960
   tgttcaccac gcgcaacaag aaaatcccgc gcgaggcgct gcaaaacaag gtcattttcc 13020
   acgtcaacaa ggacgtgaag atcacctaca ccggcgtcga gctgcgggcc gacgatgacg 13080
   aactggtgtg gcagcaggtg ttggagtacg cgaagcgcac ccctatcggc gagccgatca 13140
   ccttcacgtt ctacgagctt tgccaggacc tgggctggtc gatcaatggc cggtattaca 13200
   cgaaggccga ggaatgcctg tcgcgcctac aggcgacggc gatgggcttc acgtccgacc 13260
   gcgttgggca cctggaatcg gtgtcgctgc tgcaccgctt ccgcgtcctg gaccgtggca 13320
   agaaaacgtc ccgttgccag gtcctgatcg acgaggaaat cgtcgtgctg tttgctggcg 13380
   accactacac gaaattcata tgggagaagt accgcaagct gtcgccgacg gcccgacgga 13440
   tgttcgacta tttcagctcg caccgggagc cgtacccgct caagctggaa accttccgcc 13500
   tcatgtgcgg atcggattcc acccgcgtga agaagtggcg cgagcaggtc ggcgaagcct 13560
   gcgaagagtt gcgaggcagc ggcctggtgg aacacgcctg ggtcaatgat gacctggtgc 13620
   attgcaaacg ctagggcctt gtggggtcag ttccggctgg gggttcagca gccagcgctt 13680
   tactggcatt tcaggaacaa gcgggcactg ctcgacgcac ttgcttcgct cagtatcgct 13740
   cgggacgcac ggcgcgctct acgaactgcc gataaacaga ggattaaaat tgacaattgt 13800
   gattaaggct cagattcgac ggcttggagc ggccgacgtg caggatttcc gcgagatccg 13860
   attgtcggcc ctgaagaaag ctccagagat gttcgggtcc gtttacgagc acgaggagaa 13920
   aaagcccatg gaggcgttcg ctgaacggtt gcgagatgcc gtggcattcg gcgcctacat 13980
   cgacggcgag atcattgggc tgtcggtctt caaacaggag gacggcccca aggacgctca 14040
   caaggcgcat ctgtccggcg ttttcgtgga gcccgaacag cgaggccgag gggtcgccgg 14100
   tatgctgctg cgggcgttgc cggcgggttt attgctcgtg atgatcgtcc gacagattcc 14160
   aacgggaatc tggtggatgc gcatcttcat cctcggcgca cttaatattt cgctattctg 14220
   gagcttgttg tttatttcgg tctaccgcct gccgggcggg gtcgcggcga cggtaggcgc 14280
   tgtgcagccg ctgatggtcg tgttcatctc tgccgctctg ctaggtagcc cgatacgatt 14340
   gatggcggtc ctgggggcta tttgcggaac tgcgggcgtg gcgctgttgg tgttgacacc 14400
   aaacgcagcg ctagatcctg tcggcgtcgc agcgggcctg gcgggggcgg tttccatggc 14460
   gttcggaacc gtgctgaccc gcaagtggca acctcccgtg cctctgctca cctttaccgc 14520
   ctggcaactg gcggccggag gacttctgct cgttccagta gctttagtgt ttgatccgcc 14580
   aatcccgatg cctacaggaa ccaatgttct cggcctggcg tggctcggcc tgatcggagc 14640
   gggtttaacc tacttccttt ggttccgggg gatctcgcga ctcgaaccta cagttgtttc 14700
   cttactgggc tttctcagcc gggatggcgc taagaagcta ttgccgccga tcttcatatg 14760
   cggtgtgaaa taccgcacag atgcgtaagg agaaaatacc gcatcaggcg ctcttccgct 14820
   tcctcgctca ctgactcgct gcgctcggtc gttcggctgc ggcgagcggt atcagctcac 14880
   tcaaaggcgg taatacggtt atccacagaa tcaggggata acgcaggaaa gaacatgtga 14940
   gcaaaaggcc agcaaaaggc caggaaccgt aaaaaggccg cgttgctggc gtttttccat 15000
   aggctccgcc cccctgacga gcatcacaaa aatcgacgct caagtcagag gtggcgaaac 15060
   ccgacaggac tataaagata ccaggcgttt ccccctggaa gctccctcgt gcgctctcct 15120
   gttccgaccc tgccgcttac cggatacctg tccgcctttc tcccttcggg aagcgtggcg 15180
   ctttctcaat gctcacgctg taggtatctc agttcggtgt aggtcgttcg ctccaagctg 15240
   ggctgtgtgc acgaaccccc cgttcagccc gaccgctgcg ccttatccgg taactatcgt 15300
   cttgagtcca acccggtaag acacgactta tcgccactgg cagcagccac tggtaacagg 15360
   attagcagag cgaggtatgt aggcggtgct acagagttct tgaagtggtg gcctaactac 15420
   ggctacacta gaaggacagt atttggtatc tgcgctctgc tgaagccagt taccttcgga 15480
   aaaagagttg gtagctcttg atccggcaaa caaaccaccg ctggtagcgg tggttttttt 15540
   gtttgcaagc agcagattac gcgcagaaaa aaaggatatc aagaagatcc tttgatcttt 15600
   tctacggggt ctgacgctca gtggaacgaa aactcacgtt aagggatttt ggtcatgaga 15660
   ttatcaaaaa ggatcttcac ctagatcctt ttaaattaaa aatgaagttt taaatcaatc 15720
   taaagtatat atgagtaaac ttggtctgac agttaccaat gcttaatcag tgaggcacct 15780
   atctcagcga tctgtctatt tcgttcatcc atagttgcct gactccccgt cgtgtagata 15840
   actacgatac gggagggctt accatctggc cccagtgctg caatgatacc gcgagaccca 15900
   cgctcaccgg ctccagattt atcagcaata aaccagccag ccggaagggc cgagcgcaga 15960
   agtggtcctg caactttatc cgcctccatc cagtctatta aacaagtggc agcaacggat 16020
   tcgcaaacct gtcacgcctt ttgtgccaaa agccgcgcca ggtttgcgat ccgctgtgcc 16080
   aggcgttagg cgtcatatga agatttcggt gatccctgag caggtggcgg aaacattgga 16140
   tgctgagaac catttcattg ttcgtgaagt gttcgatgtg cacctatccg accaaggctt 16200
   tgaactatct accagaagtg tgagccccta ccggaaggat tacatctcgg atgatgactc 16260
   tgatgaagac tctgcttgct atggcgcatt catcgaccaa gagcttgtcg ggaagattga 16320
   actcaactca acatggaacg atctagcctc tatcgaacac attgttgtgt cgcacacgca 16380
   ccgaggcaaa ggagtcgcgc acagtctcat cgaatttgcg aaaaagtggg cactaagcag 16440
   acagctcctt ggcatacgat tagagacaca aacgaacaat gtacctgcct gcaatttgta 16500
   cgcaaaatgt ggctttactc tcggcggcat tgacctgttc acgtataaaa ctagacctca 16560
   agtctcgaac gaaacagcga tgtactggta ctggttctcg ggagcacagg atgacgccta 16620
   acaattcatt caagccgaca ccgcttcgcg gcgcggctta attcaggagt taaacatcat 16680
   gagggaagcg gtgatcgccg aagtatcgac tcaactatca gaggtagttg gcgtcatcga 16740
   gcgccatctc gaaccgacgt tgctggccgt acatttgtac ggctccgcag tggatggcgg 16800
   cctgaagcca cacagtgata ttgatttgct ggttacggtg accgtaaggc ttgatgaaac 16860
   aacgcggcga gctttgatca acgacctttt ggaaacttcg gcttcccctg gagagagcga 16920
   gattctccgc gctgtagaag tcaccattgt tgtgcacgac gacatcattc cgtggcgtta 16980
   tccagctaag cgcgaactgc aatttggaga atggcagcgc aatgacattc ttgcaggtat 17040
   cttcgagcca gccacgatcg acattgatct ggctatcttg ctgacaaaag caagagaaca 17100
   tagcgttgcc ttggtaggtc cagcggcgga ggaactcttt gatccggttc ctgaacagga 17160
   tctatttgag gcgctaaatg aaaccttaac gctatggaac tcgccgcccg actgggctgg 17220
   cgatgagcga aatgtagtgc ttacgttgtc ccgcatttgg tacagcgcag taaccggcaa 17280
   aatcgcgccg aaggatgtcg ctgccgactg ggcaatggag cgcctgccgg cccagtatca 17340
   gcccgtcata cttgaagcta ggcaggctta tcttggacaa gaagatcgct tggcctcgcg 17400
   cgcagatcag ttggaagaat ttgttcacta cgtgaaaggc gagatcacca aggtagtcgg 17460
   caaataatgt ctaacaattc gttcaagccg acgccgcttc gcggcgcggc ttaactcaag 17520
   cgttagagag ctggggaaga ctatgcgcga tctgttgaag gtggttctaa gcctcgtact 17580
   tgcgatggca tcggggcagg cacttgctga cctgccaatt gttttagtgg atgaagctcg 17640
   tcttccctat gactactccc catccaacta cgacatttct ccaagcaact acgacaactc 17700
   cataagcaat tacgacaata gtccatcaaa ttacgacaac tctgagagca actacgataa 17760
   tagttcatcc aattacgaca atagtcgcaa cggaaatcgt aggcttatat atagcgcaaa 17820
   tgggtctcgc actttcgccg gctactacgt cattgccaac aatgggacaa cgaacttctt 17880
   ttccacatct ggcaaaagga tgttctacac cccaaaaggg gggcgcggcg tctatggcgg 17940
   caaagatggg agcttctgcg gggcattggt cgtcataaat ggccaatttt cgcttgccct 18000
   gacagataac ggcctgaaga tcatgtatct aagcaactag cctgctctct aataaaatgt 18060
   taggagcttg gctgccattt ttggggtgag gccgttcgcg gccgaggggc gcagcccctg 18120
   gggggatggg aggcccgcgt tagcgggccg ggagggttcg agaagggggg gcacccccct 18180
   tcggcgtgcg cggtcacgcg ccagggcgca gccctggtta aaaacaaggt ttataaatat 18240
   tggtttaaaa gcaggttaaa agacaggtta gcggtggccg aaaaacgggc ggaaaccctt 18300
   gcaaatgctg gattttctgc ctgtggacag cccctcaaat gtcaataggt gcgcccctca 18360
   tctgtcagca ctctgcccct caagtgtcaa ggatcgcgcc cctcatctgt cagtagtcgc 18420
   gcccctcaag tgtcaatacc gcagggcact tatccccagg cttgtccaca tcatctgtgg 18480
   gaaactcgcg taaaatcagg cgttttcgcc gatttgcgag gctggccagc tccacgtcgc 18540
   cggccgaaat cgagcctgcc cctcatctgt caacgccgcg ccgggtgagt cggcccctca 18600
   agtgtcaacg tccgcccctc atctgtcagt gagggccaag ttttccgcga ggtatccaca 18660
   acgccggcgg ccggccgcgg tgtctcgcac acggcttcga cggcgtttct ggcgcgtttg 18720
   cagggccata gacggccgcc agcccagcgg cgagggcaac cagcccggtg agcgtcggaa 18780
   aggg 18784
<210> 31
   <211> 18802
   <212> DNA
   <213> Artificial Sequence
<220>
   <223> Artificial
<400> 31
   tcgacatcgt caacgttcac ttctaaagaa atagcgccac tcagcttcct cagcggcttt     60
   atccagcgat ttcctattat gtcggcatag ttctcaagat cgacagcctg tcacggttaa    120
   gcgagaaatg aataagaagg ctgataattc ggatctctgc gaggagatga tatttgatca    180
   caggcagcaa cgctctgtca tcgttacaat caacatgcta ccctccgcga gatcatccgt    240
   gtttcaaacc cggcagctta gttgccgttc ttccgaatag catcgggtaa catgagcaaa    300
   gtctgccgcc ttacaacggc tctcccgctg acgccgtccc ggactgatgg gctgcctgta    360
   tcgagtggtg attttgtgcc gagctgccgg tcggggagct gttggctggc tggtggcagg    420
   atatattgtg gtgtaaacaa attgacgctt agacaactta ataacacatt gcggacgttt    480
   ttaatgtact gaattcgccg ctcggtgtgt cgtagatact agcccctggg gcacttttga    540
   aatttgaata agatttatgt aatcagtctt ttaggtttga ccggttctgc cgcttttttt    600
   aaaattggat ttgtaataat aaaacgcaat tgtttgttat tgtggcgctc tatcatagat    660
   gtcgctataa acctattcag cacaatatat tgttttcatt ttaatattgt acatataagt    720
   agtagggtac aatcagtaaa ttgaacggag aatattattc ataaaaatac gatagtaacg    780
   ggtgatatat tcattagaat gaaccgaaac cggcggtaag gatctgagct acacatgctc    840
   aggtttttta caacgtgcac aacagaattg aaagcaaata tcatgcgatc ataggcgtct    900
   cgcatatctc attaaacaag tgaagatttg attcaaactc cattgagagc cctgactatg    960
   cattcggttt gacccttcca ggttgagaga cgatagcccc ctaccttaat taaggggccc 1020
   cccctcgagg tcgacggtat cgataagctt gatatcgaat tcctgcagcc agggggatcc 1080
   cccgggtcat cagatctcgg tgacgggcag gaccggacgg ggcggtaccg gcaggctgaa 1140
   gtccagctgc cagaaaccca cgtcatgcca gttcccgtgc ttgaagccgg ccgcccgcag 1200
   catgccgcgg ggggcatatc cgagcgcctc gtgcatgcgc acgctcgggt cgttgggcag 1260
   cccgatgaca gcgaccacgc tcttgaagcc ctgtgcctcc agggacttca gcaggtgggt 1320
   gtagagcgtg gagcccagtc ccgtccgctg gtggcggggg gagacgtaca cggtcgactc 1380
   ggccgtccag tcgtaggcgt tgcgtgcctt ccaggggccc gcgtaggcga tgccggcgac 1440
   ctcgccgtcc acctcggcga cgagccaggg atagcgctcc cgcagacgga cgaggtcgtc 1500
   cgtccactcc tgcggttcct gcggctcggt acggaagttg accgtgcttg tctcgatgta 1560
   gtggttgacg atggtgcaga ccgccggcat gtccgcctcg gtggcacggc ggatgtcggc 1620
   cgggcgtcgt tctgggctca tggtagatcc cctcgagaga gatagatttg tagagagaga 1680
   ctggtgattt cagcgtgtcc tctccaaatg aaatgaactt ccttatatag aggaaggtct 1740
   tgcgaaggat agtgggattg tgcgtcatcc cttacgtcag tggagatatc acatcaatcc 1800
   acttgctttg aagacgtggt tggaacgtct tctttttcca cgatgctcct cgtgggtggg 1860
   ggtccatctt tgggaccact gtcggcagag gcatcttgaa cgatagcctt tcctttatcg 1920
   caatgatggc atttgtaggt gccaccttcc ttttctactg tccttttgat gaagtgacag 1980
   atagctgggc aatggaatcc gaggaggttt cccgatatta ccctttgttg aaaagtctca 2040
   atagcccttt ggtcttctga gactgtatct ttgatattct tggagtagac gagagtgtcg 2100
   tgctccacca tgttatcaca tcaatccact tgctttgaag acgtggttgg aacgtcttct 2160
   ttttccacga tgctcctcgt gggtgggggt ccatctttgg gaccactgtc ggcagaggca 2220
   tcttgaacga tagcctttcc tttatcgcaa tgatggcatt tgtaggtgcc accttccttt 2280
   tctactgtcc ttttgatgaa gtgacagata gctgggcaat ggaatccgag gaggtttccc 2340
   gatattaccc tttgttgaaa agtctcaata gccctttggt cttctgagac tgtatctttg 2400
   atattcttgg agtagacgag agtgtcgtgc tccaccatgt tgctgatagt gaccttaggc 2460
   gacttttgaa cgcgcaataa tggtttctga cgtatgtgct tagctcatta aactccagaa 2520
   acccattaac gcttacaatt tccattcgcc attcaggctg cgcaactgtt gggaagggcg 2580
   atcggtgcgg gcctcttcgc tattacgcca gctggcgaaa gggggatgtg ctgcaaggcg 2640
   attaagttgg gtaacgccag ggttttccca gtcacgacgt tgtaaaacga cggccagtga 2700
   attgtaatac gactcactat agggcgaatt gggcccgacg tcgcatgctc ccggccgcca 2760
   tggccgcggg atatcactag tgcggccgct cgacgaatta attccaatcc cacaaaaatc 2820
   tgagcttaac agcacagttg ctcctctcag agcagaatcg ggtattcaac accctcatat 2880
   caactactac gttgtgtata acggtccaca tgccggtata tacgatgact ggggttgtac 2940
   aaaggcggca acaaacggcg ttcccggagt tgcacacaag aaatttgcca ctattacaga 3000
   ggcaagagca gcagctgacg cgtacacaac aagtcagcaa acagacaggt tgaacttcat 3060
   ccccaaagga gaagctcaac tcaagcccaa gagctttgct aaggccctaa caagcccacc 3120
   aaagcaaaaa gcccactggc tcacgctagg aaccaaaagg cccagcagtg atccagcccc 3180
   aaaagagatc tcctttgccc cggagattac aatggacgat ttcctctatc tttacgatct 3240
   aggaaggaag ttcgaaggtg aaggtgacga cactatgttc accactgata atgagaaggt 3300
   tagcctcttc aatttcagaa agaatgctga cccacagatg gttagagagg cctacgcagc 3360
   aggtctcatc aagacgatct acccgagtaa caatctccag gagatcaaat accttcccaa 3420
   gaaggttaaa gatgcagtca aaagattcag gactaattgc atcaagaaca cagagaaaga 3480
   catatttctc aagatcagaa gtactattcc agtatggacg attcaaggct tgcttcataa 3540
   accaaggcaa gtaatagaga ttggagtctc taaaaaggta gttcctactg aatctaaggc 3600
   catgcatgga gtctaagatt caaatcgagg atctaacaga actcgccgtg aagactggcg 3660
   aacagttcat acagagtctt ttacgactca atgacaagaa gaaaatcttc gtcaacatgg 3720
   tggagcacga cactctggtc tactccaaaa atgtcaaaga tacagtctca gaagaccaaa 3780
   gggctattga gacttttcaa caaaggataa tttcgggaaa cctcctcgga ttccattgcc 3840
   cagctatctg tcacttcatc gaaaggacag tagaaaagga aggtggctcc tacaaatgcc 3900
   atcattgcga taaaggaaag gctatcattc aagatctctc tgccgacagt ggtcccaaag 3960
   atggaccccc acccacgagg agcatcgtgg aaaaagaaga cgttccaacc acgtcttcaa 4020
   agcaagtgga ttgatgtgac atctccactg acgtaaggga tgacgcacaa tcccactatc 4080
   cttcgcaaga cccttcctct atataaggaa gttcatttca tttggagagg acacgctcga 4140
   ggaattcggt accccatcac aagtttgtac aaaaaagcag gctgcggccg cttgctccct 4200
   taaaaaaaac catggcatgt cattacggat gtggacaaca gcaacagact tgtgcacctc 4260
   atctttgtca gcttcaacct agagcatgta gagttgtgaa ggctgctact gctgttactg 4320
   taaatttctg tgttccttat tctctcaaaa tcttcgattt tgttttcgtt cgatcccaat 4380
   ttcgtatatg ttctttggtt tagattctgt taatcttaga tcgaagacga ttttctgggt 4440
   ttgatcgtta gatatcatct taattctcga ttagggtttc atagatatca tccgatttgt 4500
   tcaaataatt tgagttttgt cgaataatta ctcttcgatt tgtgatttct atctagatct 4560
   ggtgttagtt tctagtttgt gcgatcgaat ttgtcgatta atctgagttt ttctgattaa 4620
   caggctggag gatctcttct tgttctctct ggacttactc tcgctggaac tgttatcgct 4680
   ctcactatcg ctacacctct tctcgttatc ttctctcctg ttctcgttcc tgctgtgatc 4740
   actatcttcc ttctcggagc tggatttctt gcttctggtg gatttggagt tgctgctctc 4800
   tctgttctct cttggatcta cagatacctc actggatgta aacatcctcc aggtgctgat 4860
   tgtcttgagt ctgcttgtaa gactaagctc gcttcttgtg ctagagagat gaaggattgt 4920
   agagcagagc aattctcttg tcagcctgtt gctggatctc agacttctta atgaacatat 4980
   ggtcctgctt taatgagata tgcgagacgc ctatgatcgc atgatatttg ctttcaattc 5040
   tgttgtgcac gttgtaaaaa acctgagcat gtgtagctca gatccttacc gccggtttcg 5100
   gttcattcta atgaatatat cacccgttac tatcgtattt ttatgaataa tattctccgt 5160
   tcaatttact gattgtaccc tactacttat atgtacaata ttaaaatgaa aacaatatat 5220
   tgtgctgaat aggtttatag cgacatctat gatagagcgc cacaataaca aacaattgcg 5280
   ttttattatt acaaatccaa ttttaaaaaa agcggcagaa ccggtcaaac ctaaaagact 5340
   gattacataa atcttattca aatttcaaaa ggccccaggg gctagtatct acgacacacc 5400
   gagcggcgaa ctaataacgt tcactgaagg gaactccggt tccccgccgg cgcgcatggg 5460
   tgagattcct tgaagttgag tattggccgt ccgctctacc gaaagttacg ggcaccattc 5520
   aacccggtcc agcacggcgg ccgggtaacc gacttgctgc cccgagaatt atgcagcatt 5580
   tttttggtgt atgtgggccc caaatgaagt gcaggtcaaa ccttgacagt gacgacaaat 5640
   cgttgggcgg gtccagggcg aattttgcga caacatgtcg aggctcagca ggacctgcag 5700
   gcatgcaagc tagcttacta gtgatgcata ttctatagtg tcacctaaat cttcgacgaa 5760
   ttaattccaa tcccacaaaa atctgagctt aacagcacag ttgctcctct cagagcagaa 5820
   tcgggtattc aacaccctca tatcaactac tacgttgtgt ataacggtcc acatgccggt 5880
   atatacgatg actggggttg tacaaaggcg gcaacaaacg gcgttcccgg agttgcacac 5940
   aagaaatttg ccactattac agaggcaaga gcagcagctg acgcgtacac aacaagtcag 6000
   caaacagaca ggttgaactt catccccaaa ggagaagctc aactcaagcc caagagcttt 6060
   gctaaggccc taacaagccc accaaagcaa aaagcccact ggctcacgct aggaaccaaa 6120
   aggcccagca gtgatccagc cccaaaagag atctcctttg ccccggagat tacaatggac 6180
   gatttcctct atctttacga tctaggaagg aagttcgaag gtgaaggtga cgacactatg 6240
   ttcaccactg ataatgagaa ggttagcctc ttcaatttca gaaagaatgc tgacccacag 6300
   atggttagag aggcctacgc agcaggtctc atcaagacga tctacccgag taacaatctc 6360
   caggagatca aataccttcc caagaaggtt aaagatgcag tcaaaagatt caggactaat 6420
   tgcatcaaga acacagagaa agacatattt ctcaagatca gaagtactat tccagtatgg 6480
   acgattcaag gcttgcttca taaaccaagg caagtaatag agattggagt ctctaaaaag 6540
   gtagttccta ctgaatctaa ggccatgcat ggagtctaag attcaaatcg aggatctaac 6600
   agaactcgcc gtgaagactg gcgaacagtt catacagagt cttttacgac tcaatgacaa 6660
   gaagaaaatc ttcgtcaaca tggtggagca cgacactctg gtctactcca aaaatgtcaa 6720
   agatacagtc tcagaagacc aaagggctat tgagactttt caacaaagga taatttcggg 6780
   aaacctcctc ggattccatt gcccagctat ctgtcacttc atcgaaagga cagtagaaaa 6840
   ggaaggtggc tcctacaaat gccatcattg cgataaagga aaggctatca ttcaagatct 6900
   ctctgccgac agtggtccca aagatggacc cccacccacg aggagcatcg tggaaaaaga 6960
   agacgttcca accacgtctt caaagcaagt ggattgatgt gacatctcca ctgacgtaag 7020
   ggatgacgca caatcccact atccttcgca agacccttcc tctatataag gaagttcatt 7080
   tcatttggag aggacacggg atccttgctc cgttaaaaaa aaccatggct atcctcgatt 7140
   ctgctggtgt tactactgtg actgagaatg gtggtggaga gttcgttgat ctcgatagac 7200
   tcagaagaag aaagtctaga tctgtaaatt tctgtgttcc ttattctctc aaaatcttcg 7260
   attttgtttt cgttcgatcc caatttcgta tatgttcttt ggtttagatt ctgttaatct 7320
   tagatcgaag acgattttct gggtttgatc gttagatatc atcttaattc tcgattaggg 7380
   tttcatagat atcatccgat ttgttcaaat aatttgagtt ttgtcgaata attactcttc 7440
   gatttgtgat ttctatctag atctggtgtt agtttctagt ttgtgcgatc gaatttgtcg 7500
   attaatctga gtttttctga ttaacaggat tcttctaacg gacttctcct ctctggatct 7560
   gataacaact ctccttctga tgatgttggt gctcctgctg atgtgagaga tagaatcgat 7620
   tctgtggtga acgatgatgc tcaaggtact gctaacctcg ctggtgataa taacggtgga 7680
   ggtgataaca atggtggagg aagaggtgga ggtgaaggta gaggaaacgc tgatgctact 7740
   ttcacttaca gaccatctgt gcctgctcat agaagagcta gagagtctcc tctctcttct 7800
   gatgctatct tcaagcagtc tcacgctgga cttttcaacc tctgtgtggt ggttcttatc 7860
   gctgtgaact ctagactcat catcgagaac ctcatgaagt acggatggct catcagaact 7920
   gatttctggt tctcttctag atctctcaga gattggcctc ttttcatgtg ctgcatctca 7980
   ctctcaatct tccctctcgc tgcttttact gttgagaagc tcgtgctcca gaagtatatc 8040
   gctgaacctg tggtgatctt cctccacatc atcatcacta tgactgaggt tctctaccct 8100
   gtttacgtga ctctcagatg cgattctgct ttcctctctg gtgttactct tatgctcctc 8160
   acttgcattg tgtggcttaa gctcgtgtct tacgctcaca cttcttacga tatcagatct 8220
   ctcgctaacg ctgctgataa ggctaaccct gaagtgtctt actacgtgtc tctcaagtct 8280
   ctcgcttact tcatggttgc tcctacactt tgttaccagc catcttaccc tagatctgct 8340
   tgcattagaa agggatgggt ggcaagacaa ttcgctaagt tggtgatctt cactggattc 8400
   atgggattca tcatcgagca gtacatcaac cctattgtga gaaactctaa gcaccctctc 8460
   aagggtgatc ttctctacgc tatcgagaga gttcttaagc tctctgtgcc taacctttat 8520
   gtgtggctct gcatgttcta ctgtttcttc cacctctggc ttaacatcct tgctgagttg 8580
   ctttgcttcg gagatagaga gttctacaag gattggtgga acgctaagtc tgttggagat 8640
   tattggagaa tgtggaacat gcctgtgcat aagtggatgg tgcgtcacat ctacttccct 8700
   tgcctcagat ctaagatccc taagactctc gctatcatta tcgctttcct cgtgtctgct 8760
   gttttccatg agttgtgtat cgctgttcct tgcagacttt tcaagctttg ggctttcctc 8820
   ggaatcatgt tccaggttcc actcgtgttc atcactaact acctccaaga gagattcgga 8880
   tctactgttg gaaacatgat tttctggttc attttctgca tcttcggaca gcctatgtgc 8940
   gttctcctct actaccacga tctcatgaac agaaagggat ctatgtctta atgaaggatc 9000
   cacccagctt tcttgtacaa agtggtgatg ggttcgaaat cgataagctt ggatcctcta 9060
   gagtcctgct ttaatgagat atgcgagacg cctatgatcg catgatattt gctttcaatt 9120
   ctgttgtgca cgttgtaaaa aacctgagca tgtgtagctc agatccttac cgccggtttc 9180
   ggttcattct aatgaatata tcacccgtta ctatcgtatt tttatgaata atattctccg 9240
   ttcaatttac tgattgtacc ctactactta tatgtacaat attaaaatga aaacaatata 9300
   ttgtgctgaa taggtttata gcgacatcta tgatagagcg ccacaataac aaacaattgc 9360
   gttttattat tacaaatcca attttaaaaa aagcggcaga accggtcaaa cctaaaagac 9420
   tgattacata aatcttattc aaatttcaaa aggccccagg ggctagtatc tacgacacac 9480
   cgagcggcga actaataacg ttcactgaag ggaactccgg ttccccgccg gcgcgcatgg 9540
   gtgagattcc ttgaagttga gtattggccg tccgctctac cgaaagttac gggcaccatt 9600
   caacccggtc cagcacggcg gccgggtaac cgacttgctg ccccgagaat tatgcagcat 9660
   ttttttggtg tatgtgggcc ccaaatgaag tgcaggtcaa accttgacag tgacgacaaa 9720
   tcgttgggcg ggtccagggc gaattttgcg acaacatgtc gaggctcagc aggacctgca 9780
   ggcatgcaag ctagcttact agtgatgcat attctatagt gtcacctaaa tctgcggccg 9840
   cctgcaggtc gatatgggag agctcccaac gcgttggatg catagcttga gtattctata 9900
   gtgtcaccta aatagcttgg cgtaatcatg gtcatagctg tttcctgtgt gaaattgtta 9960
   tccgctcaca attccacaca acatacgagc cggaagcata aagtgtaaag cctggggtgc 10020
   ctaatgagtg agctaactca cattaattgc gttgcgctca ctgcccgctt tccagtcggg 10080
   aaacctgtcg tgccagctgc attaatgaat cggccaacgc gcggggagag gcggtttgcg 10140
   tattggggct gagtggctcc ttcaacgttg cggttctgtc agttccaaac gtaaaacggc 10200
   ttgtcccgcg tcatcggcgg gggtcataac gtgactccct taattctccg ctcatgatca 10260
   gattgtcgtt tcccgccttc agtttaaact atcagtgttt gacaggatat attggcgggt 10320
   aaacctaaga gaaaagagcg tttattagaa taatcggata tttaaaaggg cgtgaaaagg 10380
   tttatccgtt cgtccatttg tatgtgcatg ccaaccacag ggttcccctc gggagtgctt 10440
   ggcattccgt gcgataatga cttctgttca accacccaaa cgtcggaaag cctgacgacg 10500
   gagcagcatt ccaaaaagat cccttggctc gtctgggtcg gctagaaggt cgagtgggct 10560
   gctgtggctt gatccctcaa cgcggtcgcg gacgtagcgc agcgccgaaa aatcctcgat 10620
   cgcaaatccg acgctgtcga aaatcgtgat ctgcttgtcg ctctttcggc cgacgtcctg 10680
   gccagtcatc acgcgccaaa gttccgtcac aggatgatct ggcgcgagtt gctggatctc 10740
   gccttcaatc cgggtctgtg gcgggaactc cacgaaaata tccgaacgca gcaagatgtc 10800
   gacggatctt ttccgctgca taaccctgct tcggggtcat tatagcgatt ttttcggtat 10860
   atccatcctt tttcgcacga tatacaggat tttgccaaag ggttcgtgta gactttcctt 10920
   ggtgtatcca acggcgtcag ccgggcagga taggtgaagt aggcccaccc gcgagcgggt 10980
   gttccttctt cactgtccct tattcgcacc tggcggtgct caacgggaat cctgctctgc 11040
   gaggctggcc ggctaccgcc ggcgtaacag atgagggcaa gcggatggct gatgaaacca 11100
   agccaaccag gggtgatgct gccaacttac tgatttagtg tatgatggtg tttttgaggt 11160
   gctccagtgg cttctgtttc tatcagctgt ccctcctgtt cagctactga cggggtggtg     11220
   cgtaacggca aaagcaccgc cggacatcag cgctatctct gctctcactg ccgtaaaaca     11280
   tggcaactgc agttcactta caccgcttct caacccggta cgcaccagaa aatcattgat     11340
   atggccatga atggcgttgg atgccgggca acagcccgca ttatgggcgt tggcctcaac     11400
   acgattttac gtcacttaaa aaactcaggc cgcagtcggt aacctcgcgc atacagccgg     11460
   gcagtgacgt catcgtctgc gcggaaatgg acgaacagtg gggctatgtc ggggctaaat     11520
   cgcgccagcg ctggctgttt tacgcgtatg acagtctccg gaagacggtt gttgcgcacg     11580
   tattcggtga acgcactatg gcgacgctgg ggcgtcttat gagcctgctg tcaccctttg     11640
   acgtggtgat atggatgacg gatggctggc cgctgtatga atcccgcctg aagggaaagc     11700
   tgcacgtaat cagcaagcga tatacgcagc gaattgagcg gcataacctg aatctgaggc     11760
   agcacctggc acggctggga cggaagtcgc tgtcgttctc aaaatcggtg gagctgcatg     11820
   acaaagtcat cgggcattat ctgaacataa aacactatca ataagttgga gtcattaccc     11880
   aaccaggaag ggcagcccac ctatcaaggt gtactgcctt ccagacgaac gaagagcgat     11940
   tgaggaaaag gcggcggcgg ccggcatgag cctgtcggcc tacctgctgg ccgtcggcca     12000
   gggctacaaa atcacgggcg tcgtggacta tgagcacgtc cgcgagctgg cccgcatcaa     12060
   tggcgacctg ggccgcctgg gcggcctgct gaaactctgg ctcaccgacg acccgcgcac     12120
   ggcgcggttc ggtgatgcca cgatcctcgc cctgctggcg aagatcgaag agaagcagga     12180
   cgagcttggc aaggtcatga tgggcgtggt ccgcccgagg gcagagccat gactttttta     12240
   gccgctaaaa cggccggggg gtgcgcgtga ttgccaagca cgtccccatg cgctccatca     12300
   agaagagcga cttcgcggag ctggtattcg tgcagggcaa gattcggaat accaagtacg     12360
   agaaggacgg ccagacggtc tacgggaccg acttcattgc cgataaggtg gattatctgg     12420
   acaccaaggc accaggcggg tcaaatcagg aataagggca cattgccccg gcgtgagtcg     12480
   gggcaatccc gcaaggaggg tgaatgaatc ggacgtttga ccggaaggca tacaggcaag     12540
   aactgatcga cgcggggttt tccgccgagg atgccgaaac catcgcaagc cgcaccgtca     12600
   tgcgtgcgcc ccgcgaaacc ttccagtccg tcggctcgat ggtccagcaa gctacggcca     12660
   agatcgagcg cgacagcgtg caactggctc cccctgccct gcccgcgcca tcggccgccg     12720
   tggagcgttc gcgtcgtctc gaacaggagg cggcaggttt ggcgaagtcg atgaccatcg     12780
   acacgcgagg aactatgacg accaagaagc gaaaaaccgc cggcgaggac ctggcaaaac     12840
   aggtcagcga ggccaagcag gccgcgttgc tgaaacacac gaagcagcag atcaaggaaa     12900
   tgcagctttc cttgttcgat attgcgccgt ggccggacac gatgcgagcg atgccaaacg     12960
   acacggcccg ctctgccctg ttcaccacgc gcaacaagaa aatcccgcgc gaggcgctgc     13020
   aaaacaaggt cattttccac gtcaacaagg acgtgaagat cacctacacc ggcgtcgagc     13080
   tgcgggccga cgatgacgaa ctggtgtggc agcaggtgtt ggagtacgcg aagcgcaccc     13140
   ctatcggcga gccgatcacc ttcacgttct acgagctttg ccaggacctg ggctggtcga     13200
   tcaatggccg gtattacacg aaggccgagg aatgcctgtc gcgcctacag gcgacggcga 13260
   tgggcttcac gtccgaccgc gttgggcacc tggaatcggt gtcgctgctg caccgcttcc 13320
   gcgtcctgga ccgtggcaag aaaacgtccc gttgccaggt cctgatcgac gaggaaatcg 13380
   tcgtgctgtt tgctggcgac cactacacga aattcatatg ggagaagtac cgcaagctgt 13440
   cgccgacggc ccgacggatg ttcgactatt tcagctcgca ccgggagccg tacccgctca 13500
   agctggaaac cttccgcctc atgtgcggat cggattccac ccgcgtgaag aagtggcgcg 13560
   agcaggtcgg cgaagcctgc gaagagttgc gaggcagcgg cctggtggaa cacgcctggg 13620
   tcaatgatga cctggtgcat tgcaaacgct agggccttgt ggggtcagtt ccggctgggg 13680
   gttcagcagc cagcgcttta ctggcatttc aggaacaagc gggcactgct cgacgcactt 13740
   gcttcgctca gtatcgctcg ggacgcacgg cgcgctctac gaactgccga taaacagagg 13800
   attaaaattg acaattgtga ttaaggctca gattcgacgg cttggagcgg ccgacgtgca 13860
   ggatttccgc gagatccgat tgtcggccct gaagaaagct ccagagatgt tcgggtccgt 13920
   ttacgagcac gaggagaaaa agcccatgga ggcgttcgct gaacggttgc gagatgccgt 13980
   ggcattcggc gcctacatcg acggcgagat cattgggctg tcggtcttca aacaggagga 14040
   cggccccaag gacgctcaca aggcgcatct gtccggcgtt ttcgtggagc ccgaacagcg 14100
   aggccgaggg gtcgccggta tgctgctgcg ggcgttgccg gcgggtttat tgctcgtgat 14160
   gatcgtccga cagattccaa cgggaatctg gtggatgcgc atcttcatcc tcggcgcact 14220
   taatatttcg ctattctgga gcttgttgtt tatttcggtc taccgcctgc cgggcggggt 14280
   cgcggcgacg gtaggcgctg tgcagccgct gatggtcgtg ttcatctctg ccgctctgct 14340
   aggtagcccg atacgattga tggcggtcct gggggctatt tgcggaactg cgggcgtggc 14400
   gctgttggtg ttgacaccaa acgcagcgct agatcctgtc ggcgtcgcag cgggcctggc 14460
   gggggcggtt tccatggcgt tcggaaccgt gctgacccgc aagtggcaac ctcccgtgcc 14520
   tctgctcacc tttaccgcct ggcaactggc ggccggagga cttctgctcg ttccagtagc 14580
   tttagtgttt gatccgccaa tcccgatgcc tacaggaacc aatgttctcg gcctggcgtg 14640
   gctcggcctg atcggagcgg gtttaaccta cttcctttgg ttccggggga tctcgcgact 14700
   cgaacctaca gttgtttcct tactgggctt tctcagccgg gatggcgcta agaagctatt 14760
   gccgccgatc ttcatatgcg gtgtgaaata ccgcacagat gcgtaaggag aaaataccgc 14820
   atcaggcgct cttccgcttc ctcgctcact gactcgctgc gctcggtcgt tcggctgcgg 14880
   cgagcggtat cagctcactc aaaggcggta atacggttat ccacagaatc aggggataac 14940
   gcaggaaaga acatgtgagc aaaaggccag caaaaggcca ggaaccgtaa aaaggccgcg 15000
   ttgctggcgt ttttccatag gctccgcccc cctgacgagc atcacaaaaa tcgacgctca 15060
   agtcagaggt ggcgaaaccc gacaggacta taaagatacc aggcgtttcc ccctggaagc 15120
   tccctcgtgc gctctcctgt tccgaccctg ccgcttaccg gatacctgtc cgcctttctc 15180
   ccttcgggaa gcgtggcgct ttctcaatgc tcacgctgta ggtatctcag ttcggtgtag 15240
   gtcgttcgct ccaagctggg ctgtgtgcac gaaccccccg ttcagcccga ccgctgcgcc 15300
   ttatccggta actatcgtct tgagtccaac ccggtaagac acgacttatc gccactggca 15360
   gcagccactg gtaacaggat tagcagagcg aggtatgtag gcggtgctac agagttcttg 15420
   aagtggtggc ctaactacgg ctacactaga aggacagtat ttggtatctg cgctctgctg 15480
   aagccagtta ccttcggaaa aagagttggt agctcttgat ccggcaaaca aaccaccgct 15540
   ggtagcggtg gtttttttgt ttgcaagcag cagattacgc gcagaaaaaa aggatatcaa 15600
   gaagatcctt tgatcttttc tacggggtct gacgctcagt ggaacgaaaa ctcacgttaa 15660
   gggattttgg tcatgagatt atcaaaaagg atcttcacct agatcctttt aaattaaaaa 15720
   tgaagtttta aatcaatcta aagtatatat gagtaaactt ggtctgacag ttaccaatgc 15780
   ttaatcagtg aggcacctat ctcagcgatc tgtctatttc gttcatccat agttgcctga 15840
   ctccccgtcg tgtagataac tacgatacgg gagggcttac catctggccc cagtgctgca 15900
   atgataccgc gagacccacg ctcaccggct ccagatttat cagcaataaa ccagccagcc 15960
   ggaagggccg agcgcagaag tggtcctgca actttatccg cctccatcca gtctattaaa 16020
   caagtggcag caacggattc gcaaacctgt cacgcctttt gtgccaaaag ccgcgccagg 16080
   tttgcgatcc gctgtgccag gcgttaggcg tcatatgaag atttcggtga tccctgagca 16140
   ggtggcggaa acattggatg ctgagaacca tttcattgtt cgtgaagtgt tcgatgtgca 16200
   cctatccgac caaggctttg aactatctac cagaagtgtg agcccctacc ggaaggatta 16260
   catctcggat gatgactctg atgaagactc tgcttgctat ggcgcattca tcgaccaaga 16320
   gcttgtcggg aagattgaac tcaactcaac atggaacgat ctagcctcta tcgaacacat 16380
   tgttgtgtcg cacacgcacc gaggcaaagg agtcgcgcac agtctcatcg aatttgcgaa 16440
   aaagtgggca ctaagcagac agctccttgg catacgatta gagacacaaa cgaacaatgt 16500
   acctgcctgc aatttgtacg caaaatgtgg ctttactctc ggcggcattg acctgttcac 16560
   gtataaaact agacctcaag tctcgaacga aacagcgatg tactggtact ggttctcggg 16620
   agcacaggat gacgcctaac aattcattca agccgacacc gcttcgcggc gcggcttaat 16680
   tcaggagtta aacatcatga gggaagcggt gatcgccgaa gtatcgactc aactatcaga 16740
   ggtagttggc gtcatcgagc gccatctcga accgacgttg ctggccgtac atttgtacgg 16800
   ctccgcagtg gatggcggcc tgaagccaca cagtgatatt gatttgctgg ttacggtgac 16860
   cgtaaggctt gatgaaacaa cgcggcgagc tttgatcaac gaccttttgg aaacttcggc 16920
   ttcccctgga gagagcgaga ttctccgcgc tgtagaagtc accattgttg tgcacgacga 16980
   catcattccg tggcgttatc cagctaagcg cgaactgcaa tttggagaat ggcagcgcaa 17040
   tgacattctt gcaggtatct tcgagccagc cacgatcgac attgatctgg ctatcttgct 17100
   gacaaaagca agagaacata gcgttgcctt ggtaggtcca gcggcggagg aactctttga 17160
   tccggttcct gaacaggatc tatttgaggc gctaaatgaa accttaacgc tatggaactc 17220
   gccgcccgac tgggctggcg atgagcgaaa tgtagtgctt acgttgtccc gcatttggta 17280
   cagcgcagta accggcaaaa tcgcgccgaa ggatgtcgct gccgactggg caatggagcg 17340
   cctgccggcc cagtatcagc ccgtcatact tgaagctagg caggcttatc ttggacaaga 17400
   agatcgcttg gcctcgcgcg cagatcagtt ggaagaattt gttcactacg tgaaaggcga 17460
   gatcaccaag gtagtcggca aataatgtct aacaattcgt tcaagccgac gccgcttcgc 17520
   ggcgcggctt aactcaagcg ttagagagct ggggaagact atgcgcgatc tgttgaaggt 17580
   ggttctaagc ctcgtacttg cgatggcatc ggggcaggca cttgctgacc tgccaattgt 17640
   tttagtggat gaagctcgtc ttccctatga ctactcccca tccaactacg acatttctcc 17700
   aagcaactac gacaactcca taagcaatta cgacaatagt ccatcaaatt acgacaactc 17760
   tgagagcaac tacgataata gttcatccaa ttacgacaat agtcgcaacg gaaatcgtag 17820
   gcttatatat agcgcaaatg ggtctcgcac tttcgccggc tactacgtca ttgccaacaa 17880
   tgggacaacg aacttctttt ccacatctgg caaaaggatg ttctacaccc caaaaggggg 17940
   gcgcggcgtc tatggcggca aagatgggag cttctgcggg gcattggtcg tcataaatgg 18000
   ccaattttcg cttgccctga cagataacgg cctgaagatc atgtatctaa gcaactagcc 18060
   tgctctctaa taaaatgtta ggagcttggc tgccattttt ggggtgaggc cgttcgcggc 18120
   cgaggggcgc agcccctggg gggatgggag gcccgcgtta gcgggccggg agggttcgag 18180
   aagggggggc accccccttc ggcgtgcgcg gtcacgcgcc agggcgcagc cctggttaaa 18240
   aacaaggttt ataaatattg gtttaaaagc aggttaaaag acaggttagc ggtggccgaa 18300
   aaacgggcgg aaacccttgc aaatgctgga ttttctgcct gtggacagcc cctcaaatgt 18360
   caataggtgc gcccctcatc tgtcagcact ctgcccctca agtgtcaagg atcgcgcccc 18420
   tcatctgtca gtagtcgcgc ccctcaagtg tcaataccgc agggcactta tccccaggct 18480
   tgtccacatc atctgtggga aactcgcgta aaatcaggcg ttttcgccga tttgcgaggc 18540
   tggccagctc cacgtcgccg gccgaaatcg agcctgcccc tcatctgtca acgccgcgcc 18600
   gggtgagtcg gcccctcaag tgtcaacgtc cgcccctcat ctgtcagtga gggccaagtt 18660
   ttccgcgagg tatccacaac gccggcggcc ggccgcggtg tctcgcacac ggcttcgacg 18720
   gcgtttctgg cgcgtttgca gggccataga cggccgccag cccagcggcg agggcaacca 18780
   gcccggtgag cgtcggaaag gg 18802
<210> 32
   <211> 18823
   <212> DNA
   <213> Artificial Sequence
<220>
   <223> Artificial
<400> 32
   tcgacatcgt caacgttcac ttctaaagaa atagcgccac tcagcttcct cagcggcttt 60
   atccagcgat ttcctattat gtcggcatag ttctcaagat cgacagcctg tcacggttaa 120
   gcgagaaatg aataagaagg ctgataattc ggatctctgc gaggagatga tatttgatca 180
   caggcagcaa cgctctgtca tcgttacaat caacatgcta ccctccgcga gatcatccgt 240
   gtttcaaacc cggcagctta gttgccgttc ttccgaatag catcgggtaa catgagcaaa 300
   gtctgccgcc ttacaacggc tctcccgctg acgccgtccc ggactgatgg gctgcctgta 360
   tcgagtggtg attttgtgcc gagctgccgg tcggggagct gttggctggc tggtggcagg 420
   atatattgtg gtgtaaacaa attgacgctt agacaactta ataacacatt gcggacgttt 480
   ttaatgtact gaattcgccg ctcggtgtgt cgtagatact agcccctggg gcacttttga 540
   aatttgaata agatttatgt aatcagtctt ttaggtttga ccggttctgc cgcttttttt 600
   aaaattggat ttgtaataat aaaacgcaat tgtttgttat tgtggcgctc tatcatagat 660
   gtcgctataa acctattcag cacaatatat tgttttcatt ttaatattgt acatataagt 720
   agtagggtac aatcagtaaa ttgaacggag aatattattc ataaaaatac gatagtaacg 780
   ggtgatatat tcattagaat gaaccgaaac cggcggtaag gatctgagct acacatgctc 840
   aggtttttta caacgtgcac aacagaattg aaagcaaata tcatgcgatc ataggcgtct 900
   cgcatatctc attaaacaag tgaagatttg attcaaactc cattgagagc cctgactatg 960
   cattcggttt gacccttcca ggttgagaga cgatagcccc ctaccttaat taaggggccc 1020
   cccctcgagg tcgacggtat cgataagctt gatatcgaat tcctgcagcc agggggatcc 1080
   cccgggtcat cagatctcgg tgacgggcag gaccggacgg ggcggtaccg gcaggctgaa 1140
   gtccagctgc cagaaaccca cgtcatgcca gttcccgtgc ttgaagccgg ccgcccgcag 1200
   catgccgcgg ggggcatatc cgagcgcctc gtgcatgcgc acgctcgggt cgttgggcag 1260
   cccgatgaca gcgaccacgc tcttgaagcc ctgtgcctcc agggacttca gcaggtgggt 1320
   gtagagcgtg gagcccagtc ccgtccgctg gtggcggggg gagacgtaca cggtcgactc 1380
   ggccgtccag tcgtaggcgt tgcgtgcctt ccaggggccc gcgtaggcga tgccggcgac 1440
   ctcgccgtcc acctcggcga cgagccaggg atagcgctcc cgcagacgga cgaggtcgtc 1500
   cgtccactcc tgcggttcct gcggctcggt acggaagttg accgtgcttg tctcgatgta 1560
   gtggttgacg atggtgcaga ccgccggcat gtccgcctcg gtggcacggc ggatgtcggc 1620
   cgggcgtcgt tctgggctca tggtagatcc cctcgagaga gatagatttg tagagagaga 1680
   ctggtgattt cagcgtgtcc tctccaaatg aaatgaactt ccttatatag aggaaggtct 1740
   tgcgaaggat agtgggattg tgcgtcatcc cttacgtcag tggagatatc acatcaatcc 1800
   acttgctttg aagacgtggt tggaacgtct tctttttcca cgatgctcct cgtgggtggg 1860
   ggtccatctt tgggaccact gtcggcagag gcatcttgaa cgatagcctt tcctttatcg 1920
   caatgatggc atttgtaggt gccaccttcc ttttctactg tccttttgat gaagtgacag 1980
   atagctgggc aatggaatcc gaggaggttt cccgatatta ccctttgttg aaaagtctca 2040
   atagcccttt ggtcttctga gactgtatct ttgatattct tggagtagac gagagtgtcg 2100
   tgctccacca tgttatcaca tcaatccact tgctttgaag acgtggttgg aacgtcttct 2160
   ttttccacga tgctcctcgt gggtgggggt ccatctttgg gaccactgtc ggcagaggca 2220
   tcttgaacga tagcctttcc tttatcgcaa tgatggcatt tgtaggtgcc accttccttt   2280
   tctactgtcc ttttgatgaa gtgacagata gctgggcaat ggaatccgag gaggtttccc   2340
   gatattaccc tttgttgaaa agtctcaata gccctttggt cttctgagac tgtatctttg   2400
   atattcttgg agtagacgag agtgtcgtgc tccaccatgt tgctgatagt gaccttaggc   2460
   gacttttgaa cgcgcaataa tggtttctga cgtatgtgct tagctcatta aactccagaa   2520
   acccattaac gcttacaatt tccattcgcc attcaggctg cgcaactgtt gggaagggcg   2580
   atcggtgcgg gcctcttcgc tattacgcca gctggcgaaa gggggatgtg ctgcaaggcg   2640
   attaagttgg gtaacgccag ggttttccca gtcacgacgt tgtaaaacga cggccagtga   2700
   attgtaatac gactcactat agggcgaatt gggcccgacg tcgcatgctc ccggccgcca   2760
   tggccgcggg atatcactag tgcggccgct cgacgaatta attccaatcc cacaaaaatc   2820
   tgagcttaac agcacagttg ctcctctcag agcagaatcg ggtattcaac accctcatat   2880
   caactactac gttgtgtata acggtccaca tgccggtata tacgatgact ggggttgtac   2940
   aaaggcggca acaaacggcg ttcccggagt tgcacacaag aaatttgcca ctattacaga   3000
   ggcaagagca gcagctgacg cgtacacaac aagtcagcaa acagacaggt tgaacttcat   3060
   ccccaaagga gaagctcaac tcaagcccaa gagctttgct aaggccctaa caagcccacc   3120
   aaagcaaaaa gcccactggc tcacgctagg aaccaaaagg cccagcagtg atccagcccc   3180
   aaaagagatc tcctttgccc cggagattac aatggacgat ttcctctatc tttacgatct   3240
   aggaaggaag ttcgaaggtg aaggtgacga cactatgttc accactgata atgagaaggt   3300
   tagcctcttc aatttcagaa agaatgctga cccacagatg gttagagagg cctacgcagc   3360
   aggtctcatc aagacgatct acccgagtaa caatctccag gagatcaaat accttcccaa   3420
   gaaggttaaa gatgcagtca aaagattcag gactaattgc atcaagaaca cagagaaaga   3480
   catatttctc aagatcagaa gtactattcc agtatggacg attcaaggct tgcttcataa   3540
   accaaggcaa gtaatagaga ttggagtctc taaaaaggta gttcctactg aatctaaggc   3600
   catgcatgga gtctaagatt caaatcgagg atctaacaga actcgccgtg aagactggcg   3660
   aacagttcat acagagtctt ttacgactca atgacaagaa gaaaatcttc gtcaacatgg   3720
   tggagcacga cactctggtc tactccaaaa atgtcaaaga tacagtctca gaagaccaaa   3780
   gggctattga gacttttcaa caaaggataa tttcgggaaa cctcctcgga ttccattgcc   3840
   cagctatctg tcacttcatc gaaaggacag tagaaaagga aggtggctcc tacaaatgcc   3900
   atcattgcga taaaggaaag gctatcattc aagatctctc tgccgacagt ggtcccaaag   3960
   atggaccccc acccacgagg agcatcgtgg aaaaagaaga cgttccaacc acgtcttcaa   4020
   agcaagtgga ttgatgtgac atctccactg acgtaaggga tgacgcacaa tcccactatc   4080
   cttcgcaaga cccttcctct atataaggaa gttcatttca tttggagagg acacgctcga   4140
   ggaattcggt accccatcac aagtttgtac aaaaaagcag gctgcggccg cttgctccct   4200
   taaaaaaaac catggcatgt cattacggaa tggcatgtca ttacggatgt ggacaacagc   4260
   aacagacttg tgcacctcat ctttgtcagc ttcaacctag agcatgtaga gttgtgaagg 4320
   ctgctactgc tgttactgta aatttctgtg ttccttattc tctcaaaatc ttcgattttg 4380
   ttttcgttcg atcccaattt cgtatatgtt ctttggttta gattctgtta atcttagatc 4440
   gaagacgatt ttctgggttt gatcgttaga tatcatctta attctcgatt agggtttcat 4500
   agatatcatc cgatttgttc aaataatttg agttttgtcg aataattact cttcgatttg 4560
   tgatttctat ctagatctgg tgttagtttc tagtttgtgc gatcgaattt gtcgattaat 4620
   ctgagttttt ctgattaaca ggctggagga tctcttcttg ttctctctgg acttactctc 4680
   gctggaactg ttatcgctct cactatcgct acacctcttc tcgttatctt ctctcctgtt 4740
   ctcgttcctg ctgtgatcac tatcttcctt ctcggagctg gatttcttgc ttctggtgga 4800
   tttggagttg ctgctctctc tgttctctct tggatctaca gatacctcac tggatgtaaa 4860
   catcctccag gtgctgattg tcttgagtct gcttgtaaga ctaagctcgc ttcttgtgct 4920
   agagagatga aggattgtag agcagagcaa ttctcttgtc agcctgttgc tggatgttct 4980
   cagacttctt aatgaacata tggtcctgct ttaatgagat atgcgagacg cctatgatcg 5040
   catgatattt gctttcaatt ctgttgtgca cgttgtaaaa aacctgagca tgtgtagctc 5100
   agatccttac cgccggtttc ggttcattct aatgaatata tcacccgtta ctatcgtatt 5160
   tttatgaata atattctccg ttcaatttac tgattgtacc ctactactta tatgtacaat 5220
   attaaaatga aaacaatata ttgtgctgaa taggtttata gcgacatcta tgatagagcg 5280
   ccacaataac aaacaattgc gttttattat tacaaatcca attttaaaaa aagcggcaga 5340
   accggtcaaa cctaaaagac tgattacata aatcttattc aaatttcaaa aggccccagg 5400
   ggctagtatc tacgacacac cgagcggcga actaataacg ttcactgaag ggaactccgg 5460
   ttccccgccg gcgcgcatgg gtgagattcc ttgaagttga gtattggccg tccgctctac 5520
   cgaaagttac gggcaccatt caacccggtc cagcacggcg gccgggtaac cgacttgctg 5580
   ccccgagaat tatgcagcat ttttttggtg tatgtgggcc ccaaatgaag tgcaggtcaa 5640
   accttgacag tgacgacaaa tcgttgggcg ggtccagggc gaattttgcg acaacatgtc 5700
   gaggctcagc aggacctgca ggcatgcaag ctagcttact agtgatgcat attctatagt 5760
   gtcacctaaa tcttcgacga attaattcca atcccacaaa aatctgagct taacagcaca 5820
   gttgctcctc tcagagcaga atcgggtatt caacaccctc atatcaacta ctacgttgtg 5880
   tataacggtc cacatgccgg tatatacgat gactggggtt gtacaaaggc ggcaacaaac 5940
   ggcgttcccg gagttgcaca caagaaattt gccactatta cagaggcaag agcagcagct 6000
   gacgcgtaca caacaagtca gcaaacagac aggttgaact tcatccccaa aggagaagct 6060
   caactcaagc ccaagagctt tgctaaggcc ctaacaagcc caccaaagca aaaagcccac 6120
   tggctcacgc taggaaccaa aaggcccagc agtgatccag ccccaaaaga gatctccttt 6180
   gccccggaga ttacaatgga cgatttcctc tatctttacg atctaggaag gaagttcgaa 6240
   ggtgaaggtg acgacactat gttcaccact gataatgaga aggttagcct cttcaatttc 6300
   agaaagaatg ctgacccaca gatggttaga gaggcctacg cagcaggtct catcaagacg 6360
   atctacccga gtaacaatct ccaggagatc aaataccttc ccaagaaggt taaagatgca 6420
   gtcaaaagat tcaggactaa ttgcatcaag aacacagaga aagacatatt tctcaagatc 6480
   agaagtacta ttccagtatg gacgattcaa ggcttgcttc ataaaccaag gcaagtaata 6540
   gagattggag tctctaaaaa ggtagttcct actgaatcta aggccatgca tggagtctaa 6600
   gattcaaatc gaggatctaa cagaactcgc cgtgaagact ggcgaacagt tcatacagag 6660
   tcttttacga ctcaatgaca agaagaaaat cttcgtcaac atggtggagc acgacactct 6720
   ggtctactcc aaaaatgtca aagatacagt ctcagaagac caaagggcta ttgagacttt 6780
   tcaacaaagg ataatttcgg gaaacctcct cggattccat tgcccagcta tctgtcactt 6840
   catcgaaagg acagtagaaa aggaaggtgg ctcctacaaa tgccatcatt gcgataaagg 6900
   aaaggctatc attcaagatc tctctgccga cagtggtccc aaagatggac ccccacccac 6960
   gaggagcatc gtggaaaaag aagacgttcc aaccacgtct tcaaagcaag tggattgatg 7020
   tgacatctcc actgacgtaa gggatgacgc acaatcccac tatccttcgc aagacccttc 7080
   ctctatataa ggaagttcat ttcatttgga gaggacacgg gatccttgct ccgttaaaaa 7140
   aaaccatggc tatcctcgat tctgctggtg ttactactgt gactgagaat ggtggtggag 7200
   agttcgttga tctcgataga ctcagaagaa gaaagtctag atctgtaaat ttctgtgttc 7260
   cttattctct caaaatcttc gattttgttt tcgttcgatc ccaatttcgt atatgttctt 7320
   tggtttagat tctgttaatc ttagatcgaa gacgattttc tgggtttgat cgttagatat 7380
   catcttaatt ctcgattagg gtttcataga tatcatccga tttgttcaaa taatttgagt 7440
   tttgtcgaat aattactctt cgatttgtga tttctatcta gatctggtgt tagtttctag 7500
   tttgtgcgat cgaatttgtc gattaatctg agtttttctg attaacagga ttcttctaac 7560
   ggacttctcc tctctggatc tgataacaac tctccttctg atgatgttgg tgctcctgct 7620
   gatgtgagag atagaatcga ttctgtggtg aacgatgatg ctcaaggtac tgctaacctc 7680
   gctggtgata ataacggtgg aggtgataac aatggtggag gaagaggtgg aggtgaaggt 7740
   agaggaaacg ctgatgctac tttcacttac agaccatctg tgcctgctca tagaagagct 7800
   agagagtctc ctctctcttc tgatgctatc ttcaagcagt ctcacgctgg acttttcaac 7860
   ctctgtgtgg tggttcttat cgctgtgaac tctagactca tcatcgagaa cctcatgaag 7920
   tacggatggc tcatcagaac tgatttctgg ttctcttcta gatctctcag agattggcct 7980
   cttttcatgt gctgcatctc actctcaatc ttccctctcg ctgcttttac tgttgagaag 8040
   ctcgtgctcc agaagtatat cgctgaacct gtggtgatct tcctccacat catcatcact 8100
   atgactgagg ttctctaccc tgtttacgtg actctcagat gcgattctgc tttcctctct 8160
   ggtgttactc ttatgctcct cacttgcatt gtgtggctta agctcgtgtc ttacgctcac 8220
   acttcttacg atatcagatc tctcgctaac gctgctgata aggctaaccc tgaagtgtct 8280
   tactacgtgt ctctcaagtc tctcgcttac ttcatggttg ctcctacact ttgttaccag 8340
   ccatcttacc ctagatctgc ttgcattaga aagggatggg tggcaagaca attcgctaag 8400
   ttggtgatct tcactggatt catgggattc atcatcgagc agtacatcaa ccctattgtg 8460
   agaaactcta agcaccctct caagggtgat cttctctacg ctatcgagag agttcttaag 8520
   ctctctgtgc ctaaccttta tgtgtggctc tgcatgttct actgtttctt ccacctctgg 8580
   cttaacatcc ttgctgagtt gctttgcttc ggagatagag agttctacaa ggattggtgg 8640
   aacgctaagt ctgttggaga ttattggaga atgtggaaca tgcctgtgca taagtggatg 8700
   gtgcgtcaca tctacttccc ttgcctcaga tctaagatcc ctaagactct cgctatcatt 8760
   atcgctttcc tcgtgtctgc tgttttccat gagttgtgta tcgctgttcc ttgcagactt 8820
   ttcaagcttt gggctttcct cggaatcatg ttccaggttc cactcgtgtt catcactaac 8880
   tacctccaag agagattcgg atctactgtt ggaaacatga ttttctggtt cattttctgc 8940
   atcttcggac agcctatgtg cgttctcctc tactaccacg atctcatgaa cagaaaggga 9000
   tctatgtctt aatgaaggat ccacccagct ttcttgtaca aagtggtgat gggttcgaaa 9060
   tcgataagct tggatcctct agagtcctgc tttaatgaga tatgcgagac gcctatgatc 9120
   gcatgatatt tgctttcaat tctgttgtgc acgttgtaaa aaacctgagc atgtgtagct 9180
   cagatcctta ccgccggttt cggttcattc taatgaatat atcacccgtt actatcgtat 9240
   ttttatgaat aatattctcc gttcaattta ctgattgtac cctactactt atatgtacaa 9300
   tattaaaatg aaaacaatat attgtgctga ataggtttat agcgacatct atgatagagc 9360
   gccacaataa caaacaattg cgttttatta ttacaaatcc aattttaaaa aaagcggcag 9420
   aaccggtcaa acctaaaaga ctgattacat aaatcttatt caaatttcaa aaggccccag 9480
   gggctagtat ctacgacaca ccgagcggcg aactaataac gttcactgaa gggaactccg 9540
   gttccccgcc ggcgcgcatg ggtgagattc cttgaagttg agtattggcc gtccgctcta 9600
   ccgaaagtta cgggcaccat tcaacccggt ccagcacggc ggccgggtaa ccgacttgct 9660
   gccccgagaa ttatgcagca tttttttggt gtatgtgggc cccaaatgaa gtgcaggtca 9720
   aaccttgaca gtgacgacaa atcgttgggc gggtccaggg cgaattttgc gacaacatgt 9780
   cgaggctcag caggacctgc aggcatgcaa gctagcttac tagtgatgca tattctatag 9840
   tgtcacctaa atctgcggcc gcctgcaggt cgatatggga gagctcccaa cgcgttggat 9900
   gcatagcttg agtattctat agtgtcacct aaatagcttg gcgtaatcat ggtcatagct 9960
   gtttcctgtg tgaaattgtt atccgctcac aattccacac aacatacgag ccggaagcat 10020
   aaagtgtaaa gcctggggtg cctaatgagt gagctaactc acattaattg cgttgcgctc 10080
   actgcccgct ttccagtcgg gaaacctgtc gtgccagctg cattaatgaa tcggccaacg 10140
   cgcggggaga ggcggtttgc gtattggggc tgagtggctc cttcaacgtt gcggttctgt 10200
   cagttccaaa cgtaaaacgg cttgtcccgc gtcatcggcg ggggtcataa cgtgactccc 10260
   ttaattctcc gctcatgatc agattgtcgt ttcccgcctt cagtttaaac tatcagtgtt 10320
   tgacaggata tattggcggg taaacctaag agaaaagagc gtttattaga ataatcggat 10380
   atttaaaagg gcgtgaaaag gtttatccgt tcgtccattt gtatgtgcat gccaaccaca 10440
   gggttcccct cgggagtgct tggcattccg tgcgataatg acttctgttc aaccacccaa 10500
   acgtcggaaa gcctgacgac ggagcagcat tccaaaaaga tcccttggct cgtctgggtc 10560
   ggctagaagg tcgagtgggc tgctgtggct tgatccctca acgcggtcgc ggacgtagcg 10620
   cagcgccgaa aaatcctcga tcgcaaatcc gacgctgtcg aaaatcgtga tctgcttgtc 10680
   gctctttcgg ccgacgtcct ggccagtcat cacgcgccaa agttccgtca caggatgatc 10740
   tggcgcgagt tgctggatct cgccttcaat ccgggtctgt ggcgggaact ccacgaaaat 10800
   atccgaacgc agcaagatgt cgacggatct tttccgctgc ataaccctgc ttcggggtca 10860
   ttatagcgat tttttcggta tatccatcct ttttcgcacg atatacagga ttttgccaaa 10920
   gggttcgtgt agactttcct tggtgtatcc aacggcgtca gccgggcagg ataggtgaag 10980
   taggcccacc cgcgagcggg tgttccttct tcactgtccc ttattcgcac ctggcggtgc 11040
   tcaacgggaa tcctgctctg cgaggctggc cggctaccgc cggcgtaaca gatgagggca 11100
   agcggatggc tgatgaaacc aagccaacca ggggtgatgc tgccaactta ctgatttagt 11160
   gtatgatggt gtttttgagg tgctccagtg gcttctgttt ctatcagctg tccctcctgt 11220
   tcagctactg acggggtggt gcgtaacggc aaaagcaccg ccggacatca gcgctatctc 11280
   tgctctcact gccgtaaaac atggcaactg cagttcactt acaccgcttc tcaacccggt 11340
   acgcaccaga aaatcattga tatggccatg aatggcgttg gatgccgggc aacagcccgc 11400
   attatgggcg ttggcctcaa cacgatttta cgtcacttaa aaaactcagg ccgcagtcgg 11460
   taacctcgcg catacagccg ggcagtgacg tcatcgtctg cgcggaaatg gacgaacagt 11520
   ggggctatgt cggggctaaa tcgcgccagc gctggctgtt ttacgcgtat gacagtctcc 11580
   ggaagacggt tgttgcgcac gtattcggtg aacgcactat ggcgacgctg gggcgtctta 11640
   tgagcctgct gtcacccttt gacgtggtga tatggatgac ggatggctgg ccgctgtatg 11700
   aatcccgcct gaagggaaag ctgcacgtaa tcagcaagcg atatacgcag cgaattgagc 11760
   ggcataacct gaatctgagg cagcacctgg cacggctggg acggaagtcg ctgtcgttct 11820
   caaaatcggt ggagctgcat gacaaagtca tcgggcatta tctgaacata aaacactatc 11880
   aataagttgg agtcattacc caaccaggaa gggcagccca cctatcaagg tgtactgcct 11940
   tccagacgaa cgaagagcga ttgaggaaaa ggcggcggcg gccggcatga gcctgtcggc 12000
   ctacctgctg gccgtcggcc agggctacaa aatcacgggc gtcgtggact atgagcacgt 12060
   ccgcgagctg gcccgcatca atggcgacct gggccgcctg ggcggcctgc tgaaactctg 12120
   gctcaccgac gacccgcgca cggcgcggtt cggtgatgcc acgatcctcg ccctgctggc 12180
   gaagatcgaa gagaagcagg acgagcttgg caaggtcatg atgggcgtgg tccgcccgag 12240
   ggcagagcca tgactttttt agccgctaaa acggccgggg ggtgcgcgtg attgccaagc 12300
   acgtccccat gcgctccatc aagaagagcg acttcgcgga gctggtattc gtgcagggca 12360
   agattcggaa taccaagtac gagaaggacg gccagacggt ctacgggacc gacttcattg 12420
   ccgataaggt ggattatctg gacaccaagg caccaggcgg gtcaaatcag gaataagggc     12480
   acattgcccc ggcgtgagtc ggggcaatcc cgcaaggagg gtgaatgaat cggacgtttg     12540
   accggaaggc atacaggcaa gaactgatcg acgcggggtt ttccgccgag gatgccgaaa     12600
   ccatcgcaag ccgcaccgtc atgcgtgcgc cccgcgaaac cttccagtcc gtcggctcga     12660
   tggtccagca agctacggcc aagatcgagc gcgacagcgt gcaactggct ccccctgccc      12720
   tgcccgcgcc atcggccgcc gtggagcgtt cgcgtcgtct cgaacaggag gcggcaggtt     12780
   tggcgaagtc gatgaccatc gacacgcgag gaactatgac gaccaagaag cgaaaaaccg     12840
   ccggcgagga cctggcaaaa caggtcagcg aggccaagca ggccgcgttg ctgaaacaca     12900
   cgaagcagca gatcaaggaa atgcagcttt ccttgttcga tattgcgccg tggccggaca     12960
   cgatgcgagc gatgccaaac gacacggccc gctctgccct gttcaccacg cgcaacaaga     13020
   aaatcccgcg cgaggcgctg caaaacaagg tcattttcca cgtcaacaag gacgtgaaga     13080
   tcacctacac cggcgtcgag ctgcgggccg acgatgacga actggtgtgg cagcaggtgt     13140
   tggagtacgc gaagcgcacc cctatcggcg agccgatcac cttcacgttc tacgagcttt     13200
   gccaggacct gggctggtcg atcaatggcc ggtattacac gaaggccgag gaatgcctgt     13260
   cgcgcctaca ggcgacggcg atgggcttca cgtccgaccg cgttgggcac ctggaatcgg     13320
   tgtcgctgct gcaccgcttc cgcgtcctgg accgtggcaa gaaaacgtcc cgttgccagg     13380
   tcctgatcga cgaggaaatc gtcgtgctgt ttgctggcga ccactacacg aaattcatat     13440
   gggagaagta ccgcaagctg tcgccgacgg cccgacggat gttcgactat ttcagctcgc     13500
   accgggagcc gtacccgctc aagctggaaa ccttccgcct catgtgcgga tcggattcca     13560
   cccgcgtgaa gaagtggcgc gagcaggtcg gcgaagcctg cgaagagttg cgaggcagcg     13620
   gcctggtgga acacgcctgg gtcaatgatg acctggtgca ttgcaaacgc tagggccttg     13680
   tggggtcagt tccggctggg ggttcagcag ccagcgcttt actggcattt caggaacaag     13740
   cgggcactgc tcgacgcact tgcttcgctc agtatcgctc gggacgcacg gcgcgctcta     13800
   cgaactgccg ataaacagag gattaaaatt gacaattgtg attaaggctc agattcgacg     13860
   gcttggagcg gccgacgtgc aggatttccg cgagatccga ttgtcggccc tgaagaaagc     13920
   tccagagatg ttcgggtccg tttacgagca cgaggagaaa aagcccatgg aggcgttcgc     13980
   tgaacggttg cgagatgccg tggcattcgg cgcctacatc gacggcgaga tcattgggct     14040
   gtcggtcttc aaacaggagg acggccccaa ggacgctcac aaggcgcatc tgtccggcgt     14100
   tttcgtggag cccgaacagc gaggccgagg ggtcgccggt atgctgctgc gggcgttgcc     14160
   ggcgggttta ttgctcgtga tgatcgtccg acagattcca acgggaatct ggtggatgcg     14220
   catcttcatc ctcggcgcac ttaatatttc gctattctgg agcttgttgt ttatttcggt     14280
   ctaccgcctg ccgggcgggg tcgcggcgac ggtaggcgct gtgcagccgc tgatggtcgt     14340
   gttcatctct gccgctctgc taggtagccc gatacgattg atggcggtcc tgggggctat     14400
   ttgcggaact gcgggcgtgg cgctgttggt gttgacacca aacgcagcgc tagatcctgt     14460
   cggcgtcgca gcgggcctgg cgggggcggt ttccatggcg ttcggaaccg tgctgacccg 14520
   caagtggcaa cctcccgtgc ctctgctcac ctttaccgcc tggcaactgg cggccggagg 14580
   acttctgctc gttccagtag ctttagtgtt tgatccgcca atcccgatgc ctacaggaac 14640
   caatgttctc ggcctggcgt ggctcggcct gatcggagcg ggtttaacct acttcctttg 14700
   gttccggggg atctcgcgac tcgaacctac agttgtttcc ttactgggct ttctcagccg 14760
   ggatggcgct aagaagctat tgccgccgat cttcatatgc ggtgtgaaat accgcacaga 14820
   tgcgtaagga gaaaataccg catcaggcgc tcttccgctt cctcgctcac tgactcgctg 14880
   cgctcggtcg ttcggctgcg gcgagcggta tcagctcact caaaggcggt aatacggtta 14940
   tccacagaat caggggataa cgcaggaaag aacatgtgag caaaaggcca gcaaaaggcc 15000
   aggaaccgta aaaaggccgc gttgctggcg tttttccata ggctccgccc ccctgacgag 15060
   catcacaaaa atcgacgctc aagtcagagg tggcgaaacc cgacaggact ataaagatac 15120
   caggcgtttc cccctggaag ctccctcgtg cgctctcctg ttccgaccct gccgcttacc 15180
   ggatacctgt ccgcctttct cccttcggga agcgtggcgc tttctcaatg ctcacgctgt 15240
   aggtatctca gttcggtgta ggtcgttcgc tccaagctgg gctgtgtgca cgaacccccc 15300
   gttcagcccg accgctgcgc cttatccggt aactatcgtc ttgagtccaa cccggtaaga 15360
   cacgacttat cgccactggc agcagccact ggtaacagga ttagcagagc gaggtatgta 15420
   ggcggtgcta cagagttctt gaagtggtgg cctaactacg gctacactag aaggacagta 15480
   tttggtatct gcgctctgct gaagccagtt accttcggaa aaagagttgg tagctcttga 15540
   tccggcaaac aaaccaccgc tggtagcggt ggtttttttg tttgcaagca gcagattacg 15600
   cgcagaaaaa aaggatatca agaagatcct ttgatctttt ctacggggtc tgacgctcag 15660
   tggaacgaaa actcacgtta agggattttg gtcatgagat tatcaaaaag gatcttcacc 15720
   tagatccttt taaattaaaa atgaagtttt aaatcaatct aaagtatata tgagtaaact 15780
   tggtctgaca gttaccaatg cttaatcagt gaggcaccta tctcagcgat ctgtctattt 15840
   cgttcatcca tagttgcctg actccccgtc gtgtagataa ctacgatacg ggagggctta 15900
   ccatctggcc ccagtgctgc aatgataccg cgagacccac gctcaccggc tccagattta 15960
   tcagcaataa accagccagc cggaagggcc gagcgcagaa gtggtcctgc aactttatcc 16020
   gcctccatcc agtctattaa acaagtggca gcaacggatt cgcaaacctg tcacgccttt 16080
   tgtgccaaaa gccgcgccag gtttgcgatc cgctgtgcca ggcgttaggc gtcatatgaa 16140
   gatttcggtg atccctgagc aggtggcgga aacattggat gctgagaacc atttcattgt 16200
   tcgtgaagtg ttcgatgtgc acctatccga ccaaggcttt gaactatcta ccagaagtgt 16260
   gagcccctac cggaaggatt acatctcgga tgatgactct gatgaagact ctgcttgcta 16320
   tggcgcattc atcgaccaag agcttgtcgg gaagattgaa ctcaactcaa catggaacga 16380
   tctagcctct atcgaacaca ttgttgtgtc gcacacgcac cgaggcaaag gagtcgcgca 16440
   cagtctcatc gaatttgcga aaaagtgggc actaagcaga cagctccttg gcatacgatt 16500
   agagacacaa acgaacaatg tacctgcctg caatttgtac gcaaaatgtg gctttactct 16560
   cggcggcatt gacctgttca cgtataaaac tagacctcaa gtctcgaacg aaacagcgat 16620
   gtactggtac tggttctcgg gagcacagga tgacgcctaa caattcattc aagccgacac 16680
   cgcttcgcgg cgcggcttaa ttcaggagtt aaacatcatg agggaagcgg tgatcgccga 16740
   agtatcgact caactatcag aggtagttgg cgtcatcgag cgccatctcg aaccgacgtt 16800
   gctggccgta catttgtacg gctccgcagt ggatggcggc ctgaagccac acagtgatat 16860
   tgatttgctg gttacggtga ccgtaaggct tgatgaaaca acgcggcgag ctttgatcaa 16920
   cgaccttttg gaaacttcgg cttcccctgg agagagcgag attctccgcg ctgtagaagt 16980
   caccattgtt gtgcacgacg acatcattcc gtggcgttat ccagctaagc gcgaactgca 17040
   atttggagaa tggcagcgca atgacattct tgcaggtatc ttcgagccag ccacgatcga 17100
   cattgatctg gctatcttgc tgacaaaagc aagagaacat agcgttgcct tggtaggtcc 17160
   agcggcggag gaactctttg atccggttcc tgaacaggat ctatttgagg cgctaaatga 17220
   aaccttaacg ctatggaact cgccgcccga ctgggctggc gatgagcgaa atgtagtgct 17280
   tacgttgtcc cgcatttggt acagcgcagt aaccggcaaa atcgcgccga aggatgtcgc 17340
   tgccgactgg gcaatggagc gcctgccggc ccagtatcag cccgtcatac ttgaagctag 17400
   gcaggcttat cttggacaag aagatcgctt ggcctcgcgc gcagatcagt tggaagaatt 17460
   tgttcactac gtgaaaggcg agatcaccaa ggtagtcggc aaataatgtc taacaattcg 17520
   ttcaagccga cgccgcttcg cggcgcggct taactcaagc gttagagagc tggggaagac 17580
   tatgcgcgat ctgttgaagg tggttctaag cctcgtactt gcgatggcat cggggcaggc 17640
   acttgctgac ctgccaattg ttttagtgga tgaagctcgt cttccctatg actactcccc 17700
   atccaactac gacatttctc caagcaacta cgacaactcc ataagcaatt acgacaatag 17760
   tccatcaaat tacgacaact ctgagagcaa ctacgataat agttcatcca attacgacaa 17820
   tagtcgcaac ggaaatcgta ggcttatata tagcgcaaat gggtctcgca ctttcgccgg 17880
   ctactacgtc attgccaaca atgggacaac gaacttcttt tccacatctg gcaaaaggat 17940
   gttctacacc ccaaaagggg ggcgcggcgt ctatggcggc aaagatggga gcttctgcgg 18000
   ggcattggtc gtcataaatg gccaattttc gcttgccctg acagataacg gcctgaagat 18060
   catgtatcta agcaactagc ctgctctcta ataaaatgtt aggagcttgg ctgccatttt 18120
   tggggtgagg ccgttcgcgg ccgaggggcg cagcccctgg ggggatggga ggcccgcgtt 18180
   agcgggccgg gagggttcga gaaggggggg cacccccctt cggcgtgcgc ggtcacgcgc 18240
   cagggcgcag ccctggttaa aaacaaggtt tataaatatt ggtttaaaag caggttaaaa 18300
   gacaggttag cggtggccga aaaacgggcg gaaacccttg caaatgctgg attttctgcc 18360
   tgtggacagc ccctcaaatg tcaataggtg cgcccctcat ctgtcagcac tctgcccctc 18420
   aagtgtcaag gatcgcgccc ctcatctgtc agtagtcgcg cccctcaagt gtcaataccg 18480
   cagggcactt atccccaggc ttgtccacat catctgtggg aaactcgcgt aaaatcaggc 18540
   gttttcgccg atttgcgagg ctggccagct ccacgtcgcc ggccgaaatc gagcctgccc 18600
   ctcatctgtc aacgccgcgc cgggtgagtc ggcccctcaa gtgtcaacgt ccgcccctca 18660
   tctgtcagtg agggccaagt tttccgcgag gtatccacaa cgccggcggc cggccgcggt 18720
   gtctcgcaca cggcttcgac ggcgtttctg gcgcgtttgc agggccatag acggccgcca 18780
   gcccagcggc gagggcaacc agcccggtga gcgtcggaaa ggg 18823
<210> 33
   <211> 520
   <212> PRT
   <213> Artificial Sequence
<220>
   <223> Artificial
<400> 33
   Met Ala Ile Leu Asp Ser Ala Gly Val Thr Thr Val Thr Glu Asn Gly
   1               5                   10                  15
   Gly Gly Glu Phe Val Asp Leu Asp Arg Leu Arg Arg Arg Lys Ser Arg
               20                  25                  30
   Ser Asp Ser Ser Asn Gly Leu Leu Leu Ser Gly Ser Asp Asn Asn Ser
           35                  40                  45
   Pro Ser Asp Asp Val Gly Ala Pro Ala Asp Val Arg Asp Arg Ile Asp
       50                  55                  60
   Ser Val Val Asn Asp Asp Ala Gln Gly Thr Ala Asn Leu Ala Gly Asp
   65                  70                  75                  80
   Asn Asn Gly Gly Gly Asp Asn Asn Gly Gly Gly Arg Gly Gly Gly Glu
                   85                  90                  95
   Gly Arg Gly Asn Ala Asp Ala Thr Phe Thr Tyr Arg Pro Ser Val Pro
               100                 105                 110
   Ala His Arg Arg Ala Arg Glu Ser Pro Leu Ser Ser Asp Ala Ile Phe
           115                 120                 125
   Lys Gln Ser His Ala Gly Leu Phe Asn Leu Cys Val Val Val Leu Ile
       130                 135                 140
   Ala Val Asn Ser Arg Leu Ile Ile Glu Asn Leu Met Lys Tyr Gly Trp
   145                 150                 155                 160
   Leu Ile Arg Thr Asp Phe Trp Phe Ser Ser Arg Ser Leu Arg Asp Trp
                   165                 170                 175
   Pro Leu Phe Met Cys Cys Ile Ser Leu Ser Ile Phe Pro Leu Ala Ala
               180                 185                 190
   Phe Thr Val Glu Lys Leu Val Leu Gln Lys Tyr Ile Ala Glu Pro Val
           195                 200                 205
   Val Ile Phe Leu His Ile Ile Ile Thr Met Thr Glu Val Leu Tyr Pro
       210                 215                 220
   Val Tyr Val Thr Leu Arg Cys Asp Ser Ala Phe Leu Ser Gly Val Thr
   225                 230                 235                 240
   Leu Met Leu Leu Thr Cys Ile Val Trp Leu Lys Leu Val Ser Tyr Ala
                   245                 250                 255
   His Thr Ser Tyr Asp Ile Arg Ser Leu Ala Asn Ala Ala Asp Lys Ala
               260                 265                 270
   Asn Pro Glu Val Ser Tyr Tyr Val Ser Leu Lys Ser Leu Ala Tyr Phe
           275                 280                 285
   Met Val Ala Pro Thr Leu Cys Tyr Gln Pro Ser Tyr Pro Arg Ser Ala
       290                 295                 300
   Cys Ile Arg Lys Gly Trp Val Ala Arg Gln Phe Ala Lys Leu Val Ile
   305                 310                 315                 320
   Phe Thr Gly Phe Met Gly Phe Ile Ile Glu Gln Tyr Ile Asn Pro Ile
                   325                 330                 335
   Val Arg Asn Ser Lys His Pro Leu Lys Gly Asp Leu Leu Tyr Ala Ile
               340                 345                 350
   Glu Arg Val Leu Lys Leu Ser Val Pro Asn Leu Tyr Val Trp Leu Cys
           355                 360                 365
   Met Phe Tyr Cys Phe Phe His Leu Trp Leu Asn Ile Leu Ala Glu Leu
       370                 375                 380
   Leu Cys Phe Gly Asp Arg Glu Phe Tyr Lys Asp Trp Trp Asn Ala Lys
   385                 390                 395                 400
   Ser Val Gly Asp Tyr Trp Arg Met Trp Asn Met Pro Val His Lys Trp
                   405                 410                 415
   Met Val Arg His Ile Tyr Phe Pro Cys Leu Arg Ser Lys Ile Pro Lys
               420                 425                 430
   Thr Leu Ala Ile Ile Ile Ala Phe Leu Val Ser Ala Val Phe His Glu
           435                 440                 445
   Leu Cys Ile Ala Val Pro Cys Arg Leu Phe Lys Leu Trp Ala Phe Leu
       450                 455                 460
   Gly Ile Met Phe Gln Val Pro Leu Val Phe Ile Thr Asn Tyr Leu Gln
   465 470 475 480
   Glu Arg Phe Gly Ser Thr Val Gly Asn Met Ile Phe Trp Phe Ile Phe
   485 490 495
   Cys Ile Phe Gly Gln Pro Met Cys Val Leu Leu Tyr Tyr His Asp Leu
   500 505 510
   Met Asn Arg Lys Gly Ser Met Ser
   515 520
<210> 34
   <211> 501
   <212> DNA
   <213> S. indicum
<400> 34
   atggcggacc gcgaccgtcc acacccccac caaattcaag tccaccctca acatccgcac 60
   cgctatgaag gtggcgtcaa gtctctcctc cctcaaaagg gcccctccac cacccagatt 120
   ctcgccataa tcaccctcct tcccatcagc ggcacgcttc tttgcctagc tgggatcacg 180
   ctcgtcggga ccctcatcgg acttgcagtc gccaccccag tcttcgtgat cttcagccct 240
   gttctggttc ccgcagccat actgatagcc ggcgcggtca cggcgttttt gacgtccggg 300
   gcttttgggc tgacggggct ttcgtcgctt tcttgggttc tgaattcatt cagacgggcg 360
   acggggcagg ggccgttgga gtacgcgaag cgaggcgtgc aggaggggac tttgtatgtg 420
   ggagagaaga cgaagcaagc gggcgaagcg attaagagca cagccaagga aggagggcga 480
   gaagggactg cacggacttg a 501
<210> 35
   <211> 166
   <212> PRT
   <213> S. indicum
<400> 35
   Met Ala Asp Arg Asp Arg Pro His Pro His Gln Ile Gln Val His Pro
   1 5 10 15
   Gln His Pro His Arg Tyr Glu Gly Gly Val Lys Ser Leu Leu Pro Gln
   20 25 30
   Lys Gly Pro Ser Thr Thr Gln Ile Leu Ala Ile Ile Thr Leu Leu Pro
   35 40 45
   Ile Ser Gly Thr Leu Leu Cys Leu Ala Gly Ile Thr Leu Val Gly Thr
   50 55 60
   Leu Ile Gly Leu Ala Val Ala Thr Pro Val Phe Val Ile Phe Ser Pro
   65 70 75 80
   Val Leu Val Pro Ala Ala Ile Leu Ile Ala Gly Ala Val Thr Ala Phe
   85 90 95
   Leu Thr Ser Gly Ala Phe Gly Leu Thr Gly Leu Ser Ser Leu Ser Trp
   100 105 110
   Val Leu Asn Ser Phe Arg Arg Ala Thr Gly Gln Gly Pro Leu Glu Tyr
   115 120 125
   Ala Lys Arg Gly Val Gln Glu Gly Thr Leu Tyr Val Gly Glu Lys Thr
   130 135 140
   Lys Gln Ala Gly Glu Ala Ile Lys Ser Thr Ala Lys Glu Gly Gly Arg
   145 150 155 160
   Glu Gly Thr Ala Arg Thr
   165
<210> 36
   <211> 559
   <212> DNA
   <213> S. indicum
<400> 36
   ggcacgagcg ccgtccccat ggcggacgaa ccccacgacc agcgccccac cgacgtcatc      60
   aagagctacc tccccgaaaa gggtccctcc acctctcaag tcctcgccgt cgtgaccctc     120
   ttccccctcg gcgccgtcct cctctgccta gccggtctca ttcttaccgg gaccatcatc    180
   ggcctcgccg tcgccacccc gctcttcgtc atcttcagcc ccatcttggt ccccgccgcc     240
   ctaaccatcg ccctagccgt caccggtttc ttgacctccg gagctttcgg catcaccgcc     300
   ctgtcctcga tttcgtggtt gctgaactac gttaggcgaa tgcgggggag cttgccagag     360
   cagctggatc atgcacggcg gcgcgtgcag gagacggtgg gccagaagac aagggaggcg     420
   gggcagagaa gccaagatgt aataagaccg tgaggttttt ggatattaga tgttggttaa     480
   tttgtgtgtt taatgtatat atgaggggtt gaataagtta ataaaattgc ggatttggta     540
   caaaaaaaaa aaaaaaaaa                                                  559
<210> 37
   <211> 144
   <212> PRT
   <213> S. indicum
<400> 37
   Met Ala Asp Glu Pro His Asp Gln Arg Pro Thr Asp Val Ile Lys Ser
   1               5                   10                  15
   Tyr Leu Pro Glu Lys Gly Pro Ser Thr Ser Gln Val Leu Ala Val Val
               20                  25                  30
   Thr Leu Phe Pro Leu Gly Ala Val Leu Leu Cys Leu Ala Gly Leu Ile
           35                  40                  45
   Leu Thr Gly Thr Ile Ile Gly Leu Ala Val Ala Thr Pro Leu Phe Val
       50                  55                  60
   Ile Phe Ser Pro Ile Leu Val Pro Ala Ala Leu Thr Ile Ala Leu Ala
   65                  70                  75                  80
   Val Thr Gly Phe Leu Thr Ser Gly Ala Phe Gly Ile Thr Ala Leu Ser
                   85                  90                  95
   Ser Ile Ser Trp Leu Leu Asn Tyr Val Arg Arg Met Arg Gly Ser Leu
               100                 105                 110
   Pro Glu Gln Leu Asp His Ala Arg Arg Arg Val Gln Glu Thr Val Gly
           115                 120                 125
   Gln Lys Thr Arg Glu Ala Gly Gln Arg Ser Gln Asp Val Ile Arg Pro
       130                 135                 140
<210> 38
   <211> 1800
   <212> DNA
   <213> A. thaliana
<400> 38
   ccatggctat acccaacctc ggtcttggtc acaccaggaa ctctctggta agctagctcc      60
   actccccaga aacaaccggc gccaaattgc cggaattgct gacctgaaga cggaacatca     120
   tcgtcgggtc cttgggcgat tgcggcggaa gatgggtcag cttgggcttg aggacgagac     180
   ccgaatcgag tctgttgaaa ggttgttcat tgggatttgt atacggagat tggtcgtcga     240
   gaggtttgag ggaaaggaca aatgggtttg gctctggaga aagagagtgc ggctttagag     300
   agagaattga gaggtttaga gagagatgcg gcggcgatga cgggaggaga gacgacgagg     360
   acctgcatta tcaaagcagt gacgtggtga aatttggaac ttttaagagg cagatagatt     420
   tattatttgt atccattttc ttcattgttc tagaatgtcg cggaacaaat tttaaaacta     480
   aatcctaaat ttttctaatt ttgttgccaa tagtggatat gtgggccgta tagaaggaat     540
   ctattgaagg cccaaaccca tactgacgag cccaaaggtt cgttttgcgt tttatgtttc     600
   ggttcgatgc caacgccaca ttctgagcta ggcaaaaaac aaacgtgtct ttgaatagac     660
   tcctctcgtt aacacatgca gcggctgcat ggtgacgcca ttaacacgtg gcctacaatt     720
   gcatgatgtc tccattgaca cgtgacttct cgtctccttt cttaatatat ctaacaaaca     780
   ctcctacctc ttccaaaata tatacacatc tttttgatca atctctcatt caaaatctca     840
   ttctctctag taaacaagaa caaaaaaatg gcggatacag ctagaggaac ccatcacgat     900
   atcatcggca gagaccagta cccgatgatg ggccgagacc gagaccagta ccagatgtcc     960
   ggacgaggat ctgactactc caagtctagg cagattgcta aagctgcaac tgctgtcaca    1020
   gctggtggtt ccctccttgt tctctccagc cttacccttg ttggaactgt catagctttg    1080
   actgttgcaa cacctctgct cgttatcttc agcccaatcc ttgtcccggc tctcatcaca 1140
   gttgcactcc tcatcaccgg ttttctttcc tctggagggt ttggcattgc cgctataacc 1200
   gttttctctt ggatttacaa gtaagcacac atttatcatc ttacttcata attttgtgca 1260
   atatgtgcat gcatgtgttg agccagtagc tttggatcaa tttttttggt cgaataacaa 1320
   atgtaacaat aagaaattgc aaattctagg gaacatttgg ttaactaaat acgaaatttg 1380
   acctagctag cttgaatgtg tctgtgtata tcatctatat aggtaaaatg cttggtatga 1440
   tacctattga ttgtgaatag gtacgcaacg ggagagcacc cacagggatc agacaagttg 1500
   gacagtgcaa ggatgaagtt gggaagcaaa gctcaggatc tgaaagacag agctcagtac 1560
   tacggacagc aacatactgg tggggaacat gaccgtgacc gtactcgtgg tggccagcac 1620
   actacttaag ttaccccact gatgtcatcg tcatagtcca ataactccaa tgtcggggag 1680
   ttagtttatg aggaataaag tgtttagaat ttgatcaggg ggagataata aaagccgagt 1740
   ttgaatcttt ttgttataag taatgtttat gtgtgtttct atatgttgtc aaatggtacc 1800
<210> 39
   <211> 173
   <212> PRT
   <213> A. thaliana
<400> 39
   Met Ala Asp Thr Ala Arg Gly Thr His His Asp Ile Ile Gly Arg Asp
   1 5 10 15
   Gln Tyr Pro Met Met Gly Arg Asp Arg Asp Gln Tyr Gln Met Ser Gly
   20 25 30
   Arg Gly Ser Asp Tyr Ser Lys Ser Arg Gln Ile Ala Lys Ala Ala Thr
   35 40 45
   Ala Val Thr Ala Gly Gly Ser Leu Leu Val Leu Ser Ser Leu Thr Leu
   50 55 60
   Val Gly Thr Val Ile Ala Leu Thr Val Ala Thr Pro Leu Leu Val Ile
   65 70 75 80
   Phe Ser Pro Ile Leu Val Pro Ala Leu Ile Thr Val Ala Leu Leu Ile
   85 90 95
   Thr Gly Phe Leu Ser Ser Gly Gly Phe Gly Ile Ala Ala Ile Thr Val
   100 105 110
   Phe Ser Trp Ile Tyr Lys Tyr Ala Thr Gly Glu His Pro Gln Gly Ser
   115 120 125
   Asp Lys Leu Asp Ser Ala Arg Met Lys Leu Gly Ser Lys Ala Gln Asp
   130 135 140
   Leu Lys Asp Arg Ala Gln Tyr Tyr Gly Gln Gln His Thr Gly Gly Glu
   145 150 155 160
   His Asp Arg Asp Arg Thr Arg Gly Gly Gln His Thr Thr
   165 170
<210> 40
   <211> 450
   <212> DNA
   <213> A. thaliana
<400> 40
   atggcggatc atcaacaaca tcagcaacaa caacaaccaa taatgaggag tctccatgaa 60
   tcatcaccat cgactcggca gatagtgaga ttcgtaacgg cagctacgat cggcctatca 120
   ctcctcgtgc tctcaggact aacactaacc ggaacggtga tcggtttgat cgtagcgacg 180
   ccgttgatgg ttctgttcag cccggtgttg gtaccggcag tgataacgat agggcttctg 240
   acgatgggat tcctattctc cggtggttgt ggggtggcag cagctacggc gttaacgtgg 300
   atttataagt acgttaccgg aaaacacccg atgggagcgg ataaggtgga ttacgcgagg 360
   atgaggatag cggagaaagc caaagagttg ggacattata cgcactcgca gccacaacaa 420
   acacaccaaa ccacaacaac tactcattag 450
<210> 41
   <211> 149
   <212> PRT
   <213> A. thaliana
<400> 41
   Met Ala Asp His Gln Gln His Gln Gln Gln Gln Gln Pro Ile Met Arg
   1 5 10 15
   Ser Leu His Glu Ser Ser Pro Ser Thr Arg Gln Ile Val Arg Phe Val
   20 25 30
   Thr Ala Ala Thr Ile Gly Leu Ser Leu Leu Val Leu Ser Gly Leu Thr
   35 40 45
   Leu Thr Gly Thr Val Ile Gly Leu Ile Val Ala Thr Pro Leu Met Val
   50 55 60
   Leu Phe Ser Pro Val Leu Val Pro Ala Val Ile Thr Ile Gly Leu Leu
   65 70 75 80
   Thr Met Gly Phe Leu Phe Ser Gly Gly Cys Gly Val Ala Ala Ala Thr
   85 90 95
   Ala Leu Thr Trp Ile Tyr Lys Tyr Val Thr Gly Lys His Pro Met Gly
   100 105 110
   Ala Asp Lys Val Asp Tyr Ala Arg Met Arg Ile Ala Glu Lys Ala Lys
   115 120 125
   Glu Leu Gly His Tyr Thr His Ser Gln Pro Gln Gln Thr His Gln Thr
   130 135 140
   Thr Thr Thr Thr His
   145
<210> 42
   <211> 625
   <212> DNA
   <213> H. annuus
<400> 42
   actaccacca cttacgaccg tcatttcacc accacccaac cccactaccg ccaagacgac      60
   aggtcccgct acgaccagca aacccattcc cagtccacca gcaggacact cgccatcatc     120
   gccctacttc ctgtcggcgg aatcttactc ggcttagccg ctctcacatt catcgggacg     180
   cttatcgggc tcgccctcgc caccccgctt ttcgtcatat tcagcccgat catcgtgccg     240
   gccgttctaa caatcgggct tgctgttaca ggctttttgg cgtcggggac gttcgggttg     300
   acgggtttga gctcattgtc gtatttgttc aatatggtta ggcagacggc tgggtcggtg     360
   cccgagtcct tggattatgt taaggggacg ttgcaggatg ccggtgagta tgccgggcag     420
   aagacgaagg atttcgggca gaagattcag agcacggctc atgagatggg tgatcagggg     480
   caggttggtg ttcatgctca agttggtggc gggaaagaag ggcgaaaaag cggtgatcgg     540
   acttgaggat tcaaggttga tattgtggaa taataatgtt gatgtaagtt tttagtgtta     600
   tcaaagcttt gtttgtttgt ttgta                                           625
<210> 43
   <211> 181
   <212> PRT
   <213> H. annuus
<400> 43
   Thr Thr Thr Thr Tyr Asp Arg His Phe Thr Thr Thr Gln Pro His Tyr
   1               5                   10                  15
   Arg Gln Asp Asp Arg Ser Arg Tyr Asp Gln Gln Thr His Ser Gln Ser
               20                  25                  30
   Thr Ser Arg Thr Leu Ala Ile Ile Ala Leu Leu Pro Val Gly Gly Ile
           35                  40                  45
   Leu Leu Gly Leu Ala Ala Leu Thr Phe Ile Gly Thr Leu Ile Gly Leu
       50                  55                  60
   Ala Leu Ala Thr Pro Leu Phe Val Ile Phe Ser Pro Ile Ile Val Pro
   65                  70                  75                  80
   Ala Val Leu Thr Ile Gly Leu Ala Val Thr Gly Phe Leu Ala Ser Gly
   85 90 95
   Thr Phe Gly Leu Thr Gly Leu Ser Ser Leu Ser Tyr Leu Phe Asn Met
   100 105 110
   Val Arg Gln Thr Ala Gly Ser Val Pro Glu Ser Leu Asp Tyr Val Lys
   115 120 125
   Gly Thr Leu Gln Asp Ala Gly Glu Tyr Ala Gly Gln Lys Thr Lys Asp
   130 135 140
   Phe Gly Gln Lys Ile Gln Ser Thr Ala His Glu Met Gly Asp Gln Gly
   145 150 155 160
   Gln Val Gly Val His Ala Gln Val Gly Gly Gly Lys Glu Gly Arg Lys
   165 170 175
   Ser Gly Asp Arg Thr
   180
<210> 44
   <211> 737
   <212> DNA
   <213> B. napus
<400> 44
   gaattccatt caaactagta aacaatgggg atactcagga agaaaaaaca cgagcgaaag 60
   ccgtcgttta agagtgtttt aaccgcaata ttagctacac atgccgcaac attcctcttg 120
   ttgatcgccg gtgtatccct cgccggcaca gccgccgcat ttatcgctac catgccacta 180
   ttcgtagtat tcagtccgat tctcgtacca gctggtatta ccactggttt actgactacg 240
   ggtttagcag ccgccggtgg cgccggcgcg actgctgtca ccatcatcct gtggctctac 300
   aagcgagcaa cgggcaaggc gccgccaaaa gtcctagaaa aagtcttgaa aaagataata 360
   ccaggtgctg cagctgcacc agcagccgct ccaggagccg ctccagcagc ggcgccagca 420
   gccgcaccag ctgtggcgcc agcagccgca ccagctgctg cgccagcacc taagccagca 480
   gccccaccag cacctaagcc agcagccgca ccgagtatat gaaaagaagt ggtgggcatg 540
   agtaaaggtt gatatggaaa actggataca tagaaaaaag agtaatccaa cttttaaaaa 600
   ataaataaca acttcacgtg gggatagaaa aattttcaaa tattatttta ctaatggatg 660
   tcgcggtaca aaataataac aaatgtaagc ctttttattg tatagtattt taagaacgaa 720
   gctatgtagc gttgaca 737
<210> 45
   <211> 165
   <212> PRT
   <213> B. napus
<400> 45
   Met Gly Ile Leu Arg Lys Lys Lys His Glu Arg Lys Pro Ser Phe Lys
   1               5                   10                  15
   Ser Val Leu Thr Ala Ile Leu Ala Thr His Ala Ala Thr Phe Leu Leu
               20                  25                  30
   Leu Ile Ala Gly Val Ser Leu Ala Gly Thr Ala Ala Ala Phe Ile Ala
           35                  40                  45
   Thr Met Pro Leu Phe Val Val Phe Ser Pro Ile Leu Val Pro Ala Gly
       50                  55                  60
   Ile Thr Thr Gly Leu Leu Thr Thr Gly Leu Ala Ala Ala Gly Gly Ala
   65                  70                  75                  80
   Gly Ala Thr Ala Val Thr Ile Ile Leu Trp Leu Tyr Lys Arg Ala Thr
                   85                  90                  95
   Gly Lys Ala Pro Pro Lys Val Leu Glu Lys Val Leu Lys Lys Ile Ile
               100                 105                 110
   Pro Gly Ala Ala Ala Ala Pro Ala Ala Ala Pro Gly Ala Ala Pro Ala
           115                 120                 125
   Ala Ala Pro Ala Ala Ala Pro Ala Val Ala Pro Ala Ala Ala Pro Ala
       130                 135                 140
   Ala Ala Pro Ala Pro Lys Pro Ala Ala Pro Pro Ala Pro Lys Pro Ala
   145                 150                 155                 160
   Ala Ala Pro Ser Ile
                   165
<210> 46
   <211> 1153
   <212> DNA
   <213> Z. mays
<400> 46
   aactactccg tcactttgtt tgcaaagctc ctcctcgatc catcgatcac tgcaccggcc 60
   ggcggcaccg cgctcgcagg ggctagccaa cgagacggca gcaatggcgg accgtgaccg 120
   cagcggcatc tacggcggcg cccacgccac ctacgggcag cagcagcagc agggaggagg 180
   cgggcgcccg atgggtgagc aggtgaaggg catgctccac gacaaggggc cgacggcgtc 240
   gcaggcgctg acggtggcga cgctgttccc gctgggcggg ctgctgctgg tgctgtcggg 300
   gctggcgctg acggcctccg tggtggggct ggccgtggcc acgccggtgt tcctgatctt 360
   cagccccgtg ctggtccccg ccgcgctgct catcgggacg gccgtcatgg ggttcctcac 420
   gtcgggcgcg ctggggctcg ggggcctgtc ctcgctcacg tgcctcgcca acacggcgcg 480
   gcaggcgttc cagcgcaccc cggactacgt ggaggaggcg caccgcagga tggcggaggc 540
   cgcggcgcac gcgggccaca agaccgcgca ggcaggccag gccatccagg gcagggcgca 600
   ggaggccggc gccgggggag gtgcaggtgc cggcgctggc ggcggcggca gggcttcctc 660
   gtaagcaagt catccatgca tggattatgg atagatgcgc gcgtgcgtgt ctatcagtat 720
   cagcagccag cagggtcgtc gcggaatgct gtgttcctgt acgtgtgggt gaccgtcctt 780
   ccgtccttcg tctttctccc cccgagtgtg tgttacgtat gtcctggtgt tcgtcgtgtg 840
   tgttcatcgc cgctccagtt gaattccggt gtctgttcat cgccgctcca ggtcgtagat 900
   gtgaatatac tttgctaggg gaataagtga taagtctgtc tggaaggtaa tgtttgagct 960
   ttgctagtgt ggctgggcac tctggtcact ggttgtgttg tgcatgcatc agctgtatga 1020
   tcgtcgtctg ttgtggaaaa ttggtcaatg tattctcttg ctgaataatt tgtgacatct 1080
   aattgttatg tatcgtctct ttgctgaata atcagtttct gatttatctt gcattaaaaa 1140
   aaaaaaaaaa aaa 1153
<210> 47
   <211> 186
   <212> PRT
   <213> Z. mays
<400> 47
   Met Ala Asp Arg Asp Arg Ser Gly Ile Tyr Gly Gly Ala His Ala Thr
   1               5                   10                  15
   Tyr Gly Gln Gln Gln Gln Gln Gly Gly Gly Gly Arg Pro Met Gly Glu
               20                  25                  30
   Gln Val Lys Gly Met Leu His Asp Lys Gly Pro Thr Ala Ser Gln Ala
           35                  40                  45
   Leu Thr Val Ala Thr Leu Phe Pro Leu Gly Gly Leu Leu Leu Val Leu
       50                  55                  60
   Ser Gly Leu Ala Leu Thr Ala Ser Val Val Gly Leu Ala Val Ala Thr
   65                  70                  75                  80
   Pro Val Phe Leu Ile Phe Ser Pro Val Leu Val Pro Ala Ala Leu Leu
                   85                  90                  95
   Ile Gly Thr Ala Val Met Gly Phe Leu Thr Ser Gly Ala Leu Gly Leu
               100                 105                 110
   Gly Gly Leu Ser Ser Leu Thr Cys Leu Ala Asn Thr Ala Arg Gln Ala
           115                 120                 125
   Phe Gln Arg Thr Pro Asp Tyr Val Glu Glu Ala His Arg Arg Met Ala
       130                 135                 140
   Glu Ala Ala Ala His Ala Gly His Lys Thr Ala Gln Ala Gly Gln Ala
   145                 150                 155                 160
   Ile Gln Gly Arg Ala Gln Glu Ala Gly Ala Gly Gly Gly Ala Gly Ala
                   165                 170                 175
   Gly Ala Gly Gly Gly Gly Arg Ala Ser Ser
               180                 185
<210> 48
   <211> 447
   <212> DNA
   <213> O. sativa
<400> 48
   atgggtgatc agcacagagg agtgatcggc ggtggcggct acggtgaccg tggtggccag 60
   gagcagcagg agaagcagcc cttcatgatg acggctctga agacggtgac cgcggcgacg 120
   gccgggggct cgattctggt gctgtccggg ctgatcctgg ccgggaccgt catcgcgctc 180
   acggtggcca ccccggtgtt ggtcatcttc agccccgtgc tggtaccggc ggccatcgcg 240
   ctggcgctca tggcggccgg gttcgtcacc tcggttggtc tcggtgtagc cgcgctctcc 300
   gttttctcgt ggatgtacaa gtacctcacc gggaagcacc cgccgggcgc cgaccatctg 360
   gaccacacca aggcgagggt cgcgtccaag ctccgcgaca tcaaggaggc ggcgcatcac 420
   ctcatcgacc aggcgcaggc gtcttag 447
<210> 49
   <211> 148
   <212> PRT
   <213> O. sativa
<400> 49
   Met Gly Asp Gln His Arg Gly Val Ile Gly Gly Gly Gly Tyr Gly Asp
   1 5 10 15
   Arg Gly Gly Gln Glu Gln Gln Glu Lys Gln Pro Phe Met Met Thr Ala
   20 25 30
   Leu Lys Thr Val Thr Ala Ala Thr Ala Gly Gly Ser Ile Leu Val Leu
   35 40 45
   Ser Gly Leu Ile Leu Ala Gly Thr Val Ile Ala Leu Thr Val Ala Thr
   50 55 60
   Pro Val Leu Val Ile Phe Ser Pro Val Leu Val Pro Ala Ala Ile Ala
   65 70 75 80
   Leu Ala Leu Met Ala Ala Gly Phe Val Thr Ser Val Gly Leu Gly Val
   85 90 95
   Ala Ala Leu Ser Val Phe Ser Trp Met Tyr Lys Tyr Leu Thr Gly Lys
   100 105 110
   His Pro Pro Gly Ala Asp His Leu Asp His Thr Lys Ala Arg Val Ala
   115 120 125
   Ser Lys Leu Arg Asp Ile Lys Glu Ala Ala His His Leu Ile Asp Gln
   130 135 140
   Ala Gln Ala Ser
   145
<210> 50
   <211> 709
   <212> DNA
   <213> B. oleracea
<400> 50
   gccgtttttt cagaatgttc tcttttatct tcccattgct gaacgttata aagcttatta        60
   tagcttccgt gacctcctta gtctgcttag cgttttcttg tgtgacactc ggtggttcag       120
   ccgtggcatt aatcgtatcc acaccacttt tcatcatatt tagtccaatt ctcgtacctg       180
   ccactattgc cactaccctc ctagccagtg ggctcatggc gggtaccacc ctcggactga       240
   ccggcatagg tctcatcacg gggctcgtta ggacggcagg aggagttaca ttggccgaat        300
   caccgataag aagaattata ataaatagaa ttaaagcaag acttgggggt ggcggcggtt       360
   cacgtctggc aatgctcaaa aaaattctgg gactcattaa aaagttgcgt ggtatgtctt       420
   caggtggagc agcacctgcg ctgaagcagc accagcagct gcgcccgcgg atggagctgc       480
   acccgcggca cctgcaccga cctaacaaag aacgttggtt catgctgttc caatatgtag       540
   cacataaaaa ttgtgtaata attaacttaa gaatttatga ttcggaaact aaaaagaaaa       600
   tagccctttt actatctttt atacaatata gttttctatg taataatgtt taatttgctt       660
   ataactataa aagactcatg catagttgat taggaaaaaa aaaaaaaaa                   709
<210> 51
   <211> 216
   <212> PRT
   <213> B. oleracea
<400> 51
   Arg Phe Phe Arg Met Phe Ser Phe Ile Phe Pro Leu Leu Asn Val Ile
   1               5                   10                  15
   Lys Leu Ile Ile Ala Ser Val Thr Ser Leu Val Cys Leu Ala Phe Ser
               20                  25                  30
   Cys Val Thr Leu Gly Gly Ser Ala Val Ala Leu Ile Val Ser Thr Pro
           35                  40                  45
   Leu Phe Ile Ile Phe Ser Pro Ile Leu Val Pro Ala Thr Ile Ala Thr
       50                  55                  60
   Thr Leu Leu Ala Ser Gly Leu Met Ala Gly Thr Thr Leu Gly Leu Thr
   65                  70                  75                  80
   Gly Ile Gly Leu Ile Thr Gly Leu Val Arg Thr Ala Gly Gly Val Thr
                   85                  90                  95
   Leu Ala Glu Ser Pro Ile Arg Arg Ile Ile Ile Asn Arg Ile Lys Ala
               100                 105                 110
   Arg Leu Gly Gly Gly Gly Gly Ser Arg Leu Ala Met Leu Lys Lys Ile
           115                 120                 125
   Leu Gly Leu Ile Lys Lys Leu Arg Gly Met Ser Ser Gly Gly Ala Ala
       130                 135                 140
   Pro Ala Leu Lys Gln His Gln Gln Leu Arg Pro Arg Met Glu Leu His
   145                 150                 155                 160
   Pro Arg His Leu His Arg Pro Asn Lys Glu Arg Trp Phe Met Leu Phe
                   165                 170                 175
   Gln Tyr Val Ala His Lys Asn Cys Val Ile Ile Asn Leu Arg Ile Tyr
               180                 185                 190
   Asp Ser Glu Thr Lys Lys Lys Ile Ala Leu Leu Leu Ser Phe Ile Gln
           195                 200                 205
   Tyr Ser Phe Leu Cys Asn Asn Val
       210                 215
<210> 52
   <211> 447
   <212> DNA
   <213> C. arabica
<400> 52
   atggctgagc actaccagct gcagcaacgc cccacagagg ccgtcaaaag cttccttcct       60
   cagaagggtc catcaacttc acatgtgtta gcagttgtca cgctcctccc agttgcggga      120
   gtcctgctag gcctttccgg gctgattctc gtcggaacgg tcatcggtct ggcggtgaca      180
   accccgcttt tcgttatctt tagccccatt ttggtcccag ctgtatttgc cctagggctg      240
   gccctggccg ggttcttgac ctccggtgct ttcgggatca ctgcacttgc ttcattgtcg      300
   tggatgctga actacatccg actcatgaag gcgtcttccc aggagcaaat ggacctcgca      360
   aagtggcgcg tgcaggacac tgccggccaa gttggtcaga aagcgagaga cgtgggccag      420
   agaactcaag atgtagccag agcatga                                           447
<210> 53
   <211> 148
   <212> PRT
   <213> C. arabica
<400> 53
   Met Ala Glu His Tyr Gln Leu Gln Gln Arg Pro Thr Glu Ala Val Lys
   1 5 10 15
   Ser Phe Leu Pro Gln Lys Gly Pro Ser Thr Ser His Val Leu Ala Val
   20 25 30
   Val Thr Leu Leu Pro Val Ala Gly Val Leu Leu Gly Leu Ser Gly Leu
   35 40 45
   Ile Leu Val Gly Thr Val Ile Gly Leu Ala Val Thr Thr Pro Leu Phe
   50 55 60
   Val Ile Phe Ser Pro Ile Leu Val Pro Ala Val Phe Ala Leu Gly Leu
   65 70 75 80
   Ala Leu Ala Gly Phe Leu Thr Ser Gly Ala Phe Gly Ile Thr Ala Leu
   85 90 95
   Ala Ser Leu Ser Trp Met Leu Asn Tyr Ile Arg Leu Met Lys Ala Ser
   100 105 110
   Ser Gln Glu Gln Met Asp Leu Ala Lys Trp Arg Val Gln Asp Thr Ala
   115 120 125
   Gly Gln Val Gly Gln Lys Ala Arg Asp Val Gly Gln Arg Thr Gln Asp
   130 135 140
   Val Ala Arg Ala
   145
<210> 54
   <211> 348
   <212> PRT
   <213> S. indicum
<400> 54
   Met Asp Leu Ile His Thr Phe Leu Asn Leu Ile Ala Pro Pro Phe Thr
   1               5                   10                  15
   Phe Phe Phe Leu Leu Phe Phe Leu Pro Pro Phe Gln Ile Phe Lys Phe
               20                  25                  30
   Phe Leu Ser Ile Leu Gly Thr Leu Phe Ser Glu Asp Val Ala Gly Lys
           35                  40                  45
   Val Val Val Ile Thr Gly Ala Ser Ser Gly Ile Gly Glu Ser Leu Ala
       50                  55                  60
   Tyr Glu Tyr Ala Lys Arg Gly Ala Cys Leu Val Leu Ala Ala Arg Arg
   65                  70                  75                  80
   Glu Arg Ser Leu Gln Glu Val Ala Glu Arg Ala Arg Asp Leu Gly Ser
                   85                  90                  95
   Pro Asp Val Val Val Val Arg Ala Asp Val Ser Lys Ala Glu Asp Cys
               100                 105                 110
   Arg Lys Val Val Asp Gln Thr Met Asn Arg Phe Gly Arg Leu Asp His
           115                 120                 125
   Leu Val Asn Asn Ala Gly Ile Met Ser Val Ser Met Leu Glu Glu Val
       130                 135                 140
   Glu Asp Ile Thr Gly Tyr Arg Glu Thr Met Asp Ile Asn Phe Trp Gly
   145                 150                 155                 160
   Tyr Val Tyr Met Thr Arg Phe Ala Ala Pro Tyr Leu Arg Asn Ser Arg
                   165                 170                 175
   Gly Arg Ile Val Val Leu Ser Ser Ser Ser Ser Trp Met Pro Thr Pro
               180                 185                 190
   Arg Met Ser Phe Tyr Asn Ala Ser Lys Ala Ala Ile Ser Gln Phe Phe
           195                 200                 205
   Glu Thr Leu Arg Val Glu Phe Gly Pro Asp Ile Gly Ile Thr Leu Val
       210                 215                 220
   Thr Pro Gly Phe Ile Glu Ser Glu Leu Thr Gln Gly Lys Phe Tyr Asn
   225                 230                 235                 240
   Ala Gly Glu Arg Val Ile Asp Gln Asp Met Arg Asp Val Gln Val Ser
                   245                 250                 255
   Thr Thr Pro Ile Leu Arg Val Glu Ser Ala Ala Arg Ser Ile Val Arg
               260                 265                 270
   Ser Ala Ile Arg Gly Glu Arg Tyr Val Thr Glu Pro Ala Trp Phe Arg
           275                 280                 285
   Val Thr Tyr Trp Trp Lys Leu Phe Cys Pro Glu Val Met Glu Trp Val
       290                 295                 300
   Phe Arg Leu Met Tyr Leu Ala Ser Pro Gly Glu Pro Glu Lys Glu Thr
   305                 310                 315                 320
   Phe Gly Lys Lys Val Leu Asp Tyr Thr Gly Val Lys Ser Leu Leu Tyr
                   325                 330                 335
   Pro Glu Thr Val Gln Val Pro Glu Pro Lys Asn Asp
               340                 345
<210> 55
   <211> 348
   <212> PRT
   <213> S. indicum
<400> 55
   Met Asp Leu Ile His Thr Phe Leu Asn Leu Ile Ala Pro Pro Phe Thr
   1               5                   10                  15
   Phe Phe Phe Leu Leu Phe Phe Leu Pro Pro Phe Gln Ile Phe Lys Phe
               20                  25                  30
   Phe Leu Ser Ile Leu Gly Thr Leu Phe Ser Glu Asp Val Ala Gly Lys
           35                  40                  45
   Val Val Val Ile Thr Gly Ala Ser Ser Gly Ile Gly Glu Ser Leu Ala
       50                  55                  60
   Tyr Glu Tyr Ala Lys Arg Gly Ala Cys Leu Val Leu Ala Ala Arg Arg
   65                  70                  75                  80
   Glu Arg Ser Leu Gln Glu Val Ala Glu Arg Ala Arg Asp Leu Gly Ser
                   85                  90                  95
   Pro Asp Val Val Val Val Arg Ala Asp Val Ser Lys Ala Glu Asp Cys
               100                 105                 110
   Arg Lys Val Val Asp Gln Thr Met Asn Arg Phe Gly Arg Leu Asp His
           115                 120                 125
   Leu Val Asn Asn Ala Gly Ile Met Ser Val Ser Met Leu Glu Glu Val
       130                 135                 140
   Glu Asp Ile Thr Gly Tyr Arg Glu Thr Met Asp Ile Asn Phe Trp Gly
   145                 150                 155                 160
   Tyr Val Tyr Met Thr Arg Phe Ala Ala Pro Tyr Leu Arg Asn Ser Arg
                   165                 170                 175
   Gly Arg Ile Val Val Leu Ser Ser Ser Ser Ser Trp Met Pro Thr Pro
               180                 185                 190
   Arg Met Ser Phe Tyr Asn Ala Ser Lys Ala Ala Ile Ser Gln Phe Phe
           195                 200                 205
   Glu Thr Leu Arg Val Glu Phe Gly Pro Asp Ile Gly Ile Thr Leu Val
       210                 215                 220
   Thr Pro Gly Phe Ile Glu Ser Glu Leu Thr Gln Gly Lys Phe Tyr Asn
   225                 230                 235                 240
   Ala Gly Glu Arg Val Ile Asp Gln Asp Met Arg Asp Val Gln Val Ser
                   245                 250                 255
   Thr Thr Pro Ile Leu Arg Val Glu Ser Ala Ala Arg Ser Ile Val Arg
   260 265 270
   Ser Ala Ile Arg Gly Glu Arg Tyr Val Thr Glu Pro Ala Trp Phe Arg
   275 280 285
   Val Thr Tyr Trp Trp Lys Leu Phe Cys Pro Glu Val Met Glu Trp Val
   290 295 300
   Phe Arg Leu Met Tyr Leu Ala Ser Pro Gly Glu Pro Glu Lys Glu Thr
   305 310 315 320
   Phe Gly Lys Lys Val Leu Asp Tyr Thr Gly Val Lys Ser Leu Leu Tyr
   325 330 335
   Pro Glu Thr Val Gln Val Pro Glu Pro Lys Asn Asp
   340 345
<210> 56
   <211> 1266
   <212> DNA
   <213> B. napus
<400> 56
   ggtcgacggc cattacggcc gggatcccgt taatgtgtat tgtgttttga tcgaagatgg     60
   aattgataaa cgatttcctc aatctaactg cacctttctt tactttcttt ggtctttgct    120
   tcttcttgcc ccctttttat ttcttcaagt tcgtgcagtc tatcttctcg acaattttct     180
   ctgaaaacgt atatgggaaa gtggttctca tcactggtgc ttcctccggg atcggcgagc    240
   aattggcata tgagtacgca agtaaaggtg catgtttggc tctgactgcc cgaaggaaga    300
   accgtctaga ggaagtggca gagattgctc gtgaagttgg atcccctaat gttgtcacag   360
   ttcacgctga tgtttccaaa cctgatgatt gtagacgaat cgtcgatgag accatctccc    420
   attttggcag attggatcat cttgtaaaca atgctggaat aatgcaaatt tcaatgttcg    480
   aaaacattga agaaataact aggacaagag cagttatgga tactaacttt tggggagcgg    540
   tttatacaac tcgtgctgcg cttccgtacc ttcgacaaag caatggtaag attgtggcta    600
   tgtcgtcctc tgcggcatgg ctaaccgccc caaggatgag cttttataat gctagcaaag    660
   cagctttgtt gaacttcttc gagacgttga ggattgagct tggtagcgat gtacacatta    720
   caatcgtcac acctggttat attgaatctg aactcacaca aggcaagtac gtctctggtg    780
   aaggcgagct agtagtcaac caagacatta gagatgttca aattggagca tttccggtaa    840
   cgtcagtatc aggtcgtgcc aaggggatag tgaaaggtgt gtgtaggaaa gagagatacg    900
   tgaccgaacc atcgtggttt aaggtgacgt acctttggaa agtgttttgt ccggaactga    960
   tcgagtgggg ttgcagattg atgttcttgt ccggacatgg tacgccggag gaaaatgcac   1020
   tcaacaagaa gatcctggac atacctggtg tacgtagtgc tctataccct gaacctatca   1080
   gaacgccaga aatcaagtcg gagtagagtg aggttgatac ttaataagtg tctcataaag   1140
   tggagccatg ttttgtaaat ggactttcta ttatgcacat gttactatga tgtatctgtt   1200
   tgtttatgtg tataagaata agtgaacttt ggagctcaaa aaaaaaaaaa aaaaaaaaaa   1260
   aaaaaa                                                              1266
<210> 57
   <211> 349
   <212> PRT
   <213> B. napus
<400> 57
   Met Glu Leu Ile Asn Asp Phe Leu Asn Leu Thr Ala Pro Phe Phe Thr
   1               5                   10                  15
   Phe Phe Gly Leu Cys Phe Phe Leu Pro Pro Phe Tyr Phe Phe Lys Phe
               20                  25                  30
   Val Gln Ser Ile Phe Ser Thr Ile Phe Ser Glu Asn Val Tyr Gly Lys
           35                  40                  45
   Val Val Leu Ile Thr Gly Ala Ser Ser Gly Ile Gly Glu Gln Leu Ala
       50                  55                  60
   Tyr Glu Tyr Ala Ser Lys Gly Ala Cys Leu Ala Leu Thr Ala Arg Arg
   65                  70                  75                  80
   Lys Asn Arg Leu Glu Glu Val Ala Glu Ile Ala Arg Glu Val Gly Ser
                   85                  90                  95
   Pro Asn Val Val Thr Val His Ala Asp Val Ser Lys Pro Asp Asp Cys
               100                 105                 110
   Arg Arg Ile Val Asp Glu Thr Ile Ser His Phe Gly Arg Leu Asp His
           115                 120                 125
   Leu Val Asn Asn Ala Gly Ile Met Gln Ile Ser Met Phe Glu Asn Ile
       130                 135                 140
   Glu Glu Ile Thr Arg Thr Arg Ala Val Met Asp Thr Asn Phe Trp Gly
   145                 150                 155                 160
   Ala Val Tyr Thr Thr Arg Ala Ala Leu Pro Tyr Leu Arg Gln Ser Asn
                   165                 170                 175
   Gly Lys Ile Val Ala Met Ser Ser Ser Ala Ala Trp Leu Thr Ala Pro
               180                 185                 190
   Arg Met Ser Phe Tyr Asn Ala Ser Lys Ala Ala Leu Leu Asn Phe Phe
           195                 200                 205
   Glu Thr Leu Arg Ile Glu Leu Gly Ser Asp Val His Ile Thr Ile Val
   210 215 220
   Thr Pro Gly Tyr Ile Glu Ser Glu Leu Thr Gln Gly Lys Tyr Val Ser
   225 230 235 240
   Gly Glu Gly Glu Leu Val Val Asn Gln Asp Ile Arg Asp Val Gln Ile
   245 250 255
   Gly Ala Phe Pro Val Thr Ser Val Ser Gly Arg Ala Lys Gly Ile Val
   260 265 270
   Lys Gly Val Cys Arg Lys Glu Arg Tyr Val Thr Glu Pro Ser Trp Phe
   275 280 285
   Lys Val Thr Tyr Leu Trp Lys Val Phe Cys Pro Glu Leu Ile Glu Trp
   290 295 300
   Gly Cys Arg Leu Met Phe Leu Ser Gly His Gly Thr Pro Glu Glu Asn
   305 310 315 320
   Ala Leu Asn Lys Lys Ile Leu Asp Ile Pro Gly Val Arg Ser Ala Leu
   325 330 335
   Tyr Pro Glu Pro Ile Arg Thr Pro Glu Ile Lys Ser Glu
   340 345
<210> 58
   <211> 1188
   <212> DNA
   <213> Z. mays
<400> 58
   attgcgcaca ccttgatgct gggcatgagc aggacggggt tggccggagc ggcgctgcgg    60
   gtggcgctga cggcgctgct gccgctggtc ctgccggcgt actacgtgta caagctgacc   120
   acgtacctcc tcggcgccgt cttccccgag gacgtcgccg gcaaggtggt actcatcacc   180
   ggcgcctcct ccggcatcgg cgagcacctg gcctatgagt acgcgaagcg gggagcctac   240
   ctggcgctgg tggcgaggag ggaggcgagc ctccgcgagg tcggcgacgt cgcgctgggg   300
   ctgggctcgc cgggcgtcct cgtcctcccg gctgacgtct ccaagcctcg ggactgcgag   360
   ggcttcatcg acgacacgat tagctacttc ggtagactgg atcacctggt gaacaacgcg   420
   tccatctggc aagtgtgcaa gttcgaagag atccaggacg tcaggcactt gagagccctg   480
   atggacatca acttctgggg ccacgtgtac ccaacccggc tcgccatccc tcacctcagg   540
   agaagccgtg gccggatcgt gggcgtcacc tccaactcgt cctacatatt catcgggagg   600
   aacaccttct acaatgccag caaggcggcg gcgctcagct tctacgacac cctgaggatg   660
   gagctgggca gcgacatccg catcaccgag gtggtgccag gcgtggtgga gtctgagatc   720
   accaagggga agatgctcac caagggaggc gagatgaagg tggaccagga cgaaagagac   780
   gccatcctgg ggccgacgcc ggccgagccc gtgggcgact tcgccaggac cgtggtgcgc   840
   gacgtgtgcc ggggcgcgag gtacgtgttc gagcccaggt ggtacatggg cgtctacttg   900
   ctgcgggcct gcctcccgga agtcctggcc tggaactccc gcctgctcac tgtcgacacg   960
   gtcggcgcgt ccaccacgga cacgctcgga aagtggctgg tcgagctgcc cggcgtgcgc  1020
   cgcgtcgtgc agccgccgtc gctccgctcg ccggagatca aggactagtg acggtgatcg  1080
   tgtacgttct gtggccatgg atagcactag ctgtatgaga ccgaagttcc tttagacatc  1140
   gacacgatta ataaaggact caattatctt aaaaaaaaaa aaaaaaaa                1188
<210> 59
   <211> 350
   <212> PRT
   <213> Z. mays
<400> 59
   Met Leu Gly Met Ser Arg Thr Gly Leu Ala Gly Ala Ala Leu Arg Val
   1               5                   10                  15
   Ala Leu Thr Ala Leu Leu Pro Leu Val Leu Pro Ala Tyr Tyr Val Tyr
               20                  25                  30
   Lys Leu Thr Thr Tyr Leu Leu Gly Ala Val Phe Pro Glu Asp Val Ala
           35                  40                  45
   Gly Lys Val Val Leu Ile Thr Gly Ala Ser Ser Gly Ile Gly Glu His
       50                  55                  60
   Leu Ala Tyr Glu Tyr Ala Lys Arg Gly Ala Tyr Leu Ala Leu Val Ala
   65                  70                  75                  80
   Arg Arg Glu Ala Ser Leu Arg Glu Val Gly Asp Val Ala Leu Gly Leu
                   85                  90                  95
   Gly Ser Pro Gly Val Leu Val Leu Pro Ala Asp Val Ser Lys Pro Arg
               100                 105                 110
   Asp Cys Glu Gly Phe Ile Asp Asp Thr Ile Ser Tyr Phe Gly Arg Leu
           115                 120                 125
   Asp His Leu Val Asn Asn Ala Ser Ile Trp Gln Val Cys Lys Phe Glu
       130                 135                 140
   Glu Ile Gln Asp Val Arg His Leu Arg Ala Leu Met Asp Ile Asn Phe
   145                 150                 155                 160
   Trp Gly His Val Tyr Pro Thr Arg Leu Ala Ile Pro His Leu Arg Arg
                   165                 170                 175
   Ser Arg Gly Arg Ile Val Gly Val Thr Ser Asn Ser Ser Tyr Ile Phe
               180                 185                 190
   Ile Gly Arg Asn Thr Phe Tyr Asn Ala Ser Lys Ala Ala Ala Leu Ser
   195 200 205
   Phe Tyr Asp Thr Leu Arg Met Glu Leu Gly Ser Asp Ile Arg Ile Thr
   210 215 220
   Glu Val Val Pro Gly Val Val Glu Ser Glu Ile Thr Lys Gly Lys Met
   225 230 235 240
   Leu Thr Lys Gly Gly Glu Met Lys Val Asp Gln Asp Glu Arg Asp Ala
   245 250 255
   Ile Leu Gly Pro Thr Pro Ala Glu Pro Val Gly Asp Phe Ala Arg Thr
   260 265 270
   Val Val Arg Asp Val Cys Arg Gly Ala Arg Tyr Val Phe Glu Pro Arg
   275 280 285
   Trp Tyr Met Gly Val Tyr Leu Leu Arg Ala Cys Leu Pro Glu Val Leu
   290 295 300
   Ala Trp Asn Ser Arg Leu Leu Thr Val Asp Thr Val Gly Ala Ser Thr
   305 310 315 320
   Thr Asp Thr Leu Gly Lys Trp Leu Val Glu Leu Pro Gly Val Arg Arg
   325 330 335
   Val Val Gln Pro Pro Ser Leu Arg Ser Pro Glu Ile Lys Asp
   340 345 350
<210> 60
   <211> 890
   <212> DNA
   <213> B. napus
<400> 60
   gtttccaaac ctgatgattg tagacgaatc gtcgatgaga ccatctccca ttttggcaga 60
   ttggatcatc ttgtaaacaa tgctggaata atgcagattt caatgttcga aaacattgaa 120
   gaaataacta ggacaagagc agttatggat actaactttt ggggatcggt ttatacaact 180
   cgtgctgcgc ttccgtacct tcgacaaagc aatggtaaga ttgtggccat gtcgtcctct 240
   gcggcatggc taaccgcccc aaggatgagc ttttataatg ctagcaaagc cgcattgttg 300
   aacttcttcg agacgttgag gattgagctt ggcagcgatg tacacattac aatcgtcaca 360
   cctggttata ttgaatctga actcacacaa ggcaagtact tctctggtga aggcgagcta 420
   gtagtcaacc aagacattag agatgttcaa attggagcat ttccggtaac gtcggtatca 480
   ggttgtgcca aggggatagt gaaaggtgtg tgtaggaaac agagatacgt gaccgaacca 540
   tcgtggttta aggtgacgta cctttggaaa gtgttttgtc cggaactgat cgagtggggt 600
   tgcaggttgc tgttcttgtc cggacatggt acgtcggaga aaaatgcact caacaagaag 660
   atcttggaca tacctggtgt acgtagtgct ctataccctg aatctatcag aacgccagaa 720
   atcaagtcgg agtagagtga ggttgatact taataagtgt ctcatatagt ggagccatgt 780
   tttgtaaatg gactttctat tatgcacatg ttactatgat gtatccgttt gtttatgtgt 840
   ataagaataa gtgaactttg gagctccaaa aaaaaaaaaa aaaaaaaaaa 890
<210> 61
   <211> 244
   <212> PRT
   <213> B. napus
<400> 61
   Val Ser Lys Pro Asp Asp Cys Arg Arg Ile Val Asp Glu Thr Ile Ser
   1               5                   10                  15
   His Phe Gly Arg Leu Asp His Leu Val Asn Asn Ala Gly Ile Met Gln
               20                  25                  30
   Ile Ser Met Phe Glu Asn Ile Glu Glu Ile Thr Arg Thr Arg Ala Val
           35                  40                  45
   Met Asp Thr Asn Phe Trp Gly Ser Val Tyr Thr Thr Arg Ala Ala Leu
       50                  55                  60
   Pro Tyr Leu Arg Gln Ser Asn Gly Lys Ile Val Ala Met Ser Ser Ser
   65                  70                  75                  80
   Ala Ala Trp Leu Thr Ala Pro Arg Met Ser Phe Tyr Asn Ala Ser Lys
                   85                  90                  95
   Ala Ala Leu Leu Asn Phe Phe Glu Thr Leu Arg Ile Glu Leu Gly Ser
               100                 105                 110
   Asp Val His Ile Thr Ile Val Thr Pro Gly Tyr Ile Glu Ser Glu Leu
           115                 120                 125
   Thr Gln Gly Lys Tyr Phe Ser Gly Glu Gly Glu Leu Val Val Asn Gln
       130                 135                 140
   Asp Ile Arg Asp Val Gln Ile Gly Ala Phe Pro Val Thr Ser Val Ser
   145                 150                 155                 160
   Gly Cys Ala Lys Gly Ile Val Lys Gly Val Cys Arg Lys Gln Arg Tyr
                   165                 170                 175
   Val Thr Glu Pro Ser Trp Phe Lys Val Thr Tyr Leu Trp Lys Val Phe
               180                 185                 190
   Cys Pro Glu Leu Ile Glu Trp Gly Cys Arg Leu Leu Phe Leu Ser Gly
           195                 200                 205
   His Gly Thr Ser Glu Lys Asn Ala Leu Asn Lys Lys Ile Leu Asp Ile
       210                 215                 220
   Pro Gly Val Arg Ser Ala Leu Tyr Pro Glu Ser Ile Arg Thr Pro Glu
   225                 230                 235                 240
   Ile Lys Ser Glu
<210> 62
   <211> 1030
   <212> DNA
   <213> S. indicum
<400> 62
   ggcacgagag agaaaaaagg tgattttgtc aagggaaata tggcaactca tgttttggct      60
   gctgcggcgg agagaaatgc tgcgttggcg ccggacgccc cgcttgctcc ggtgactatg     120
   gagcgcccag tgcgcactga cttggagact tcgatcccga agccctatat ggcaagagga     180
   ttggttgcac ctgatatgga tcaccccaac ggaacaccag gccatgtgca tgataatttg     240
   agtgtgctgc aacagcattg tgctttcttt gatcaggatg ataacggaat catctatcca     300
   tgggagactt actctggact tcgccaaatt ggtttcaatg tgatagcttc ccttataatg     360
   gctatcgtca ttaatgtggc gctgagttat cctactctcc cgggttggat tccttctcct     420
   tttttcccca tatatttgta caacatacac aaggccaaac atggaagcga ctccggaacc     480
   tatgatactg aaggaaggta cctacctatg aattttgaga acctgttcag caagcatgcc     540
   cggacaatgc ccgataggct cactctaggg gagctatgga gcatgactga agctaacaga     600
   gaagcatttg acattttcgg ctggatcgca agcaaaatgg agtggactct cctctacatt     660
   cttgcaagag accaggacgg tttcctgtcg aaagaagcca tcaggcggtg ttacgatggc     720
   agtttgttcg agtactgtgc aaagatgcaa aggggagccg aggacaagat gaaatgaagg     780
   aaatcggcta tcgcggtagg tgtaagttat gatgtggtgt gtatgatgga ttgaaagtgc     840
   cagtgcttaa gttgtgtggc agagtcttgt gtaataacct ttgtgtacag atttaaggtc     900
   tcggaattgg tgtaactgtg gagaagatgt tgactcctgt ttttgttcaa taagtccaac     960
   tcttgacatt tggttggttt gcagggaaag atggggaatt ttgttttccg aaaaaaaaaa    1020
   aaaaaaaaaa                                                           1030
<210> 63
   <211> 245
   <212> PRT
   <213> S. indicum
<400> 63
   Met Ala Thr His Val Leu Ala Ala Ala Ala Glu Arg Asn Ala Ala Leu
   1               5                   10                  15
   Ala Pro Asp Ala Pro Leu Ala Pro Val Thr Met Glu Arg Pro Val Arg
   20                  25                  30
   Thr Asp Leu Glu Thr Ser Ile Pro Lys Pro Tyr Met Ala Arg Gly Leu
           35                  40                  45
   Val Ala Pro Asp Met Asp His Pro Asn Gly Thr Pro Gly His Val His
       50                  55                  60
   Asp Asn Leu Ser Val Leu Gln Gln His Cys Ala Phe Phe Asp Gln Asp
   65                  70                  75                  80
   Asp Asn Gly Ile Ile Tyr Pro Trp Glu Thr Tyr Ser Gly Leu Arg Gln
                   85                  90                  95
   Ile Gly Phe Asn Val Ile Ala Ser Leu Ile Met Ala Ile Val Ile Asn
               100                 105                 110
   Val Ala Leu Ser Tyr Pro Thr Leu Pro Gly Trp Ile Pro Ser Pro Phe
           115                 120                 125
   Phe Pro Ile Tyr Leu Tyr Asn Ile His Lys Ala Lys His Gly Ser Asp
       130                 135                 140
   Ser Gly Thr Tyr Asp Thr Glu Gly Arg Tyr Leu Pro Met Asn Phe Glu
   145                 150                 155                 160
   Asn Leu Phe Ser Lys His Ala Arg Thr Met Pro Asp Arg Leu Thr Leu
                   165                 170                 175
   Gly Glu Leu Trp Ser Met Thr Glu Ala Asn Arg Glu Ala Phe Asp Ile
               180                 185                 190
   Phe Gly Trp Ile Ala Ser Lys Met Glu Trp Thr Leu Leu Tyr Ile Leu
           195                 200                 205
   Ala Arg Asp Gln Asp Gly Phe Leu Ser Lys Glu Ala Ile Arg Arg Cys
       210                 215                 220
   Tyr Asp Gly Ser Leu Phe Glu Tyr Cys Ala Lys Met Gln Arg Gly Ala
   225                 230                 235                 240
   Glu Asp Lys Met Lys
                   245
<210> 64
   <211> 985
   <212> DNA
   <213> G. max
<400> 64
   atacgagaga gtgagtaatc gagaaagaga agaagcatat atatcaaaat ggctgcagag 60
   atggagaggg agtcattgat aactgaagct cctaatgcac cagttactgc acagagaagg 120
   gtcagaaatg acttagaaaa ttctctacca aaaccatact tgccaagagc attgaaagct 180
   cctgatacgg gtcacccaaa tggaacagca ggccacaggc accacaactt atctgttctt 240
   cagcagcatt gtgctttttt tgatcaagat gacaatggaa tcatttaccc ttgggaaact 300
   tacatggggc tgcgttctat tggatttaat gttgttgcat ctgttattat ggctattgtt 360
   atcaatgttg gattgagtta ccccactcta cctaattggt tcccttctct cctttttcct 420
   atctacatac acaacataca caaagcaaag catgggagtg actctggagt ttatgacaca 480
   gaaggacgtt atgtgccagc aaatattgag aacatattca gtaagtatgc tcgtacagta 540
   cctgacaagc tcacacttgg ggagctctgg gacttgacag agggaaaccg aaatgctttt 600
   gacatatttg gctggcttgc agcaaaattt gaatgggggg ttctgtacat tctggcaagg 660
   gatgaggaag gtttcctgtc taaagaagct gttagaagat gctttgatgg gagcttattt 720
   gaatactgtg ctaaaatgca tactactagt gatgccaaga tgagttgaaa ttggattatg 780
   cagtgtgcat agttacgtgt gatgtgtcgt ttctgttaga catgttagtg gtgtaatgtg 840
   aaaaaatagt aaaaaaataa acttattagg aactcgtggt ctgggattga tcagcataat 900
   aaagagttta ctgtctctag catatgttgt tctcgagatg taatcctttc ctcacgtgtg 960
   gaaagaagtt actctggttc ctaca 985
<210> 65
   <211> 239
   <212> PRT
   <213> G. max
<400> 65
   Met Ala Ala Glu Met Glu Arg Glu Ser Leu Ile Thr Glu Ala Pro Asn
   1 5 10 15
   Ala Pro Val Thr Ala Gln Arg Arg Val Arg Asn Asp Leu Glu Asn Ser
   20 25 30
   Leu Pro Lys Pro Tyr Leu Pro Arg Ala Leu Lys Ala Pro Asp Thr Gly
   35 40 45
   His Pro Asn Gly Thr Ala Gly His Arg His His Asn Leu Ser Val Leu
   50 55 60
   Gln Gln His Cys Ala Phe Phe Asp Gln Asp Asp Asn Gly Ile Ile Tyr
   65 70 75 80
   Pro Trp Glu Thr Tyr Met Gly Leu Arg Ser Ile Gly Phe Asn Val Val
   85 90 95
   Ala Ser Val Ile Met Ala Ile Val Ile Asn Val Gly Leu Ser Tyr Pro
   100 105 110
   Thr Leu Pro Asn Trp Phe Pro Ser Leu Leu Phe Pro Ile Tyr Ile His
   115 120 125
   Asn Ile His Lys Ala Lys His Gly Ser Asp Ser Gly Val Tyr Asp Thr
   130 135 140
   Glu Gly Arg Tyr Val Pro Ala Asn Ile Glu Asn Ile Phe Ser Lys Tyr
   145 150 155 160
   Ala Arg Thr Val Pro Asp Lys Leu Thr Leu Gly Glu Leu Trp Asp Leu
   165 170 175
   Thr Glu Gly Asn Arg Asn Ala Phe Asp Ile Phe Gly Trp Leu Ala Ala
   180 185 190
   Lys Phe Glu Trp Gly Val Leu Tyr Ile Leu Ala Arg Asp Glu Glu Gly
   195 200 205
   Phe Leu Ser Lys Glu Ala Val Arg Arg Cys Phe Asp Gly Ser Leu Phe
   210 215 220
   Glu Tyr Cys Ala Lys Met His Thr Thr Ser Asp Ala Lys Met Ser
   225 230 235
<210> 66
   <211> 1326
   <212> DNA
   <213> Z. mays
<400> 66
   atcccagcag caggtagcag cagcgcatga ctttgtatct gcaactcgtt tcgactagcc      60
   tgcacgccgg gagccctcgt ctcgcccttc tccacctccg aaggtagggt acctgatact     120
   tccctgcccc gcgcatcgca tccgcggttt tagtttgctg gcctcggttc cgatcgattc     180
   ctatcctccc tccgggcggg cgtccaccga cttcatcatc cactcgccgg ccgaaccgat     240
   ttgtttcgtt tgccgttcga tccgcagatc ctgcgagttc aacccgcgag tgagcatgtc     300
   gtcctactcc ccgccgccgc cgccgccgcg ggaccagtcc atggacaccg aggcacccaa     360
   cgcgcccatc accagggagc ggaggctcaa ccccgatctg caggagcagc tccccaagcc     420
   atatctcgcg agagctctcg aggcggtgga cccgagccac ccgcagggga ccaaggggcg     480
   cgacccccgc ggcatgagcg tgcttcagca gcacgccgcc ttcttcgacc gcaatggcga     540
   cggcgtcatc tacccctggg agacgtttca aggactgcga gcgataggat gtggactcac     600
   tgtatcattc gcgttctcca tactgatcaa cctcttcctc agttatccca ctcagccggg     660
   atggttacct tctcctttgc tgtccatccg tatagacaac atccacaagg gtaagcacgg     720
   gagtgattct gaaacctacg acactgaagg gaggtttgat ccatcaaagt tcgatgctat     780
   attcagtaag tacggtcgaa cccatcctaa tgctataaca agagacgagt taagctcaat     840
   gcttcaagga aaccgcaata cgtacgattt ccttggctgg ttggccgctg ccggtgaatg     900
   gctcttactc tacagcttgg cgaaagacaa ggatggcctc ttgcagcgcg aaactgtccg     960
   tggtctattt gatgggagcc tatttgagcg actggaagac gacaacaaca agaagaaatc    1020
   gtcatgaatg ctgagcagcc ttgtacagct cagggaagtg ctgtcagtac aaaactacca    1080
   gatataccat tggtcgtgtt caaataacaa atgcttcggc tttgttcatc cgtcattaac    1140
   tatgagtgct gggatttgtt tgtatgtgtg tcgtgctacc agtttcttct cctgtcgtct    1200
   cacacaggta ctgaattacg catgtgtttt ctagtgttcg tgcggaagct gtattataag    1260
   ctgaaaaatg tgcgtttgaa atttatgggc aaaactgtct tcttggtctt aaaaaaaaaa    1320
   aaaaaa                                                               1326
<210> 67
   <211> 243
   <212> PRT
   <213> Z. mays
<400> 67
   Met Ser Ser Tyr Ser Pro Pro Pro Pro Pro Pro Arg Asp Gln Ser Met
   1               5                   10                  15
   Asp Thr Glu Ala Pro Asn Ala Pro Ile Thr Arg Glu Arg Arg Leu Asn
               20                  25                  30
   Pro Asp Leu Gln Glu Gln Leu Pro Lys Pro Tyr Leu Ala Arg Ala Leu
           35                  40                  45
   Glu Ala Val Asp Pro Ser His Pro Gln Gly Thr Lys Gly Arg Asp Pro
       50                  55                  60
   Arg Gly Met Ser Val Leu Gln Gln His Ala Ala Phe Phe Asp Arg Asn
   65                  70                  75                  80
   Gly Asp Gly Val Ile Tyr Pro Trp Glu Thr Phe Gln Gly Leu Arg Ala
                   85                  90                  95
   Ile Gly Cys Gly Leu Thr Val Ser Phe Ala Phe Ser Ile Leu Ile Asn
               100                 105                 110
   Leu Phe Leu Ser Tyr Pro Thr Gln Pro Gly Trp Leu Pro Ser Pro Leu
           115                 120                 125
   Leu Ser Ile Arg Ile Asp Asn Ile His Lys Gly Lys His Gly Ser Asp
       130                 135                 140
   Ser Glu Thr Tyr Asp Thr Glu Gly Arg Phe Asp Pro Ser Lys Phe Asp
   145                 150                 155                 160
   Ala Ile Phe Ser Lys Tyr Gly Arg Thr His Pro Asn Ala Ile Thr Arg
                   165                 170                 175
   Asp Glu Leu Ser Ser Met Leu Gln Gly Asn Arg Asn Thr Tyr Asp Phe
               180                 185                 190
   Leu Gly Trp Leu Ala Ala Ala Gly Glu Trp Leu Leu Leu Tyr Ser Leu
           195                 200                 205
   Ala Lys Asp Lys Asp Gly Leu Leu Gln Arg Glu Thr Val Arg Gly Leu
       210                 215                 220
   Phe Asp Gly Ser Leu Phe Glu Arg Leu Glu Asp Asp Asn Asn Lys Lys
   225                 230                 235                 240
   Lys Ser Ser
<210> 68
   <211> 1058
   <212> DNA
   <213> B. napus
<400> 68
   cttcccattc tcctctagag caaaaaagag cgagagatga gtacggcgac tgagataatg      60
   gagagagacg caatggctgc ggtggctccc tacgctccgg tcacctttca ccgccgtgct     120
   cgtgttgaca tggatgatag acttcctaaa ccttatatgc caagagcact gcaagcaccc     180
   gacagagagc atccgtatgg aaccccaggc cataagaatt atggacttag tgttcttcaa     240
   cagcacgtcg ccttcttcga tttagatgat aatggaatta tctacccttg ggagacctac     300
   tctggactgc gaatgcttgg tttcaatatc attgtatcgc ttatcgcagc cgctgtaatc     360
   aatttggccc ttagctatgc tactcttccg ggatggtttc cttcgccgtt cttcccaata     420
   tacatacaca atatacacaa gtcaaagcat gggagcgact caagaacata tgacaatgaa     480
   gggaggttta tgcctgtgaa tcttgagttg atatttagca aatatgcgaa aacattgcca     540
   gacaagttga gtcttggaga attatgggag atgacacaag gacaacgtga cgcatgggac     600
   atcttcggat ggttcgcaag caaaatagag tgggggttgt tgtacttgct agcgagggat     660
   gaagaagggt ttctgtcaaa agaagcgatt aggaggtgtt ttgacgggag cttgttcgag     720
   tattgtgcca agatatacgc aggtatcaat gaagacaaga cagcctacta ctaaaagtaa     780
   atggtagagg agctttaggc tgataatcgt cgatgtgaat gtaacttgtg tctaaagcag     840
   agtccatgtg tttgttatgt tatgtcaaaa tctgtaaggt agaagtatga tcagttgcag     900
   ctggtataga aaaacttcta tgttgaataa tagtatgttt tgttgttgtg tttgtgtttg     960
   tatcaaccct tttttagtta tttccagttc aaatgtaatt tttcattgtt gttacttggt    1020
   attgagaatc atttcttgta taaaaaaaaa aaaaaaaa                             1058
<210> 69
   <211> 245
   <212> PRT
   <213> B. napus
<400> 69
   Met Ser Thr Ala Thr Glu Ile Met Glu Arg Asp Ala Met Ala Ala Val
   1               5                   10                  15
   Ala Pro Tyr Ala Pro Val Thr Phe His Arg Arg Ala Arg Val Asp Met
               20                  25                  30
   Asp Asp Arg Leu Pro Lys Pro Tyr Met Pro Arg Ala Leu Gln Ala Pro
           35                  40                  45
   Asp Arg Glu His Pro Tyr Gly Thr Pro Gly His Lys Asn Tyr Gly Leu
       50                  55                  60
   Ser Val Leu Gln Gln His Val Ala Phe Phe Asp Leu Asp Asp Asn Gly
   65                  70                  75                  80
   Ile Ile Tyr Pro Trp Glu Thr Tyr Ser Gly Leu Arg Met Leu Gly Phe
                   85                  90                  95
   Asn Ile Ile Val Ser Leu Ile Ala Ala Ala Val Ile Asn Leu Ala Leu
               100                 105                 110
   Ser Tyr Ala Thr Leu Pro Gly Trp Phe Pro Ser Pro Phe Phe Pro Ile
           115                 120                 125
   Tyr Ile His Asn Ile His Lys Ser Lys His Gly Ser Asp Ser Arg Thr
       130                 135                 140
   Tyr Asp Asn Glu Gly Arg Phe Met Pro Val Asn Leu Glu Leu Ile Phe
   145                 150                 155                 160
   Ser Lys Tyr Ala Lys Thr Leu Pro Asp Lys Leu Ser Leu Gly Glu Leu
                   165                 170                 175
   Trp Glu Met Thr Gln Gly Gln Arg Asp Ala Trp Asp Ile Phe Gly Trp
               180                 185                 190
   Phe Ala Ser Lys Ile Glu Trp Gly Leu Leu Tyr Leu Leu Ala Arg Asp
           195                 200                 205
   Glu Glu Gly Phe Leu Ser Lys Glu Ala Ile Arg Arg Cys Phe Asp Gly
       210                 215                 220
   Ser Leu Phe Glu Tyr Cys Ala Lys Ile Tyr Ala Gly Ile Asn Glu Asp
   225                 230                 235                 240
   Lys Thr Ala Tyr Tyr
                   245
<210> 70
   <211> 1013
   <212> DNA
   <213> C. revoluta
<400> 70
   acgcgggggc agttgaggtg atcctcaata tcgtagcgaa gatcgcccca aatggcttcc      60
   gtcgagtctc ttcaaactac ggctctaagg gcaccagtta cactggaacg tagagtgaat     120
   cccaatttgg atgatgaaat tccaaagcca tttctaccaa gagcgcttgt agctgtagat     180
   acagagcatt tagatggatc cccgggacac cagcataaca acatgagtgt tcttcagcag     240
   catgttgcat ttttcgatcg aaatcatgac ggaattgtgt atccttggga aacatacgaa     300
   ggcttccgtg ccataggatt taacatcgtc atttccttaa tgtctgcact gttcatcaac     360
   atagccttga gctatcttac tcttcctgga tggataccgt cactgctctt tccaatacat     420
   ataaacagga ttcatcgagc aaaacatgga agtgattcag aggtgtatga caccgagggg     480
   aggtttgttc catcgaaatt cgaggaaatt ttcactaaat atgcacgtgt acgtccagat     540
   aggctcacat tctctgaaat actgttggcc ttgacggagg ccaatagaaa cgccaatgac     600
   cctttcggat ggttaaccag caaggccaag tggggcctcc tctaccttct tgccaaggat     660
   gatcaaggtt ttctgccgaa agaggcagtc agaggagttt atgatggcag tttgtttgag     720
   ttattggaaa agcaacgatc atcccgtaag cagaaataaa atggcctttt gaattagacc     780
   ctgaaagagc atgagtgaga aataaactgc acataaatgg cccactagcc catctatgct     840
   gtgaaaaata tgatgtcatt agtcattgtc agttagcata ttttaacaag catgactttg     900
   tacatattgc atgccgtatc atgaattatg ttcttggttg tgtgatctag actccatttc     960
   ttaatgaaat gtcttcctta tttcataaaa aaaaaaaaaa aaaaaaaaaa aaa           1013
<210> 71
   <211> 235
   <212> PRT
   <213> C. revoluta
<400> 71
   Met Ala Ser Val Glu Ser Leu Gln Thr Thr Ala Leu Arg Ala Pro Val
   1               5                   10                  15
   Thr Leu Glu Arg Arg Val Asn Pro Asn Leu Asp Asp Glu Ile Pro Lys
               20                  25                  30
   Pro Phe Leu Pro Arg Ala Leu Val Ala Val Asp Thr Glu His Leu Asp
           35                  40                  45
   Gly Ser Pro Gly His Gln His Asn Asn Met Ser Val Leu Gln Gln His
       50                  55                  60
   Val Ala Phe Phe Asp Arg Asn His Asp Gly Ile Val Tyr Pro Trp Glu
   65                  70                  75                  80
   Thr Tyr Glu Gly Phe Arg Ala Ile Gly Phe Asn Ile Val Ile Ser Leu
                   85                  90                  95
   Met Ser Ala Leu Phe Ile Asn Ile Ala Leu Ser Tyr Leu Thr Leu Pro
               100                 105                 110
   Gly Trp Ile Pro Ser Leu Leu Phe Pro Ile His Ile Asn Arg Ile His
           115                 120                 125
   Arg Ala Lys His Gly Ser Asp Ser Glu Val Tyr Asp Thr Glu Gly Arg
       130                 135                 140
   Phe Val Pro Ser Lys Phe Glu Glu Ile Phe Thr Lys Tyr Ala Arg Val
   145                 150                 155                 160
   Arg Pro Asp Arg Leu Thr Phe Ser Glu Ile Leu Leu Ala Leu Thr Glu
                   165                 170                 175
   Ala Asn Arg Asn Ala Asn Asp Pro Phe Gly Trp Leu Thr Ser Lys Ala
               180                 185                 190
   Lys Trp Gly Leu Leu Tyr Leu Leu Ala Lys Asp Asp Gln Gly Phe Leu
           195                 200                 205
   Pro Lys Glu Ala Val Arg Gly Val Tyr Asp Gly Ser Leu Phe Glu Leu
       210                 215                 220
   Leu Glu Lys Gln Arg Ser Ser Arg Lys Gln Lys
   225                 230                 235
<210> 72
   <211> 720
   <212> DNA
   <213> C. sativus
<400> 72
   atgtctttga cgatggaaaa cgaagccacc gccaccgagg cccgattcgc gcccgtcact      60
   cgggagcgaa gggttcgcaa cgacctcgaa accacgctcc ccaagccata tttggcgagg     120
   gctttggtgg cggcggactc caaccacccc acggggacgg tggggcataa acactatgga     180
   atgacagtgc ttcaacagca tgttgctttc tttgaccaag atgataatgg tatcgtttac     240
   ccttgggaga cttatgttgg gctaagagcg attggattca acatgataat gtccctagtt     300
   atggccgtta ttataaattt cgccatgagc tatcgcactc aacaaggatg gattccatca     360
   cctttctttc ccatctatat ttacaatatt cacagagaca aacatggcag cgacactgga     420
   acctatgaca ctgaaggaag gtatatagcg gcgaatttcg agaacatgtt cagcaagtac     480
   gctagaacac aaccggacaa gttgtcgctt ggcgagatat gggacatgac tgaagccaac     540
   cgtctagcgt tcgaccccta cggatggata gcggcgaaac tggaatggtt catattatac     600
   gtgctggcta gggacgagga cgggtatctg tcgaaagagg ccgtaagaag atgttatgat     660
   ggaagtttgt tcgagtattg tgcaaagatg aatatgagtg ctcaatacaa gatgtattga     720
<210> 73
   <211> 239
   <212> PRT
   <213> C. sativus
<400> 73
   Met Ser Leu Thr Met Glu Asn Glu Ala Thr Ala Thr Glu Ala Arg Phe
   1               5                   10                  15
   Ala Pro Val Thr Arg Glu Arg Arg Val Arg Asn Asp Leu Glu Thr Thr
               20                  25                  30
   Leu Pro Lys Pro Tyr Leu Ala Arg Ala Leu Val Ala Ala Asp Ser Asn
           35                  40                  45
   His Pro Thr Gly Thr Val Gly His Lys His Tyr Gly Met Thr Val Leu
       50                  55                  60
   Gln Gln His Val Ala Phe Phe Asp Gln Asp Asp Asn Gly Ile Val Tyr
   65                  70                  75                  80
   Pro Trp Glu Thr Tyr Val Gly Leu Arg Ala Ile Gly Phe Asn Met Ile
                   85                  90                  95
   Met Ser Leu Val Met Ala Val Ile Ile Asn Phe Ala Met Ser Tyr Arg
               100                 105                 110
   Thr Gln Gln Gly Trp Ile Pro Ser Pro Phe Phe Pro Ile Tyr Ile Tyr
           115                 120                 125
   Asn Ile His Arg Asp Lys His Gly Ser Asp Thr Gly Thr Tyr Asp Thr
       130                 135                 140
   Glu Gly Arg Tyr Ile Ala Ala Asn Phe Glu Asn Met Phe Ser Lys Tyr
   145                 150                 155                 160
   Ala Arg Thr Gln Pro Asp Lys Leu Ser Leu Gly Glu Ile Trp Asp Met
                   165                 170                 175
   Thr Glu Ala Asn Arg Leu Ala Phe Asp Pro Tyr Gly Trp Ile Ala Ala
               180                 185                 190
   Lys Leu Glu Trp Phe Ile Leu Tyr Val Leu Ala Arg Asp Glu Asp Gly
           195                 200                 205
   Tyr Leu Ser Lys Glu Ala Val Arg Arg Cys Tyr Asp Gly Ser Leu Phe
       210                 215                 220
   Glu Tyr Cys Ala Lys Met Asn Met Ser Ala Gln Tyr Lys Met Tyr
   225                 230                 235
<210> 74
   <211> 2074
   <212> DNA
   <213> A. thaliana
<400> 74
   tgaatccttt ttcctttctt cttcttcttc tcttcagaga aaactttgct tctctttcta      60
   taaggaacca gacacgaatc ccattcccac cgatttctta gcttcttcct tcaatccgct     120
   ctttccctct ccattagatt ctgtttcctc tttcaatttc ttctgcatgc ttctcgattc     180
   tctctgacgc ctcttttctc ccgacgctgt ttcgtcaaac gcttttcgaa atggcgattt     240
   tggattctgc tggcgttact acggtgacgg agaacggtgg cggagagttc gtcgatcttg     300
   ataggcttcg tcgacggaaa tcgagatcgg attcttctaa cggacttctt ctctctggtt     360
   ccgataataa ttctccttcg gatgatgttg gagctcccgc cgacgttagg gatcggattg     420
   attccgttgt taacgatgac gctcagggaa cagccaattt ggccggagat aataacggtg     480
   gtggcgataa taacggtggt ggaagaggcg gcggagaagg aagaggaaac gccgatgcta     540
   cgtttacgta tcgaccgtcg gttccagctc atcggagggc gagagagagt ccacttagct     600
   ccgacgcaat cttcaaacag agccatgccg gattattcaa cctctgtgta gtagttctta     660
   ttgctgtaaa cagtagactc atcatcgaaa atcttatgaa gtatggttgg ttgatcagaa     720
   cggatttctg gtttagttca agatcgctgc gagattggcc gcttttcatg tgttgtatat     780
   ccctttcgat ctttcctttg gctgccttta cggttgagaa attggtactt cagaaataca     840
   tatcagaacc tgttgtcatc tttcttcata ttattatcac catgacagag gttttgtatc     900
   cagtttacgt caccctaagg tgtgattctg cttttttatc aggtgtcact ttgatgctcc     960
   tcacttgcat tgtgtggcta aagttggttt cttatgctca tactagctat gacataagat    1020
   ccctagccaa tgcagctgat aaggccaatc ctgaagtctc ctactacgtt agcttgaaga    1080
   gcttggcata tttcatggtc gctcccacat tgtgttatca gccaagttat ccacgttctg    1140
   catgtatacg gaagggttgg gtggctcgtc aatttgcaaa actggtcata ttcaccggat    1200
   tcatgggatt tataatagaa caatatataa atcctattgt caggaactca aagcatcctt    1260
   tgaaaggcga tcttctatat gctattgaaa gagtgttgaa gctttcagtt ccaaatttat    1320
   atgtgtggct ctgcatgttc tactgcttct tccacctttg gttaaacata ttggcagagc    1380
   ttctctgctt cggggatcgt gaattctaca aagattggtg gaatgcaaaa agtgtgggag    1440
   attactggag aatgtggaat atgcctgttc ataaatggat ggttcgacat atatacttcc    1500
   cgtgcttgcg cagcaagata ccaaagacac tcgccattat cattgctttc ctagtctctg    1560
   cagtctttca tgagctatgc atcgcagttc cttgtcgtct cttcaagcta tgggcttttc    1620
   ttgggattat gtttcaggtg cctttggtct tcatcacaaa ctatctacag gaaaggtttg    1680
   gctcaacggt ggggaacatg atcttctggt tcatcttctg cattttcgga caaccgatgt    1740
   gtgtgcttct ttattaccac gacctgatga accgaaaagg atcgatgtca tgaaacaact    1800
   gttcaaaaaa tgactttctt caaacatcta tggcctcgtt ggatctccgt tgatgttgtg    1860
   gtggttctga tgctaaaacg acaaatagtg ttataaccat tgaagaagaa aagaaaatta    1920
   gagttgttgt atctgcaaaa attttggtag agacacgcga acccgtttgg attttgttat 1980
   ggtgtaaaga aatttcaatc aaaaaactgt tgtaataatt gttaccaaaa agaaatgctt 2040
   ttctggaaac gaggggaaaa atagtagttt tgtt 2074
<210> 75
   <211> 520
   <212> PRT
   <213> A. thaliana
<400> 75
   Met Ala Ile Leu Asp Ser Ala Gly Val Thr Thr Val Thr Glu Asn Gly
   1               5                   10                  15
   Gly Gly Glu Phe Val Asp Leu Asp Arg Leu Arg Arg Arg Lys Ser Arg
               20                  25                  30
   Ser Asp Ser Ser Asn Gly Leu Leu Leu Ser Gly Ser Asp Asn Asn Ser
           35                  40                  45
   Pro Ser Asp Asp Val Gly Ala Pro Ala Asp Val Arg Asp Arg Ile Asp
       50                  55                  60
   Ser Val Val Asn Asp Asp Ala Gln Gly Thr Ala Asn Leu Ala Gly Asp
   65                  70                  75                  80
   Asn Asn Gly Gly Gly Asp Asn Asn Gly Gly Gly Arg Gly Gly Gly Glu
                   85                  90                  95
   Gly Arg Gly Asn Ala Asp Ala Thr Phe Thr Tyr Arg Pro Ser Val Pro
               100                 105                 110
   Ala His Arg Arg Ala Arg Glu Ser Pro Leu Ser Ser Asp Ala Ile Phe
           115                 120                 125
   Lys Gln Ser His Ala Gly Leu Phe Asn Leu Cys Val Val Val Leu Ile
       130                 135                 140
   Ala Val Asn Ser Arg Leu Ile Ile Glu Asn Leu Met Lys Tyr Gly Trp
   145                 150                 155                 160
   Leu Ile Arg Thr Asp Phe Trp Phe Ser Ser Arg Ser Leu Arg Asp Trp
                   165                 170                 175
   Pro Leu Phe Met Cys Cys Ile Ser Leu Ser Ile Phe Pro Leu Ala Ala
               180                 185                 190
   Phe Thr Val Glu Lys Leu Val Leu Gln Lys Tyr Ile Ser Glu Pro Val
           195                 200                 205
   Val Ile Phe Leu His Ile Ile Ile Thr Met Thr Glu Val Leu Tyr Pro
       210                 215                 220
   Val Tyr Val Thr Leu Arg Cys Asp Ser Ala Phe Leu Ser Gly Val Thr
   225                 230                 235                 240
   Leu Met Leu Leu Thr Cys Ile Val Trp Leu Lys Leu Val Ser Tyr Ala
                   245                 250                 255
   His Thr Ser Tyr Asp Ile Arg Ser Leu Ala Asn Ala Ala Asp Lys Ala
               260                 265                 270
   Asn Pro Glu Val Ser Tyr Tyr Val Ser Leu Lys Ser Leu Ala Tyr Phe
           275                 280                 285
   Met Val Ala Pro Thr Leu Cys Tyr Gln Pro Ser Tyr Pro Arg Ser Ala
       290                 295                 300
   Cys Ile Arg Lys Gly Trp Val Ala Arg Gln Phe Ala Lys Leu Val Ile
   305                 310                 315                 320
   Phe Thr Gly Phe Met Gly Phe Ile Ile Glu Gln Tyr Ile Asn Pro Ile
                   325                 330                 335
   Val Arg Asn Ser Lys His Pro Leu Lys Gly Asp Leu Leu Tyr Ala Ile
               340                 345                 350
   Glu Arg Val Leu Lys Leu Ser Val Pro Asn Leu Tyr Val Trp Leu Cys
           355                 360                 365
   Met Phe Tyr Cys Phe Phe His Leu Trp Leu Asn Ile Leu Ala Glu Leu
       370                 375                 380
   Leu Cys Phe Gly Asp Arg Glu Phe Tyr Lys Asp Trp Trp Asn Ala Lys
   385                 390                 395                 400
   Ser Val Gly Asp Tyr Trp Arg Met Trp Asn Met Pro Val His Lys Trp
                   405                 410                 415
   Met Val Arg His Ile Tyr Phe Pro Cys Leu Arg Ser Lys Ile Pro Lys
               420                 425                 430
   Thr Leu Ala Ile Ile Ile Ala Phe Leu Val Ser Ala Val Phe His Glu
           435                 440                 445
   Leu Cys Ile Ala Val Pro Cys Arg Leu Phe Lys Leu Trp Ala Phe Leu
       450                 455                 460
   Gly Ile Met Phe Gln Val Pro Leu Val Phe Ile Thr Asn Tyr Leu Gln
   465                 470                 475                 480
   Glu Arg Phe Gly Ser Thr Val Gly Asn Met Ile Phe Trp Phe Ile Phe
   485 490 495
   Cys Ile Phe Gly Gln Pro Met Cys Val Leu Leu Tyr Tyr His Asp Leu
   500 505 510
   Met Asn Arg Lys Gly Ser Met Ser
   515 520
<210> 76
   <211> 2090
   <212> DNA
   <213> T. majus
<400> 76
   acgcggggag ttttcaaaat catattatgc tttttcttca ctactgcatg aactttcttt 60
   ctacttcttg caactgattt gtaatcctta cacatgtttc tagttttctc catataaaaa 120
   aaatattctc tgagcttctc gattctctag agagagaagg ccaaaaaaaa atggcggtgg 180
   cagagtcgtc acagaacacg acaaccatga gtggtcacgg cgactcggat ctcaacaatt 240
   tccgtagaag gaaaccgagt tcctccgtga ttgaaccttc gtcgtccggt tttacatcca 300
   ccaatggcgt accggcgact ggccacgtgg ctgagaatcg tgaccaggat cgggtagggg 360
   ctatggagaa cgcaacagga tcggtcaact taattggaaa tggtggaggc gtggttatcg 420
   ggaatgaaga gaaacaggta ggggagactg atatacgatt cacttaccgg ccttcgtttc 480
   cggctcatcg gagggtgagg gagagtcctc ttagctctga tgcaatcttc aaacagagcc 540
   atgcgggttt attcaacttg tgtatagtag tgctcattgc agtaaacagt aggcttatca 600
   tcgaaaatct tatgaagtat ggttggttga tcgatactgg tttctggttt agctcaagat 660
   cactgggtga ttggtccatc tttatgtgct gtcttacact cccaattttc ccacttgctg 720
   cttttattgt tgaaaagctg gtgcagcgaa atcatatatc tgaacttgtt gctgttctcc 780
   ttcatgtaat cgtttctacc gctgcagttt tatatccagt tattgtgatc ttaacgtgtg 840
   attcggtgta tatgtctggt gtggtattga tgctctttgg ttgcattatg tggttgaagc 900
   tggtgtcata tgcacatact agttctgata ttagaacact ggccaaatct ggctataagg 960
   gggatgcgca ccccaattca accattgtga gttgctcata tgatgttagc ttgaagagtt 1020
   tggcatactt catggttgct ccgacattat gttaccagcc tagctatcct cgttcgtcgt 1080
   gtatccgcaa gggttgggtt gttcgtcaat ttgtcaaact aatagttttc ataggactca 1140
   tggggttcat tatagaacaa tatattaatc ctatcgttcg aaattccaaa cacccattga 1200
   aaggagattt tttatatgca atagaaagag ttttgaagct ttcagttcca aatctatatg 1260
   tttggctttg catgttctac tcttttttcc acctctggtt gaacatactg gctgagcttc 1320
   ttcgctttgg tgatcgtgaa ttctacaaag attggtggaa tgcaaaaact gttgcggagt 1380
   attggaaaat gtggaatatg cctgttcata gatggatggt tcgtcatcta tattttccct 1440
   gtttgaggaa tgggataccc aaggaaggtg ccattattat cgcgttctta gtttctggtg 1500
   ctttccatga gctctgcatt gcagttcctt gccacgtatt caagttatgg gcctttatag 1560
   gcattatgtt tcaggttccc ttggtattga ttacgaatta tctacaagaa aagttcagta 1620
   attctatggt gggcaatatg atcttctggt tcatcttctg catacttggc caacctatgt 1680
   gtgtccttct atattaccat gacctgataa atctaaagga aaagtgaaaa aatggaagtt 1740
   gcctatgctc agagtattcc tatcccaatg cacacattat atggttctgt acaatctgtg 1800
   cccccttcat cctttacacg tacccatgct ggttcctgca cgatgatttg ccttttgttt 1860
   gtaagcaata tttggagaga gtccaattta ggaagtgact agtgtggctt atatcttgta 1920
   tactaccttt agtcatgggg gggtttttat attactagta ccaaaagtca agttgtatat 1980
   gatttacggt ttagtttctt tcatgttttt tgtttttgtg taaatatacg tttcatatat 2040
   cactgttttt tcaaagtaaa atcaataata ccccatagat gttgaaactg 2090
<210> 77
   <211> 518
   <212> PRT
   <213> T. majus
<400> 77
   Met Ala Val Ala Glu Ser Ser Gln Asn Thr Thr Thr Met Ser Gly His
   1               5                   10                  15
   Gly Asp Ser Asp Leu Asn Asn Phe Arg Arg Arg Lys Pro Ser Ser Ser
               20                  25                  30
   Val Ile Glu Pro Ser Ser Ser Gly Phe Thr Ser Thr Asn Gly Val Pro
           35                  40                  45
   Ala Thr Gly His Val Ala Glu Asn Arg Asp Gln Asp Arg Val Gly Ala
       50                  55                  60
   Met Glu Asn Ala Thr Gly Ser Val Asn Leu Ile Gly Asn Gly Gly Gly
   65                  70                  75                  80
   Val Val Ile Gly Asn Glu Glu Lys Gln Val Gly Glu Thr Asp Ile Arg
                   85                  90                  95
   Phe Thr Tyr Arg Pro Ser Phe Pro Ala His Arg Arg Val Arg Glu Ser
               100                 105                 110
   Pro Leu Ser Ser Asp Ala Ile Phe Lys Gln Ser His Ala Gly Leu Phe
           115                 120                 125
   Asn Leu Cys Ile Val Val Leu Ile Ala Val Asn Ser Arg Leu Ile Ile
       130                 135                 140
   Glu Asn Leu Met Lys Tyr Gly Trp Leu Ile Asp Thr Gly Phe Trp Phe
   145                 150                 155                 160
   Ser Ser Arg Ser Leu Gly Asp Trp Ser Ile Phe Met Cys Cys Leu Thr
                   165                 170                 175
   Leu Pro Ile Phe Pro Leu Ala Ala Phe Ile Val Glu Lys Leu Val Gln
               180                 185                 190
   Arg Asn His Ile Ser Glu Leu Val Ala Val Leu Leu His Val Ile Val
           195                 200                 205
   Ser Thr Ala Ala Val Leu Tyr Pro Val Ile Val Ile Leu Thr Cys Asp
       210                 215                 220
   Ser Val Tyr Met Ser Gly Val Val Leu Met Leu Phe Gly Cys Ile Met
   225                 230                 235                 240
   Trp Leu Lys Leu Val Ser Tyr Ala His Thr Ser Ser Asp Ile Arg Thr
                   245                 250                 255
   Leu Ala Lys Ser Gly Tyr Lys Gly Asp Ala His Pro Asn Ser Thr Ile
               260                 265                 270
   Val Ser Cys Ser Tyr Asp Val Ser Leu Lys Ser Leu Ala Tyr Phe Met
           275                 280                 285
   Val Ala Pro Thr Leu Cys Tyr Gln Pro Ser Tyr Pro Arg Ser Ser Cys
       290                 295                 300
   Ile Arg Lys Gly Trp Val Val Arg Gln Phe Val Lys Leu Ile Val Phe
   305                 310                 315                 320
   Ile Gly Leu Met Gly Phe Ile Ile Glu Gln Tyr Ile Asn Pro Ile Val
                   325                 330                 335
   Arg Asn Ser Lys His Pro Leu Lys Gly Asp Phe Leu Tyr Ala Ile Glu
               340                 345                 350
   Arg Val Leu Lys Leu Ser Val Pro Asn Leu Tyr Val Trp Leu Cys Met
           355                 360                 365
   Phe Tyr Ser Phe Phe His Leu Trp Leu Asn Ile Leu Ala Glu Leu Leu
       370                 375                 380
   Arg Phe Gly Asp Arg Glu Phe Tyr Lys Asp Trp Trp Asn Ala Lys Thr
   385                 390                 395                 400
   Val Ala Glu Tyr Trp Lys Met Trp Asn Met Pro Val His Arg Trp Met
                   405                 410                 415
   Val Arg His Leu Tyr Phe Pro Cys Leu Arg Asn Gly Ile Pro Lys Glu
               420                 425                 430
   Gly Ala Ile Ile Ile Ala Phe Leu Val Ser Gly Ala Phe His Glu Leu
           435                 440                 445
   Cys Ile Ala Val Pro Cys His Val Phe Lys Leu Trp Ala Phe Ile Gly
       450                 455                 460
   Ile Met Phe Gln Val Pro Leu Val Leu Ile Thr Asn Tyr Leu Gln Glu
   465                 470                 475                 480
   Lys Phe Ser Asn Ser Met Val Gly Asn Met Ile Phe Trp Phe Ile Phe
                   485                 490                 495
   Cys Ile Leu Gly Gln Pro Met Cys Val Leu Leu Tyr Tyr His Asp Leu
               500                 505                 510
   Ile Asn Leu Lys Glu Lys
           515
<210> 78
   <211> 1485
   <212> DNA
   <213> Z. mays
<400> 78
   atggccccgc ccccctccat gcctgccgcc tccgatcgcg ccggccctgg ccgcgacgcg      60
   ggcgactcgt cctcccttcg cctccgccgc gccccctcag ccgacgccgg cgaccttgcc     120
   ggcgattcct cgggaggctt gcgggagaac ggcgagccgc aatcgccgac gaatccgccg     180
   ccgcaggagc agcagcagca cgagatgcta tactaccgcg cgtcggcgcc cgcccaccgc     240
   cgcgtcaagg agagccccct cagctctgac gccatcttcc ggcagagcca tgctggtctt     300
   ctgaatctat gcattgttgt tctgatcgca gtgaacagca gactcattat tgagaattta     360
   atgaagtatg gcctgttgat aagagctgga ttttggttta gtgcaagatc gctgggtgac     420
   tggccccttc taatgtgctg cctcactcta ccagttttcc cactagttgc actcatggct     480
   gagaagctga tcacaagaaa gctcattggt gaacatgtgg ttattctact ccatatcatt     540
   attacaacat ctgccattgt ctatccagtt gttgtgactc ttaagtgtga ctcagcagta     600
   ctatctggat ttgtgctaat gtttcttgcg agcatcatgt ggatgaagct tgtctcttat     660
   gcacatacaa attatgatat aagggtattg tccaaaagta ctgaaaaggg tgctgcatat     720
   ggaaattatg tcgatcctga gaatatgaaa gatccaacct ttaaaagtct agtgtacttt     780
   atgttggccc caacactttg ttaccagcca acttatcctc aaactacatg tattagaaag     840
   ggttgggtga cccagcaact cataaagtgc gtggttttta caggcttgat gggcttcata     900
   attgagcaat atataaaccc aattgtgaag aattccaaac atccactgaa agggaatttt     960
   ttgaatgcta tagaaagagt cttaaaactc tcagtgccaa cattatatgt atggctttgc    1020
   atgttctatt gcttttttca tttatggctg aacattgtag ctgaactcct ctgtttcggt    1080
   gaccgtgaat tctataagga ctggtggaat gccaaaactg ttgaagagta ctggaggatg    1140
   tggaacatgc ctgttcataa gtggatcatc agacacatat attttccatg tataaggaaa    1200
   ggcttttcca ggggtgtagc tattctaatc tcgtttctgg tttcagctgt atttcatgag    1260
   atatgtattg cggtgccttg ccacattttc aaattctggg cattttctgg gatcatgttt    1320
   cagataccct tggtattctt gacaagatat ctccatgcta cgttcaagca tgtaatggtg    1380
   ggcaacatga tattttggtt cttcttcagt atagtcggac agccgatgtg tgtccttcta    1440
   tactaccatg acgtcatgaa caggcaggcc caggcaagta gatag                    1485
<210> 79
   <211> 494
   <212> PRT
   <213> Z. mays
<400> 79
   Met Ala Pro Pro Pro Ser Met Pro Ala Ala Ser Asp Arg Ala Gly Pro
   1               5                   10                  15
   Gly Arg Asp Ala Gly Asp Ser Ser Ser Leu Arg Leu Arg Arg Ala Pro
               20                  25                  30
   Ser Ala Asp Ala Gly Asp Leu Ala Gly Asp Ser Ser Gly Gly Leu Arg
           35                  40                  45
   Glu Asn Gly Glu Pro Gln Ser Pro Thr Asn Pro Pro Pro Gln Glu Gln
       50                  55                  60
   Gln Gln His Glu Met Leu Tyr Tyr Arg Ala Ser Ala Pro Ala His Arg
   65                  70                  75                  80
   Arg Val Lys Glu Ser Pro Leu Ser Ser Asp Ala Ile Phe Arg Gln Ser
                   85                  90                  95
   His Ala Gly Leu Leu Asn Leu Cys Ile Val Val Leu Ile Ala Val Asn
               100                 105                 110
   Ser Arg Leu Ile Ile Glu Asn Leu Met Lys Tyr Gly Leu Leu Ile Arg
           115                 120                 125
   Ala Gly Phe Trp Phe Ser Ala Arg Ser Leu Gly Asp Trp Pro Leu Leu
       130                 135                 140
   Met Cys Cys Leu Thr Leu Pro Val Phe Pro Leu Val Ala Leu Met Ala
   145                 150                 155                 160
   Glu Lys Leu Ile Thr Arg Lys Leu Ile Gly Glu His Val Val Ile Leu
                   165                 170                 175
   Leu His Ile Ile Ile Thr Thr Ser Ala Ile Val Tyr Pro Val Val Val
               180                 185                 190
   Thr Leu Lys Cys Asp Ser Ala Val Leu Ser Gly Phe Val Leu Met Phe
   195 200 205
   Leu Ala Ser Ile Met Trp Met Lys Leu Val Ser Tyr Ala His Thr Asn
   210 215 220
   Tyr Asp Ile Arg Val Leu Ser Lys Ser Thr Glu Lys Gly Ala Ala Tyr
   225 230 235 240
   Gly Asn Tyr Val Asp Pro Glu Asn Met Lys Asp Pro Thr Phe Lys Ser
   245 250 255
   Leu Val Tyr Phe Met Leu Ala Pro Thr Leu Cys Tyr Gln Pro Thr Tyr
   260 265 270
   Pro Gln Thr Thr Cys Ile Arg Lys Gly Trp Val Thr Gln Gln Leu Ile
   275 280 285
   Lys Cys Val Val Phe Thr Gly Leu Met Gly Phe Ile Ile Glu Gln Tyr
   290 295 300
   Ile Asn Pro Ile Val Lys Asn Ser Lys His Pro Leu Lys Gly Asn Phe
   305 310 315 320
   Leu Asn Ala Ile Glu Arg Val Leu Lys Leu Ser Val Pro Thr Leu Tyr
   325 330 335
   Val Trp Leu Cys Met Phe Tyr Cys Phe Phe His Leu Trp Leu Asn Ile
   340 345 350
   Val Ala Glu Leu Leu Cys Phe Gly Asp Arg Glu Phe Tyr Lys Asp Trp
   355 360 365
   Trp Asn Ala Lys Thr Val Glu Glu Tyr Trp Arg Met Trp Asn Met Pro
   370 375 380
   Val His Lys Trp Ile Ile Arg His Ile Tyr Phe Pro Cys Ile Arg Lys
   385 390 395 400
   Gly Phe Ser Arg Gly Val Ala Ile Leu Ile Ser Phe Leu Val Ser Ala
   405 410 415
   Val Phe His Glu Ile Cys Ile Ala Val Pro Cys His Ile Phe Lys Phe
   420 425 430
   Trp Ala Phe Ser Gly Ile Met Phe Gln Ile Pro Leu Val Phe Leu Thr
   435 440 445
   Arg Tyr Leu His Ala Thr Phe Lys His Val Met Val Gly Asn Met Ile
   450 455 460
   Phe Trp Phe Phe Phe Ser Ile Val Gly Gln Pro Met Cys Val Leu Leu
   465 470 475 480
   Tyr Tyr His Asp Val Met Asn Arg Gln Ala Gln Ala Ser Arg
   485 490
<210> 80
   <211> 1330
   <212> DNA
   <213> A. thaliana
<400> 80
   agttaaaaga ttggttattt gggctctgca ctcaagtgag agagaagata gatagatctg     60
   agtagaatct tcgattcatt attcgttgtc gtcgttcatc tgtgagaagc ggacaaacca    120
   aagaatccac cggagctagt gatatgggtg gttccagaga gttccgagct gaggaacatt    180
   caaatcaatt ccactctatc atcgccatgg ccatctggct tggcgccatt cacttcaacg    240
   tcgctcttgt tctctgttct ctcattttcc ttcctccttc tctatctctc atggtcttgg    300
   gcttgctctc tctgtttatc tttatcccaa tcgatcatcg tagcaaatat ggtcgtaagc    360
   tcgctaggta catatgcaag cacgcgtgta attatttccc cgtctctctg tacgtcgagg    420
   attacgaagc tttccagcct aatcgtgcct atgtctttgg ttatgaacca cattcggtgc    480
   taccgattgg agttgttgct ctttgtgatc tcacagggtt tatgcctatt cctaacatta    540
   aagttcttgc aagtagtgct atattctaca ctccctttct aaggcatata tggacatggt    600
   tagggctcac cgctgcttct aggaagaatt tcacttccct tttggattct ggctacagtt    660
   gtgttcttgt acctggtggt gtgcaggaga cttttcatat gcaacatgat gctgagaatg    720
   tcttcctttc aaggagaaga ggatttgtgc gcatagccat ggaacagggg agccctctgg    780
   ttccagtatt ctgctttggt caggcacgcg tgtacaaatg gtggaagccg gattgtgatc    840
   tctatcttaa actatctaga gcaatcagat tcaccccgat ctgcttctgg ggagtttttg    900
   gatcaccatt accgtgtcga cagcctatgc atgtggtcgt tggtaaacca atagaagtca    960
   caaaaactct gaagccaact gacgaagaga ttgctaagtt tcatggccag tatgtggaag   1020
   cgcttaggga tctgtttgag aggcacaagt cccgagtcgg ctatgatctt gagctgaaaa   1080
   ttctttgaac aaaatctcca atggaaataa ttacttgtgt gtatccttca ttaattgtta   1140
   ccttggagct ggatttggac ttaatataaa tgactacatc atgtagtcta catgtattgc   1200
   atgtctttag catcgactgt tgaagtaatg gaatacgttt ataaagcctg taaattacat   1260
   gtcgtcttgc acaagagtat gtggtaataa taacatttga cccaaaaata atactagtta   1320
   aatttttcct                                                          1330
<210> 81
   <211> 314
   <212> PRT
   <213> A. thaliana
<400> 81
   Met Gly Gly Ser Arg Glu Phe Arg Ala Glu Glu His Ser Asn Gln Phe
   1               5                   10                  15
   His Ser Ile Ile Ala Met Ala Ile Trp Leu Gly Ala Ile His Phe Asn
               20                  25                  30
   Val Ala Leu Val Leu Cys Ser Leu Ile Phe Leu Pro Pro Ser Leu Ser
           35                  40                  45
   Leu Met Val Leu Gly Leu Leu Ser Leu Phe Ile Phe Ile Pro Ile Asp
       50                  55                  60
   His Arg Ser Lys Tyr Gly Arg Lys Leu Ala Arg Tyr Ile Cys Lys His
   65                  70                  75                  80
   Ala Cys Asn Tyr Phe Pro Val Ser Leu Tyr Val Glu Asp Tyr Glu Ala
                   85                  90                  95
   Phe Gln Pro Asn Arg Ala Tyr Val Phe Gly Tyr Glu Pro His Ser Val
               100                 105                 110
   Leu Pro Ile Gly Val Val Ala Leu Cys Asp Leu Thr Gly Phe Met Pro
           115                 120                 125
   Ile Pro Asn Ile Lys Val Leu Ala Ser Ser Ala Ile Phe Tyr Thr Pro
       130                 135                 140
   Phe Leu Arg His Ile Trp Thr Trp Leu Gly Leu Thr Ala Ala Ser Arg
   145                 150                 155                 160
   Lys Asn Phe Thr Ser Leu Leu Asp Ser Gly Tyr Ser Cys Val Leu Val
                   165                 170                 175
   Pro Gly Gly Val Gln Glu Thr Phe His Met Gln His Asp Ala Glu Asn
               180                 185                 190
   Val Phe Leu Ser Arg Arg Arg Gly Phe Val Arg Ile Ala Met Glu Gln
           195                 200                 205
   Gly Ser Pro Leu Val Pro Val Phe Cys Phe Gly Gln Ala Arg Val Tyr
       210                 215                 220
   Lys Trp Trp Lys Pro Asp Cys Asp Leu Tyr Leu Lys Leu Ser Arg Ala
   225                 230                 235                 240
   Ile Arg Phe Thr Pro Ile Cys Phe Trp Gly Val Phe Gly Ser Pro Leu
                   245                 250                 255
   Pro Cys Arg Gln Pro Met His Val Val Val Gly Lys Pro Ile Glu Val
               260                 265                 270
   Thr Lys Thr Leu Lys Pro Thr Asp Glu Glu Ile Ala Lys Phe His Gly
   275 280 285
   Gln Tyr Val Glu Ala Leu Arg Asp Leu Phe Glu Arg His Lys Ser Arg
   290 295 300
   Val Gly Tyr Asp Leu Glu Leu Lys Ile Leu
   305 310
<210> 82
   <211> 954
   <212> DNA
   <213> B. napus
<400> 82
   atgggcaaag tcagagactt tggagctgag gatcatatcc catcaaacat attccatgca      60
   gtgaccgcca tatccatctg cctcagcgcc atttacttga acctcgctct tgttctcatc     120
   tccctcttct tcctcccaac ttctctctcc ctcctggtct tgggcctgct ctctctgttt     180
   atcatcatcc ctatagatga tcgtagcaag tacggtctta agctggctag gtacatatgc     240
   aagcacgcgg ctagttactt ccccgttact ctgcatgtcg aagactacga agctttcaag     300
   cctgatcgct cctatgtatt tggttatgaa ccacactcgg tgtggcccat tggagctgtt     360
   gcacttgttg atctggcagg gtttatgcct cttcctaaca tcaaacttct tgcaagcaat     420
   gctatattct acacgccgtt tctaaggcac atgtgggcat ggttagggct cgcctctgct     480
   tctaggaaga gtttctcttc tcttctggag tctggctata gttgtatcct tgtacctggt     540
   ggtgtgcagg aaacatttca cttgcaacat gatgttgaga acgtcttcct ttcatcgaga     600
   agaggatttg tgcgcatcgc catggaacaa ggggcacctc ttgttccagt tttctgcttt     660
   ggtcagtccc gtgcgtacaa gtggtggaag ccggattgtg acctttattt taaactagca     720
   agagcgatca ggtttactcc tatctgtttc tggggagttt tcggatcccc aataccatat     780
   agacacccta ttcatgtggt ggttggtaaa ccaatacaag ttgcaaagtc tctgcagcca     840
   actgatgaag agattgatga gttgcatggc cagtttgtgg aagcgcttaa ggatctgttt     900
   gagaggcaca aggccggagc aggctactct gatctgcagt tgaacattct ttga           954
<210> 83
   <211> 317
   <212> PRT
   <213> B. napus
<400> 83
   Met Gly Lys Val Arg Asp Phe Gly Ala Glu Asp His Ile Pro Ser Asn
   1 5 10 15
   Ile Phe His Ala Val Thr Ala Ile Ser Ile Cys Leu Ser Ala Ile Tyr
   20 25 30
   Leu Asn Leu Ala Leu Val Leu Ile Ser Leu Phe Phe Leu Pro Thr Ser
   35 40 45
   Leu Ser Leu Leu Val Leu Gly Leu Leu Ser Leu Phe Ile Ile Ile Pro
   50 55 60
   Ile Asp Asp Arg Ser Lys Tyr Gly Leu Lys Leu Ala Arg Tyr Ile Cys
   65 70 75 80
   Lys His Ala Ala Ser Tyr Phe Pro Val Thr Leu His Val Glu Asp Tyr
   85 90 95
   Glu Ala Phe Lys Pro Asp Arg Ser Tyr Val Phe Gly Tyr Glu Pro His
   100 105 110
   Ser Val Trp Pro Ile Gly Ala Val Ala Leu Val Asp Leu Ala Gly Phe
   115 120 125
   Met Pro Leu Pro Asn Ile Lys Leu Leu Ala Ser Asn Ala Ile Phe Tyr
   130 135 140
   Thr Pro Phe Leu Arg His Met Trp Ala Trp Leu Gly Leu Ala Ser Ala
   145 150 155 160
   Ser Arg Lys Ser Phe Ser Ser Leu Leu Glu Ser Gly Tyr Ser Cys Ile
   165 170 175
   Leu Val Pro Gly Gly Val Gln Glu Thr Phe His Leu Gln His Asp Val
   180 185 190
   Glu Asn Val Phe Leu Ser Ser Arg Arg Gly Phe Val Arg Ile Ala Met
   195 200 205
   Glu Gln Gly Ala Pro Leu Val Pro Val Phe Cys Phe Gly Gln Ser Arg
   210 215 220
   Ala Tyr Lys Trp Trp Lys Pro Asp Cys Asp Leu Tyr Phe Lys Leu Ala
   225 230 235 240
   Arg Ala Ile Arg Phe Thr Pro Ile Cys Phe Trp Gly Val Phe Gly Ser
   245 250 255
   Pro Ile Pro Tyr Arg His Pro Ile His Val Val Val Gly Lys Pro Ile
   260 265 270
   Gln Val Ala Lys Ser Leu Gln Pro Thr Asp Glu Glu Ile Asp Glu Leu
   275 280 285
   His Gly Gln Phe Val Glu Ala Leu Lys Asp Leu Phe Glu Arg His Lys
   290 295 300
   Ala Gly Ala Gly Tyr Ser Asp Leu Gln Leu Asn Ile Leu
   305 310 315
<210> 84
   <211> 1637
   <212> DNA
   <213> A. hypogaea
<400> 84
   aatgaacttg acataaagtg gttgtttgta acaccccatt tagtgttttg cttagatgtt      60
   gagagttcta taaacttttg tactatttgg taccccgtaa ttaatagaaa tagaaatgtg     120
   ataatggttc tatgtttcat tccagaaaaa aattgtcatt ttaaaaagtt ttcttaaatt     180
   ctgaatggga atgatgatca ttcagatcaa taaggttaac acttttttat atgatatttt     240
   atgtaatctg attaattttt ttttggtgac aaaaaactcg tgccgaattc ggcacgaggt     300
   caaaacctca gaagagagaa aaggagaatt tggttcctaa ttaattctca ccatcaacga     360
   tggaggtttc aggcgccgtt ctaaggaatg tcacgtgccc ttccttttct gtgcacgtga     420
   gttcccgtcg tcgtggtggt gatagttgtg ttacagtgcc ggtgaggatg agaaaaaagg     480
   cggtggtgcg ttgttgctgc gggttcagtg attcggggca tgtgcagtat tacggggacg     540
   agaagaagaa ggagaatgga accgctatgt tgagcaccaa gaagaagctc aagatgctga     600
   agaaacgtgt ccttttcgat gatcttcaag gaaacctgac ttgggatgct gctatggttt     660
   tgatgaagca gctagagcaa gtaagggcag aggagaagga attgaagaaa aaaaggaagc     720
   aagagaagaa ggaggcaaaa ctcaaagcct ctaagatgaa caccaatcct gattgcgaat     780
   cgtcatcgtc atcgtcatca tctgaatctg aatctgaatc aagtgagagt gaatgtgaca     840
   atgaggtggt tgacatgaag aagaacatta aggttggtgt tgccgttgct gttgccgatt     900
   ccccacgaaa ggcggaaacc atgattctat acacctccct tgttgcccga gatgttagtg     960
   ctaatcatca tcatcataat gccgtggaat tattctctag aaacaatgac atatcagttg    1020
   gaagcattaa tggtggcctt aagaatgaga atactgcggt tattaccact gaagctattc    1080
   ctcagaagag gattgaggta tgcatgggaa acaagtgcaa gaaatccgga tctattgcat    1140
   tgttgcaaga atttgagaga gtggttggtg ctgaaggagg tgctgctgct gcagttgttg    1200
   gatgcaagtg catggggaag tgcaagagtg cacctaatgt gaggattcag aactctactg    1260
   cagataaaat agctgagggg ttcaatgatt cagttaaggt tccagctaac cctctttgca    1320
   ttggggttgc atggaggatg ttgaaaccat tgtggcttag attcttgggc gagaatcagg    1380
   aaagtactaa tgaataattt gctggtatgc tgtttggaaa attgtatata cgtagtgcca    1440
   gaacctatca gattgttgtt ttattttata taaacataga ctgcatattg ttgtgagatt    1500
   cgatttcctc atttattgga acttccagag cctgatttgt gtccattcga gctcgactca    1560
   aagatttaca tggcctgctc aatctatgaa ttcaaatttg agggccctgt ttggcattaa    1620
   tattaatata ttaatat                                                   1637
<210> 85
   <211> 345
   <212> PRT
   <213> A. hypogaea
<400> 85
   Met Glu Val Ser Gly Ala Val Leu Arg Asn Val Thr Cys Pro Ser Phe
   1               5                   10                  15
   Ser Val His Val Ser Ser Arg Arg Arg Gly Gly Asp Ser Cys Val Thr
               20                  25                  30
   Val Pro Val Arg Met Arg Lys Lys Ala Val Val Arg Cys Cys Cys Gly
           35                  40                  45
   Phe Ser Asp Ser Gly His Val Gln Tyr Tyr Gly Asp Glu Lys Lys Lys
       50                  55                  60
   Glu Asn Gly Thr Ala Met Leu Ser Thr Lys Lys Lys Leu Lys Met Leu
   65                  70                  75                  80
   Lys Lys Arg Val Leu Phe Asp Asp Leu Gln Gly Asn Leu Thr Trp Asp
                   85                  90                  95
   Ala Ala Met Val Leu Met Lys Gln Leu Glu Gln Val Arg Ala Glu Glu
               100                 105                 110
   Lys Glu Leu Lys Lys Lys Arg Lys Gln Glu Lys Lys Glu Ala Lys Leu
           115                 120                 125
   Lys Ala Ser Lys Met Asn Thr Asn Pro Asp Cys Glu Ser Ser Ser Ser
       130                 135                 140
   Ser Ser Ser Ser Glu Ser Glu Ser Glu Ser Ser Glu Ser Glu Cys Asp
   145                 150                 155                 160
   Asn Glu Val Val Asp Met Lys Lys Asn Ile Lys Val Gly Val Ala Val
                   165                 170                 175
   Ala Val Ala Asp Ser Pro Arg Lys Ala Glu Thr Met Ile Leu Tyr Thr
               180                 185                 190
   Ser Leu Val Ala Arg Asp Val Ser Ala Asn His His His His Asn Ala
           195                 200                 205
   Val Glu Leu Phe Ser Arg Asn Asn Asp Ile Ser Val Gly Ser Ile Asn
       210                 215                 220
   Gly Gly Leu Lys Asn Glu Asn Thr Ala Val Ile Thr Thr Glu Ala Ile
   225                 230                 235                 240
   Pro Gln Lys Arg Ile Glu Val Cys Met Gly Asn Lys Cys Lys Lys Ser
                   245                 250                 255
   Gly Ser Ile Ala Leu Leu Gln Glu Phe Glu Arg Val Val Gly Ala Glu
   260 265 270
   Gly Gly Ala Ala Ala Ala Val Val Gly Cys Lys Cys Met Gly Lys Cys
   275 280 285
   Lys Ser Ala Pro Asn Val Arg Ile Gln Asn Ser Thr Ala Asp Lys Ile
   290 295 300
   Ala Glu Gly Phe Asn Asp Ser Val Lys Val Pro Ala Asn Pro Leu Cys
   305 310 315 320
   Ile Gly Val Ala Trp Arg Met Leu Lys Pro Leu Trp Leu Arg Phe Leu
   325 330 335
   Gly Glu Asn Gln Glu Ser Thr Asn Glu
   340 345
<210> 86
   <211> 2811
   <212> DNA
   <213> A. thaliana
<400> 86
   cccaagtgta atattgttat taatacatgg gctatactaa aagccccacg aaaagtttac      60
   tgaactattt gaggcccaac aagagcctat cggattaacg cctactgcag aagaaaatct     120
   gtctgcactc cacccaagaa aacgcagact aattaatgaa atcaacgaaa cggataggtc     180
   gggtctaagg ttgaccatga accgcaacct gaaccaggag caaagtggtc aagttttgcc     240
   atccggtccg agtcccttgg aggaataata ccagaacaga aaaaaacaga aaagtcgaca     300
   ataaacaaaa gagacaaatt tgatttgatt ggttccagaa attcgcagag aaacagctct     360
   ttgtctctct cgactgatct aacaatccct aatctgtgtt ctaaattcct ggacgagatt     420
   tgacaaagtc cgtatagctt aacctggttt aatttcaagt gacagatatg ccccttattc     480
   atcggaaaaa gccgacggag aaaccatcga cgccgccatc tgaagaggtg gtgcacgatg     540
   aggattcgca aaagaaacca cacgaatctt ccaaatccca ccataagaaa tcgaacggag     600
   gagggaagtg gtcgtgcatc gattcttgtt gttggttcat tgggtgtgtg tgtgtaacct     660
   ggtggtttct tctcttcctt tacaacgcaa tgcctgcgag cttccctcag tatgtaacgg     720
   agcgaatcac gggtcctttg cctgacccgc ccggtgttaa gctcaaaaaa gaaggtctta     780
   aggcgaaaca tcctgttgtc ttcattcctg ggattgtcac cggtgggctc gagctttggg     840
   aaggcaaaca atgcgctgat ggtttattta gaaaacgttt gtggggtgga acttttggtg     900
   aagtctacaa aaggcctcta tgttgggtgg aacacatgtc acttgacaat gaaactgggt     960
   tggatccagc tggtattaga gttcgagctg tatcaggact cgtggctgct gactactttg    1020
   ctcctggcta ctttgtctgg gcagtgctga ttgctaacct tgcacatatt ggatatgaag    1080
   agaaaaatat gtacatggct gcatatgact ggcggctttc gtttcagaac acagaggtac    1140
   gtgatcagac tcttagccgt atgaaaagta atatagagtt gatggtttct accaacggtg    1200
   gaaaaaaagc agttatagtt ccgcattcca tgggggtctt gtattttcta cattttatga 1260
   agtgggttga ggcaccagct cctctgggtg gcgggggtgg gccagattgg tgtgcaaagt 1320
   atattaaggc ggtgatgaac attggtggac catttcttgg tgttccaaaa gctgttgcag 1380
   ggcttttctc tgctgaagca aaggatgttg cagttgccag agcgattgcc ccaggattct 1440
   tagacaccga tatatttaga cttcagacct tgcagcatgt aatgagaatg acacgcacat 1500
   gggactcaac aatgtctatg ttaccgaagg gaggtgacac gatatggggc gggcttgatt 1560
   ggtcaccgga gaaaggccac acctgttgtg ggaaaaagca aaagaacaac gaaacttgtg 1620
   gtgaagcagg tgaaaacgga gtttccaaga aaagtcctgt taactatgga aggatgatat 1680
   cttttgggaa agaagtagca gaggctgcgc catctgagat taataatatt gattttcgag 1740
   gtgctgtcaa aggtcagagt atcccaaatc acacctgtcg tgacgtgtgg acagagtacc 1800
   atgacatggg aattgctggg atcaaagcta tcgctgagta taaggtctac actgctggtg 1860
   aagctataga tctactacat tatgttgctc ctaagatgat ggcgcgtggt gccgctcatt 1920
   tctcttatgg aattgctgat gatttggatg acaccaagta tcaagatccc aaatactggt 1980
   caaatccgtt agagacaaaa ttaccgaatg ctcctgagat ggaaatctac tcattatacg 2040
   gagtggggat accaacggaa cgagcatacg tatacaagct taaccagtct cccgacagtt 2100
   gcatcccctt tcagatattc acttctgctc acgaggagga cgaagatagc tgtctgaaag 2160
   caggagttta caatgtggat ggggatgaaa cagtacccgt cctaagtgcc gggtacatgt 2220
   gtgcaaaagc gtggcgtggc aagacaagat tcaacccttc cggaatcaag acttatataa 2280
   gagaatacaa tcactctccg ccggctaacc tgttggaagg gcgcgggacg cagagtggtg 2340
   cccatgttga tatcatggga aactttgctt tgatcgaaga tatcatgagg gttgccgccg 2400
   gaggtaacgg gtctgatata ggacatgacc aggtccactc tggcatattt gaatggtcgg 2460
   agcgtattga cctgaagctg tgaatatcat gatctcttta agctgtcctg tcagcttatg 2520
   tgaatccaat actttgaaag agagatcatc atcaattcat catcatcgtc atcatcatga 2580
   tgctcaactc acaaagaagc ctgagaatga tactttggtg cgaaattctc aatacctctt 2640
   taatattctt attgaatgta aattatacaa tcctatctaa tgtttgaacg ataacacaaa 2700
   acttgctgcg ccatgtttgt ttgtcttgtc aaaagcatca atttgtgggt tatacgtagt 2760
   gtagaggatg attcaaattt gtgataaatt tggtaatcaa agttaattct g          2811
<210> 87
   <211> 671
   <212> PRT
   <213> A. thaliana
<400> 87
   Met Pro Leu Ile His Arg Lys Lys Pro Thr Glu Lys Pro Ser Thr Pro
   1 5 10 15
   Pro Ser Glu Glu Val Val His Asp Glu Asp Ser Gln Lys Lys Pro His
               20                  25                  30
   Glu Ser Ser Lys Ser His His Lys Lys Ser Asn Gly Gly Gly Lys Trp
           35                  40                  45
   Ser Cys Ile Asp Ser Cys Cys Trp Phe Ile Gly Cys Val Cys Val Thr
       50                  55                  60
   Trp Trp Phe Leu Leu Phe Leu Tyr Asn Ala Met Pro Ala Ser Phe Pro
   65                  70                  75                  80
   Gln Tyr Val Thr Glu Arg Ile Thr Gly Pro Leu Pro Asp Pro Pro Gly
                   85                  90                  95
   Val Lys Leu Lys Lys Glu Gly Leu Lys Ala Lys His Pro Val Val Phe
               100                 105                 110
   Ile Pro Gly Ile Val Thr Gly Gly Leu Glu Leu Trp Glu Gly Lys Gln
           115                 120                 125
   Cys Ala Asp Gly Leu Phe Arg Lys Arg Leu Trp Gly Gly Thr Phe Gly
       130                 135                 140
   Glu Val Tyr Lys Arg Pro Leu Cys Trp Val Glu His Met Ser Leu Asp
   145                 150                 155                 160
   Asn Glu Thr Gly Leu Asp Pro Ala Gly Ile Arg Val Arg Ala Val Ser
                   165                 170                 175
   Gly Leu Val Ala Ala Asp Tyr Phe Ala Pro Gly Tyr Phe Val Trp Ala
               180                 185                 190
   Val Leu Ile Ala Asn Leu Ala His Ile Gly Tyr Glu Glu Lys Asn Met
           195                 200                 205
   Tyr Met Ala Ala Tyr Asp Trp Arg Leu Ser Phe Gln Asn Thr Glu Val
       210                 215                 220
   Arg Asp Gln Thr Leu Ser Arg Met Lys Ser Asn Ile Glu Leu Met Val
   225                 230                 235                 240
   Ser Thr Asn Gly Gly Lys Lys Ala Val Ile Val Pro His Ser Met Gly
                   245                 250                 255
   Val Leu Tyr Phe Leu His Phe Met Lys Trp Val Glu Ala Pro Ala Pro
               260                 265                 270
   Leu Gly Gly Gly Gly Gly Pro Asp Trp Cys Ala Lys Tyr Ile Lys Ala
           275                 280                 285
   Val Met Asn Ile Gly Gly Pro Phe Leu Gly Val Pro Lys Ala Val Ala
   290 295 300
   Gly Leu Phe Ser Ala Glu Ala Lys Asp Val Ala Val Ala Arg Ala Ile
   305 310 315 320
   Ala Pro Gly Phe Leu Asp Thr Asp Ile Phe Arg Leu Gln Thr Leu Gln
   325 330 335
   His Val Met Arg Met Thr Arg Thr Trp Asp Ser Thr Met Ser Met Leu
   340 345 350
   Pro Lys Gly Gly Asp Thr Ile Trp Gly Gly Leu Asp Trp Ser Pro Glu
   355 360 365
   Lys Gly His Thr Cys Cys Gly Lys Lys Gln Lys Asn Asn Glu Thr Cys
   370 375 380
   Gly Glu Ala Gly Glu Asn Gly Val Ser Lys Lys Ser Pro Val Asn Tyr
   385 390 395 400
   Gly Arg Met Ile Ser Phe Gly Lys Glu Val Ala Glu Ala Ala Pro Ser
   405 410 415
   Glu Ile Asn Asn Ile Asp Phe Arg Gly Ala Val Lys Gly Gln Ser Ile
   420 425 430
   Pro Asn His Thr Cys Arg Asp Val Trp Thr Glu Tyr His Asp Met Gly
   435 440 445
   Ile Ala Gly Ile Lys Ala Ile Ala Glu Tyr Lys Val Tyr Thr Ala Gly
   450 455 460
   Glu Ala Ile Asp Leu Leu His Tyr Val Ala Pro Lys Met Met Ala Arg
   465 470 475 480
   Gly Ala Ala His Phe Ser Tyr Gly Ile Ala Asp Asp Leu Asp Asp Thr
   485 490 495
   Lys Tyr Gln Asp Pro Lys Tyr Trp Ser Asn Pro Leu Glu Thr Lys Leu
   500 505 510
   Pro Asn Ala Pro Glu Met Glu Ile Tyr Ser Leu Tyr Gly Val Gly Ile
   515 520 525
   Pro Thr Glu Arg Ala Tyr Val Tyr Lys Leu Asn Gln Ser Pro Asp Ser
   530 535 540
   Cys Ile Pro Phe Gln Ile Phe Thr Ser Ala His Glu Glu Asp Glu Asp
   545 550 555 560
   Ser Cys Leu Lys Ala Gly Val Tyr Asn Val Asp Gly Asp Glu Thr Val
   565 570 575
   Pro Val Leu Ser Ala Gly Tyr Met Cys Ala Lys Ala Trp Arg Gly Lys
   580 585 590
   Thr Arg Phe Asn Pro Ser Gly Ile Lys Thr Tyr Ile Arg Glu Tyr Asn
   595 600 605
   His Ser Pro Pro Ala Asn Leu Leu Glu Gly Arg Gly Thr Gln Ser Gly
   610 615 620
   Ala His Val Asp Ile Met Gly Asn Phe Ala Leu Ile Glu Asp Ile Met
   625 630 635 640
   Arg Val Ala Ala Gly Gly Asn Gly Ser Asp Ile Gly His Asp Gln Val
   645 650 655
   His Ser Gly Ile Phe Glu Trp Ser Glu Arg Ile Asp Leu Lys Leu
   660 665 670
<210> 88
   <211> 2473
   <212> DNA
   <213> R. communis
<400> 88
   cttgaatttt gtttcgatta ctttaaaaaa aattgccttt ttctgaaagc gcttctagtt      60
   tgatcttaat ggattattta cttttcagaa attagtagct caaacaaaat taagagagaa     120
   aaagagatcg tgggtttttt caaagaaaaa gtttcaaaca gaaagcacaa actttccgat     180
   gtggcttgcg aggaagttgg ggatgacata aattcttctg ccagtccgaa gttttggata     240
   taaagcagtg gtttttgtat tttccttttt ccttttctgt atcgtttagt aaagtcacat     300
   ttggcttatt gggtttgttt tatttcctct gtatttgctt tctgtacaaa gactatcaat     360
   aattagttaa taagcttaac aaatttaaaa atcttatttt tctttaaacc cagaaatgcc     420
   tgtaattcgg aggaaaaaac ccacttctga acccaacaaa aattcagcat cagactcaaa     480
   aacgccaagc gaggaagagg aacatgaaca agaacaggaa caagaagaag ataaaaataa     540
   caaaaagaaa tacccaaaga agaagagcag tgaaatcaat gcaaaaaaat ggtcatgcat     600
   agacagctgt tgttggtttg ttggttgcat ctgcgtgacg tggtgggttt tactatttct     660
   ttacaatgca gtgcctgcgt ctttgcctca atacgtaact gaggcaatca cgggtccttt     720
   acccgatcca cctggtgtta agctgaaaaa agagggatta acagcaaagc atccagtggt     780
   ttttgtacct gggattgtta ccgcggggct tgaattgtgg gaaggccatc agtgtgctga     840
   tgggctgttt aggaaacggc tctggggtgg aacttttgga gaagtttata agaggcctct     900
   ctgctgggta gagcatatgt ctctagacaa tgaaactgga ttggatcctc ctggtataag     960
   ggtcaggcca gtctctggac ttgtggctgc tgattacttt gctccaggct attttgtgtg    1020
   ggctgttctg attgctaatt tggcacgcat tggatatgag gagaaaacaa tgttcatggc    1080
   ctcatacgat tggagacttt catttcagaa cactgaggtc cgtgaccaaa cattaagccg     1140
   gatgaagagt aatatagaac ttatggtttc tatcaatggt ggaaataagg cagttattgt     1200
   tccacattcc atgggtgttt tgtactttct gcattttatg aagtgggttg aggcaccagc     1260
   tccaatggga ggaggtggtg gaccagattg gtgtgctaag catatcaagg cagtcatgaa     1320
   cattggtggt ccatttttag gtgttcccaa agctgttgct gggcttttct cggctgaagc     1380
   aagagatatt gcagttgcca gggccatagc accaggtttc ttagataatg atatgttccg     1440
   cctacaaaca ttgcaacaca tgatgaggat gtctcgcaca tgggattcga ccatgtcaat     1500
   gataccaaga ggtggggaca ctatctgggg cgatcttgat tggtcacctg aagaaggtta     1560
   cattcctaga aagaaaaggc agagaaataa tgcaactgat aatgtaaacg aaggtggggc     1620
   cgaaagtgag atttctcaaa gaaagattgt tagatatgga agaatgatat catttgggaa     1680
   aaatatagca gaggcacctt catatgatat tgaaaggatt gactttaggg atgctgttaa     1740
   aggtcgtagt gtggcaaata atacctgcct tgatgtgtgg actgaatacc atgaaatggg     1800
   attcggaggt attaaagccg ttgcagagta taaggtctac actgctggat ctactataga     1860
   gctgcttcag tttgtcgccc caaaaatgat ggagcgtggt agtgctcatt tttcttatgg     1920
   aattgctgac aatttggagg acccaaaata tgagcactac aaatactggt caaatcccct     1980
   ggagacaaag ttacctaatg ctccagaaat ggaaatattt tccatgtatg gagttggcat     2040
   accaacagaa agagcttatg tttatgagtt ttctcctgct gctgagtgct acattccatt     2100
   tcagattgat acatcagcta atgatggcga tgaagatggc tgtctgaaag atggagtcta     2160
   tactgttgat ggggatgaga ctgttcctgt tttaagtgca ggcttcatgt gtgctaaagc     2220
   ttggcgtggg aaaaccagat ttaatccttc aggaagtcga acatacatta gagagtacga     2280
   tcattctcct ccagctaatt tgctagaggg ccgaggcacc caaagtggtg cccatgttga     2340
   tataatgggt aattttgctt taatcgagga tattatgagg gtggcagccg gggctacagg     2400
   agaagatttg ggaggcgatc aagtgtattc agatatcttt aagtggtctc agaagatcaa     2460
   attaccactg taa                                                        2473
<210> 89
   <211> 685
   <212> PRT
   <213> R. communis
<400> 89
   Met Pro Val Ile Arg Arg Lys Lys Pro Thr Ser Glu Pro Asn Lys Asn
   1               5                   10                  15
   Ser Ala Ser Asp Ser Lys Thr Pro Ser Glu Glu Glu Glu His Glu Gln
               20                  25                  30
   Glu Gln Glu Gln Glu Glu Asp Lys Asn Asn Lys Lys Lys Tyr Pro Lys
           35                  40                  45
   Lys Lys Ser Ser Glu Ile Asn Ala Lys Lys Trp Ser Cys Ile Asp Ser
       50                  55                  60
   Cys Cys Trp Phe Val Gly Cys Ile Cys Val Thr Trp Trp Val Leu Leu
   65                  70                  75                  80
   Phe Leu Tyr Asn Ala Val Pro Ala Ser Leu Pro Gln Tyr Val Thr Glu
                   85                  90                  95
   Ala Ile Thr Gly Pro Leu Pro Asp Pro Pro Gly Val Lys Leu Lys Lys
               100                 105                 110
   Glu Gly Leu Thr Ala Lys His Pro Val Val Phe Val Pro Gly Ile Val
           115                 120                 125
   Thr Ala Gly Leu Glu Leu Trp Glu Gly His Gln Cys Ala Asp Gly Leu
       130                 135                 140
   Phe Arg Lys Arg Leu Trp Gly Gly Thr Phe Gly Glu Val Tyr Lys Arg
   145                 150                 155                 160
   Pro Leu Cys Trp Val Glu His Met Ser Leu Asp Asn Glu Thr Gly Leu
                   165                 170                 175
   Asp Pro Pro Gly Ile Arg Val Arg Pro Val Ser Gly Leu Val Ala Ala
               180                 185                 190
   Asp Tyr Phe Ala Pro Gly Tyr Phe Val Trp Ala Val Leu Ile Ala Asn
           195                 200                 205
   Leu Ala Arg Ile Gly Tyr Glu Glu Lys Thr Met Phe Met Ala Ser Tyr
       210                 215                 220
   Asp Trp Arg Leu Ser Phe Gln Asn Thr Glu Val Arg Asp Gln Thr Leu
   225                 230                 235                 240
   Ser Arg Met Lys Ser Asn Ile Glu Leu Met Val Ser Ile Asn Gly Gly
                   245                 250                 255
   Asn Lys Ala Val Ile Val Pro His Ser Met Gly Val Leu Tyr Phe Leu
               260                 265                 270
   His Phe Met Lys Trp Val Glu Ala Pro Ala Pro Met Gly Gly Gly Gly
           275                 280                 285
   Gly Pro Asp Trp Cys Ala Lys His Ile Lys Ala Val Met Asn Ile Gly
       290                 295                 300
   Gly Pro Phe Leu Gly Val Pro Lys Ala Val Ala Gly Leu Phe Ser Ala
   305                 310                 315                 320
   Glu Ala Arg Asp Ile Ala Val Ala Arg Ala Ile Ala Pro Gly Phe Leu
                   325                 330                 335
   Asp Asn Asp Met Phe Arg Leu Gln Thr Leu Gln His Met Met Arg Met
               340                 345                 350
   Ser Arg Thr Trp Asp Ser Thr Met Ser Met Ile Pro Arg Gly Gly Asp
           355                 360                 365
   Thr Ile Trp Gly Asp Leu Asp Trp Ser Pro Glu Glu Gly Tyr Ile Pro
       370                 375                 380
   Arg Lys Lys Arg Gln Arg Asn Asn Ala Thr Asp Asn Val Asn Glu Gly
   385                 390                 395                 400
   Gly Ala Glu Ser Glu Ile Ser Gln Arg Lys Ile Val Arg Tyr Gly Arg
                   405                 410                 415
   Met Ile Ser Phe Gly Lys Asn Ile Ala Glu Ala Pro Ser Tyr Asp Ile
               420                 425                 430
   Glu Arg Ile Asp Phe Arg Asp Ala Val Lys Gly Arg Ser Val Ala Asn
           435                 440                 445
   Asn Thr Cys Leu Asp Val Trp Thr Glu Tyr His Glu Met Gly Phe Gly
       450                 455                 460
   Gly Ile Lys Ala Val Ala Glu Tyr Lys Val Tyr Thr Ala Gly Ser Thr
   465                 470                 475                 480
   Ile Glu Leu Leu Gln Phe Val Ala Pro Lys Met Met Glu Arg Gly Ser
                   485                 490                 495
   Ala His Phe Ser Tyr Gly Ile Ala Asp Asn Leu Glu Asp Pro Lys Tyr
               500                 505                 510
   Glu His Tyr Lys Tyr Trp Ser Asn Pro Leu Glu Thr Lys Leu Pro Asn
           515                 520                 525
   Ala Pro Glu Met Glu Ile Phe Ser Met Tyr Gly Val Gly Ile Pro Thr
       530                 535                 540
   Glu Arg Ala Tyr Val Tyr Glu Phe Ser Pro Ala Ala Glu Cys Tyr Ile
   545                 550                 555                 560
   Pro Phe Gln Ile Asp Thr Ser Ala Asn Asp Gly Asp Glu Asp Gly Cys
                   565                 570                 575
   Leu Lys Asp Gly Val Tyr Thr Val Asp Gly Asp Glu Thr Val Pro Val
               580                 585                 590
   Leu Ser Ala Gly Phe Met Cys Ala Lys Ala Trp Arg Gly Lys Thr Arg
   595 600 605
   Phe Asn Pro Ser Gly Ser Arg Thr Tyr Ile Arg Glu Tyr Asp His Ser
   610 615 620
   Pro Pro Ala Asn Leu Leu Glu Gly Arg Gly Thr Gln Ser Gly Ala His
   625 630 635 640
   Val Asp Ile Met Gly Asn Phe Ala Leu Ile Glu Asp Ile Met Arg Val
   645 650 655
   Ala Ala Gly Ala Thr Gly Glu Asp Leu Gly Gly Asp Gln Val Tyr Ser
   660 665 670
   Asp Ile Phe Lys Trp Ser Gln Lys Ile Lys Leu Pro Leu
   675 680 685
<210> 90
   <211> 380
   <212> PRT
   <213> B. oleraceae
<400> 90
   Met Arg Asn Glu Ile Gln Asn Glu Thr Ala Gln Thr Asp Gln Thr Gln
   1               5                   10                  15
   Gly Ser Met Phe Ser Phe Phe Asp Leu Phe Pro Phe Leu Leu Pro Met
               20                  25                  30
   Phe Glu Val Ile Lys Met Val Val Ala Ser Val Ala Ser Val Val Tyr
           35                  40                  45
   Leu Gly Phe Ala Gly Val Thr Leu Ser Gly Ser Ala Val Ala Leu Ala
       50                  55                  60
   Val Ser Thr Pro Leu Phe Ile Ile Phe Ser Pro Ile Leu Leu Pro Ala
   65                  70                  75                  80
   Ile Ala Ala Thr Thr Val Leu Ala Ala Gly Leu Gly Ser Lys Lys Val
                   85                  90                  95
   Ala Ala Ala Pro Ala Ala Ser Pro Ser Leu Ser Leu Leu Gly Ile Pro
               100                 105                 110
   Glu Ser Ile Lys Pro Ser Asn Val Ile Pro Glu Ser Ile Lys Pro Ser
           115                 120                 125
   Asn Ile Ile Pro Glu Ser Ile Lys Pro Ser Asn Ile Ile Pro Glu Ser
       130                 135                 140
   Val Lys Pro Ser Asn Ile Lys Asp Lys Ile Lys Asp Thr Ile Gly Lys
   145                 150                 155                 160
   Val Lys Asn Lys Ile Asn Ala Lys Lys Glu Glu Lys Ser Lys Gly Lys
                   165                 170                 175
   Ser Glu Asp Ser Ser Lys Gly Lys Gly Lys Ser Lys Gly Glu Asp Thr
               180                 185                 190
   Thr Thr Asp Glu Asp Lys Pro Gly Ser Gly Gly Lys His Gly Lys Gly
           195                 200                 205
   Glu Ser Lys His Gly Lys Gly Glu Ser Thr His Gly Lys Gly Gly Lys
       210                 215                 220
   His Gly Ser Glu Gly Ser Ser Met Asp Glu Gly Lys His Gly Gly Lys
   225                 230                 235                 240
   His Gly Ser Gly Gly Ser Pro Met Gly Val Gly Lys His Gly Ser Gly
                   245                 250                 255
   Gly Lys His Glu Ser Gly Gly Ser Pro Met Gly Gly Gly Lys His Gly
               260                 265                 270
   Ser Gly Gly Lys His Glu Ser Gly Gly Ala Ser Met Gly Gly Gly Lys
           275                 280                 285
   His Gly Ser Gly Gly Arg His Glu Gly Gly Gly Ser Ala Met Gly Gly
       290                 295                 300
   Gly Lys His Gly Ser Gly Gly Lys His Gly Ser Glu Gly Lys His Gly
   305                 310                 315                 320
   Gly Glu Gly Ser Ser Met Gly Lys Asn Ser Leu Ser Lys Asn Lys Lys
                   325                 330                 335
   Glu Phe His Tyr Arg Asp Gln Ala Met Asp Ala Ser Ser Thr Ser Glu
               340                 345                 350
   Ser Ser Asp Gly Ser Ser Asp Gly Ser Ser Ser Asp Gly Ser Ser Ser
           355                 360                 365
   Asp Gly Ser Ser His Gly Ser Gly Gly Lys His Ile
       370                 375                 380
<210> 91
   <211> 186
   <212> PRT
   <213> Z. mays
<400> 91
   Met Ala Asp Arg Asp Arg Ser Gly Ile Tyr Gly Gly Ala His Ala Thr
   1               5                   10                  15
   Tyr Gly Gln Gln Gln Gln Gln Gly Gly Gly Gly Arg Pro Met Gly Glu
               20                  25                  30
   Gln Val Lys Gly Met Leu His Asp Lys Gly Pro Thr Ala Ser Gln Ala
           35                  40                  45
   Leu Thr Val Ala Thr Leu Phe Pro Leu Gly Gly Leu Leu Leu Val Leu
       50                  55                  60
   Ser Gly Leu Ala Leu Thr Ala Ser Val Val Gly Leu Ala Val Ala Thr
   65                  70                  75                  80
   Pro Val Phe Leu Ile Phe Ser Pro Val Leu Val Pro Ala Ala Leu Leu
                   85                  90                  95
   Ile Gly Thr Ala Val Met Gly Phe Leu Thr Ser Gly Ala Leu Gly Leu
               100                 105                 110
   Gly Gly Leu Ser Ser Leu Thr Cys Leu Ala Asn Thr Ala Arg Gln Ala
           115                 120                 125
   Phe Gln Arg Thr Pro Asp Tyr Val Glu Glu Ala His Arg Arg Met Ala
       130                 135                 140
   Glu Ala Ala Ala His Ala Gly His Lys Thr Ala Gln Ala Gly Gln Ala
   145                 150                 155                 160
   Ile Gln Gly Arg Ala Gln Glu Ala Gly Ala Gly Gly Gly Ala Gly Ala
                   165                 170                 175
   Gly Ala Gly Gly Gly Gly Arg Ala Ser Ser
               180                 185
<210> 92
   <211> 148
   <212> PRT
   <213> O. sativa
<400> 92
   Met Gly Asp Gln His Arg Gly Val Ile Gly Gly Gly Gly Tyr Gly Asp
   1               5                   10                  15
   Arg Gly Gly Gln Glu Gln Gln Glu Lys Gln Pro Phe Met Met Thr Ala
               20                  25                  30
   Leu Lys Thr Val Thr Ala Ala Thr Ala Gly Gly Ser Ile Leu Val Leu
           35                  40                  45
   Ser Gly Leu Ile Leu Ala Gly Thr Val Ile Ala Leu Thr Val Ala Thr
       50                  55                  60
   Pro Val Leu Val Ile Phe Ser Pro Val Leu Val Pro Ala Ala Ile Ala
   65                  70                  75                  80
   Leu Ala Leu Met Ala Ala Gly Phe Val Thr Ser Val Gly Leu Gly Val
                   85                  90                  95
   Ala Ala Leu Ser Val Phe Ser Trp Met Tyr Lys Tyr Leu Thr Gly Lys
               100                 105                 110
   His Pro Pro Gly Ala Asp His Leu Asp His Thr Lys Ala Arg Val Ala
           115                 120                 125
   Ser Lys Leu Arg Asp Ile Lys Glu Ala Ala His His Leu Ile Asp Gln
       130                 135                 140
   Ala Gln Ala Ser
   145
<210> 93
   <211> 245
   <212> PRT
   <213> S. indicum
<400> 93
   Met Ala Thr His Val Leu Ala Ala Ala Ala Glu Arg Asn Ala Ala Leu
   1 5 10 15
   Ala Pro Asp Ala Pro Leu Ala Pro Val Thr Met Glu Arg Pro Val Arg
   20 25 30
   Thr Asp Leu Glu Thr Ser Ile Pro Lys Pro Tyr Met Ala Arg Gly Leu
   35 40 45
   Val Ala Pro Asp Met Asp His Pro Asn Gly Thr Pro Gly His Val His
   50 55 60
   Asp Asn Leu Ser Val Leu Gln Gln His Cys Ala Phe Phe Asp Gln Asp
   65 70 75 80
   Asp Asn Gly Ile Ile Tyr Pro Trp Glu Thr Tyr Ser Gly Leu Arg Gln
   85 90 95
   Ile Gly Phe Asn Val Ile Ala Ser Leu Ile Met Ala Ile Val Ile Asn
   100 105 110
   Val Ala Leu Ser Tyr Pro Thr Leu Pro Gly Trp Ile Pro Ser Pro Phe
   115 120 125
   Phe Pro Ile Tyr Leu Tyr Asn Ile His Lys Ala Lys His Gly Ser Asp
   130 135 140
   Ser Gly Thr Tyr Asp Thr Glu Gly Arg Tyr Leu Pro Met Asn Phe Glu
   145 150 155 160
   Asn Leu Phe Ser Lys His Ala Arg Thr Met Pro Asp Arg Leu Thr Leu
   165 170 175
   Gly Glu Leu Trp Ser Met Thr Glu Ala Asn Arg Glu Ala Phe Asp Ile
   180 185 190
   Phe Gly Trp Ile Ala Ser Lys Met Glu Trp Thr Leu Leu Tyr Ile Leu
   195 200 205
   Ala Arg Asp Gln Asp Gly Phe Leu Ser Lys Glu Ala Ile Arg Arg Cys
   210 215 220
   Tyr Asp Gly Ser Leu Phe Glu Tyr Cys Ala Lys Met Gln Arg Gly Ala
   225 230 235 240
   Glu Asp Lys Met Lys
   245
<210> 94
   <211> 239
   <212> PRT
   <213> G. max
<400> 94
   Met Ala Ala Glu Met Glu Arg Glu Ser Leu Ile Thr Glu Ala Pro Asn
   1               5                   10                  15
   Ala Pro Val Thr Ala Gln Arg Arg Val Arg Asn Asp Leu Glu Asn Ser
               20                  25                  30
   Leu Pro Lys Pro Tyr Leu Pro Arg Ala Leu Lys Ala Pro Asp Thr Gly
           35                  40                  45
   His Pro Asn Gly Thr Ala Gly His Arg His His Asn Leu Ser Val Leu
       50                  55                  60
   Gln Gln His Cys Ala Phe Phe Asp Gln Asp Asp Asn Gly Ile Ile Tyr
   65                  70                  75                  80
   Pro Trp Glu Thr Tyr Met Gly Leu Arg Ser Ile Gly Phe Asn Val Val
                   85                  90                  95
   Ala Ser Val Ile Met Ala Ile Val Ile Asn Val Gly Leu Ser Tyr Pro
               100                 105                 110
   Thr Leu Pro Asn Trp Phe Pro Ser Leu Leu Phe Pro Ile Tyr Ile His
           115                 120                 125
   Asn Ile His Lys Ala Lys His Gly Ser Asp Ser Gly Val Tyr Asp Thr
       130                 135                 140
   Glu Gly Arg Tyr Val Pro Ala Asn Ile Glu Asn Ile Phe Ser Lys Tyr
   145                 150                 155                 160
   Ala Arg Thr Val Pro Asp Lys Leu Thr Leu Gly Glu Leu Trp Asp Leu
                   165                 170                 175
   Thr Glu Gly Asn Arg Asn Ala Phe Asp Ile Phe Gly Trp Leu Ala Ala
               180                 185                 190
   Lys Phe Glu Trp Gly Val Leu Tyr Ile Leu Ala Arg Asp Glu Glu Gly
           195                 200                 205
   Phe Leu Ser Lys Glu Ala Val Arg Arg Cys Phe Asp Gly Ser Leu Phe
       210                 215                 220
   Glu Tyr Cys Ala Lys Met His Thr Thr Ser Asp Ala Lys Met Ser
   225                 230                 235
<210> 95
   <211> 243
   <212> PRT
   <213> Z. mays
<400> 95
   Met Ser Ser Tyr Ser Pro Pro Pro Pro Pro Pro Arg Asp Gln Ser Met
   1               5                   10                  15
   Asp Thr Glu Ala Pro Asn Ala Pro Ile Thr Arg Glu Arg Arg Leu Asn
               20                  25                  30
   Pro Asp Leu Gln Glu Gln Leu Pro Lys Pro Tyr Leu Ala Arg Ala Leu
           35                  40                  45
   Glu Ala Val Asp Pro Ser His Pro Gln Gly Thr Lys Gly Arg Asp Pro
       50                  55                  60
   Arg Gly Met Ser Val Leu Gln Gln His Ala Ala Phe Phe Asp Arg Asn
   65                  70                  75                  80
   Gly Asp Gly Val Ile Tyr Pro Trp Glu Thr Phe Gln Gly Leu Arg Ala
                   85                  90                  95
   Ile Gly Cys Gly Leu Thr Val Ser Phe Ala Phe Ser Ile Leu Ile Asn
               100                 105                 110
   Leu Phe Leu Ser Tyr Pro Thr Gln Pro Gly Trp Leu Pro Ser Pro Leu
           115                 120                 125
   Leu Ser Ile Arg Ile Asp Asn Ile His Lys Gly Lys His Gly Ser Asp
   130 135 140
   Ser Glu Thr Tyr Asp Thr Glu Gly Arg Phe Asp Pro Ser Lys Phe Asp
   145 150 155 160
   Ala Ile Phe Ser Lys Tyr Gly Arg Thr His Pro Asn Ala Ile Thr Arg
   165 170 175
   Asp Glu Leu Ser Ser Met Leu Gln Gly Asn Arg Asn Thr Tyr Asp Phe
   180 185 190
   Leu Gly Trp Leu Ala Ala Ala Gly Glu Trp Leu Leu Leu Tyr Ser Leu
   195 200 205
   Ala Lys Asp Lys Asp Gly Leu Leu Gln Arg Glu Thr Val Arg Gly Leu
   210 215 220
   Phe Asp Gly Ser Leu Phe Glu Arg Leu Glu Asp Asp Asn Asn Lys Lys
   225 230 235 240
   Lys Ser Ser
<210> 96
   <211> 348
   <212> PRT
   <213> S. indicum
<400> 96
   Met Asp Leu Ile His Thr Phe Leu Asn Leu Ile Ala Pro Pro Phe Thr
   1               5                   10                  15
   Phe Phe Phe Leu Leu Phe Phe Leu Pro Pro Phe Gln Ile Phe Lys Phe
               20                  25                  30
   Phe Leu Ser Ile Leu Gly Thr Leu Phe Ser Glu Asp Val Ala Gly Lys
           35                  40                  45
   Val Val Val Ile Thr Gly Ala Ser Ser Gly Ile Gly Glu Ser Leu Ala
       50                  55                  60
   Tyr Glu Tyr Ala Lys Arg Gly Ala Cys Leu Val Leu Ala Ala Arg Arg
   65                  70                  75                  80
   Glu Arg Ser Leu Gln Glu Val Ala Glu Arg Ala Arg Asp Leu Gly Ser
                   85                  90                  95
   Pro Asp Val Val Val Val Arg Ala Asp Val Ser Lys Ala Glu Asp Cys
               100                 105                 110
   Arg Lys Val Val Asp Gln Thr Met Asn Arg Phe Gly Arg Leu Asp His
           115                 120                 125
   Leu Val Asn Asn Ala Gly Ile Met Ser Val Ser Met Leu Glu Glu Val
   130 135 140
   Glu Asp Ile Thr Gly Tyr Arg Glu Thr Met Asp Ile Asn Phe Trp Gly
   145 150 155 160
   Tyr Val Tyr Met Thr Arg Phe Ala Ala Pro Tyr Leu Arg Asn Ser Arg
   165 170 175
   Gly Arg Ile Val Val Leu Ser Ser Ser Ser Ser Trp Met Pro Thr Pro
   180 185 190
   Arg Met Ser Phe Tyr Asn Ala Ser Lys Ala Ala Ile Ser Gln Phe Phe
   195 200 205
   Glu Thr Leu Arg Val Glu Phe Gly Pro Asp Ile Gly Ile Thr Leu Val
   210 215 220
   Thr Pro Gly Phe Ile Glu Ser Glu Leu Thr Gln Gly Lys Phe Tyr Asn
   225 230 235 240
   Ala Gly Glu Arg Val Ile Asp Gln Asp Met Arg Asp Val Gln Val Ser
   245 250 255
   Thr Thr Pro Ile Leu Arg Val Glu Ser Ala Ala Arg Ser Ile Val Arg
   260 265 270
   Ser Ala Ile Arg Gly Glu Arg Tyr Val Thr Glu Pro Ala Trp Phe Arg
   275 280 285
   Val Thr Tyr Trp Trp Lys Leu Phe Cys Pro Glu Val Met Glu Trp Val
   290 295 300
   Phe Arg Leu Met Tyr Leu Ala Ser Pro Gly Glu Pro Glu Lys Glu Thr
   305 310 315 320
   Phe Gly Lys Lys Val Leu Asp Tyr Thr Gly Val Lys Ser Leu Leu Tyr
   325 330 335
   Pro Glu Thr Val Gln Val Pro Glu Pro Lys Asn Asp
   340 345
<210> 97
   <211> 349
   <212> PRT
   <213> Brassica napus
<400> 97
   Met Glu Leu Ile Asn Asp Phe Leu Asn Leu Thr Ala Pro Phe Phe Thr
   1               5                   10                  15
   Phe Phe Gly Leu Cys Phe Phe Leu Pro Pro Phe Tyr Phe Phe Lys Phe
               20                  25                  30
   Val Gln Ser Ile Phe Ser Thr Ile Phe Ser Glu Asn Val Tyr Gly Lys
           35                  40                  45
   Val Val Leu Ile Thr Gly Ala Ser Ser Gly Ile Gly Glu Gln Leu Ala
       50                  55                  60
   Tyr Glu Tyr Ala Ser Lys Gly Ala Cys Leu Ala Leu Thr Ala Arg Arg
   65                  70                  75                  80
   Lys Asn Arg Leu Glu Glu Val Ala Glu Ile Ala Arg Glu Val Gly Ser
                   85                  90                  95
   Pro Asn Val Val Thr Val His Ala Asp Val Ser Lys Pro Asp Asp Cys
               100                 105                 110
   Arg Arg Ile Val Asp Glu Thr Ile Ser His Phe Gly Arg Leu Asp His
           115                 120                 125
   Leu Val Asn Asn Ala Gly Ile Met Gln Ile Ser Met Phe Glu Asn Ile
       130                 135                 140
   Glu Glu Ile Thr Arg Thr Arg Ala Val Met Asp Thr Asn Phe Trp Gly
   145                 150                 155                 160
   Ala Val Tyr Thr Thr Arg Ala Ala Leu Pro Tyr Leu Arg Gln Ser Asn
                   165                 170                 175
   Gly Lys Ile Val Ala Met Ser Ser Ser Ala Ala Trp Leu Thr Ala Pro
               180                 185                 190
   Arg Met Ser Phe Tyr Asn Ala Ser Lys Ala Ala Leu Leu Asn Phe Phe
           195                 200                 205
   Glu Thr Leu Arg Ile Glu Leu Gly Ser Asp Val His Ile Thr Ile Val
       210                 215                 220
   Thr Pro Gly Tyr Ile Glu Ser Glu Leu Thr Gln Gly Lys Tyr Val Ser
   225                 230                 235                 240
   Gly Glu Gly Glu Leu Val Val Asn Gln Asp Ile Arg Asp Val Gln Ile
                   245                 250                 255
   Gly Ala Phe Pro Val Thr Ser Val Ser Gly Arg Ala Lys Gly Ile Val
               260                 265                 270
   Lys Gly Val Cys Arg Lys Glu Arg Tyr Val Thr Glu Pro Ser Trp Phe
           275                 280                 285
   Lys Val Thr Tyr Leu Trp Lys Val Phe Cys Pro Glu Leu Ile Glu Trp
   290 295 300
   Gly Cys Arg Leu Met Phe Leu Ser Gly His Gly Thr Pro Glu Glu Asn
   305 310 315 320
   Ala Leu Asn Lys Lys Ile Leu Asp Ile Pro Gly Val Arg Ser Ala Leu
   325 330 335
   Tyr Pro Glu Pro Ile Arg Thr Pro Glu Ile Lys Ser Glu
   340 345
<210> 98
   <211> 350
   <212> PRT
   <213> zea mays
<400> 98
   Met Leu Gly Met Ser Arg Thr Gly Leu Ala Gly Ala Ala Leu Arg Val
   1               5                   10                  15
   Ala Leu Thr Ala Leu Leu Pro Leu Val Leu Pro Ala Tyr Tyr Val Tyr
               20                  25                  30
   Lys Leu Thr Thr Tyr Leu Leu Gly Ala Val Phe Pro Glu Asp Val Ala
           35                  40                  45
   Gly Lys Val Val Leu Ile Thr Gly Ala Ser Ser Gly Ile Gly Glu His
       50                  55                  60
   Leu Ala Tyr Glu Tyr Ala Lys Arg Gly Ala Tyr Leu Ala Leu Val Ala
   65                  70                  75                  80
   Arg Arg Glu Ala Ser Leu Arg Glu Val Gly Asp Val Ala Leu Gly Leu
                   85                  90                  95
   Gly Ser Pro Gly Val Leu Val Leu Pro Ala Asp Val Ser Lys Pro Arg
               100                 105                 110
   Asp Cys Glu Gly Phe Ile Asp Asp Thr Ile Ser Tyr Phe Gly Arg Leu
           115                 120                 125
   Asp His Leu Val Asn Asn Ala Ser Ile Trp Gln Val Cys Lys Phe Glu
       130                 135                 140
   Glu Ile Gln Asp Val Arg His Leu Arg Ala Leu Met Asp Ile Asn Phe
   145                 150                 155                 160
   Trp Gly His Val Tyr Pro Thr Arg Leu Ala Ile Pro His Leu Arg Arg
                   165                 170                 175
   Ser Arg Gly Arg Ile Val Gly Val Thr Ser Asn Ser Ser Tyr Ile Phe
   180 185 190
   Ile Gly Arg Asn Thr Phe Tyr Asn Ala Ser Lys Ala Ala Ala Leu Ser
   195 200 205
   Phe Tyr Asp Thr Leu Arg Met Glu Leu Gly Ser Asp Ile Arg Ile Thr
   210 215 220
   Glu Val Val Pro Gly Val Val Glu Ser Glu Ile Thr Lys Gly Lys Met
   225 230 235 240
   Leu Thr Lys Gly Gly Glu Met Lys Val Asp Gln Asp Glu Arg Asp Ala
   245 250 255
   Ile Leu Gly Pro Thr Pro Ala Glu Pro Val Gly Asp Phe Ala Arg Thr
   260 265 270
   Val Val Arg Asp Val Cys Arg Gly Ala Arg Tyr Val Phe Glu Pro Arg
   275 280 285
   Trp Tyr Met Gly Val Tyr Leu Leu Arg Ala Cys Leu Pro Glu Val Leu
   290 295 300
   Ala Trp Asn Ser Arg Leu Leu Thr Val Asp Thr Val Gly Ala Ser Thr
   305 310 315 320
   Thr Asp Thr Leu Gly Lys Trp Leu Val Glu Leu Pro Gly Val Arg Arg
   325 330 335
   Val Val Gln Pro Pro Ser Leu Arg Ser Pro Glu Ile Lys Asp
   340 345 350
<210> 99
   <211> 380
   <212> PRT
   <213> Brassica oleracea
<400> 99
   Met Arg Asn Cys Ile Gln Asn Glu Thr Ala Gln Thr Cys Gln Thr Gln
   1               5                   10                  15
   Gly Ser Met Phe Ser Phe Phe Cys Leu Phe Pro Phe Leu Leu Pro Met
               20                  25                  30
   Phe Glu Val Ile Lys Met Val Val Ala Ser Val Ala Ser Val Val Tyr
           35                  40                  45
   Leu Gly Phe Ala Gly Val Thr Leu Ser Gly Ser Ala Val Ala Leu Ala
       50                  55                  60
   Val Ser Thr Pro Leu Phe Ile Ile Phe Ser Pro Ile Leu Leu Pro Ala
   65 70 75 80
   Ile Ala Ala Thr Thr Val Leu Ala Ala Gly Leu Gly Ser Lys Lys Val
   85 90 95
   Ala Ala Ala Pro Ala Ala Ser Pro Ser Leu Ser Leu Leu Gly Ile Pro
   100 105 110
   Glu Ser Ile Lys Pro Ser Asn Val Ile Pro Glu Ser Ile Lys Pro Ser
   115 120 125
   Asn Ile Ile Pro Glu Ser Ile Lys Pro Ser Asn Ile Ile Pro Glu Ser
   130 135 140
   Val Lys Pro Ser Asn Ile Lys Asp Lys Ile Lys Asp Thr Ile Gly Lys
   145 150 155 160
   Val Lys Asn Lys Ile Asn Ala Lys Lys Glu Glu Lys Ser Lys Gly Lys
   165 170 175
   Ser Glu Asp Ser Ser Lys Gly Lys Gly Lys Ser Lys Gly Glu Asp Thr
   180 185 190
   Thr Thr Asp Glu Asp Lys Pro Gly Ser Gly Gly Lys His Gly Lys Gly
   195 200 205
   Glu Ser Lys His Gly Lys Gly Glu Ser Thr His Gly Lys Gly Gly Lys
   210 215 220
   His Gly Ser Glu Gly Ser Ser Met Asp Glu Gly Lys His Gly Gly Lys
   225 230 235 240
   His Gly Ser Gly Gly Ser Pro Met Gly Val Gly Lys His Gly Ser Gly
   245 250 255
   Gly Lys His Glu Ser Gly Gly Ser Pro Met Gly Gly Gly Lys His Gly
   260 265 270
   Ser Gly Gly Lys His Glu Ser Gly Gly Ala Ser Met Gly Gly Gly Lys
   275 280 285
   His Gly Ser Gly Gly Arg His Glu Gly Gly Gly Ser Ala Met Gly Gly
   290 295 300
   Gly Lys His Gly Ser Gly Gly Lys His Gly Ser Glu Gly Lys His Gly
   305 310 315 320
   Gly Glu Gly Ser Ser Met Gly Lys Asn Ser Leu Ser Lys Asn Lys Lys
   325 330 335
   Glu Phe His Tyr Arg Asp Gln Ala Met Cys Ala Ser Ser Thr Ser Glu
   340 345 350
   Ser Ser Asp Gly Ser Ser Cys Gly Ser Ser Ser Asp Gly Ser Ser Ser
   355 360 365
   Cys Gly Ser Ser His Gly Ser Gly Gly Lys His Ile
   370 375 380
<210> 100
   <211> 186
   <212> PRT
   <213> zea mays
<400> 100
   Met Ala Asp Arg Cys Arg Ser Gly Ile Tyr Gly Gly Ala His Ala Thr
   1 5 10 15
   Tyr Gly Gln Gln Gln Gln Gln Gly Gly Gly Gly Arg Pro Met Gly Cys
   20 25 30
   Gln Val Lys Gly Met Leu His Cys Lys Gly Pro Thr Ala Ser Gln Ala
   35 40 45
   Leu Thr Val Ala Thr Leu Phe Pro Leu Gly Gly Leu Leu Leu Val Leu
   50 55 60
   Ser Gly Leu Ala Leu Thr Ala Ser Val Val Gly Leu Ala Val Ala Thr
   65 70 75 80
   Pro Val Phe Leu Ile Phe Ser Pro Val Leu Val Pro Ala Ala Leu Leu
   85 90 95
   Ile Gly Thr Ala Val Met Gly Phe Leu Thr Ser Gly Ala Leu Gly Leu
   100 105 110
   Gly Gly Leu Ser Ser Leu Thr Cys Leu Ala Asn Thr Ala Arg Gln Ala
   115 120 125
   Phe Gln Arg Thr Pro Asp Tyr Val Cys Glu Ala His Arg Arg Met Ala
   130 135 140
   Cys Ala Ala Ala His Ala Gly His Lys Thr Ala Gln Ala Gly Gln Ala
   145 150 155 160
   Ile Gln Gly Arg Ala Gln Cys Ala Gly Ala Gly Gly Gly Ala Gly Ala
   165 170 175
   Gly Ala Gly Gly Gly Gly Arg Ala Ser Ser
   180 185
<210> 101
   <211> 148
   <212> PRT
   <213> oryza sativa
<400> 101
   Met Gly Asp Gln His Cys Gly Val Ile Gly Gly Gly Gly Tyr Gly Cys
   1               5                   10                  15
   Arg Gly Gly Gln Glu Gln Gln Cys Lys Gln Pro Phe Met Met Thr Ala
               20                  25                  30
   Leu Lys Thr Val Thr Ala Ala Thr Ala Gly Gly Ser Ile Leu Val Leu
           35                  40                  45
   Ser Gly Leu Ile Leu Ala Gly Thr Val Ile Ala Leu Thr Val Ala Thr
       50                  55                  60
   Pro Val Leu Val Ile Phe Ser Pro Val Leu Val Pro Ala Ala Ile Ala
   65                  70                  75                  80
   Leu Ala Leu Met Ala Ala Gly Phe Val Thr Ser Val Gly Leu Gly Val
                   85                  90                  95
   Ala Ala Leu Ser Val Phe Ser Trp Met Tyr Lys Tyr Leu Thr Gly Lys
               100                 105                 110
   His Pro Pro Gly Ala Asp His Leu Cys His Thr Lys Ala Arg Val Ala
           115                 120                 125
   Ser Lys Leu Arg Cys Ile Lys Glu Ala Ala His His Leu Ile Cys Gln
       130                 135                 140
   Ala Gln Ala Ser
   145
<210> 102
   <211> 245
   <212> PRT
   <213> S. indicum
<400> 102
   Met Ala Thr His Val Leu Ala Ala Ala Ala Cys Arg Asn Ala Ala Leu
   1               5                   10                  15
   Ala Pro Cys Ala Pro Leu Ala Pro Val Thr Met Cys Arg Pro Val Arg
               20                  25                  30
   Thr Asp Leu Glu Thr Ser Ile Pro Lys Pro Tyr Met Ala Arg Gly Leu
           35                  40                  45
   Val Ala Pro Asp Met Asp His Pro Asn Gly Thr Pro Gly His Val His
       50                  55                  60
   Asp Asn Leu Ser Val Leu Gln Gln His Cys Ala Phe Phe Asp Gln Asp
   65                  70                  75                  80
   Asp Asn Gly Ile Ile Tyr Pro Trp Glu Thr Tyr Ser Gly Leu Arg Gln
                   85                  90                  95
   Ile Gly Phe Asn Val Ile Ala Ser Leu Ile Met Ala Ile Val Ile Asn
               100                 105                 110
   Val Ala Leu Ser Tyr Pro Thr Leu Pro Gly Trp Ile Pro Ser Pro Phe
           115                 120                 125
   Phe Pro Ile Tyr Leu Tyr Asn Ile His Lys Ala Lys His Gly Ser Asp
       130                 135                 140
   Ser Gly Thr Tyr Asp Thr Glu Gly Arg Tyr Leu Pro Met Asn Phe Glu
   145                 150                 155                 160
   Asn Leu Phe Ser Lys His Ala Arg Thr Met Pro Asp Arg Leu Thr Leu
                   165                 170                 175
   Gly Glu Leu Trp Ser Met Thr Glu Ala Asn Arg Glu Ala Phe Asp Ile
               180                 185                 190
   Phe Gly Trp Ile Ala Ser Lys Met Glu Trp Thr Leu Leu Tyr Ile Leu
           195                 200                 205
   Ala Arg Cys Gln Asp Gly Phe Leu Ser Cys Glu Ala Ile Arg Arg Cys
       210                 215                 220
   Tyr Asp Gly Ser Leu Phe Glu Tyr Cys Ala Lys Met Gln Arg Gly Ala
   225                 230                 235                 240
   Glu Asp Lys Met Cys
                   245
<210> 103
   <211> 239
   <212> PRT
   <213> G. soja
<400> 103
   Met Ala Ala Cys Met Glu Arg Glu Ser Leu Ile Thr Cys Ala Pro Asn
   1               5                   10                  15
   Ala Pro Val Thr Ala Gln Arg Cys Val Arg Asn Asp Leu Glu Asn Ser
               20                  25                  30
   Leu Pro Lys Pro Tyr Leu Pro Arg Ala Leu Lys Ala Pro Asp Thr Gly
           35                  40                  45
   His Pro Asn Gly Thr Ala Gly His Arg His His Asn Leu Ser Val Leu
       50                  55                  60
   Gln Gln His Cys Ala Phe Phe Asp Gln Asp Asp Asn Gly Ile Ile Tyr
   65                  70                  75                  80
   Pro Trp Glu Thr Tyr Met Gly Leu Arg Ser Ile Gly Phe Asn Val Val
                   85                  90                  95
   Ala Ser Val Ile Met Ala Ile Val Ile Asn Val Gly Leu Ser Tyr Pro
               100                 105                 110
   Thr Leu Pro Asn Trp Phe Pro Ser Leu Leu Phe Pro Ile Tyr Ile His
           115                 120                 125
   Asn Ile His Lys Ala Lys His Gly Ser Asp Ser Gly Val Tyr Asp Thr
       130                 135                 140
   Glu Gly Arg Tyr Val Pro Ala Asn Ile Glu Asn Ile Phe Ser Lys Tyr
   145                 150                 155                 160
   Ala Arg Thr Val Pro Asp Lys Leu Thr Leu Gly Glu Leu Trp Asp Leu
                   165                 170                 175
   Thr Glu Gly Asn Arg Asn Ala Phe Asp Ile Phe Gly Trp Leu Ala Ala
               180                 185                 190
   Lys Phe Glu Trp Gly Val Leu Tyr Ile Leu Ala Arg Cys Glu Glu Gly
           195                 200                 205
   Phe Leu Ser Lys Cys Ala Val Arg Arg Cys Phe Asp Gly Ser Leu Phe
       210                 215                 220
   Glu Tyr Cys Ala Lys Met His Thr Thr Ser Asp Ala Cys Met Ser
   225                 230                 235
<210> 104
   <211> 243
   <212> PRT
   <213> Z. mays
<400> 104
   Met Ser Ser Tyr Ser Pro Pro Pro Pro Pro Pro Arg Asp Gln Ser Met
   1               5                   10                  15
   Asp Thr Cys Ala Pro Asn Ala Pro Ile Thr Cys Glu Arg Arg Leu Asn
               20                  25                  30
   Pro Asp Leu Gln Cys Gln Leu Pro Lys Pro Tyr Leu Ala Arg Ala Leu
           35                  40                  45
   Glu Ala Val Asp Pro Ser His Pro Gln Gly Thr Lys Gly Arg Asp Pro
       50                  55                  60
   Arg Gly Met Ser Val Leu Gln Gln His Ala Ala Phe Phe Asp Arg Asn
   65 70 75 80
   Gly Asp Gly Val Ile Tyr Pro Trp Glu Thr Phe Gln Gly Leu Arg Ala
   85 90 95
   Ile Gly Cys Gly Leu Thr Val Ser Phe Ala Phe Ser Ile Leu Ile Asn
   100 105 110
   Leu Phe Leu Ser Tyr Pro Thr Gln Pro Gly Trp Leu Pro Ser Pro Leu
   115 120 125
   Leu Ser Ile Arg Ile Asp Asn Ile His Lys Gly Lys His Gly Ser Asp
   130 135 140
   Ser Glu Thr Tyr Asp Thr Glu Gly Arg Phe Asp Pro Ser Lys Phe Asp
   145 150 155 160
   Ala Ile Phe Ser Lys Tyr Gly Arg Thr His Pro Asn Ala Ile Thr Arg
   165 170 175
   Asp Glu Leu Ser Ser Met Leu Gln Gly Asn Arg Asn Thr Tyr Asp Phe
   180 185 190
   Leu Gly Trp Leu Ala Ala Ala Gly Glu Trp Leu Leu Leu Tyr Ser Leu
   195 200 205
   Ala Lys Asp Lys Asp Gly Leu Leu Gln Arg Cys Thr Val Arg Gly Leu
   210 215 220
   Phe Asp Gly Ser Leu Phe Cys Arg Leu Glu Asp Asp Asn Asn Lys Lys
   225 230 235 240
   Cys Ser Ser
<210> 105
   <211> 348
   <212> PRT -
   <213> S. indicum
<400> 105
   Met Asp Leu Ile Cys Thr Phe Leu Asn Leu Ile Ala Pro Pro Phe Thr
   1 5 10 15
   Phe Phe Phe Leu Leu Phe Phe Leu Pro Pro Phe Cys Ile Phe Lys Phe
   20 25 30
   Phe Leu Cys Ile Leu Gly Thr Leu Phe Ser Glu Asp Val Ala Gly Lys
   35 40 45
   Val Val Val Ile Thr Gly Ala Ser Ser Gly Ile Gly Glu Ser Leu Ala
       50                  55                  60
   Tyr Glu Tyr Ala Lys Arg Gly Ala Cys Leu Val Leu Ala Ala Arg Arg
   65                  70                  75                  80
   Glu Arg Ser Leu Gln Glu Val Ala Glu Arg Ala Arg Asp Leu Gly Ser
                   85                  90                  95
   Pro Asp Val Val Val Val Arg Ala Asp Val Ser Lys Ala Glu Asp Cys
               100                 105                 110
   Arg Lys Val Val Asp Gln Thr Met Asn Arg Phe Gly Arg Leu Asp His
           115                 120                 125
   Leu Val Asn Asn Ala Gly Ile Met Ser Val Ser Met Leu Glu Glu Val
       130                 135                 140
   Glu Asp Ile Thr Gly Tyr Arg Glu Thr Met Asp Ile Asn Phe Trp Gly
   145                 150                 155                 160
   Tyr Val Tyr Met Thr Arg Phe Ala Ala Pro Tyr Leu Arg Asn Ser Arg
                   165                 170                 175
   Gly Arg Ile Val Val Leu Ser Ser Ser Ser Ser Trp Met Pro Thr Pro
               180                 185                 190
   Arg Met Ser Phe Tyr Asn Ala Ser Lys Ala Ala Ile Ser Gln Phe Phe
           195                 200                 205
   Glu Thr Leu Arg Val Glu Phe Gly Pro Asp Ile Gly Ile Thr Leu Val
       210                 215                 220
   Thr Pro Gly Phe Ile Glu Ser Glu Leu Thr Gln Gly Lys Phe Tyr Asn
   225                 230                 235                 240
   Ala Gly Glu Arg Val Ile Asp Gln Asp Met Arg Asp Val Gln Val Ser
                   245                 250                 255
   Thr Thr Pro Ile Leu Arg Val Glu Ser Ala Ala Arg Ser Ile Val Arg
               260                 265                 270
   Ser Ala Ile Arg Gly Glu Arg Tyr Val Thr Glu Pro Ala Trp Phe Arg
           275                 280                 285
   Val Thr Tyr Trp Trp Lys Leu Phe Cys Pro Glu Val Met Glu Trp Val
       290                 295                 300
   Phe Arg Leu Met Tyr Leu Ala Ser Pro Gly Cys Pro Glu Lys Cys Thr
   305                 310                 315                 320
   Phe Gly Lys Lys Val Leu Cys Tyr Thr Gly Val Lys Ser Leu Leu Tyr
   325 330 335
   Pro Glu Thr Val Gln Val Pro Glu Pro Lys Asn Asp
   340 345
<210> 106
   <211> 349
   <212> PRT
   <213> Brassica napus
<400> 106
   Met Glu Leu Ile Asn Cys Phe Leu Asn Leu Thr Ala Pro Phe Phe Thr
   1               5                   10                  15
   Phe Phe Gly Leu Cys Phe Phe Leu Pro Pro Phe Tyr Phe Phe Cys Phe
               20                  25                  30
   Val Gln Cys Ile Phe Ser Thr Ile Phe Ser Glu Asn Val Tyr Gly Lys
           35                  40                  45
   Val Val Leu Ile Thr Gly Ala Ser Ser Gly Ile Gly Glu Gln Leu Ala
       50                  55                  60
   Tyr Glu Tyr Ala Ser Lys Gly Ala Cys Leu Ala Leu Thr Ala Arg Arg
   65                  70                  75                  80
   Lys Asn Arg Leu Glu Glu Val Ala Glu Ile Ala Arg Glu Val Gly Ser
                   85                  90                  95
   Pro Asn Val Val Thr Val His Ala Asp Val Ser Lys Pro Asp Asp Cys
               100                 105                 110
   Arg Arg Ile Val Asp Glu Thr Ile Ser His Phe Gly Arg Leu Asp His
           115                 120                 125
   Leu Val Asn Asn Ala Gly Ile Met Gln Ile Ser Met Phe Glu Asn Ile
       130                 135                 140
   Glu Glu Ile Thr Arg Thr Arg Ala Val Met Asp Thr Asn Phe Trp Gly
   145                 150                 155                 160
   Ala Val Tyr Thr Thr Arg Ala Ala Leu Pro Tyr Leu Arg Gln Ser Asn
                   165                 170                 175
   Gly Lys Ile Val Ala Met Ser Ser Ser Ala Ala Trp Leu Thr Ala Pro
               180                 185                 190
   Arg Met Ser Phe Tyr Asn Ala Ser Lys Ala Ala Leu Leu Asn Phe Phe
           195                 200                 205
   Glu Thr Leu Arg Ile Glu Leu Gly Ser Asp Val His Ile Thr Ile Val
       210                 215                 220
   Thr Pro Gly Tyr Ile Glu Ser Glu Leu Thr Gln Gly Lys Tyr Val Ser
   225                 230                 235                 240
   Gly Glu Gly Glu Leu Val Val Asn Gln Asp Ile Arg Asp Val Gln Ile
                   245                 250                 255
   Gly Ala Phe Pro Val Thr Ser Val Ser Gly Arg Ala Lys Gly Ile Val
               260                 265                 270
   Lys Gly Val Cys Arg Lys Glu Arg Tyr Val Thr Glu Pro Ser Trp Phe
           275                 280                 285
   Lys Val Thr Tyr Leu Trp Lys Val Phe Cys Pro Glu Leu Ile Glu Trp
       290                 295                 300
   Gly Cys Arg Leu Met Phe Leu Ser Gly His Gly Thr Pro Cys Glu Asn
   305                 310                 315                 320
   Ala Leu Asn Lys Lys Ile Leu Asp Ile Pro Gly Val Arg Ser Ala Leu
                   325                 330                 335
   Tyr Pro Cys Pro Ile Arg Thr Pro Cys Ile Lys Ser Glu
               340                 345
<210> 107
   <211> 349
   <212> PRT
   <213> z. mays
<400> 107
   Met Glu Leu Ile Asn Cys Phe Leu Asn Leu Thr Ala Pro Phe Phe Thr
   1               5                   10                  15
   Phe Phe Gly Leu Cys Phe Phe Leu Pro Pro Phe Tyr Phe Phe Cys Phe
               20                  25                  30
   Val Gln Cys Ile Phe Ser Thr Ile Phe Ser Glu Asn Val Tyr Gly Lys
           35                  40                  45
   Val Val Leu Ile Thr Gly Ala Ser Ser Gly Ile Gly Glu Gln Leu Ala
       50                  55                  60
   Tyr Glu Tyr Ala Ser Lys Gly Ala Cys Leu Ala Leu Thr Ala Arg Arg
   65                  70                  75                  80
   Lys Asn Arg Leu Glu Glu Val Ala Glu Ile Ala Arg Glu Val Gly Ser
                   85                  90                  95
   Pro Asn Val Val Thr Val His Ala Asp Val Ser Lys Pro Asp Asp Cys
                   100                 105                 110
   Arg Arg Ile Val Asp Glu Thr Ile Ser His Phe Gly Arg Leu Asp His
           115                 120                 125
   Leu Val Asn Asn Ala Gly Ile Met Gln Ile Ser Met Phe Glu Asn Ile
       130                 135                 140
   Glu Glu Ile Thr Arg Thr Arg Ala Val Met Asp Thr Asn Phe Trp Gly
   145                 150                 155                 160
   Ala Val Tyr Thr Thr Arg Ala Ala Leu Pro Tyr Leu Arg Gln Ser Asn
                   165                 170                 175
   Gly Lys Ile Val Ala Met Ser Ser Ser Ala Ala Trp Leu Thr Ala Pro
               180                 185                 190
   Arg Met Ser Phe Tyr Asn Ala Ser Lys Ala Ala Leu Leu Asn Phe Phe
           195                 200                 205
   Glu Thr Leu Arg Ile Glu Leu Gly Ser Asp Val His Ile Thr Ile Val
       210                 215                 220
   Thr Pro Gly Tyr Ile Glu Ser Glu Leu Thr Gln Gly Lys Tyr Val Ser
   225                 230                 235                 240
   Gly Glu Gly Glu Leu Val Val Asn Gln Asp Ile Arg Asp Val Gln Ile
                   245                 250                 255
   Gly Ala Phe Pro Val Thr Ser Val Ser Gly Arg Ala Lys Gly Ile Val
               260                 265                 270
   Lys Gly Val Cys Arg Lys Glu Arg Tyr Val Thr Glu Pro Ser Trp Phe
           275                 280                 285
   Lys Val Thr Tyr Leu Trp Lys Val Phe Cys Pro Glu Leu Ile Glu Trp
       290                 295                 300
   Gly Cys Arg Leu Met Phe Leu Ser Gly His Gly Thr Pro Cys Glu Asn
   305                 310                 315                 320
   Ala Leu Asn Lys Lys Ile Leu Asp Ile Pro Gly Val Arg Ser Ala Leu
                   325                 330                 335
   Tyr Pro Cys Pro Ile Arg Thr Pro Cys Ile Lys Ser Glu
               340                 345

## Description

### TECHNICAL FIELD

The invention relates to compositions and methods for the production and modification of oil bodies in various host cell types.

### BACKGROUND

In nature, flowering plants efficiently store energy in their seeds through the accumulation of oil, namely triacylglycerol (TAG) and store it in discreet oil bodies by embedding a phospholipid protein monolayer around the oil body. These seed crops have been used in a variety of agricultural applications as feed and more recently also as a feedstock source for biofuels. On a per weight basis, lipids have approximately double the energy content of either proteins or carbohydrates and as such, substantial focus has been placed on raising the oil content of various species, most notably plants. Beyond the energy aspect, the oil bodies themselves also have unique properties and form the basis for a number of biotechnical applications including but not limited to the purification of recombinant proteins, formation of multimeric protein complexes, emulsification and the delivery of bio-actives.

Unfortunately plant seeds represent a very small percentage of total plant biomass and with the demand for improved agricultural productivity and alternative energies it is recognised that current oil production from a number of devoted seed crops is insufficient. Research efforts have focused on not only increasing the productivity of oil production within plant seeds but also oil production in other cell types and species.

Traditional breeding and mutagenesis have offered incremental successes in this area; however genetic engineering has made the furthest strides in modifying organisms to produce elevated oil levels. While certain groups have worked along various parts of the oil synthesis pathway to up-regulate oil production within the, seed, others groups have focused on increasing oil in cell types that represent a larger portion of the biomass.

While genetic engineering has made some progress in increasing oil content in certain targets, significant challenges still remain. Further productivity increases can still be realized in oil body production in the seed and the means to produce oil bodies similar to those of a plant seed in other cell types and species has yet to be achieved.

### SUMMARY OF THE INVENTION

The present invention provides compositions and methods for producing oil bodies with varying degrees of stability. The invention involves producing modified oleosins with artificially introduced cysteine residues. The artificially introduced cysteine residues are introduced in the N- and C-terminal hydrophilic arms of the modified oleosins.

Expression of the modified oleosins allows for the creation of stable oil bodies beyond the reproductive tissue of vascular plants into new cell types and even other species. When combined with a TAG synthesising enzyme, the invention leads to the accumulation and storage of TAG in eukaryotic cells as stable oil bodies. Compared with an unmodified cell or even one expressing just a TAG synthesis enzyme, the invention allows for the accumulation of TAG in excess levels achieved by other means. For example the invention has shown that one can accumulate higher levels of stable oil bodies beyond the seed, in the vegetative portion of vascular plants.

Plants with increased levels of TAG in their vegetative tissues provide a valuable energy source for both animal feedstock and biofuel feedstock applications.

In addition recombinant modified oleosins purified from a host cell (such as *E. coli, P. pastoris, S. ceriviseae, Dunaliella, C. reinhardtii*) can be used to generate artificial oil bodies. The modified oleosins in artificial oil bodies, or those purified form transformed cells, can optionally be made to cross-link via the cysteine residues in the modified oleosin. The degree of cross-linking may be controlled manipulating the redox environment. The degree of cross-linking can also be tailored by altering the number of cysteines in the modified oleosins.

Using combinations of these techniques the oil bodies formed with the modified oleosins can be tailored for their emulsification properties, to regulate thermal stability, chemical stability, and peptidase resistance.

The modified oleosins can also be fused to a protein of interest, to form a fusion protein. The fusion protein (modified oleosin plus protein of interest) can be recombinantly expressed in a cell or organism. In this way oil bodies containing the expressed fusion proteins can be used to purify and deliver the protein of interest, for a variety of applications.

In addition the oil bodies can protect, or at least delay, degradation and/or biohydrogenation, of TAG, within the stomach and/or rumen of an animal, allowing the intact individual lipids from the TAG to be absorbed by the animal in the intestine. Therefore, the invention is also useful in terms of dietary intake of an animal, particularly through expression of the modified oleosins in plants.

### Polynucleotides encoding modified oleosins with artificially introduced cysteines

In the first aspect the invention provides a polynucleotide encoding a modified oleosin including at least one artificially introduced cysteine. The cysteines are artificially introduced into the N-terminal hydrophilic region of the oleosin, or into the C-terminal hydrophilic region of the oleosin.

In one embodiment, the modified oleosin includes at least two cysteines, at least one of which is artificially introduced. In a further embodiment, the modified oleosin includes at least two to at least thirteen (i.e., 2, 3, 4, 5, 6, 7, 8, 9, 10, 1, 12, 13, 14 or more) artificially introduced cysteines. The cysteines are artificially introduced in the N-terminal hydrophilic region of the oleosin, or in the C-terminal hydrophilic region of the oleosin. In a further embodiment the modified oleosin incudes at least one cysteine in the N-terminal hydrophilic region, and at least one cysteine in the C-terminal hydrophilic region. In a further embodiment the cysteines are distributed substantially evenly over the N-terminal and C-terminal hydrophilic regions of the oleosin.

In a further embodiment the polynucleotide encodes a fusion protein including the modified oleosin fused to a protein of interest.

### Constructs

In a further aspect the invention provides a genetic construct comprising a polynucleotide of the invention. In a further aspect the invention provides an expression construct comprising a polynucleotide of the invention. In one embodiment the polynucleotide in the construct is operably linked to a promoter sequence. In one embodiment the promoter sequence is capable of driving expression of the polynucleotide in a vegetative tissue of a plant. In a further embodiment the promoter sequence is capable of driving expression of the polynucleotide in a seed of a plant. In a further embodiment the promoter sequence is capable of driving expression of the polynucleotide in the pollen of a plant. In a further embodiment the promoter sequence is capable of driving expression of the polynucleotide in an *E*. *coli* cell. In a further embodiment the promoter sequence is capable of driving expression of the polynucleotide in a yeast cell. In a further embodiment the promoter sequence is capable of driving expression of the polynucleotide in an algal cell.

Also described herein is a construct containing a polynucleotide that encodes a modified neutral lipid protein. In one embodiment, the construct also contains a second polynucleotide that encodes a triacylglycerol (TAG) synthesizing enzyme. In various embodiments, the construct can be linked to a promoter sequence capable of driving its expression in various host cells. Also provided is use of the constructs to induce a host cell to express a modified oleosin and/or a TAG synthesizing enzyme. In various embodiments, the construct expressing a modified oleosin and the construct expressing a TAG synthesizing enzyme may be driven by the same or by different promoters. In yet another embodiment the construct is located in an appropriate position and orientation of a suitable functional endogenous promoter such that the expression of the construct occurs. In various embodiments, the construct can be expressed in a bacterial, plant, fungal or algal cell. In one embodiment where the construct is expressed in a plant cell, the cell may be of vegetative, seed, pollen or fruit tissue.

### Host cells

In a further aspect the invention provides a host cell comprising a construct of the invention. In a further aspect the invention provides a host cell genetically modified to comprise a polynucleotide of the invention. In a further aspect the invention provides a host cell genetically modified to express a polynucleotide of the invention.

### Host cell also expressing a TAG synthesising enzyme

In a further embodiment the host cell is also genetically modified to express a triacylglycerol (TAG) synthesising enzyme. In a further embodiment the host cell is genetically modified to comprise a nucleic acid sequence encoding a triacylglycerol (TAG) synthesising enzyme. In a further embodiment the host cell comprises an expression construct including a nucleic acid sequence encoding a triacylglycerol (TAG) synthesising enzyme.

In a further embodiment the nucleic acid is operably linked to a promoter sequence. In a further embodiment the promoter sequence is capable of driving expression of the nucleic acid sequence in a vegetative tissue of a plant. In one embodiment the promoter sequence is capable of driving expression of the nucleic acid sequence in a seed of a plant. In one embodiment the promoter sequence is capable of driving expression of the nucleic acid sequence in the pollen of a plant.

In a further embodiment the promoter sequence is capable of driving expression of the polynucleotide in an *E. coli* cell. In a further embodiment the promoter sequence is capable of driving expression of the polynucleotide in a yeast cell. In a further embodiment the promoter sequence is capable of driving expression of the polynucleotide in an algal cell.

### Host cell types

The host cell may be any type of cell. In on embodiment the host cell is a prokaryotic cell. In a further embodiment the host cell is a eukaryotic cell. In one embodiment the host cell is selected from a bacterial cell, a yeast cell, a fungal cell, an insect cell, algal cell, and a plant cell. In one embodiment the host cell is a bacterial cell. In a further embodiment the host cell is a yeast cell. In further embodiment the host cell is a fungal cell. In further embodiment the host cell is an insect cell. In further embodiment the host cell is an algal cell. In a further embodiment the host cell is a plant cell.

### Plants

In a further aspect the invention provides a plant comprising a plant cell of the invention. In a further aspect the invention provides a plant comprising a construct of the invention. In a further aspect the invention provides a plant genetically modified to comprise a polynucleotide of the invention. In a further aspect the invention provides a plant genetically modified to express a polynucleotide of the invention. In a further embodiment the plant expresses a modified oleosin encoded by the polynucleotide of the invention.

In a further embodiment the modified oleosin is expressed in a vegetative tissue of the plant. In a further embodiment the modified oleosin is expressed in a seed of the plant. In a further embodiment the modified oleosin is expressed in the pollen of the plant.

### Plant also expresses a TAG enzyme

In a further embodiment the plant is also genetically modified to express a triacylglycerol (TAG) synthesising enzyme. In a further embodiment the triacylglycerol (TAG) synthesising enzyme is expressed in the same tissue as the modified oleosin.

In a further embodiment the plant is genetically modified to comprise a nucleic acid sequence encoding a triacylglycerol (TAG) synthesising enzyme. In a further embodiment the plant comprises an expression construct including a nucleic acid sequence encoding a triacylglycerol (TAG) synthesising enzyme.

In a further embodiment the nucleic acid is operably linked to a promoter sequence.

In a further embodiment the promoter sequence is capable of driving expression of the nucleic acid sequence in a vegetative tissue of a plant. In one embodiment the promoter sequence is capable of driving expression off the nucleic acid sequence in a seed of a plant. In one embodiment the promoter sequence is capable of driving expression of the nucleic acid sequence in the pollen of a plant.

### Modified oleosin polypeptides with artificially introduced cysteines

In a further aspect the invention provides a modified oleosin including at least one artificially introduced cysteine. The cysteines are artificially introduced into the N-terminal hydrophilic region of the oleosin, or into the C-terminal hydrophilic region of the oleosin. In a further aspect the invention provides a modified oleosin encode by a polynucleotide of the invention. In one embodiment, the modified oleosin includes at least two cysteines, at least one of which is artificially introduced. In a further embodiment, the modified oleosin includes at least two to at least thirteen (i.e., 2, 3, 4, 5, 6, 7, 8, 9, 10, 1, 12, 13, 14 or more) artificially introduced cysteines.

In a further embodiment the modified oleosin includes at least one artificially introduced cysteine in the N-terminal hydrophilic region, and at least one cysteine in the C-terminal hydrophilic region. The cysteines are artificially introduced in the N-terminal hydrophilic region of the oleosin, or in the C-terminal hydrophilic region of the oleosin. Preferably the cysteins are distributed substantially evenly between the N-terminal and C-terminal hydrophilic region of the oleosin.

### Fusion proteins with modified oleosins including artificially introduced cysteines

In a further aspect the invention provides a fusion protein comprising a modified oleosin of the invention and a protein of interest. The fusion protein thus comprises a modified oleosin portion, and a protein of interest portion.

### Oil bodies comprising modified oleosins

In a further aspect the invention provides an oil body comprising a modified oleosin of the invention. In a further aspect the invention provides an oil body comprising at least two modified oleosins of the invention. In one embodiment at least two of the modified oleosins are cross-linked to each other via disulphide bridges between cysteine residues in the modified oleosins. In a further embodiment the modified oleosins are cross-linked via the artificially introduced cysteine residues in the modified oleosins.

In a further embodiment the oil body additionally comprises a fusion protein, wherein the fusion protein includes an oleosin fused to a protein of interest. In this embodiment, the oleosin in the fusion protein need not include an artificially introduced cysteine. Preferably the oleosin in the fusion protein does not include an artificially introduced cysteine.

The oil bodies of this embodiment are useful for purifying and delivering the protein of interest, as discussed in Roberts *et al.,* (2008).

However in this embodiment it is possible to take advantage of the option to vary the stability/integrity of the oil body provided by presence of the modified oleosins in the oil body, hence allowing for more stringent purification and delivery procedures.

### Oil bodies comprising fusion proteins with modified oleosisn

In a further aspect the invention provides an oil body comprising a fusion protein of the invention, the fusion protein comprising a modified oleosin of the invention and a protein of interest. The fusion protein thus comprises a modified oleosin portion, and a protein of interest portion.

In one embodiment the oil body comprises at least two fusion proteins of the invention.

In one embodiment at least two of the fusion proteins are cross-linked to each other via disulphide bridges between cysteine residues in the modified oleosin portion of the fusion proteins. In one embodiment the fusion proteins are cross-linked via the artificially introduced cysteine residues in the modified oleosin portion of the fusion proteins.

In a further embodiment the oil body comprises at least one modified oleosin of the invention. In a further embodiment at least one fusion protein is cross-linked to at least one modified oleosin, via a cysteine in the modified oleosin portion of the fusion protein and a cysteine in the modified oleosin.

Again, the oil bodies of this embodiment are useful for purifying and delivering the protein of interest, as discussed in Roberts *et al.,* (2008).

However in this embodiment it is possible to take advantage of the option to vary the stability/integrity of the oil body provided by presence of the modified oleosins in the oil body, hence allowing for more stringent purification and delivery procedures.

### Emulsion

In a further aspect the invention provides an emulsion comprising a modified oleosin of the invention. In one embodiment the emulsion comprises the modified oleosin and a suitable carrier. The carrier may be buffered, with the appropriate redox environment to retain the desired degree of cross-linking of the oleosins.

To resuspend the modified oleosin in the carrier may require sonication or high pressure homogenising, followed by exposure to the appropriate oxidising conditions.

### Compositions

In a further aspect the invention provides a composition comprising a modified oleosin of the invention. In one embodiment the composition comprises the modified oleosin and a suitable carrier. The carrier may be buffered, with the appropriate redox environment to attain the desired degree of cross-linking of the modified oleosins.

To resuspend the modified oleosins in the carrier may require sonication or high pressure homogenising, followed by exposure to the appropriate oxidising conditions.

In a further aspect the invention provides a composition comprising an oil body of the invention. In one embodiment the composition comprises the oil body and a suitable carrier. The carrier may be buffered, with the appropriate redox environment to retain the desired degree of cross-linking of the modified oleosins. In a further embodiment the invention provides a composition formulated for dermal application comprising an oil body of the invention.

### Plants, and parts thereof, comprising oil bodies of the invention

In a further aspect the invention provides a plant, or part thereof, comprising an oil body of the invention. In a further aspect the invention provides a vegetative tissue of a plant, comprising an oil body of the invention. In a further aspect the invention provides a seed of a plant, comprising an oil body of the invention.

### Animal feed comprising oil bodies of the invention

In a further aspect the invention provides an animal feed comprising an oil body of the invention. In a further aspect the invention provides an animal feed comprising a plant, or part thereof, of the invention.

### Methods for producing oil bodies

In a further aspect invention provides a method for producing an oil body, the method comprising the step of combining:
a) at least two modified oleosins, each including at least one artificially introduced cysteine,
b) triacylglycerol, and
c) phospholipid, wherein the cysteines are artificially introduced into the N-terminal hydrophilic region of the oleosins, or into the C-terminal hydrophilic region of the oleosins.

In one embodiment, the modified oleosins each include at least two cysteines, at least one of which is artificially introduced. In a further embodiment the modified oleosins each include at least one cysteine in the N-terminal hydrophilic region of the oleosin, and at least one cysteine in the C-terminal hydrophilic region of the oleosin.

In a further embodiment, the modified oleosin includes at least two to at least thirteen (i.e., 2, 3, 4, 5, 6, 7, 8, 9, 10, 1, 12, 13, 14 or more) artificially introduced cysteines.

The cysteines are artificially introduced in the N-terminal hydrophilic region of the oleosins, or in the C-terminal hydrophilic region of the oleosins. In a further embodiment the cysteines are distributed substantially evenly between the N-tcrminal and C-terminal hydrophilic region of the oleosins. In a further embodiment the modified oleosins are cross-linked via disulphide bridges between cysteine residues in the oleosins. In a further embodiment embodiment the modified oleosins are cross-linked between the artificially introduced cysteine residues in the oleosins.

In one embodiment the modified oleosins are part of fusion proteins wherein the fusion proteins comprise a modified oleosin, and a protein of interest.

In one embodiment the method comprises the additional step of regulating the degree of cross-linking of modified oleosins in the oil body by controlling the redox environment of the oil body produced.

### All components combined in vivo (in vivo oil bodies)

In one embodiment the components of a), b) and c) are combined within a host cell. In this embodiment the modified oleosins are preferably expressed in the host cell.

The host cell is preferably genetically modified to express the modified oleosins.

The host cell is preferably comprises a construct of the invention. The host cell is preferably genetically modified to comprise a polynucleotide of the invention. The host cell is preferably genetically modified to express a polynucleotide of the invention.

### Host cell also expresses a TAG synthesising enzyme

In a further embodiment the host cell is also genetically modified to express a triacylglycerol (TAG) synthesising enzyme. In a further embodiment the host cell comprises an expression construct including a nucleic acid sequence encoding a triacylglycerol (TAG) synthesising enzyme.

In a further embodiment the nucleic acid sequence is operably linked to a promoter sequence. In one embodiment the promoter sequence is capable of driving expression of the nucleic acid sequence in a vegetative tissue of a plant. In one embodiment the promoter sequence is capable of driving expression of the nucleic acid sequence in a seed of a plant. In one embodiment the promoter sequence is capable of driving expression of the nucleic acid sequence in the pollen of a plant.

In a further embodiment the host cell is also genetically modified to comprise a nucleic acid sequence encoding a triacylglycerol (TAG) synthesising enzyme. In a further embodiment the host cell is also genetically modified to express a nucleic acid sequence encoding a triacylglycerol (TAG) synthesising enzyme.

It will be understood by those skilled in the art that the polynucleotide encoding the modified oleosin and the nucleic acid sequence encoding a triacylglycerol (TAG) synthesising enzyme can be placed on the same construct or on separate constructs to be transformed into the host cell. Expression of each can be driven by the same or different promoters, which may be incuded in the construct to be transformed. It will also be understood by those skilled in the art that alternatively the polynucleotide and nucleic acid can be transformed into the cell without a promoter, but expression of either the polynucleotide and nucleic acid could be driven by a promoter or promoters endogenous to the cell transformed.

In a further embodiment the host cell forms part of an organism. In a preferred embodiment the organism is a plant.

In a further embodiment the oil is produced in the vegetative tissues of the plant.

In one embodiment of the method the plant acumulates about 50% to about 400% more lipid than does a suitable control plant. In a further embodiment of the method the plant acumulates about 100% to about 300% more lipid than does a suitable control plant. In a further embodiment of the method the plant acumulates about 150% to about 250% more lipid than does a suitable control plant. Suitable control plants include non-transformed or wild-type versions of plant of the same variety and or species as the transformed plant used in the method of the invention.

In a further embodiment the plant is processed into an animal feed.

In a further embodiment the plant is processed into a biofuel feed stock.

### Additional method step to purify the in vivo produced oil bodies

In one embodiment the method includes the additional step of purifying the oil bodies from the cell or organisim.

### Additional method step to vary degree of cross-linking of in vivo produced purified oil bodies

In a further embodiment the method comprises the additional step of regulating the degree of cross-linking of modified oleosins in the *in vivo* produced purified oil bodies
by controlling the redox environment of the purified oil bodies. In one embodiment the degree of cross-linking is increased by use of an oxidising environment. In a further embodiment the degree of cross-linking is decreased by use of a reducing environment.

### Components combined in vitro (in vitro / artificial oil bodies)

In certain embodiments the components of a), b) and c) may be combined *in vitro.*

In one embodiment, the modified oleosin of a) has been recombinantly expressed in, and purified from a host cell of the invention, before being combined with the components of b) and c).

### Additional method step to vary degree of cross-linking of in vitro / artificial oil bodies

In a further embodiment the method comprises the additional step of regulating the degree of cross-linking by controlling the redox environment in which the components of a), b) and c) are combined. In one embodiment the degree of cross-linking is increased by combining the components of a), b) and c) in on oxidising environment. In a further embodiment the degree of cross-linking is decreased by combining the components of a), b) and c) in a reducing environment. The degree of cross-linking may also be regulated after the oil body is formed, by controlling the redox environment in which the oil body is contained.

In a further aspect the invention provides a method of producing a plant that accumulates more oil than a suitable control plant the method comprising providing a plant transformed with a polynucleotide of the invention that expresses a modified oleosin encode by the polynucleotide.

In one embodiment the plant is also transformed with a polynucleotide encoding a TAG synthesising enzyme to express the TAG synthesising enzyme and thus synthesise TAG.

A plant may be produced by transforming a single plant, or plant cell, with both the polynucleotide of any one the invention and the polynucleotide encoding the TAG synthesising enzyme.

A plant may be produced by crossing a first plant transformed with a polynucleotide of any one of the invention, with second plant transformed the polynucleotide encoding the TAG synthesising enzyme, to produce the plant transformed with both a polynucleotide of the invention, and a polynucleotide encoding the TAG synthesising enzyme.

In a further embodiment the oil is TAG. In a further embodiment the oil is produced in the vegetative tissues of the plant.

In one embodiment of the method the plant acumulates about 50% to about 400% more lipid than does a suitable control plant. In a further embodiment of the method the plant acumulates about 100% to about 300% more lipid than does a suitable control plant. In a further embodiment of the method the plant acumulates about 150% to about 250% more lipid than does a suitable control plant

In a further embodiment the plant is processed into an animal feed.

In a further embodiment the plant is processed into a biofuel feed stock.

In a further aspect invention provides a method for producing an oil body in a host cell, the method comprising:
a) introducing into a host cell at least one nucleic acid molecule encoding a modified oleosin of the invention; and
b) culturing the host cell in order to express the modified oleosin.

In a further aspect invention provides a method for producing an oil body in a host cell, the method comprising:
a) introducing into a host cell at least one nucleic acid molecule encoding a modified oleosin of the invention and a nucleic acid molecule encoding a TAG synthesizing enzyme ; and
b) culturing the host cell in order to express the modified oleosin and the TAG synthesizing enzyme.

The host cell may be a host cell as herein described.

### Oil bodies

Oil bodies may be produced by a method of the invention.

### Compositions

In a further aspect the invention provides a composition comprising an oil body of the invention. In one embodiment the composition comprises the oil body and a suitable carrier. The carrier may be buffered to provide the appropriate redox environment to retain the desired degree of cross-linking of the modified oleosin. In a further :embodiment the invention provides a composition formulated for dermal application comprising an oil body of the invention.

### Plants, and parts thereof, comprising oil bodies of the invention

In a further aspect the invention provides a plant, or part thereof, comprising an oil body of the invention. In a further aspect the invention provides a vegetative tissue of a plant, comprising an oil body of the invention. In a further aspect the invention provides a seed of a plant, comprising an oil body of the invention. Pollen of a plant may comprise an oil body of the invention. In a further aspect the invention provides a fruit, or fruiting body, of a plant, comprising an oil body of the invention.

### Animal feed comprising oil bodies of the invention

In a further aspect the invention provides an animal feed comprising an oil body of the invention. In a further aspect the invention provides an animal feed comprising a plant, or part thereof, of the invention.

In one embodiment the feed is suitable for a mammalian animal including humans. In a further embodiment the feed is suitable for non-human mammals. Preferred animals include farm animals such as but not limited to cows, sheep, horses, goats, pigs, chickens, and the like.

### Plants

The modified oleosins may be modified naturally occurring oleosins. The plants from which the un-modified oleosin sequences are derived may be from any plant species that contains oleosins and polynucleotide sequences encoding oleosins.

The plant cells, in which the modified oleosins are expressed, may be from any plant species. The plants, in which the modified oleosins are expressed, may be from any plant species.

In one embodiment the plant cell or plant, is derived from a gymnosperm plant species.

In a further embodiment the plant cell or plant, is derived from an angiosperm plant species.

In a further embodiment the plant cell or plant, is derived from a from dicotyledonous plant species.

In a further embodiment the plant cell or plant, is derived from a monocotyledonous plant species.

Other preferred plants are forage plant species from a group comprising but not limited to the following genera: Zea, *Lolium, Hordium, Miscanthus, Saccharum, Festuca, Dactylis, Bromus, Thinopyrum, Trifolium, Medicago, Pheleum, Phalaris, Holcus, Glycine, Lotus, Plantago* and *Cichorium.*

Other preferred plants are leguminous plants. The leguminous plant or part thereof may encompass any plant in the plant family Leguminosae or Fabaceae. For example, the plants may be selected from forage legumes including, alfalfa, clover; leucaena; grain legumes including, beans, lentils, lupins, peas, peanuts, soy bean; bloom legumes including lupin, pharmaceutical or industrial legumes; and fallow or green manure legume species.

A particularly preferred genus is *Trifolium.* Preferred *Trifolium* species include *Trifolium repens*; *Trifolium arvense*; *Trifolium affine*; and *Trifolium occidentale.* A particularly preferred *Trifolium* species is *Trifolium repens.*

Another preferred genus is *Medicago.* Preferred *Medicago* species include *Medicago sativa* and *Medicago truncatula.* A particularly preferred *Medicago* species is *Medicago sativa,* commonly known as alfalfa.

Another preferred genus is *Glycine.* Preferred *Glycine* species include *Glycine max* and *Glycine wightii* (also known as *Neonotonia wightii*)*.* A particularly preferred *Glycine* species is *Glycine max,* commonly known as soy bean. A particularly preferred *Glycine* species is *Glycine wightii,* commonly known as perennial soybean.

Another preferred genus is *Vigna.* A particularly preferred *Vigna* species is *Vigna unguiculata* commonly known as cowpea.

Another preferred genus is *Mucana.* Preferred *Mucana* species include *Mucana pruniens.* A particularly preferred *Mucana* species is *Mucana pruniens* commonly known as velvetbean.

Another preferred genus is *Arachis.* A particularly preferred *Arachis* species is *Arachis glabrata* commonly known as perennial peanut.

Another preferred genus is *Pisum.* A preferred *Pisum* species is *Pisuin sativum* commonly known as pea.

Another preferred genus is *Lotus.* Preferred Lotus species include *Lotus corniculatus, Lotus pedunculatus, Lotus glabar, Lotus tenuis* and *Lotus uliginosus.* A preferred *Lotus* species is *Lotus corniculatus* commonly known as Birdsfoot Trefoil. Another preferred *Lotus* species is *Lotus glabar* commonly known as Narrow-leaf Birdsfoot Trefoil. Another preferred preferred *Lotus* species is *Lotus pedunculatus* commonly known as Big trefoil. Another preferred *Lotus* species is *Lotus tenuis* commonly known as Slender trefoil.

Another preferred genus is *Brassica.* A preferred *Brassica* species is *Brassica oleracea,* commonly known as forage kale and cabbage.

Other preferred species are oil seed crops including but not limited to the following genera: *Brassica, Carthumus, Helianthus, Zea* and *Sesamum.*

A preferred oil seed genera is *Brassica.* A preferred oil seed species is *Brassica napus.*

A preferred oil seed genera is *Brassica.* A preferred oil seed species is *Brassica oleraceae.*

A preferred oil seed genera is *Zea.* A preferred oil seed species is Zea mays.

A preferred oil seed genera is *Carthamus.* A preferred oil seed species is *Carthamus tinctorius.*

A preferred oil seed genera is *Helianthus.* A preferred oil seed species is *Helianthus annuus.*

A preferred oil seed genera is *Zea.* A preferred oil seed species is *Zea mays.*

A preferred oil seed genera is *Sesamum.* A preferred oil seed species is *Sesamum indicum.*

A preferred silage genera is *Zea.* A preferred silage species is Zea mays.

A preferred grain producing genera is *Hordeum.* A preferred grain producing species is *Hordeum vulgare.*

A preferred grazing genera is *Lolium.* A preferred grazing species is *Lolium perenne.*

A preferred grazing genera is *Lolium.* A preferred grazing species is *Lolium arundinaceum.*

A preferred grazing genera is *Trifolium.* A preferred grazing species is *Trifolium repens.*

A preferred grazing genera is *Hordeum.* A preferred grazing species is *Hordeum vulgare.*

Preferred plants also include forage, or animal feedstock plants. Such plants include but are not limited to the following genera: *Hiscanthus, Saccharum, Panicum.*

A preferred biofuel genera is *Miscanthus.* A preferred biofuel species is *Miscanthus giganteus.*

A preferred biofuel genera is *Saccharum.* A preferred biofuel species is *Saccharum officinarum.*

A preferred biofuel genera is *Panicum.* A preferred biofuel speices is *Panicum virgatum.*

### DETAILED DESCRIPTION OF THE INVENTION

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

On a weight for weight basis lipids have approximately double the energy content of either proteins or carbohydrates. The bulk of the world's lipids are produced by plants and the densest form of lipid is as a triacylglycerol (TAG). Dicotyledonous plants can accumulate up to approximately 60% of their seed weight as TAG which is subsequently used as an energy source for germination. As such there have been a number of efforts targeted at using seeds rich in oils to sustainably produce sufficient lipids for both animal and biofuel feed stock.

Given that there is only a limited quantity of TAG able to be produced by seeds alternative approaches are being made to produce- additional lipid (preferentially TAGs) in vegetative tissues. The majority of these approaches have pursued the up regulation or over expression of one or several enzymes in the Kennedy pathway in the leaves of plants in order to synthesise TAG. Typically however, the majority of additional lipids produced by this approach are re-mobilised within the plant by a combination of lipases and β-oxidation resulting in a limited increase in lipid content (usually 2-4% of the DM).

The TAG produced in developing seeds is typically contained within discreet structures called oil bodies (OBs) which are highly stable and remain as discrete tightly packed organelles without coalescing even when the cells desiccate or undergo freezing conditions (Siloto et al., 2006; Shimada et al., 2008). OBs consist of a TAG core surrounded by a phospholipid monolayer embedded with proteinaceous emulsifiers. The latter make up 0.5-3.5% of the OB; of this, 80-90% is oleosin with the remainder predominantly consisting of the calcium binding (caloleosin) and sterol binding (steroleosin) proteins (Lin and Tzen, 2004). The emulsification properties of oleosins derives from their three functional domains which consist of an amphipathic N-terminal arm, a highly conserved central hydrophobic core (∼72 residues) and a C-terminal amphipathic arm. Similarly, both caloleosin and steroleosin possess hydrophilic N and C-terminal arms and their own conserved hydrophobic core.

It was previously speculated that the constitutive expression of oleosin or polyoleosin (tandem head-to-tale fusions of oleosins) with TAG synthesising enzymes in the leaves would result in the formation of stable oil bodies leading to the accumulation of TAG. We have subsequently found however, that oleosin and polyoleosins are ineffective and promoting the accumulation of TAG when co-expressed with DGAT1 in plant leaves (Roberts *et al.,* unpublished data).

The current invention provides modified oleosins which contain one or more artificially introduced cysteine residues. The cysteines are artificially introduced into the N-terminal hydrophilic region of the oleosin, or into the C-terminal hydrophilic region of the oleosin.

The encapsulation of the neutral lipids by oleosins containing engineered cysteines provides an alternative mechanism to accumulate appreciable quantities of TAG in leaves without the requirement to wait until senescence and without producing extreme phenotypes. In addition the modified oleosin has a number of other applications involving modifying OB stability, emulsion properties as well as the generation and purification of recombinant proteins.

### Oil bodies

OBs generally range from 0.5-2.5µm in diameter and consist of a TAG core surrounded by a phospholipid monolayer embedded with proteinaceous emulsifiers - predominantly oleosins (Tzen et at, 1993; Tzen, et at 1997). OBs consist of only 0.5-3.5% protein; of this 80-90% is oleosin with the remainder predominantly consisting of the calcium binding (caleosin) and sterol binding (steroleosin) proteins (Lin and Tzen, 2004). The ratio ofoleosin to TAG within the plant cell influences the size and number of oil bodies within the cell (Sarmiento et al., 1997; Siloto et al., 2006).

While OBs are naturally produced predominantly in the seeds and pollen of many plants they are also found in some other organs (e.g., specific tubers).

Oleosins are comparatively small (15 to 24 kDa) proteins that are embedded in the surface of OBs.

### Oil body stability

The suitability of oil bodies, and artificial oil bodies, for the applications discussed above, among others, is limited at least in part, by their stability. One approach to address oil body stability was to generate oil bodies comprising so-called polyoleosin. Polyoleosin is the head to tail fusion of two or more oleosin units (Roberts et al., 2008). Altering the number of oleosin units enables the properties (thermal stability and degradation rate) of the oil bodies to be tailored. Expression of polyoleosin *in planta* leads to incorporation of the polyoleosin units to the oil bodies as per single oleosin units (Scott et al., 2007). Multiple oleosin units in tandem head-to-tail arrangements were used to create polyoleosin. Separate constructs (containing from one to six oleosin repeats) were specifically designed for expression *in planta* and in *E. coli.* The majority of recombinant polyoleosin accumulated in the oil bodies of transgenic plants and in the inclusion bodies of *E*. *coli.* Purified prokaryotically produced polyoleosin was used to generate artificial oil bodies. Oil body and artificial oil body thermal stability and structural integrity in proteinase-K were raised by polyoleosin.

However, there are several limiting factors determining the degree of protection/stability that polyoleosin can provide; these relate to the number of tandem repeats that can be joined before the process of translation and oil body targeting becomes limiting (Scott et al., 2007); while another limitation comes from the nature of the oleosin fusion which is achieved by generating a transcript with a head to tail fusion arrangement. This is essentially a linear protein of multimeric oleosin repeats that has a number of covalent-links and position of covalent-links per individual oleosin repeat (i.e., a maximum of one at each end). In addition this arrangement only affords protection against N-terminal degrading proteins but it does not provide any additional protection against other proteolytic enzymes that recognise specific internal peptide sequences. Furthermore, the linking between oleosin units in a polyoleosin molecule formed by tandem head to tail repeats is not readily altered *in situ.* While specific protease specific sites could be engineered into the joining regions in order to break apart fused polyoleosin molecules embedded into an oil body or artificial oil body they could not be re-fused easily.

Oleosins embedded in oil bodies have previously covalently cross-linked by the addition of cross-linking agents such as glutaraldehyde or genepin (Peng et al., 2004 & 2006), however, this random cross-linking requires the addition of cross-linking agents to oil body preparations, and is not easy to reverse.

### Artificial oil bodies

Prokaryotically expressed recombinant oleosins can be used to generate artificial oil bodies (AOBs) who's properties are very similar to plant derived OBs (Peng et al. 2004; Roux et al. 2004; Chiang et al. 2005; Chiang et al. 2007).

### Applications of oil bodies and artificial oil bodies

The unique properties of oil bodies, and their constituent oleosins, form the basis of a number of biotechnical applications including: purifying recombinant proteins; formation of multimeric protein complexes; emulsification; delivery of bioactives; generation of multivalent bioactives and even as a potential flavour enhancer (for reviews see Capuano et al., 2007 and Roberts et al., 2008).

### Emulsions

Emulsions are produced when one or more liquids that are immiscible in another liquid, usually due to different polarities and thus different hydrophobicities, are uniformly suspended within that liquid. Examples include oil droplets uniformly dispersed in water, or water droplets uniformly dispersed in oil. Generation of a relatively stable emulsion requires the use of an emulsifier, which lowers the interfacial tension between the liquids. The stability of an emulsion is generally measured in terms of the duration that the uniform dispersion persists under specified conditions. Emulsifiers are commonly used in the food and cosmetic industry; so need to have high emulsion stability and be safe for consumption and topical application.

Intact oil bodies containing oleosin naturally form a surfactant-free, oil-in water emulsion. It has been found that intact oil bodies or oil bodies in which the majority of TAG has been removed have a broad range of emulsification applications in food, topical personal care (skin creams) and pharmaceutical formulations (Harada et al., 2002; Deckers et al., 2003; Hou et al., 2003).

### Biohydrogenation

It has been demonstrated that the lipid profile of ruminant animal feed in turn influences the lipid profile of meat and dairy products (Demeyer and Doreau, 1999). Different plants have different lipid profiles; by selectively feeding animals only plants with the desired lipid profile it is possible to positively influence the lipid profile of downstream meat and dairy products. In ruminants the final lipid make up of the meat and milk is not only influenced by the dietary lipids but is also heavily influenced by biohydrogenation (Jenkins and McGuire 2006; Firkins et al., 2006; Lock and Bauman, 2004). Biohydrogenation is the hydrogenation of non-reduced compounds (such as unsaturated fats) by the biota present in the rumen. Biohydrogenation can be prevented/delayed by encapsulating the lipids in a protein or proteins that provide resistance to microbial degradation (Jenkins and Bridges 2007). The prevention of biohydrogenation by encapsulating triacylglycerides in polyoleosin or oleosins *in planta* was reported by Scott et al., (2007), Cookson et al., (2009) and Roberts et al., (2008).

### Oleosins

Oleosins are comparatively small (15 to 24 kDa) proteins which allow the OBs to become tightly packed discrete organelles without coalescing as the cells desiccate or undergo freezing conditions (Leprince *et al.,* 1998; Siloto *et al.,* 2006; Slack *et al.,* 1980; Shimada *et al.* 2008)*.*

Oleosins have three functional domains consisting of an amphipathic N-terminal arm, a highly conserved central hydrophobic core (∼72 residues) and a C-terminal amphipathic arm. The accepted topological model is one in which the N- and C-terminal amphipathic arms are located on the outside of the OBs and the central hydrophobic core is located inside the OB (Huang, 1992; Loer and Herman, 1993; Murphy 1993). The negatively charged residues of the N- and C-terminal amphipathic arms are exposed to the aqueous exterior whereas the positively charged residues are exposed to the OB interior and face the negatively charged lipids. Thus, the amphipathic arms with their outward facing negative charge are responsible for maintaining the OBs as individual entities via steric hinderance and electrostatic repulsion both *in vivo* and in isolated preparation (Tzen et al, 1992). The N-terminal amphipathic arm is highly variable and as such no specific secondary structure can describe all examples. In comparison the C-terminal arm contains a α-helical domain of 30-40 residues (Tzen et al, 2003). The central core is highly conserved and thought to be the longest hydrophobic region known to occur in nature; at the center is a conserved 12 residue proline knot motif which includes three spaced proline residues (for reviews see Frandsen et al, 2001; Tzen et al, 2003). The secondary, tertiary and quaternary structure of the central domain is still unclear. Modelling, Fourier Transformation-Infra Red (FT-IR) and Circular Dichromism (CD) evidence exists for a number of different arrangements (for review see Roberts et al., 2008).

The properties of the major oleosins is relatively conserved between plants and is characterised by the following:
- 15-25kDa protein corresponding to approximately 140-230 amino acid residues.
- The protein sequence can be divided almost equally along its length into 4 parts which correspond to a N-terminal hydrophilic region, two centre hydrophobic-regions (joined by a proline knot or knob) and a C-terminal hydrophilic region.
- The topology of oleosin is attributed to its physical properties which includes a folded hydrophobic core flanked by hydrophilic domains. This arrangement confers an amphipathic nature to oleosin resulting in the hydrophobic domain being embedded in the phospholipid monolayer (Tzen et al., 1992) while the flanking hydrophilic domains are exposed to the aqueous environment of the cytoplasm.
- Typically oleosins do not contain cysteines

Preferred oleosins for use in the invention are those which contain a central domain of approximately 70 non-polar amino acid residues (including a proline knot) uninterrupted by any charged residues, flanked by two hydrophilic arms.

### Also described for reference:

### Steroleosins

Steroleosins comprises an N-terminal anchoring segment comprising two amphipathic α-helices 912 residues in each helix) connected by a hydrophobic anchoring region of 14 residues. The soluble dehydrogenase domain contains a NADP+- binding subdomain and a sterol-binding subdomain. The apparent distinction between steroleosins-A and -B occurs in their diverse sterol-binding subdomains (Lin and Tzen, 2004). Steroleosins have a proline knob in their hydrophobic domain and contains a sterol-binding dehydrogenase in one of their hydrophilic arms.

### Caloleosins

Caloleosins (Frandsen *et al.,* 2001) have a slightly different proline knot than do the basic oleosins, and contain a calcium-binding motif and several potential phosphorylation sites in the hydrophilic arms. Similar to oleosin, caloleosin is proposed to have three structural domains, where the N- and C-terminal arms are hydrophilic while the central domain is hydrophobic and acts as the oil body anchor. The N-terminal hydrophilic domain consists of a helix-turn-helix calcium binding EF-hand motif of 28 residues including an invariable glycine residue as a structural turning point and five conserved oxygen-containing residues as calcium-binding ligands (Chen *et al.,* 1999; Frandsen *et al.,* 2001). The C-terminal hydrophilic domain contains several phosphorylation sites and near the C-terminus is an invariable cysteine that is not involved in any intra- or inter-disulfide linkages (Peng, 2004). The hydrophilic N- and C-termini of caloleosin are approximately 3 times larger than those of oleosin (Lin and Tzen, 2004). The hydrophobic domain is thought to consist of an amphipathic α-helix and an anchoring region (which includes a proline knot).

Examples of oleosin sequences suitable to be modified for use in the invention, by the addition of at least one artificially introduced cysteine, are shown in Table 1 below. The cysteines are articificially introduced into the N-terminal hydrophilic region of the oleosin, or into the C-terminal hydrophilic region of the oleosin. The sequences (both polynucleotide and polypeptide) are provided in the Sequence Listing. Steroleosin and caloleosin sequences are disclosed for reference.

**Table 1**

| **Oleosin** | **Species** | **cDNA accession no.** | **SEQ ID NO:** | **Protein accession no.** | **SEQ ID NO:** |
|---|---|---|---|---|---|
| Oleosin | *S. indicum* | AF302907 | 34 | AAG23840 | 35 |
| Oleosin | *S. indicum* | U97700 | 36 | AAB58402 | 37 |
| Oleosin | *A. thaliana* | X62353 | 38 | CAA44225 | 39 |
| Oleosin | *A. thaliana* | But023738 | 40 | AAZ23930 | 41 |
| Oleosin | *H. annuus* | X62352.1 | 42 | CAA44224.1 | 43 |
| Oleosin | *B. napus* | X82020.1 | 44 | CAA57545.1 | 45 |
| Oleosin | *Z*. *mays* | NM_001153560.1 | 46 | NP_001147032.1 | 47 |
| Oleosin | *O. sativa* | AAL4077.1 | 48 | AAL40177.1 | 49 |
| Oleosin | *B. oleracea* | AF117126.1 | 50 | AAD24547.1 | 51 |
| Oleosin | *C. arabica* | AY928084.1 | 52 | AAY14574.1 | 53 |
| Steroleosin | *S. indicum* | AAL13315 | 54 | AAL13315 | 55 |
| Steroleosin | *A. napus* | EU678274 | 56 | ACG69522 | 57 |
| Steroleosin | Z. *mays* | NM_001159142.1 | 58 | NP_001152614.1 | 59 |
| Steroleosin | *B. napus* | EF143915.1 | 60 | ABM30178.1 | 61 |
| Caloleosin | *S. indicum* | AF109921 | 62 | AAF13743 | 63 |
| Caloleosin | *G*. *max* | AF004809 | 64 | AAB71227 | 65 |
| Calolcosin | *Z. mays* | NM_001158434.1 | 66 | NP_001151906 | 67 |
| Caloleosin | *B. napus* | AY966447.1 | 68 | AAY40837 | 69 |
| Caloleosin | *C. revoluta* | FJ455154.1 | 70 | ACJ70083 | 71 |
| Caloleosin | *C. sativus* | EU232173.1 | 72 | ABY56103.1 | 73 |

Oleosin, steroleosin and caloleosins are well known to those skilled in the art. Further sequences from many different species can be readily identified by methods well-known to those skilled in the art. For example, further sequences can be easily identified by an NCBI Entrez Cross-Database Search (available at http://www.ncbi.nlm.nih.gov/sites/gquery) using any one of the terms oleosin, steroleosin and caloleosin.

### Plant lipids biosynthesis

All plant cells produce fatty acids from actetyl-CoA by a common pathway localized in plastids. Although a portion of the newly synthesized acyl chains is then used for lipid biosynthesis within the plastid (the prokaryotic pathway), a major portion is exported into the cytosol for glycerolipid assembly at the endoplasmic reticulum (ER) or other sites (the eukaryotic pathway). In addition, some of the extraplastidial glycerolipids return to the plastid, which results in considerable intermixing between the plastid and ER lipid pools (Ohlrogge and Jaworski 1997).

The simplest description of the plastidial pathway of fatty acid biosynthesis consists of two enzyme systems: acetyl-CoA carboxylase (ACCase) and fatty acid synthase (FAS). ACCase catalyzes the formation of malonyl-CoA.from acetyl-CoA, and FAS transfers the malonyl moiety to acyl carrier protein (ACP) and catalyzes the extension of the growing acyl chain with malonyl-ACP.

The initial fatty acid synthesis reaction is catalyzed by 3-ketoacyl-ACP III (KAS III) which results in the condensation of acetyl-CoA and malonyl-ACP. Subsequent condensations are catalyzed by KAS I and KAS II. Before a subsequent cycle of fatty acid synthesis begins, the 3-ketoacyl-ACP intermediate is reduced to the saturated acyl-ACP in the remaining FAS reactions, catalyzed sequentially by the 3-ketoacyl-ACP reductase, 3 hydroxyacyl-ACP dehydrase, and the enoyl-ACP reductase.

The final products of FAS are usually 16:0 and 18:0-ACP, and the final fatty acid composition of a plant cell is in large part determined by activities of several enzymes that use these acyl-ACPs at the termination phase of fatty acid synthesis. Stearoyl-ACP desatruase modifies the final. product of FAS by insertion of a cis double bond at the 9 position of the C18:0-ACP. Reactions of fatty acid synthesis are terminated by hydrolysis or transfer of the acyl chain from the ACP. Hydrolysis is catalyzed by acyl-ACP thioesterases, of which there are two main types: one thioesterase relatively specific for 18:1-ACP and a second more specific for saturated acyl-ACPs. Fatty acids that have been released from ACPs by thioesterases leave the plastid and enter into the eukaryotic lipid pathway, where they are primarily esterified to glycerolipids on the ER. Acyl transferases in the plastid, in contrast to thioesterases, terminate fatty acid synthesis by transesterifying acyl moieties from ACP to glycerol, and they are an essential part of the prokaryotic lipid pathway leading to plastid glycerolipid assembly.

### Triacylglycerol biosynthesis

The only committed step in TAG biosynthesis is the last one, i.e. the addition of a third fatty acid to an existing diacylglycerol, thus generating TAG. In plants this step is predominantly (but not exclusively) performed by one of five (predominantly ER localised) TAG synthesising enzymes including: acyl CoA: diacylglycerol acyltransferase (DGAT1); an unrelated acyl CoA: diacylglycerol acyl transferase (DGAT2); a soluble DGAT (DGAT3) which has less than 10% identity with DGAT1 or DGAT2 (Saha et al., 2006); phosphatidylcholine-sterol O-acyltransferase (PDAT); and a wax synthase (WSD1, Li et al., 2008). The DGAT1 and DGAT2 proteins are eoncoded by two distinct gene families, with DGAT1 containing approximately 500 amino acids and 10 predicted transmembrane domains and DGAT2 has only 320 amino acids and two transmembrane domains (Shockey et al., 2006).

The term "triacylglycerol synthesising enzyme" or "TAG synthesising enzyme" as used herein means an enzyme capable of catalysing the addition of a third fatty acid to an existing diacylglycerol, thus generating TAG. Preferred TAG synthesising enzymes include but are not limited to: acyl CoA: diacylglycerol acyltransferasel (DGAT1); diacylglycerol acyl transferase2 (DGAT2); phosphatidylcholine-sterol O-acyltransferase (PDAT) and cytosolic soluble form of DGAT (soluble DGAT or DGAT3).

Given that endogenous DGAT1 and DGAT2 appear to play roles in mature and senescing leaves (Kaup et al. 2002; Shockey et al. 2006), it is likely that plants possess a number of feedback mechanisms to control their activity. Indeed, Zou et al. (2008) recently identified a consensus sequence (X-Leu-X-Lys-X-X-Ser-X-X-X-Val) within *Tropaeolum majus* (garden nasturtium) DGAT1 (TmDGAT1) sequences as a targeting motif typical of members of the SNF1-related protein kinase-1 (SnRK1) with Ser being the residue for phosphorylation. The SnRK1 proteins are a class of Ser/Thr protein kinases that have been increasingly implicated in the global regulation of carbon metabolism in plants, e.g. the inactivation of sucrose phosphate synthase by phosphorylation (Halford & Hardie 1998). Zou *et al.* (2008) went on to demonstrate that the obliteration of a potential SnRK1 phosphorylation site in DGAT1 by single point mutation (Ser197A1a of TmDGAT1) led to the accumulation of significantly higher levels of TAG in the seed. This mutation increased activity by 38-80%, which led to a 20-50% increase in oil content on a per seed basis in Arabidopsis.

Phospholipid:DGA acyltransferase (PDAT) forms TAG from a molecule of phospholipid and a molecule of diacyglycerol. PDAT is quite active when expressed in yeast but does not appreciably increase TAG yields when expressed in plant seeds. PDAT and a proposed DAG:DAG transacylase are neutral lipid synthesizing enzymes that produce TAG, but are not considered part of the Kennedy Pathway.

A combination of wax ester synthase and DGAT enzyme (WS/DGAT) has been found in all neutral lipid producing prokaryotes studied so far. WS/DAGAT has extraordinary broad activity on a variety of unusual fatty acids, alcohols and even thiols. This enzyme has a putative membrane-spanning region but shows no sequence homology to the DGAT1 and DGAT2 families from eukaryotes or the WE synthase from jojoba (Jojoba is the only eukaryote that has been found to accumulate wax ester).

It should be noted that Lecithin-Cholesterol AcylTransferase (LCAT) and Acyl-coenzyme:Cholesterol. AcylTransferase (ACAT) are enzymes that produce sterol esters (a form of neutral lipid) not TAGs.

In applications requiring the increase of neutral lipids evidence suggests that the higher activity and broader specificity of DGAT1 relative to DGAT2 is preferential. Where a specific fatty acid is preferred, such as a long-chain PUFA, DGAT1 is still applicable, provided it accepts the fatty acid of choice. Plants generally incorporate long chain PUFAs in the sn-2 position. It is not known whether this is due to high activity of LPAT or low activity of DGAT1 on this substrate. For the improved specificity for PUFAs, a DGAT2 that prefers these fatty acids may be preferable, or the properties of DGAT1 could be altered using directed evolution or an equivalent procedure.

Examples of these TAG synthesising enzymes, suitable for use in the methods and compositions of the invention, from members of several plant species are provided in Table 2 below. The sequences (both polynucleotide and polypeptide are provided in the Sequence Listing)

**Table 2**

| **TAG synthesising enzyme** | **Species** | **cDNA accession no.** | **SEQ ID NO:** | **Protein accession no.** | **SEQ ID NO:** |
|---|---|---|---|---|---|
| DGAT1 | *A. thaliana* | NM_127503 | 74 | NP_179535 | 75 |
| DGAT1 | *T. majus* | AY084052 | 76 | AAM03340 | 77 |
| DGAT1 | Z. *mays* | EU039830 | 78 | ABV91586 | 79 |
| DGAT2 | *A. thaliana* | NM_115011 | 80 | NP_566952 | 81 |
| DGAT2 | *B. napus* | FJ858270 | 82 | AC090187 | 83 |
| DGAT3 (soluble DGAT) | *A. hypogaea* | AY875644 | 84 | AAX62735 | 85 |
| PDAT | *A. thaliana* | NM_121367 | 86 | NP_196868 | 87 |
| PDAT | *R. communis* | XM_00252130 4 | 88 | XP_002521350 | 89 |

The inventions also contemplates use of modified TAG synthesizing enzymes, that are modified (for example in their sequence by substitutions, insertions or additions an the like) to alter their specificity and or activity.

### TAG accumulation in leaves

A recent field survey of 302 angiosperm species in the north-central USA found that 24% have conspicuous cytosolic oil droplets in leaves, with usually one large oil droplet per mesophyll cell (Lersten *et al.,* 2006 [from Slocombe *et al* 2009]). The role of cytosolic leaf TAG is thought to be involved in carbon storage and/or membrane lipid re-modelling (for review see Slocombe *et al.,* 2009). Indeed, in senescing leaves, plastidial fatty acids are partitioned into TAG prior for further mobilization, and DGAT1 is thought to be instrumental in this process (Kaup *et al.,* 2002).

There have been several attempts to engineer plants to accumulate elevated levels of TAG in their leaves. The success of these has been somewhat limited by the relatively low level of TAG that accumulated and in some cases the majority of TAG accumulated in senescing leaves only, thus limiting the flexibility of harvesting and proportion of crop accumulating TAG at any one time (Bouvier-Nave *et al,* 2001; Xu *et al.,* 2005; Winichayakul *et al.,* 2008; Andrianov et al., 2010; Slocombe *et al.,* 2009 and references therein).

To date the attempts to accumulate TAG in leaves have predominantly focussed on three particular gene candidates including over expression of DGAT (TAG biosynthesis), mutation of TGD1 or CTS (resulting in the prevention of lipid remobilisation), and over expression of LEC1, LEC2 and WRI1 (transcriptional factors involved in storage oil and protein accumulation in developing seeds). Over expression of TAG and other neutral lipid synthesizing enzymes relies on the presence of sufficient substrate, in the expanding and or mature leaf this is assumed to be provided by the plastid (chloroplast in the case of the leaf) which synthesises lipids for membranes. In photosynthetic leaves of Arabidopsis it has been estimated that the turnover of membrane lipids is 4% of total fatty acids per day (Bao *et al,* 2000). In senescing leaves, the existing plastidal membranes provide the bulk of fatty acids for partitioning into TAG prior to further mobilization.

Over-expression of the Arabidopsis DGAT1 gene in tobacco leaves results in enhanced TAG accumulation (Bouvier-Nave *et al.,* 2001), this was later repeated and quantified by Andrianov et al., (2010). They calculated the TAG level increased 20 fold and lead to a doubling of lipid content from ∼3% to ∼6% of dry matter in mature leaves. A further increase to 6.8% was achieved by the over expression of LEC2 (a master regulator of seed maturation and seed oil storage) in mature leaves using the inducible Alc promoter (Andrianov et al., 2010). No estimation of the extractable TAG was given, nor was there any calculation on the accumulation of TAG in expanding leaves.

Mutations in a permease-like protein TRIGALACTOSYLDIACYLGLYCEROL (TGD1), in Arabidopsis thaliana caused the accumulation of TAGs, oligogalactolipids and phosphatidate; this was accompanied by a high incidence of embryo abortion and comparatively poor overall plant growth (Xu *et al.,* 2005).

Winichayakul *et al.,* (2008) over expressed Arabidopsis thaliana DGAT1 in the leaves of ryegrass (Lolium perenne) and found this lead to a 50% elevation of total extractable leaf lipid (from ∼4% to 6% of dry matter). Furthermore, the elevated lipid level was present in new leaves generated by repeated harvests spaced 2-3 weeks apart, indicating that the new emerging leaves were also capable of accumulating additional lipid. However, the elevated lipid level in these leaves typically began to decline to wild type levels when the leaves were more than 2 weeks old indicating that the lipids were being re-mobilised via catabolism (release from the glycerol backbone by lipase followed by β-oxidation).

Slocombe *et al.,* (2009) demonstrated that mutations in the CTS peroxisomal ABC transporter (cts-2) led to accumulation of up to 1.4% TAG in leaves, particularly during the onset of senescence. They also ectopically expressed LEC2 during senescence in the cts-2 background; while this did not elevate the overall accumulation of TAG over the cts-2 mutant it did increase the accumulation of seed oil type species of TAG in senescing tissue. While cts-2 blocks fatty acid breakdown it also led to a severe phenotype. Slocombe *et al.,* (2009) concluded that recycled membrane fatty acids may be able to be re-directed to TAG by expressing the seed-programme in senescing tissue or by a block in fatty acid breakdown.

Scott *et al.,* (2007) claimed that the co-expression of a triacylglyceride synthesising enzyme and polyoleosin (two or more oleosin units fused in a tandem head-to-tail arrangement) would enable the storage of lipid in a plant cell. Similarly, Cookson *et al.,* (2009) claimed that producing a single oleosin and a TAG synthesising enzyme within vegetative portions of a plant would lead to increased number of oil bodies and TAG in the vegetative tissue. Using either of these techniques leads to a maximum increase in lipid content (not necessarily in the form of TAG) of up to approximately 50%. Furthermore this level begins to decline as the leaves mature; typically in leaves greater than 2 weeks old (unpublished data).

Hence, the degree to which TAG can be accumulated in vegetative tissues appears to be limited to some extent by the fact that the endogenous fixed-carbon recovery machinery catabolises the TAG.

### Leaf senescence - recycling of lipids via TAG intermediates

Leaf senescence is a highly controlled sequence of events leading ultimately to the death of cells, tissues and finally the whole organ. This entails regulated recruitment of nutrients together with their translocation from the senescing tissue to other tissues that are still growing and developing. The chloroplast is the first organelle of mesophyll cells to show symptoms of senescence and although breakdown of thylakoid membranes is initiated early in the leaf senescence cascade, the chloroplast envelope remains relatively intact until the very late stages of senescence. DGAT1 is up-regulated during senescence of Arabidopsis leaves and this is temporally correlated with increased levels of TAG-containing fatty acids commonly found in chloroplast galactolipids. Recruitment of membrane carbon from senescing leaves, particularly senescing chloroplasts, to growing parts of the plant is a key feature of leaf senescence, and it involves de-esterification of thylakoid lipids and conversion of the resultant free fatty acids to phloem-mobile sucrose. Deesterification of thylakoid lipids appears to be mediated by one or more senescence induced galactolipases. The formation of TAG appears to be an intermediate step in the mobilisation of membrane lipid carbon to phloem mobile sucrose during senescence (Kaup *et* a/., 2002).

### Modified oleosins engineered to include artificially introduced cysteines

The modified oleosins of the invention, or for use in the methods of the invention, are modified to contain at least one artificially introduced cysteine residue.

The cysteines are artificially introduced into the N-terminal hydrophilic region of the oleosin, or into the C-terminal hydrophilic region of the oleosin. Preferably the engineered oleosins contain at least two cysteines.

The encapsulation of the neutral lipids by oleosins containing engineered cysteines provides an alternative mechanism to accumulate appreciable quantities of TAG in leaves without the requirement to wait until senescence and without producing extreme phenotypes.

Various methods well-known to those skilled in the art may be used in production of the modified oleosins with artificially introduced cysteines.

Such methods include site directed mutagenesis (US 6,448,048) in which the polynucleotide encoding an oleosin is modified to introduce a cysteine into the encoded oleosin protein.

Alternatively the polynucleotide encoding the modified oleosins, may be synthcsed in its entirety.

Further methodology for producing modified oleosins of the invention and for use in the methods of the invention, is provided in the Examples section.

The introduced cysteine may be an additional amino acid (i.e. an insertion) or may replace an existing amino acid (i.e. a replacement). Preferably the introduced cysteine replaces an existing amino acid. In a preferred embodiment the replaced amino acid is a charged residue. Preferably the charged residue is predicted to be in the hydrophilic domains and therefore likely to be located on the surface of the oil body.

The hydrophilic, and hydrophobic regions/arms of the oleosin can be easily identified by those skilled in the art using standard methodology (for example: Kyte and Doolitle (1982).

The modified oleosins of the invention are preferably range in molecular weight from 5 to 50 kDa, more preferably, 10 to 40kDa, more preferably 15 to 25 kDa.

The modified oleosins of the invention are preferably in the size range 100 to 300 amino acids, more preferably 110 to 260 amino acids, more preferably 120 to 250 amino acids, more preferably 130 to 240 amino acids, more preferably 140 to 230 amino acids.

Preferably the modified oleosins comprise an N-terminal hydrophilic region, two centre hydrophobic regions (joined by a proline knot or knob) and a C-terminal hydrophilic region.

Preferably the modified oleosins can be divided almost equally their length into four parts which correspond to the N-terminal hydrophilic region (or arm), the two centre hydrophobic regions (joined by a proline knot or knob) and a C-terminal hydrophilic region (or arm).

Preferably the topology of modified oleosin is attributed to its physical properties which include a folded hydrophobic core flanked by hydrophilic domains.

Preferably the modified oleosins can be formed into oil bodies when combined with triacylglycerol (TAG) and phospholipid.

Preferably topology confers an amphipathic nature to modified oleosin resulting in the hydrophobic domain being embedded in the phospholipid monolayer of the oil body while the flanking hydrophilic domains are exposed to the aqueous environment outside the oil body, such as in the cytoplasm.

In one embodiment the modified oleosin of the invention or used in the method of the invention, comprises a sequence with at least 70% identity the hydrophobic domain of any of the oleosin protein sequences referred to in Table 1 above.

In one embodiment the modified oleosin of the invention or used in the method of the invention, comprises a sequence with at least 70% identity to any of the protein sequences referred to in Table 1 above.

In further embodiment the modified oleosin is essentially the same as any of the oleosins referred to in Table 1 above, apart from the additional artificially introduced cysteine or cysteines.

In a further embodiment the modified oleosin of the invention or used in the method of the invention, comprises a sequence with at least 70% identity to the oleosin sequence of SEQ ID NO: 16.

In further embodiment the modified oleosin has the same amino acid sequence as that of SEQ ID NO: 16, apart from the additional artificially introduced cysteine or cysteines.

In further embodiment the modified oleosin is has the amino acid sequence of any one of SEQ ID NO: 16 to 20.

### Fusion proteins with modified oleosins

The invention also provides a fusion proteins including a modified oleosin of the invention fused to a protein of interest.

Preferably the protein of interest is at the N- or C-terminal end of the fusion protein.

Methods for recombinantly expressing fusion proteins are well known to those skilled in the art (Papapostolou and Howorka, 2009). Production of the fusion protein of the invention may typically involve fusing the coding sequence of the protein of interest to the coding sequence of the modified oleosin.

Such fusion proteins may be included in, or expressed in, the oil bodies of the invention and used to purify and deliver the protein of interest for a variety of applications, as discussed in Roberts *et al,* (2008).

However in the invention makes it possible to take advantage of the option to vary the stability/integrity of the oil body provided by presence of the modified oleosins in the oil body, hence allowing for more stringent purification and delivery procedures.

### Fusion proteins with un-modified oleosins

The invention also involves use of fusion protein including un-modified oleosin fused to a protein of interest. Production of the fusion protein of the invention may typically involve fusing the coding sequence of the protein of interest to the coding sequence of the un-modified oleosin.

Preferably the protein of interest is at the N- or C-terminal end of the fusion protein.

Such fusion proteins may be included or expressed in the oil bodies of the invention and used to purify and deliver the protein of interest for a variety of applications, as discussed in Roberts *et al.,* (2008).

The present invention however, takes advantage of the option to vary the stability/integrity of the oil body provided by presence of the modified oleosins in the oil body of the invention, hence allowing for more stringent purification and delivery procedures.

### Vegetative tissues

Vegetative tissue include, shoots, leaves, roots, stems. A preferred vegetative tissue is a leaf.

### Vegetative tissue specific promoters

An example of a vegetative specific promoter is found in US 6,229,067; and US 7,629,454; and US 7,153,953; and US 6,228,643.

### Pollen specific promoters

An example of a pollen specific promoter is found in US 7,141,424; and US 5,545,546; and US 5,412,085; and US 5,086,169; and US 7,667,097.

### Seed specific promoters,

An example of a seed specific promoter is found in US 6,342,657; and US 7,081,565; and US 7,405,345; and US 7,642,346; and US 7,371,928.

### Fruit specific promoters

An example of a fruit specific promoter is found in US 5,536,653; and US 6,127,179; and US 5,608,150; and US 4;943,674.

### Polynucleotides and fragments

The term "polynucleotide(s)," as used herein, means a single or double-stranded deoxyribonucleotide or ribonucleotide polymer of any length but preferably at least 15 nucleotides, and include as non-limiting examples, coding and non-coding sequences of a gene, sense and antisense sequences complements, exons, introns, genomic DNA, cDNA, pre-mRNA, mRNA, rRNA, siRNA, miRNA, tRNA, ribozymes, recombinant polypeptides, isolated and purified naturally occurring DNA or RNA sequences, synthetic RNA and DNA sequences, nucleic acid probes, primers and fragments.

A "fragment" of a polynucleotide sequence provided herein is a subsequence of contiguous nucleotides that is capable of specific hybridization to a target of interest, e.g., a sequence that is at least 15 nucleotides in length. The fragments of the invention comprise 15 nucleotides, preferably at least 16 nucleotides, more preferably at least 17 nucleotides, more preferably at least 18 nucleotides, more preferably at least 19 nucleotides, more preferably at least 20 nucleotides, more preferably at least 21 nucleotides, more preferably at least 22 nucleotides, more preferably at least 23 nucleotides, more preferably at least 24 nucleotides, more preferably at least 25 nucleotides, more preferably at least 26 nucleotides, more preferably at least 27 nucleotides, more preferably at least 28 nucleotides, more preferably at least 29 nucleotides, more preferably at least 30 nucleotides, more preferably at least 31 nucleotides, more preferably at least 32 nucleotides, more preferably at least 33 nucleotides, more preferably at least 34 nucleotides, more preferably at least 35 nucleotides, more preferably at least 36 nucleotides, more preferably at least 37 nucleotides, more preferably at least 38 nucleotides, more preferably at least 39 nucleotides, more preferably at least 40 nucleotides, more preferably at least 41 nucleotides, more preferably at least 42 nucleotides, more preferably at least 43 nucleotides, more preferably at least 44 nucleotides, more preferably at least 45 nucleotides, more preferably at least 46 nucleotides, more preferably at least 47 nucleotides, more preferably at least 48 nucleotides, more preferably at least 49 nucleotides, more preferably at least 50 nucleotides, more preferably at least 51 nucleotides, more preferably at least 52 nucleotides, more preferably at least 53 nucleotides, more preferably at least 54 nucleotides, more preferably at least 55 nucleotides, more preferably at least 56 nucleotides, more preferably at least 57 nucleotides, more preferably at least 58 nucleotides, more preferably at least 59 nucleotides, more preferably at least 60 nucleotides, more preferably at least 61 nucleotides, more preferably at least 62 nucleotides, more preferably at least 63 nucleotides, more preferably at least 64 nucleotides, more preferably at least 65 nucleotides, more preferably at least 66 nucleotides, more preferably at least 67 nucleotides, more preferably at least 68 nucleotides, more preferably at least 69 nucleotides, more preferably at least 70 nucleotides, more preferably at least 71 nucleotides, more preferably at least 72 nucleotides, more preferably at least 73 nucleotides, more preferably at least 74 nucleotides, more preferably at least 75 nucleotides, more preferably at least 76 nucleotides, more preferably at least 77 nucleotides, more preferably at least 78 nucleotides, more preferably at least 79 nucleotides, more preferably at least 80 nucleotides, more preferably at least 81 nucleotides, more preferably at least 82 nucleotides, more preferably at least 83 nucleotides, more preferably at least 84 nucleotides, more preferably at least 85 nucleotides, more preferably at least 86 nucleotides, more preferably at least 87 nucleotides, more preferably at least 88 nucleotides, more preferably at least 89 nucleotides, more preferably at least 90 nucleotides, more preferably at least 91 nucleotides, more preferably at least 92 nucleotides, more preferably at least 93 nucleotides, more preferably at least 94 nucleotides, more preferably at least 95 nucleotides, more preferably at least 96 nucleotides, more preferably at least 97 nucleotides, more preferably at least 98 nucleotides, more preferably at least 99 nucleotides, more preferably at least 100 nucleotides, more preferably at least 150 nucleotides, more preferably at least 200 nucleotides, more preferably at least 250 nucleotides, more preferably at least 300 nucleotides, more preferably at least 350 nucleotides, more preferably at least 400 nucleotides, more preferably at least 450 nucleotides and most preferably at least 500 nucleotides of contiguous nucleotides of a polynucleotide disclosed. A fragment of a polynucleotide sequence can be used in antisense, RNA interference (RNAi), gene silencing, triple helix or ribozyme technology, or as a primer, a probe, included in a microarray, or used in polynucleotide-based selection methods of the invention.

The term "primer" refers to a short polynucleotide, usually having a free 3'OH group, that is hybridized to a template and used for priming polymerization of a polynucleotide complementary to the target.

The term "probe" refers to a short polynucleotide that is used to detect a polynucleotide sequence that is complementary to the probe, in a hybridization-based assay. The probe may consist of a "fragment" of a polynucleotide as defined herein.

### Polypeptides and fragments

The term "polypeptide", as used herein, encompasses amino acid chains of any length but preferably at least 5 amino acids, including full-length proteins, in which amino acid residues are linked by covalent peptide bonds. Polypeptides of the present invention, or used in the methods of the invention, may be purified natural products, or may be produced partially or wholly using recombinant or synthetic techniques. The term may refer to a polypeptide, an aggregate of a polypeptide such as a dimer or other multimer, a fusion polypeptide, a polypeptide fragment, a polypeptide variant, or derivative thereof.

A "fragment" of a polypeptide is a subsequence of the polypeptide that performs a function that is required for the biological activity and/or provides three dimensional structure of the polypeptide. The term may refer to a polypeptide, an aggregate of a polypeptide such as a dimer or other multimer, a fusion polypeptide, a polypeptide fragment, a polypeptide variant, or derivative thereof capable of performing the above enzymatic activity.

The term "isolated" as applied to the polynucleotide or polypeptide sequences disclosed herein is used to refer to sequences that are removed from their natural cellular environment. An isolated molecule may be obtained by any method or combination of methods including biochemical, recombinant, and synthetic techniques.

The term "recombinant" refers to a polynucleotide sequence that is removed from sequences that surround it in its natural context and/or is recombined with sequences that are not present in its natural context.

A "recombinant" polypeptide sequence is produced by translation from a "recombinant" polynucleotide sequence.

The term "derived from" with respect to polynucleotides or polypeptides of the invention being derived from a particular genera or species, means that the polynucleotide or polypeptide has the same sequence as a polynucleotide or polypeptide found naturally in that genera or species. The polynucleotide or polypeptide, derived from a particular genera or species, may therefore be produced synthetically or recombinantly.

### Variants

As used herein, the term "variant" refers to polynucleotide or polypeptide sequences different from the specifically identified sequences, wherein one or more nucleotides or amino acid residues is deleted, substituted, or added. Variants may be naturally occurring allelic variants, or non-naturally occurring variants. Variants may be from the same or from other species and may encompass homologues, paralogues and orthologues. In certain embodiments, variants of the inventive polypeptides and polypeptides possess biological activities that are the same or similar to those of the inventive polypeptides or polypeptides. The term "variant" with reference to polypeptides and polypeptides encompasses all forms of polypeptides and polypeptides as defined herein.

### Polynucleotide variants

Variant polynucleotide sequences preferably exhibit at least 50%, more preferably at least 51%, more preferably at least 52%, more preferably at least 53%, more preferably at least 54%, more preferably at least 55%, more preferably at least 56%, more preferably at least 57%, more preferably at least 58%, more preferably at least 59%, more preferably at least 60%, more preferably at least 61%, more preferably at least 62%, more preferably at least 63%, more preferably at least 64%, more preferably at least 65%, more preferably at least 66%, more preferably at least 67%, more preferably at least 68%, more preferably at least 69%, more preferably at least 70%, more preferably at least 71%, more preferably at least 72%, more preferably at least 73%, more preferably at least 74%, more preferably at least 75%, more preferably at least 76%, more preferably at least 77%, more preferably at least 78%, more preferably at least 79%, more preferably at least 80%, more preferably at least 81 %, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and most preferably at least 99% identity to a sequence of the present invention. Identity is found over a comparison window of at least 20 nucleotide positions, preferably at least 50 nucleotide positions, more preferably at least 100 nucleotide positions, and most preferably over the entire length of a polynucleotide of the invention.

Polynucleotide sequence identity can be determined in the following manner. The subject polynucleotide sequence is compared to a candidate polynucleotide sequence using BLASTN (from the BLAST suite of programs, version 2.2.5 [Nov 2002]) in bl2seq (Tatiana A. Tatusova, Thomas L. Madden (1999), "Blast 2 sequences - a new tool for comparing protein and nucleotide sequences", FEMS Microbiol Lett. 174:247-250), which is publicly available from NCBI (ftp://ftp.ncbi.nih.gov/blast/). The default parameters of bl2seq are utilized except that filtering of low complexity parts should be turned off.

The identity of polynucleotide sequences may be examined using the following unix command line parameters:
bl2seq -i nucleotideseq1 -j nucleotideseq2 -F F -p blastn

The parameter -F F turns off filtering of low complexity sections. The parameter -p selects the appropriate algorithm for the pair of sequences. The bl2seq program reports sequence identity as both the number and percentage of identical nucleotides in a line "Identities = ".

Polynucleotide sequence identity may also be calculated over the entire length of the overlap between a candidate and subject polynucleotide sequences using global sequence alignment programs (e.g. Needleman, S. B. and Wunsch, C. D. (1970) J. Mol. Biol. 48, 443-453). A full implementation of the Needleman-Wunsch global alignment algorithm is found in the needle program in the EMBOSS package (Rice,P. Longden,I. and Bleasby,A. EMBOSS: The European Molecular Biology Open Software Suite, Trends in Genetics June 2000, vol 16, No 6. pp.276-277) which can be obtained from http://www.hgmp.mrc.ac.uk/Software/EMBOSS/. The European Bioinformatics Institute server also provides the facility to perform EMBOSS-needle global alignments between two sequences on line at http:/www.ebi.ac.uk/emboss/align/.

Alternatively the GAP program may be used which computes an optimal global alignment of two sequences without penalizing terminal gaps. GAP is described in the following paper: Huang, X. (1994) On Global Sequence Alignment. Computer Applications in the Biosciences 10, 227-235.

A preferred method for calculating polynucleotide % sequence identity is based on aligning sequences to be compared using Clustal X (Jeanmougin et al., 1998, Trends Biochem. Sci. 23, 403-5.)

Polynucleotide variants of the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.5 [Nov 2002]) from NCBI (ftp://ftp.ncbi.nih.gov/blast/).

The similarity of polynucleotide sequences may be examined using the following unix command line parameters:
bl2seq -i nucleotideseq1 -j nucleotideseq2 -F F -p tblastx

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. The size of this database is set by default in the bl2seq program. For small E values, much less than one, the E value is approximately the probability of such a random match.

Variant polynucleotide sequences preferably exhibit an E value of less than 1 x 10 -6 more preferably less than 1 x 10 -9, more preferably less than 1 x 10 -12, more preferably less than 1 x 10 -15, more preferably less than 1 x 10 -18, more preferably less than 1 x 10 -21, more preferably less than 1 x 10 -30, more preferably less than 1 x 10 -40, more preferably less than 1 x 10 -50, more preferably less than 1 x 10 -60, more preferably less than 1 x 10 -70, more preferably less than 1 x 10 -80, more preferably less than 1 x 10 -90 and most preferably less than 1 x 10-100 when compared with any one of the specifically identified sequences.

Alternatively, variant polynucleotides of the present invention, or used in the methods of the invention, hybridize to the specified polynucleotide sequences, or complements thereof under stringent conditions.

The term "hybridize under stringent conditions", and grammatical equivalents thereof, refers to the ability of a polynucleotide molecule to hybridize to a target polynucleotide molecule (such as a target polynucleotide molecule immobilized on a DNA or RNA blot, such as a Southern blot or Northern blot) under defined conditions of temperature and salt concentration. The ability to hybridize under stringent hybridization conditions can be determined by initially hybridizing under less stringent conditions then increasing the stringency to the desired stringency.

With respect to polynucleotide molecules greater than about 100 bases in length, typical stringent hybridization conditions are no more than 25 to 30° C (for example, 10° C) below the melting temperature (Tm) of the native duplex (see generally, Sambrook et al., Eds, 1987, Molecular Cloning, A Laboratory Manual, 2nd Ed. Cold Spring Harbor Press; Ausubel et al., 1987, Current Protocols in Molecular Biology, Greene Publishing,). Tm for polynucleotide molecules greater than about 100 bases can be calculated by the formula Tm = 81. 5 + 0. 41% (G + C-log (Na+). (Sambrook et al., Eds, 1987, Molecular Cloning, A Laboratory Manual, 2nd Ed. Cold Spring Harbor Press; Bolton and McCarthy, 1962, PNAS 84:1390). Typical stringent conditions for polynucleotide of greater than 100 bases in length would be hybridization conditions such as prewashing in a solution of 6X SSC, 0.2% SDS; hybridizing at 65°C, 6X SSC, 0.2% SDS overnight; followed by two washes of 30 minutes each in 1X SSC, 0.1% SDS at 65° C and two washes of 30 minutes each in 0.2X SSC, 0.1% SDS at 65°C.

With respect to polynucleotide molecules having a length less than 100 bases, exemplary stringent hybridization conditions are 5 to 10° C below Tm. On average, the Tm of a polynucleotide molecule of length less than 100 bp is reduced by approximately (500/oligonucleotide length)° C.

With respect to the DNA mimics known as peptide nucleic acids (PNAs) (Nielsen et al., Science. 1991 Dec 6;254(5037):1497-500) Tm values are higher than those for DNA-DNA or DNA-RNA hybrids, and can be calculated using the formula described in Giesen et al., Nucleic Acids Res. 1998 Nov 1;26(21):5004-6. Exemplary stringent hybridization conditions for a DNA-PNA hybrid having a length less than 100 bases are 5 to 10° C below the Tm.

Variant polynucleotides of the present invention, or used in the methods of the invention, also encompasses polynucleotides that differ from the sequences of the invention but that, as a consequence of the degeneracy of the genetic code, encode a polypeptide having similar activity to a polypeptide encoded by a polynucleotide of the present invention. A sequence alteration that does not change the amino acid sequence of the polypeptide is a "silent variation". Except for ATG (methionine) and TGG (tryptophan), other codons for the same amino acid may be changed by art recognized techniques, e.g., to optimize codon expression in a particular host organism.

Polynucleotide sequence alterations resulting in conservative substitutions of one or several amino acids in the encoded polypeptide sequence without significantly altering its biological activity are also included in the invention. A skilled artisan will be aware of methods for making phenotypically silent amino acid substitutions (see, e.g., Bowie et al., 1990, Science 247, 1306).

Variant polynucleotides due to silent variations and conservative substitutions in the encoded polypeptide sequence may be determined using the publicly available bl2seq program from the BLAST suite of programs (version 2.2.5 [Nov 2002]) from NCBI (ftp://ftp.ncbi.nih.gov/blast/) via the tblastx algorithm as previously described.

### Polypeptide variants

The term "variant" with reference to polypeptides encompasses naturally occurring, recombinantly and synthetically produced polypeptides. Variant polypeptide sequences preferably exhibit at least 50%, more preferably at least 51%, more preferably at least 52%, more preferably at least 53%, more preferably at least 54%, more preferably at least 55%, more preferably at least 56%, more preferably at least 57%, more preferably at least 58%, more preferably at least 59%, more preferably at least 60%, more preferably at least 61%, more preferably at least 62%, more preferably at least 63%, more preferably at least 64%, more preferably at least 65%, more preferably at least .66%, more preferably at least 67%, more preferably at least 68%, more preferably at least 69%, more preferably at least 70%, more preferably at least 71%, more preferably at least 72%, more preferably at least 73%, more preferably at least 74%, more preferably at least 75%, more preferably at least 76%, more preferably at least 77%, more preferably at least 78%, more preferably at least 79%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and most preferably at least 99% identity to a sequences of the present invention. Identity is found over a comparison window of at least 20 amino acid positions, preferably at least 50 amino acid positions, more preferably at least 100 amino acid positions, and most preferably over the entire length of a polypeptide of the invention.

Polypeptide sequence identity can be determined in the following manner. The subject polypeptide sequence is compared to a candidate polypeptide sequence using BLASTP (from the BLAST suite of programs, version 2.2.5 [Nov 2002]) in bl2seq, which is publicly available from NCBI (ftp://ftp.ncbi.nih.gov/blast/). The default parameters of bl2seq are utilized except that filtering of low complexity regions should be turned off.

Polypeptide sequence identity may also be calculated over the entire length of the overlap between a candidate and subject polynucleotide sequences using global sequence alignment programs. EMBOSS-needle (available at http:/www.ebi.ac.uk/emboss/align/) and GAP (Huang, X. (1994) On Global Sequence Alignment. Computer Applications in the Biosciences 10, 227-235.) as discussed above are also suitable global sequence alignment programs for calculating polypeptide sequence identity.

A preferred method for calculating polypeptide % sequence identity is based on aligning sequences to be compared using Clustal X (Jeanmougin et al., 1998, Trends Biochem. Sci. 23, 403-5.)

Polypeptide variants of the present invention, or used in the methods of the invention, also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.5 [Nov 2002]) from NCBI (ftp://ftp.ncbi.nih.gov/blast/). The similarity of polypeptide sequences may be examined using the following unix command line parameters:
bl2seq -i peptideseq1 -j peptideseq2 -F F -p blastp

Variant polypeptide sequences preferably exhibit an E value of less than 1 x 10 -6 more preferably less than 1 x 10 -9, more preferably less than 1 x 10 -12, more preferably less than 1 x 10 -15, more preferably less than 1 x 10 -18, more preferably less than 1 x 10 -21, more preferably less than 1 x 10 -30, more preferably less than 1 x 10 -40, more preferably less than 1 x 10 -50, more preferably less than 1 x 10 -60, more preferably less than 1 x 10 -70, more preferably less than 1 x 10 -80, more preferably less than 1 x 10 -90 and most preferably 1 x 10-100 when compared with any one of the specifically identified sequences.

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. For small E values, much less than one, this is approximately the probability of such a random match.

Conservative substitutions of one or several amino acids of a described polypeptide sequence without significantly altering its biological activity are also included in the invention. A skilled artisan will be aware of methods for making phenotypically silent amino acid substitutions (see, e.g., Bowie et al., 1990, Science 247, 1306).

### Constructs, vectors and components thereof

The term "genetic construct" refers to a polynucleotide molecule, usually double-stranded DNA, which may have inserted into it another polynucleotide molecule (the insert polynucleotide molecule) such as, but not limited to, a cDNA molecule. A genetic construct may contain the necessary elements that permit transcribing the insert polynucleotide molecule, and, optionally, translating the transcript into a polypeptide. The insert polynucleotide molecule may be derived from the host cell, or may be derived from a different cell or organism and/or may be a recombinant polynucleotide. Once inside the host cell the genetic construct may become integrated in the host chromosomal DNA. The genetic construct may be linked to a vector.

The term "vector" refers to a polynucleotide molecule, usually double stranded DNA, which is used to transport the genetic construct into a host cell. The vector may be capable of replication in at least one additional host system, such as *E. coli.*

The term "expression construct" refers to a genetic construct that includes the necessary elements that permit transcribing the insert polynucleotide molecule, and, optionally, translating the transcript into a polypeptide. An expression construct typically comprises in a 5' to 3' direction:
a) a promoter functional in the host cell into which the construct will be transformed,
b) the polynucleotide to be expressed, and
c) a terminator functional in the host cell into which the construct will be transformed.

The term "coding region" or "open reading frame" (ORF) refers to the sense strand of a genomic DNA sequence or a cDNA sequence that is capable of producing a transcription product and/or a polypeptide under the control of appropriate regulatory sequences. The coding sequence may, in some cases, identified by the presence of a 5' translation start codon and a 3' translation stop codon. When inserted into a genetic construct, a "coding sequence" is capable of being expressed when it is operably linked to promoter and terminator sequences.

"Operably-linked" means that the sequenced to be expressed is placed under the control of regulatory elements that include promoters, tissue-specific regulatory elements, temporal regulatory elements, enhancers, repressors and terminators.

The term "noncoding region" refers to untranslated sequences that are upstream of the translational start site and downstream of the translational stop site. These sequences are also referred to respectively as the 5' UTR and the 3' UTR. These regions include elements required for transcription initiation and termination, mRNA stability, and for regulation of translation efficiency.

Terminators are sequences, which terminate transcription, and are found in the 3' untranslated ends of genes downstream of the translated sequence. Terminators are important determinants of mRNA stability and in some cases have been found to have spatial regulatory functions.

The term "promoter" refers to nontranscribed cis-regulatory elements upstream of the coding region that regulate gene transcription. Promoters comprise cis-initiator elements which specify the transcription initiation site and conserved boxes such as the TATA box, and motifs that are bound by transcription factors. Introns within coding sequences can also regulate transcription and influence post-transcriptional processing (including splicing, capping and polyadenylation).

A promoter may be homologous with respect to the polynucleotide to be expressed. This means that the promoter and polynucleotide are found operably linked in nature.

Alternatively the promoter may be heterologous with respect to the polynucleotide to be expressed. This means that the promoter and the polynucleotide are not found operably linked in nature.

A "transgene" is a polynucleotide that is taken from one organism and introduced into a different organism by transformation. The transgene may be derived from the same species or from a different species as the species of the organism into which the transgene is introduced.

An "inverted repeat" is a sequence that is repeated, where the second half of the repeat is in the complementary strand, e.g.,
(5')GATCTA.......TAGATC(3')
(3')CTAGAT.......ATCTAG(5')

Read-through transcription will produce a transcript that undergoes complementary base-pairing to form a hairpin structure provided that there is a 3-5 bp spacer between the repeated regions.

### Host cells

Host cells may be derived from, for example, bacterial, fungal, yeast, insect, mammalian, algal or plant organisms. Host cells may also be synthetic cells. Preferred host cells are eukaryotic cells. A particularly preferred host cell is a plant cell, particularly a plant cell in a vegetative tissue of a plant.

A "transgenic plant" refers to a plant which contains new genetic material as a result of genetic manipulation or transformation. The new genetic material may be derived from a plant of the same species as the resulting transgenic plant or from a different species.

### Methods for isolating or producing polynucleotides

The polynucleotide molecules of the invention can be isolated by using a variety of techniques known to those of ordinary skill in the art. By way of example, such polypeptides can be isolated through use of the polymerase chain reaction (PCR) described in Mullis et al., Eds. 1994 The Polymerase Chain Reaction, Birkhauser, incorporated herein by reference. The polypeptides of the invention can be amplified using primers, as defined herein, derived from the polynucleotide sequences of the invention.

Further methods for isolating polynucleotides of the invention include use of all, or portions of, the polypeptides having the sequence set forth herein as hybridization probes. The technique of hybridizing labelled polynucleotide probes to polynucleotides immobilized on solid supports such as nitrocellulose filters or nylon membranes, can be used to screen the genomic or cDNA libraries. Exemplary hybridization and wash conditions are: hybridization for 20 hours at 65°C in 5. 0 X SSC, 0. 5% sodium dodecyl sulfate, 1 X Denhardt's solution; washing (three washes of twenty minutes each at 55°C) in 1. 0 X SSC, 1% (w/v) sodium dodecyl sulfate, and optionally one wash (for twenty minutes) in 0. 5 X SSC, 1% (w/v) sodium dodecyl sulfate, at 60°C. An optional further wash (for twenty minutes) can be conducted under conditions of 0.1 X SSC, 1% (w/v) sodium dodecyl sulfate, at 60°C.

The polynucleotide fragments of the invention may be produced by techniques well-known in the art such as restriction endonuclease digestion, oligonucleotide synthesis and PCR amplification.

A partial polynucleotide sequence may be used, in methods well-known in the art to identify the corresponding full length polynucleotide sequence. Such methods include PCR-based methods, 5'RACE (Frohman MA, 1993, Methods Enzymol. 218: 340-56) and hybridization- based method, computer/database -based methods. Further, by way of example, inverse PCR permits acquisition of unknown sequences, flanking the polynucleotide sequences disclosed herein, starting with primers based on a known region (Triglia et al., 1998, Nucleic Acids Res 16, 8186, incorporated herein by reference). The method uses several restriction enzymes to generate a suitable fragment in the known region of a gene. The fragment is then circularized by intramolecular ligation and used as a PCR template. Divergent primers are designed from the known region. In order to physically assemble full-length clones, standard molecular biology approaches can be utilized (Sambrook et al., Molecular Cloning: A Laboratory Manual, 2nd Ed. Cold Spring Harbor Press, 1987).

It may be beneficial, when producing a transgenic plant from a particular species, to transform such a plant with a sequence or sequences derived from that species. The benefit may be to alleviate public concerns regarding cross-species transformation in generating transgenic organisms. Additionally when down-regulation of a gene is the desired result, it may be necessary to utilise a sequence identical (or at least highly similar) to that in the plant, for which reduced expression is desired. For these reasons among others, it is desirable to be able to identify and isolate orthologues of a particular gene in several different plant species.

Variants (including orthologues) may be identified by the methods described.

### Methods for identifying variants

### Physical methods

Variant polypeptides may be identified using PCR-based methods (Mullis et al., Eds. 1994 The Polymerase Chain Reaction, Birkhauser). Typically, the polynucleotide sequence of a primer, useful to amplify variants of polynucleotide molecules of the invention by PCR, may be based on a sequence encoding a conserved region of the corresponding amino acid sequence.

Alternatively library screening methods, well known to those skilled in the art, may be employed (Sambrook et al., Molecular Cloning: A Laboratory Manual, 2nd Ed. Cold Spring Harbor Press, 1987). When identifying variants of the probe sequence, hybridization and/or wash stringency will typically be reduced relatively to when exact sequence, are sought.

Polypeptide variants may also be identified by physical methods, for example by screening expression libraries using antibodies raised against polypeptides of the invention (Sambrook et al., Molecular Cloning: A Laboratory Manual, 2nd Ed. Cold Spring Harbor Press, 1987) or by identifying polypeptides from natural sources with the aid of such antibodies.

### Computer based methods

The variant sequences of the invention, including both polynucleotide and polypeptide variants, may also be identified by computer-based methods well-known to those skilled in the art, using public domain sequence alignment algorithms and sequence similarity search tools to search sequence databases (public domain databases include Genbank, EMBL, Swiss-Prot, PIR and others). See, e.g., Nucleic Acids Res. 29: 1-10 and 11-16, 2001 for examples of online resources. Similarity searches retrieve and align target sequences for comparison with a sequence to be analyzed (i.e., a query sequence). Sequence comparison algorithms use scoring matrices to assign an overall score to each of the alignments.

An exemplary family of programs useful for identifying variants in sequence databases is the BLAST suite of programs (version 2.2.5 [Nov 2002]) including BLASTN, BLASTP, BLASTX, tBLASTN and tBLASTX, which are publicly available from (ftp://ftp.ncbi.nih.gov/blast/) or from the National Center for Biotechnology Information (NCBI), National Library of Medicine, Building 38A, Room 8N805, Bethesda, MD 20894 USA. The NCBI server also provides the facility to use the programs to screen a number of publicly available sequence databases. BLASTN compares a nucleotide query sequence against a nucleotide sequence database. BLASTP compares an amino acid query sequence against a protein sequence database. BLASTX compares a nucleotide query sequence translated in all reading frames against a protein sequence database. tBLASTN compares a protein query sequence against a nucleotide sequence database dynamically translated in all reading frames. tBLASTX compares the six-frame translations of a nucleotide query sequence against the six-frame translations of a nucleotide sequence database. The BLAST programs may be used with default parameters or the parameters may be altered as required to refine the screen.

The use of the BLAST family of algorithms, including BLASTN, BLASTP, and BLASTX, is described in the publication of Altschul et al., Nucleic Acids Res. 25: 3389-3402, 1997.

The "hits" to one or more database sequences by a queried sequence produced by BLASTN, BLASTP, BLASTX, tBLASTN, tBLASTX, or a similar algorithm, align and identify similar portions of sequences. The hits are arranged in order of the degree of similarity and the length of sequence overlap. Hits to a database sequence generally represent an overlap over only a fraction of the sequence length of the queried sequence.

The BLASTN, BLASTP, BLASTX, tBLASTN and tBLASTX algorithms also produce "Expect" values for alignments. The Expect value (E) indicates the number of hits one can "expect" to see by chance when searching a database of the same size containing random contiguous sequences. The Expect value is used as a significance threshold for determining whether the hit to a database indicates true similarity. For example, an E value of 0.1 assigned to a polynucleotide hit is interpreted as meaning that in a database of the size of the database screened, one might expect to see 0.1 matches over the aligned portion of the sequence with a similar score simply by chance. For sequences having an E value of 0.01 or less over aligned and matched portions, the probability of finding a match by chance in that database is 1% or less using the BLASTN, BLASTP, BLASTX, tBLASTN or tBLASTX algorithm.

Multiple sequence alignments of a group of related sequences can be carried out with CLUSTALW (Thompson, J.D., Higgins, D.G. and Gibson, T.J. (1994) CLUSTALW: improving the sensitivity of progressive multiple sequence alignment through sequence weighting, positions-specific gap penalties and weight matrix choice. Nucleic Acids Research, 22:4673-4680, http://www-igbmc.u-strasbg.fr/BioInfo/ClustalW/Top.html) or T-COFFEE (Cedric Notredame, Desmond G. Higgins, Jaap Heringa, T-Coffee: A novel method for fast and accurate multiple sequence alignment, J. Mol. Biol. (2000) 302: 205-217)) or PILEUP, which uses progressive, pairwise alignments. (Feng and Doolittle, 1987, J. Mol. Evol. 25, 351).

Pattern recognition software applications are available for finding motifs or signature sequences. For example, MEME (Multiple Em for Motif Elicitation) finds motifs and signature sequences in a set of sequences, and MAST (Motif Alignment and Search Tool) uses these motifs to identify similar or the same motifs in query sequences. The MAST results are provided as a series of alignments with appropriate statistical data and a visual overview of the motifs found. MEME and MAST were developed at the University of California, San Diego.

PROSITE (Bairoch and Bucher, 1994, Nucleic Acids Res. 22, 3583; Hofmann et al., 1999, Nucleic Acids Res. 27, 215) is a method of identifying the functions of uncharacterized proteins translated from genomic or cDNA sequences. The PROSITE database (www.expasy.org/prosite) contains biologically significant patterns and profiles and is designed so that it can be used with appropriate computational tools to assign a new sequence to a known family of proteins or to determine which known domain(s) are present in the sequence (Falquet et al., 2002, Nucleic Acids Res. 30, 235). Prosearch is a tool that can search SWISS-PROT and EMBL databases with a given sequence pattern or signature.

### Methods for isolating polypeptides

The polypeptides of the invention, or used in the methods of the invention, including variant polypeptides, may be prepared using peptide synthesis methods well known in the art such as direct peptide synthesis using solid phase techniques (e.g. Stewart et al., 1969, in Solid-Phase Peptide Synthesis, WH Freeman Co, San Francisco California, or automated synthesis, for example using an Applied Biosystems 431A Peptide Synthesizer (Foster City, California). Mutated forms of the polypeptides may also be produced during such synthesis.

The polypeptides and variant polypeptides of the invention, or used in the methods of the invention, may also be purified from natural sources using a variety of techniques that are well known in the art (e.g. Deutscher, 1990, Ed, Methods in Enzymology, Vol. 182, Guide to Protein Purification,).

Alternatively the polypeptides and variant polypeptides of the invention, or used in the methods of the invention, may be expressed recombinantly in suitable host cells and separated from the cells as discussed below.

### Methods for producing constructs and vectors

The genetic constructs of the present invention comprise one or more polynucleotide sequences of the invention and/or polynucleotides encoding polypeptides of the invention, and may be useful for transforming, for example, bacterial, fungal, insect, mammalian or plant organisms. The genetic constructs of the invention are intended to include expression constructs as herein defined.

Methods for producing and using genetic constructs and vectors are well known in the art and are described generally in Sambrook et al., Molecular Cloning: A Laboratory Manual, 2nd Ed. Cold Spring Harbor Press, 1987 ; Ausubel et al., Current Protocols in Molecular Biology, Greene Publishing, 1987).

### Methods for producing host cells comprising polynucleotides, constructs or vectors

The invention provides a host cell which comprises a genetic construct or vector of the invention.

Host cells comprising genetic constructs, such as expression constructs, of the invention are useful in methods well known in the art (e.g. Sambrook et al., Molecular Cloning : A Laboratory Manual, 2nd Ed. Cold Spring Harbor Press, 1987 ; Ausubel et al., Current Protocols in Molecular Biology, Greene Publishing, 1987) for recombinant production of polypeptides of the invention. Such methods may involve the culture of host cells in an appropriate medium in conditions suitable for or conducive to expression of a polypeptide of the invention. The expressed recombinant polypeptide, which may optionally be secreted into the culture, may then be separated from the medium, host cells or culture medium by methods well known in the art (e.g. Deutscher, Ed, 1990, Methods in Enzymology, Vol 182, Guide to Protein Purification).

### Methods for producing plant cells and plants comprising constructs and vectors

The invention further provides plant cells which comprise a genetic construct of the invention, and plant cells modified to alter expression of a polynucleotide or polypeptide of the invention, or used in the methods of the invention. Plants comprising such cells also form an aspect of the invention.

Methods for transforming plant cells, plants and portions thereof with polypeptides are described in Draper et al., 1988, Plant Genetic Transformation and Gene Expression. A Laboratory Manual. Blackwell Sci. Pub. Oxford, p. 365; Potrykus and Spangenburg, 1995, Gene Transfer to Plants. Springer-Verlag, Berlin.; and Gelvin et al., 1993, Plant Molecular Biol. Manual. Kluwer Acad. Pub. Dordrecht. A review of transgenic plants, including transformation techniques, is provided in Galun and Breiman, 1997, Transgenic Plants. Imperial College Press, London.

### Methods for genetic manipulation of plants

A number of plant transformation strategies are available (e.g. Birch, 1997, Ann Rev Plant Phys Plant Mol Biol, 48, 297, Hellens RP, et al (2000) Plant Mol Biol 42: 819-32, Hellens R et al Plant Meth 1: 13). For example, strategies may be designed to increase expression of a polynucleotide/polypeptide in a plant cell, organ and/or at a particular developmental stage where/when it is normally expressed or to ectopically express a polynucleotide/polypeptide in a cell, tissue, organ and/or at a particular developmental stage which/when it is not normally expressed. The expressed polynucleotide/polypeptide may be derived from the plant species to be transformed or may be derived from a different plant species.

Transformation strategies may be designed to reduce expression of a polynucleotide/polypeptide in a plant cell, tissue, organ or at a particular developmental stage which/when it is normally expressed. Such strategies are known as gene silencing strategies.

Genetic constructs for expression of genes in transgenic plants typically include promoters for driving the expression of one or more cloned polynucleotide, terminators and selectable marker sequences to detect presence of the genetic construct in the transformed plant.

The promoters suitable for use in the constructs of this invention are functional in a cell, tissue or organ of a monocot or dicot plant and include cell-, tissue- and organ-specific promoters, cell cycle specific promoters, temporal promoters, inducible promoters, constitutive promoters that are active in most plant tissues, and recombinant promoters. Choice of promoter will depend upon the temporal and spatial expression of the cloned polynucleotide, so desired. The promoters may be those normally associated with a transgene of interest, or promoters which are derived from genes of other plants, viruses, and plant pathogenic bacteria and fungi. Those skilled in the art will, without undue experimentation, be able to select promoters that are suitable for use in modifying and modulating plant traits using genetic constructs comprising the polynucleotide sequences of the invention. Examples of constitutive plant promoters include the CaMV 35S promoter, the nopaline synthase promoter and the octopine synthase promoter, and the Ubi 1 promoter from maize. Plant promoters which are active in specific tissues, respond to internal developmental signals or external abiotic or biotic stresses are described in the scientific literature. Exemplary promoters are described, e.g., in WO 02/00894, which is herein incorporated by reference.

Exemplary terminators that are commonly used in plant transformation genetic construct include, e.g., the cauliflower mosaic virus (CaMV) 35S terminator, the *Agrobacterium tumefaciens* nopaline synthase or octopine synthase terminators, the *Zea mays* zein gene terminator, the *Oryza sativa* ADP-glucose pyrophosphorylase terminator and the *Solanum tuberosum* PI-II terminator.

Selectable markers commonly used in plant transformation include the neomycin phophotransferase II gene (NPT II) which confers kanamycin resistance, the aadA gene, which confers spectinomycin and streptomycin resistance, the phosphinothricin acetyl transferase (*bar* gene) for Ignite (AgrEvo) and Basta (Hoechst) resistance, and the hygromycin phosphotransferase gene (hpt) for hygromycin resistance.

Use of genetic constructs comprising reporter genes (coding sequences which express an activity that is foreign to the host, usually an enzymatic activity and/or a visible signal (e.g., luciferase, GUS, GFP) which may be used for promoter expression analysis in plants and plant tissues are also contemplated. The reporter gene literature is reviewed in Herrera-Estrella et al., 1993, Nature 303, 209, and Schrott, 1995, In: Gene Transfer to Plants (Potrykus, T., Spangenberg. Eds) Springer Verlag. Berline, pp. 325-336.

The following are representative publications disclosing genetic transformation protocols that can be used to genetically transform the following plant species: Rice (Alam et al., 1999, Plant Cell Rep. 18, 572); apple (Yao et al., 1995, Plant Cell Reports 14, 407-412); maize (US Patent Serial Nos. 5, 177, 010 and 5, 981, 840); wheat (Ortiz et al., 1996, Plant Cell Rep. 15, 1996, 877); tomato (US Patent Serial No. 5, 159, 135); potato (Kumar et al., 1996 Plant J. 9, : 821); cassava (Li et al., 1996 Nat. Biotechnology 14, 736); lettuce (Michelmore et al., 1987, Plant Cell Rep. 6, 439); tobacco (Horsch et al., 1985, Science 227, 1229); cotton (US Patent Serial Nos. 5, 846, 797 and 5, 004, 863); grasses (US Patent Nos. 5, 187, 073 and 6. 020, 539); peppermint (Niu et al., 1998, Plant Cell Rep. 17, 165); citrus plants (Pena et al., 1995, Plant Sci.104, 183); caraway (Krens et al., 1997, Plant Cell Rep, 17, 39); banana (US Patent Serial No. 5, 792, 935); soybean (US Patent Nos. 5, 416, 011 ; 5, 569, 834 ; 5, 824, 877 ; 5, 563, 04455 and 5, 968, 830); pineapple (US Patent Serial No. 5, 952, 543); poplar (US Patent No. 4, 795, 855); monocots in general (US Patent Nos. 5, 591, 616 and 6, 037, 522); brassica (US Patent Nos. 5, 188, 958 ; 5, 463, 174 and 5, 750, 871); cereals (US Patent No. 6, 074, 877); pear (Matsuda et al., 2005, Plant Cell Rep. 24(1):45-51); Prunus (Ramesh et al., 2006 Plant Cell Rep. 25(8):821-8; Song and Sink 2005 Plant Cell Rep. 2006 ;25(2):117-23; Gonzalez Padilla et al., 2003 Plant Cell Rep.22(1):38-45); strawberry (Oosumi et al., 2006 Planta. 223(6):1219-30; Folta et al., 2006 Planta Apr 14; PMID: 16614818), rose (Li et al., 2003), Rubus (Graham et al., 1995 Methods Mol Biol. 1995;44:129-33), tomato (Dan et al., 2006, Plant Cell Reports V25:432-441), apple (Yao et al., 1995, Plant Cell Rep. 14, 407-412), Canola (Brassica napus L.).(Cardoza and Stewart, 2006 Methods Mol Biol. 343:257-66), safflower (Orlikowska et al, 1995, Plant Cell Tissue and Organ Culture 40:85-91), ryegrass (Altpeter et al, 2004 Developments in Plant Breeding 11 (7):255-250), rice (Christou et al, 1991 Nature Biotech. 9:957-962), maize (Wang et al 2009 In: Handbook of Maize pp. 609-639) and *Actinidia eriantha* (Wang et al.; 2006, Plant Cell Rep. 25,5: 425-31). Transformation of other species is also contemplated by the invention. Suitable methods and protocols are available in the scientific literature.

### Plants

The term "plant" is intended to include a whole plant, any part of a plant, a seed, a fruit, propagules and progeny of a plant.

The term 'propagule' means any part of a plant that may be used in reproduction or propagation, either sexual or asexual, including seeds and cuttings.

The plants of the invention may be grown and either self-ed or crossed with a different plant strain and the resulting hybrids, with the desired phenotypic characteristics, may be identified. Two or more generations may be grown to ensure that the subject phenotypic characteristics are stably maintained and inherited. Plants resulting from such standard breeding approaches also form an aspect of the present invention.

### Abbreviations

**Oleosin (or Ole)_0-0 means an oleosin without engineered** cysteines**.**
**Oleosin (or Ole)_1-1 means an oleosin with one engineered cysteine in each hydrophilic arm.**
**Oleosin (or Ole)_1-3 means an oleosin with one engineered cysteine in the N-terminal hydrophilic arm and three engineered cysteines in the C-terminal hydrophilic arm.**
**Oleosin (or Ole)_3-1 means an oleosin with three engineered cysteines in the N-terminal hydrophilic arm and one engineered cysteine in the C-terminal hydrophilic arm.**
**Oleosin (or Ole)_3-3 means an oleosin with three engineered cysteines in the N-terminal hydrophilic arm and three engineered cysteines in the C-terminal hydrophilic arm.**
**Oleosin (or Ole)_5-6 means an oleosin with five engineered cysteines in the N-terminal hydrophilic arm and six engineered cysteines in the C-terminal hydrophilic arm.**
**Oleosin (or Ole)_6-7 means an oleosin with six engineered cysteines in the N-terminal hydrophilic arm and seven engineered cysteines in the C-terminal hydrophilic arm.**

### BRIEF DESCRIPTION OF THE FIGURES

Figure 1 shows the sequence of the Oleosin_0-0 and DGAT1 (S205A) construct. CaMV35 is the Cauliflower Mosais Virus 35S promoter. attB1 is the GATEWAY™ recombination site. UBQ10 is the intron from the A. thaliana UBQ10 gene. OCS terminator is the octopine synthase terminator.
Figure 2 shows the Oleosin_1-1 and DGAT1 (S205A) construct arrangement, as transformed into Arabidopsis thaliana.
Figure 3 shows the sequence of the Oleosin_1-3 and DGAT1 (S205A) construct. CaMV35 is the Cauliflower Mosais Virus 35S promoter. attB1 is the GATEWAY™ recombination site. UBQ10 is the intron from the A. thaliana UBQ10 gene. OCS terminator is the-octopine synthase terminator.
Figure 4 shows the Oleosin_3-1 and DGAT1 (S205A) construct. CaMV35 is the Cauliflower Mosais Virus 35S promoter. attB1 is the GATEWAY™ recombination site. UBQ10 is the intron from the A. thaliana UBQ10 gene. OCS terminator is the octopine synthase terminator.
Figure 5 shows the Oleosin_3-3 and DGAT1 (S205A) construct. CaMV35 is the Cauliflower Mosais Virus 35S promoter. attB1 is the GATEWAY™ recombination site. UBQ10 is the intron from the A. thaliana UBQ10 gene. OCS terminator is the octopine synthase terminator.
Figure 6 shows a map of the construct pRSh1 used for transforming plants. The map shows the arrangement of the oleosins, with artificially introduced cysteines (in this case Oleo_3-3) under the control of the CaMV35s promoter as well as Arabidopsis thaliana DGAT1 (S205A) also under the control of the CaMV35s promoter. Other oleosin sequences and TAG synthesising enzyme sequences can of course be substituted for Oleo_3-3 and DGAT1 respectively.
Figure 7 shows dot blot comparison of anti-sesame seed oleosin antibodies binding to purified recombinant sesame seed oleosin with and without engineered cysteine residues.
Figure 8 shows immunoblot analysis to detect *E*. *coli* expressed oleosin cysteine proteins in AOBs. Equal volume of AOB (7.5 µL including 2x SDS loading dye without reducing agent) was loaded per lane. The mM concentration of GSSG is indicated above each lane.
Figure 9 shows SDS and SDS-UREA PAGE/immunoblot analysis of *E. coli* expressed Ole-0-0, Ole-1-1 and Ole-3-3. Samples were prepared from inclusion bodies (IB) and artificial oil bodies (AOBs) in the presence and absence of reducing agents (DTT and β-ME) or oxidising agent (GSSG), where equal amounts of protein were loaded in adjacent lanes.
Figure 10 shows immunoblot analysis of oleosin (Oleo_0-0, Oleo_1-3, Oleo_3-1, and Oleo_3-3, SEQ ID NOs 11-20) accumulation in the seeds of transgenic *Arabidopsis thaliana* expressing both DGAT1 (S205A) and a sesame oleosin under the control of CaMV35S promoters.
Figure 11 shows immunoblot analysis of oleosin (Oleo_0-0, Oleo_1-3, Oleo_3-1, and Oleo_3-3, SEQ ID NOs 11-20) accumulation in the oil bodies of transgenic *Arabidopsis thaliana* expressing both DGAT1 (S205A) and a sesame oleosin under the control of CaMV35S promoters. The appearance of the oligomeric oleosin bands (dimeric and trimeric) in the presence of oxidising agent (+) indicates the disulfide bonds are able to form on the outside of native oil bodies.
Figure 12 shows immunoblot analysis of oleosin (Oleo_0-0, Oleo_1-3, Oleo_3-1, and Oleo_3-3, SEQ ID NOs 11-20) accumulation in the leaves of transgenic *Arabidopsis thaliana* expressing both DGAT1 (S205A) and a sesame oleosin under the control of CaMV35S promoters.
Figure 13 shows immunoblot of recombinant oleosin accumulation (black arrow) in transgenic Arabidopsis leaves.

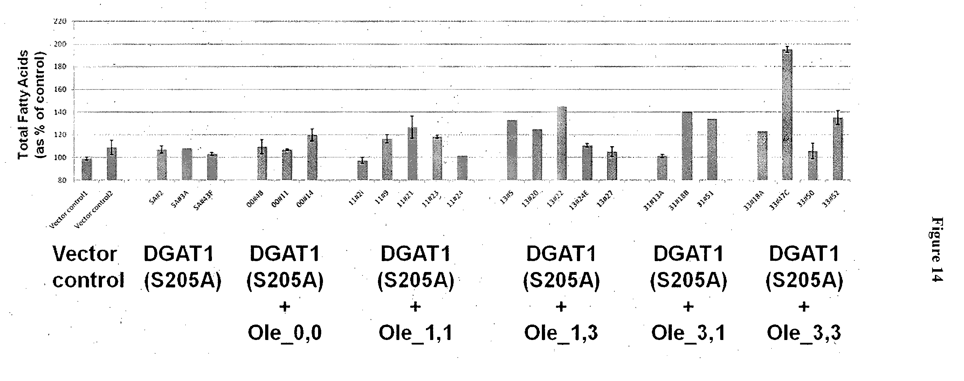

Figure 14 shows FAMES GC/MS results demonstratinging accumulation of additional lipids (black arrows) in Arabidopsis leaves over expressing DGAT1 (S205A) and Ole_3,3.

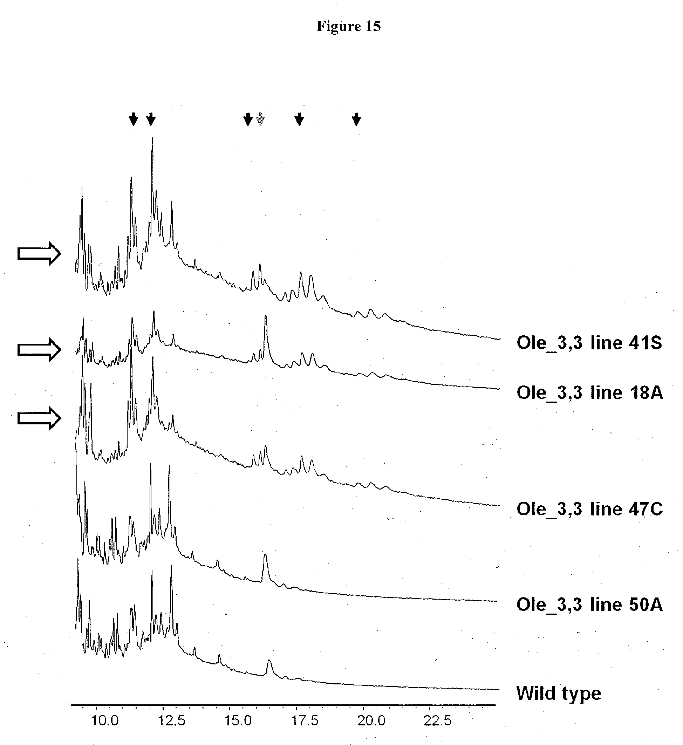

Figure 15 shows GC/MS results for total leaf lipid profile of wild type and independent lines of transgenic Arabidopsis containing DGAT1 (S205A) and Ole_3,3. Grey arrow indicates internal standard. Black arrows indicate additional neutral lipids (wax esters, sterol esters and TAGs. Open arrows show three lines (41 S, 18A and 47C) which accumulate substantial quantities of neutral lipids in their leaves compared to wild type (and line 50A).
Figure 16 shows GC/MS results showing total TAG profile of wild type and transgenic Arabidopsis (containing DGAT1 (S205A) and Ole_3,3) 2, 3, 4 and 5 weeks after germination. Black arrows indicate additional TAGs found in transgenic leaves but not wild type.
Figure 17 shows FAMES GC/MS results showing total leaf lipid profiles of wild type and transgeneic *Trifolium repens* (containing DGAT1 (S205A) and Ole_3,3).
Figure 18 shows FAMES GC/MS results showing C18:1 and C18:2 leaf lipid profiles of wild type and transgeneic *Trifolium repens* (containing DGAT1 (S205A) and Ole_3,3).

### EXAMPLES

This invention will now be illustrated with reference to the following non-limiting examples.

### EXAMPLE 1: Creating rabbit anti-sesame seed oleosin antibodies

### Generating rabbit anti-sesame seed oleosin antibodies

Full length sesame seed oleosin containing a C-terminal His tag (nucleotide sequence is shown in SEQ ID NO: 1) was expressed in *E. coli* and inclusion bodies were prepared by standard techniques. The inclusion bodies were solubilised in Binding Buffer (100 mM phosphate buffer pH 8.0, 500mMNaCl, 8M urea and 10 mM imidazole) and loaded onto a column containing equilibrated ion metal affinity chromatography (IMAC) Ni agarose (Invitrogen). Non-bound proteins were removed from the column by washing with 6 volumes of Wash Buffer (100 mM phosphate buffer pH 8.0, 500mM NaCl, 6M urea and 50 mM imidazole). Protein was eluted in 1 vol. aliquots of Elution Buffer (100 mM phosphate buffer pH 8.0, 500mM NaCl, 6M urea and 250mM imidazole). Eluted fractions were analysed by SDS-PAGE/Coomassie stain and the protein concentration measured using the Bradford's Assay. 265µg of the IMAC-purified recombinant oleosin protein was mixed with an equal amount of Freunds Complete Adjuvant to a final volume of 0.5mL. Following collection of the pre-bleed, the first injection was administered into multiple sites across the back of the neck and shoulder area of a rabbit. Booster shots containing 77µg of the purified oleosin were delivered at three and seven weeks after the primary, and a test bleed of ∼3mL was removed for preliminary analysis at nine weeks. Serum was preserved by the addition of 0.25% v/v phenol and 0.01% v/v merthiolate, and stored in 200µL aliquots at -20°C.

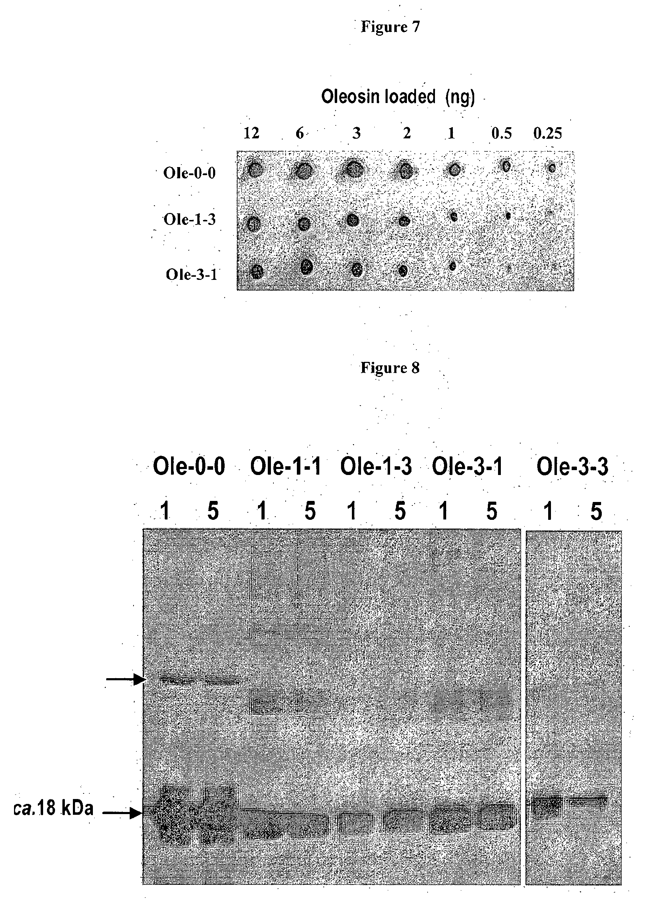

The sensitivity of the rabbit anti-sesame seed oleosin antibodies was evaluated by immunodotting which indicated that 0.25ng of sesame seed oleosin could be regularly detected with a 1/2,000 dilution of the antibody (Figure 7).

### EXAMPLE 2: Design and E. coli expression of modified oleosins containing one or more artificially introduced cysteine residue

### Construct design for expression in E. coli

A number of modified oleosin constructs for expression in *E. coli* were designed. These contained either one or three cysteine residues on the N-terminal and C-terminal hydrophilic arms. The constructs were based on the nucleotide sequence and translated polypeptide sequence from a sesame seed oleosin, GenBank clone AF091840 which contains no cysteine residues (SEQ ID NO: 16).

All clones were subcloned into pET29b using engineered NdeI/XhoI sites. In addition, a ProTrp coding sequence was added to the coding region of the 3' end of the C-terminal hydrophilic arm to mimic the amino acid residues encoded for by the NcoI site previously engineered by Peng et al (2006) Stability enhancement of native and artificial oil bodies by genipin crosslink. Taiwan Patent I250466.

Oleosin-cysteine proteins mutated to include cysteine residues in both the N- and C- terminal hydrophilic regions described here are designated Ole-1-1, Ole-1-3, Ole-3-1, and Ole-3-3 (SEQ ID NO 2, 3, 4, and 5 respectively), where the first and the second numeral digits correspond to the number of disulfide bonds in the N- and C- terminus, respectively. The standard oleosin without the cysteine residues was used as a control and was designated as Ole-0-0 (SEQ ID NO 1).

The cysteines were substituted for charged residues predicted to be on the surface of the oil bodies and are listed below.

| | | | | |
|---|---|---|---|---|
| N-terminal single cysteine | (Ole-1-x) | Glu3Cys | | |
| N-terminal triple cysteine | (Ole-3-x) | Glu3Cys | Arg12Cys | Gln23Cys |
| C-terminal single cysteine | (Ole-x-1) | Gln137cys | | |
| C-terminal triple cysteine | (Ole-x-3) | Gln112Cys | Lys123Cys | Gln137Cys |

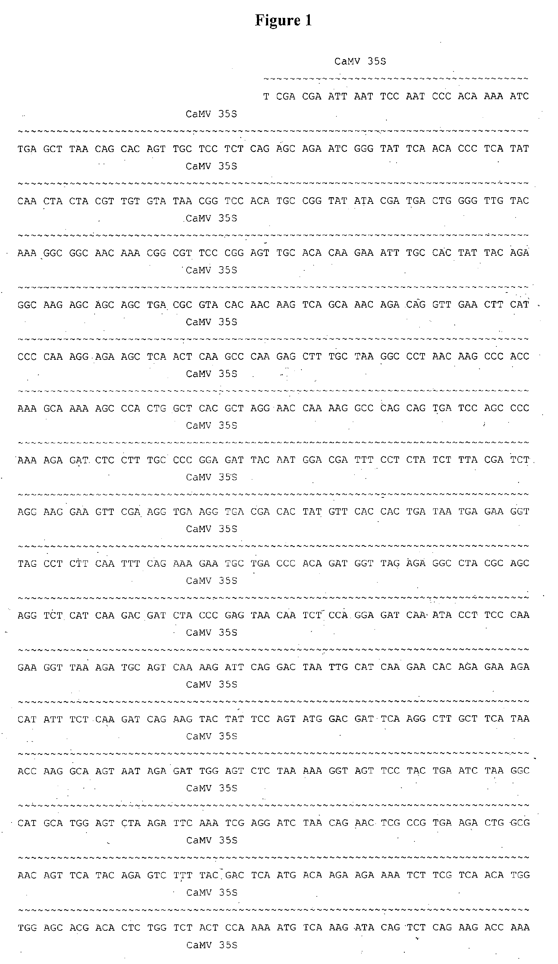

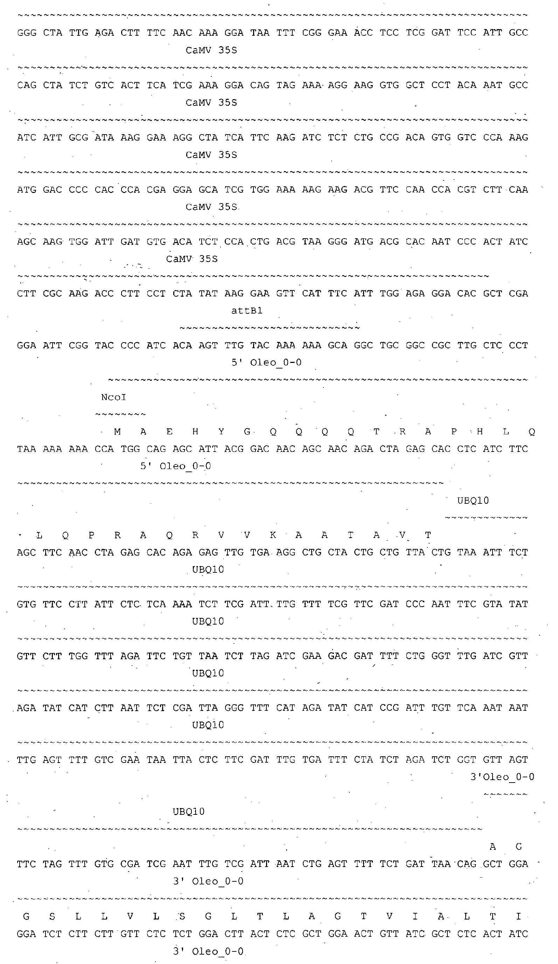

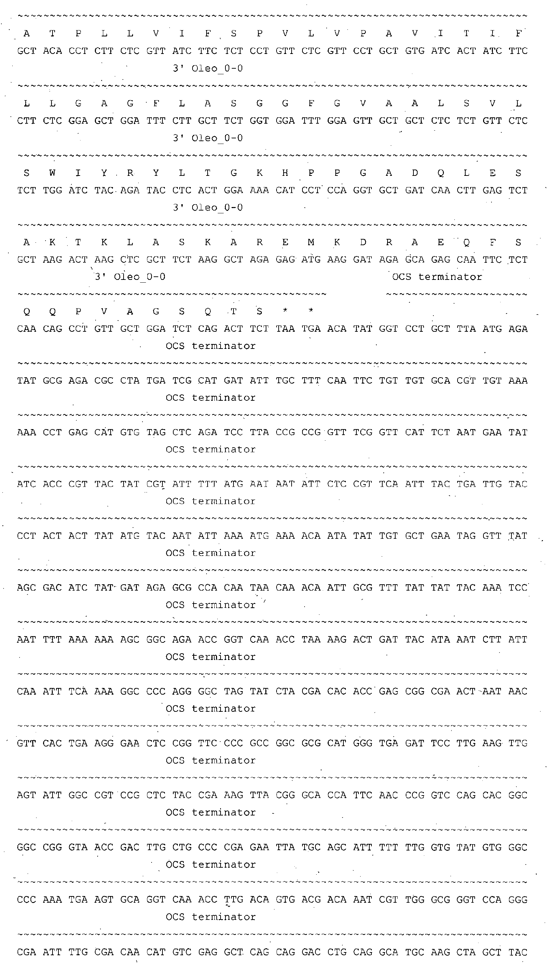

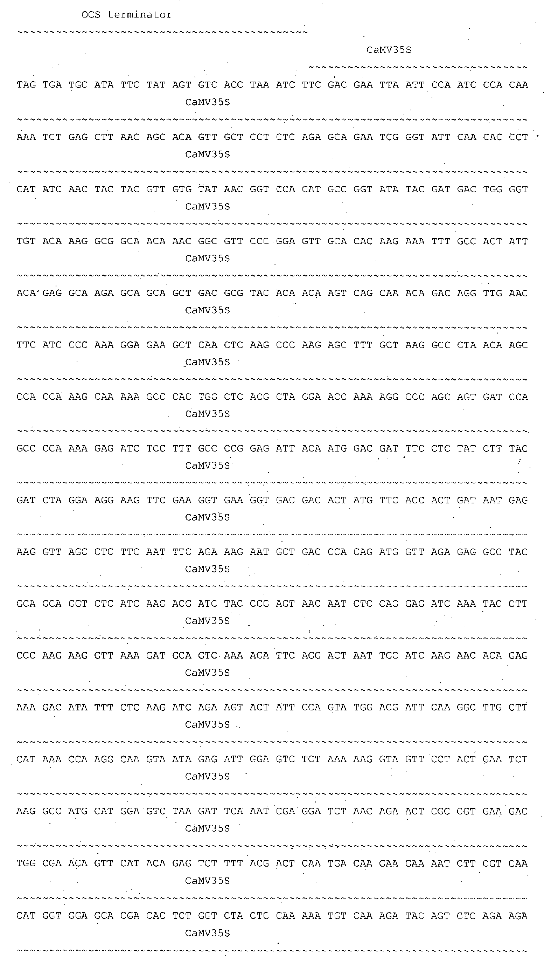

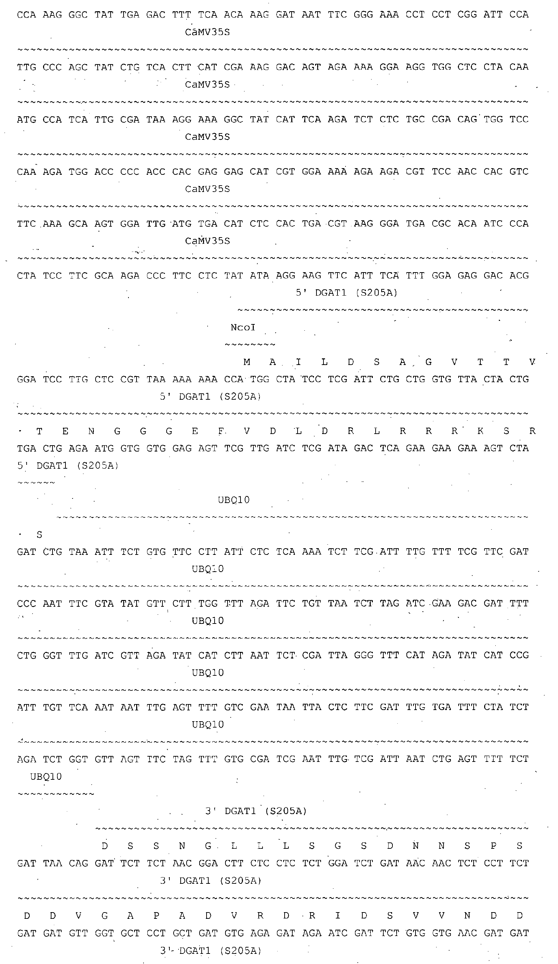

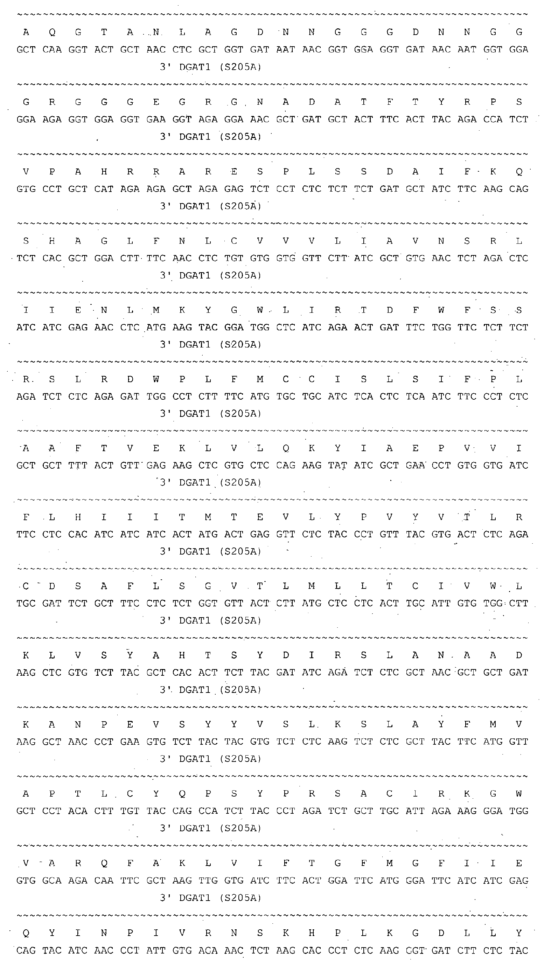

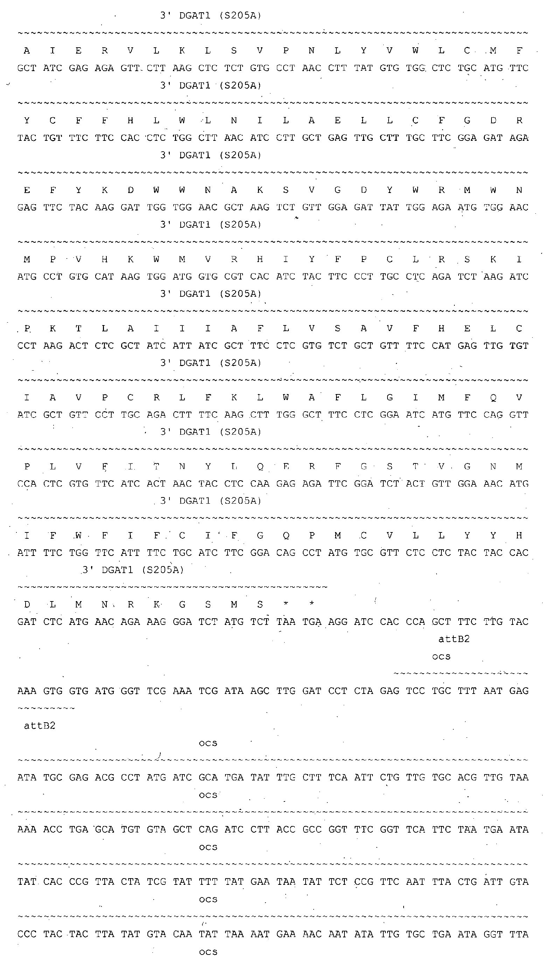

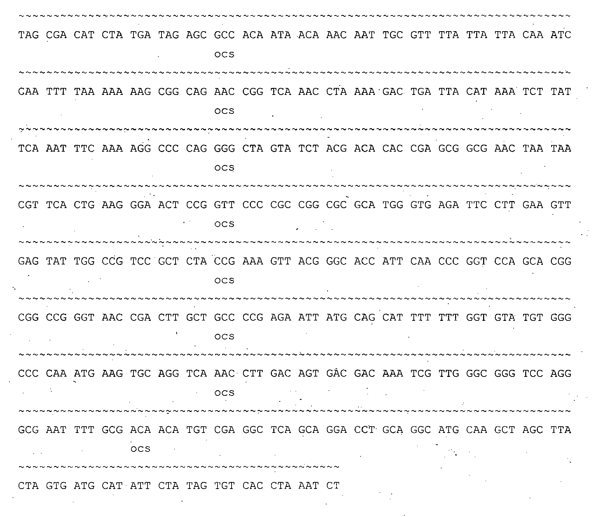

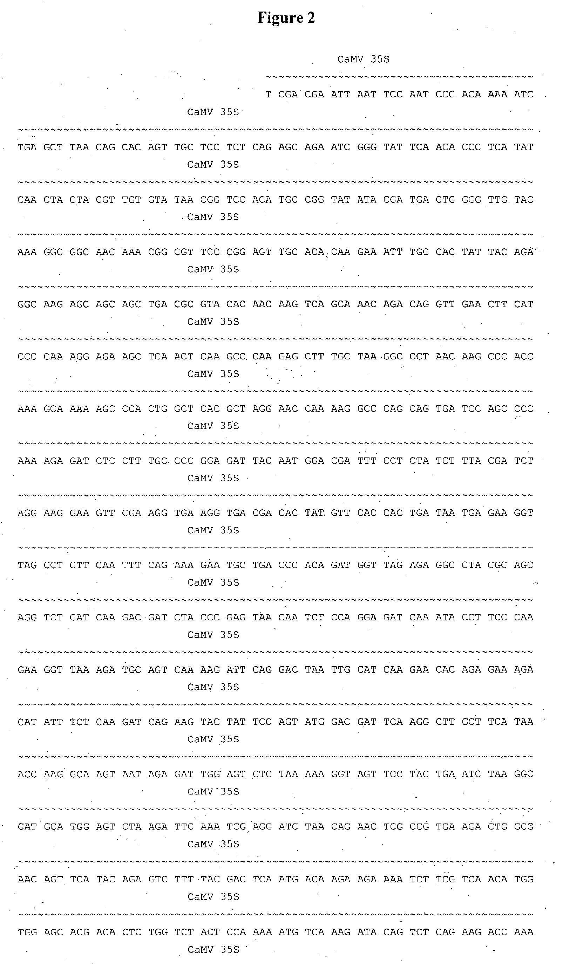

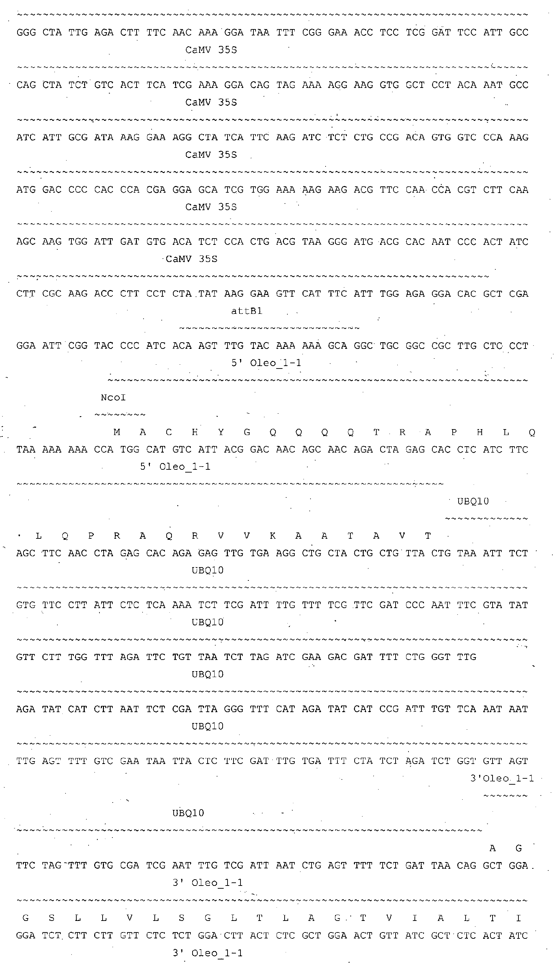

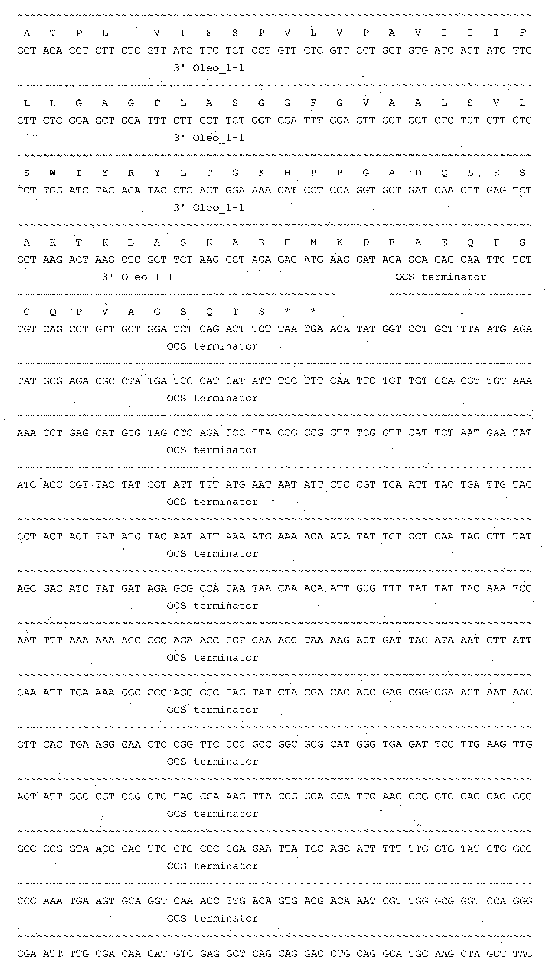

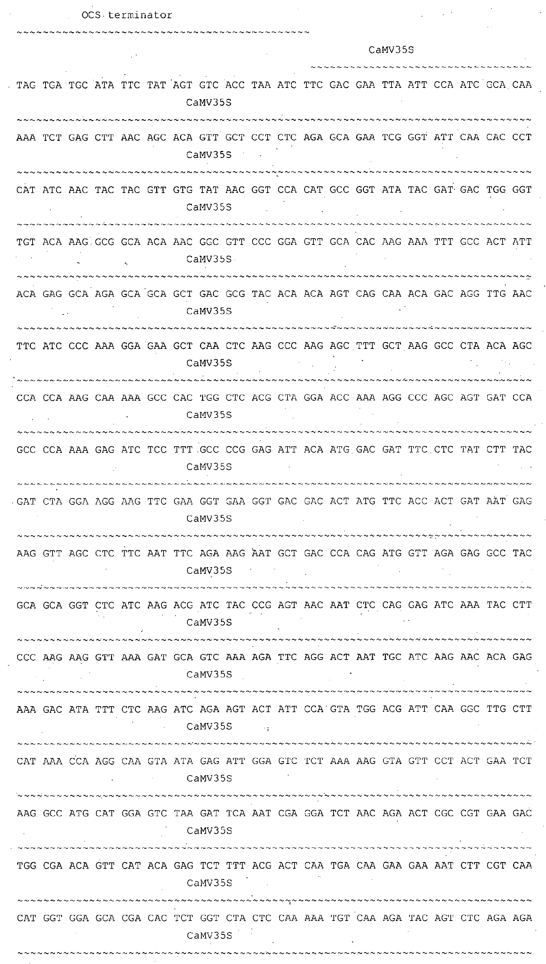

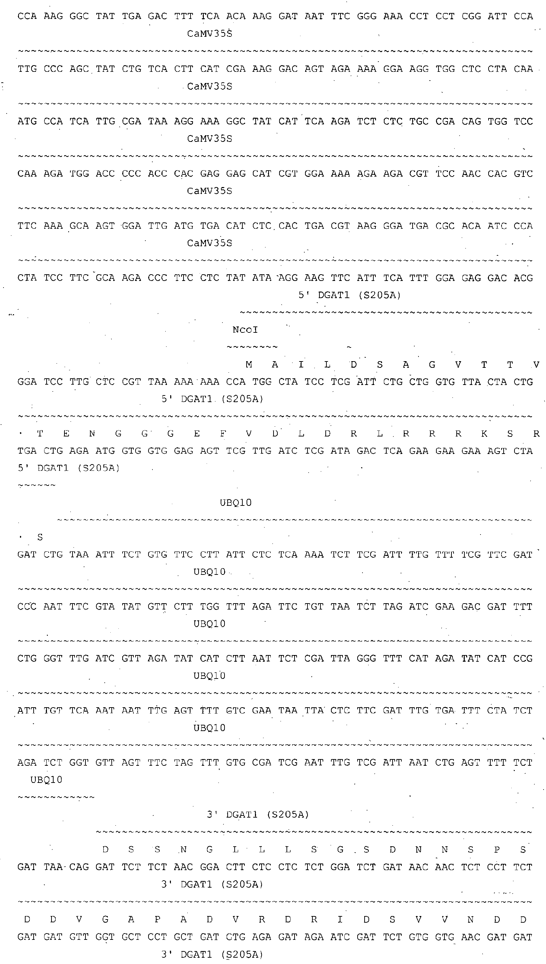

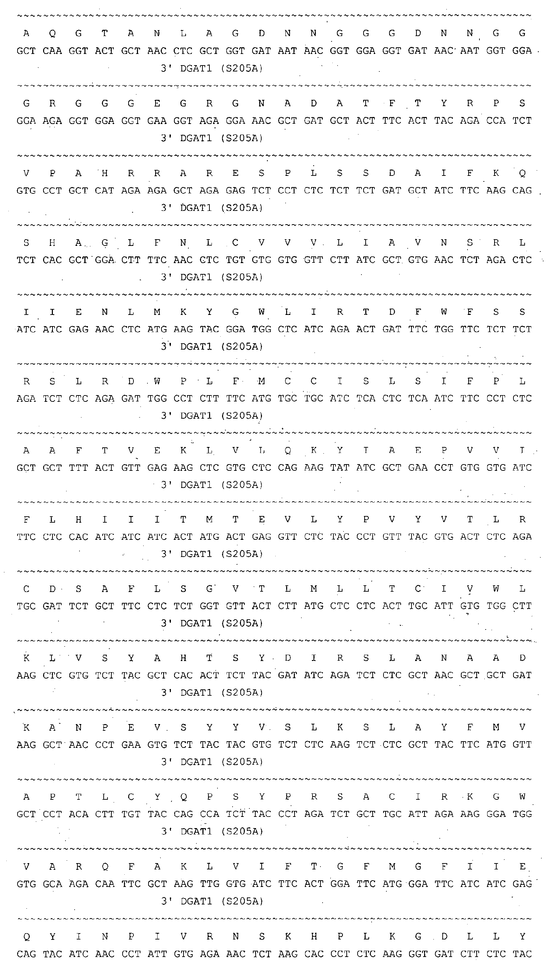

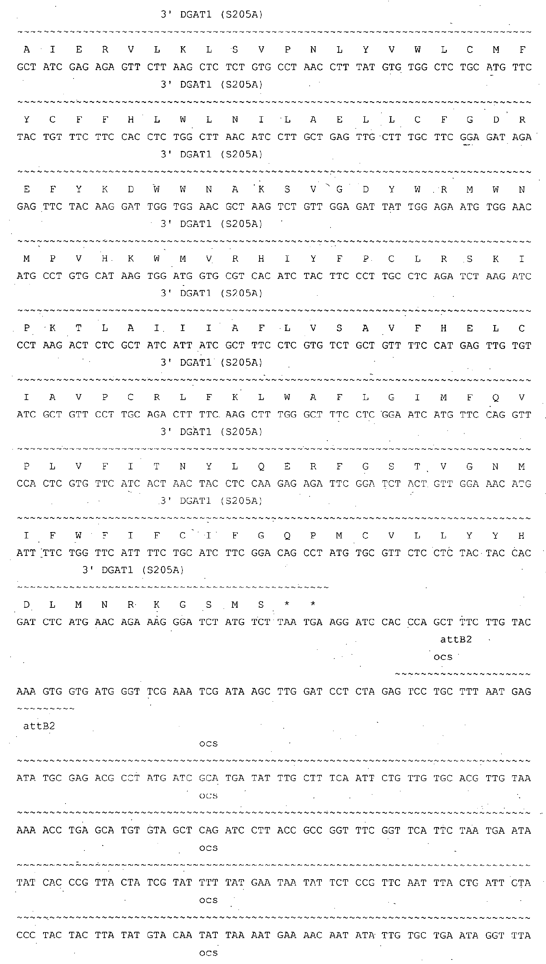

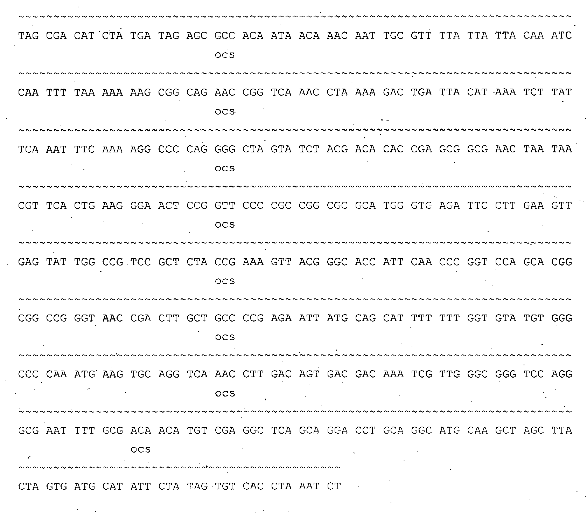

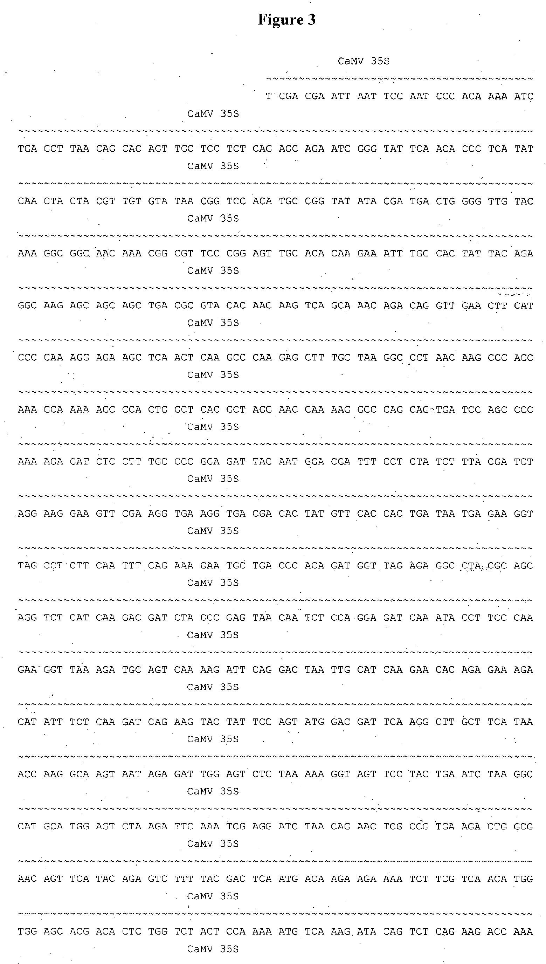

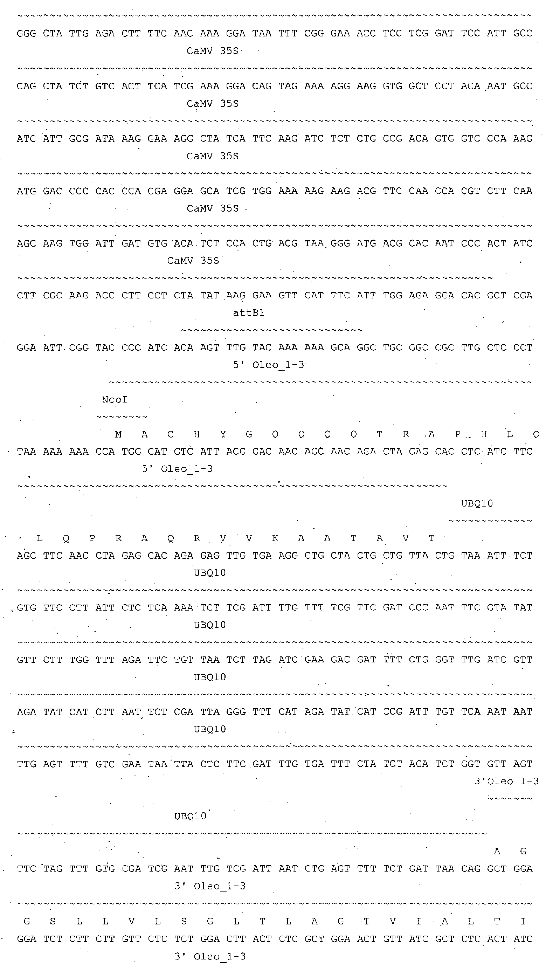

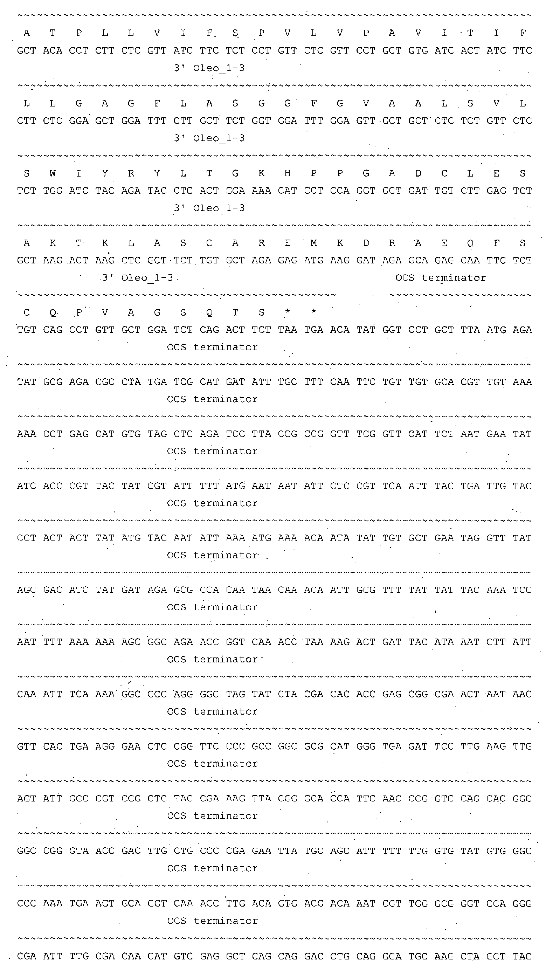

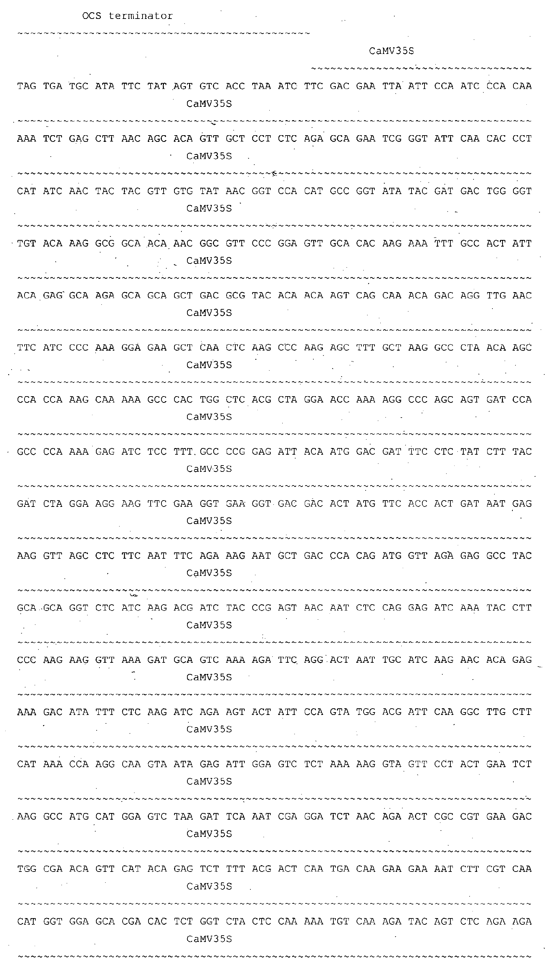

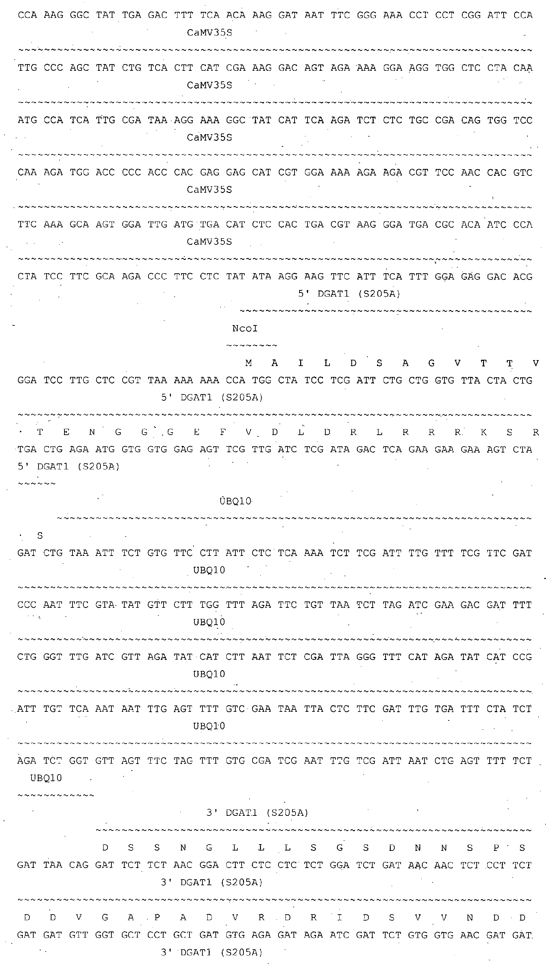

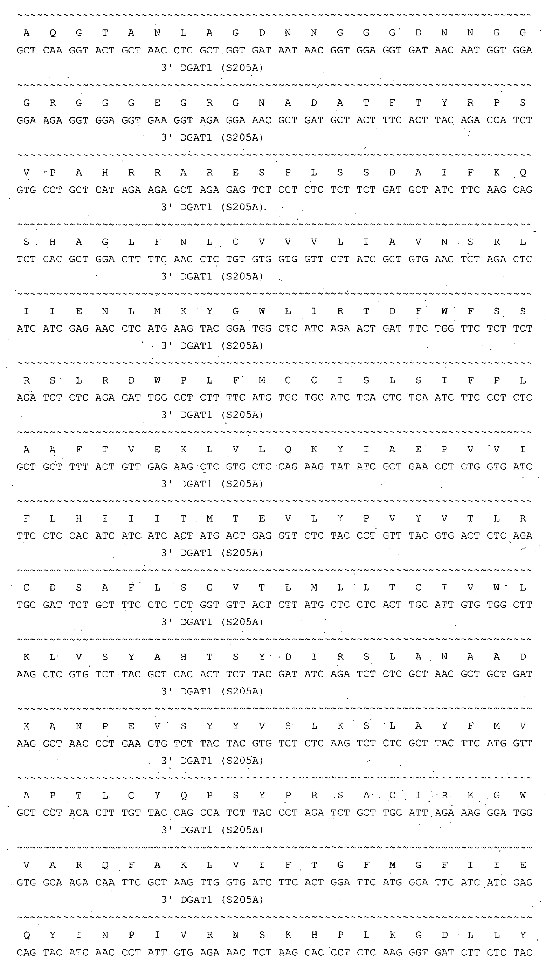

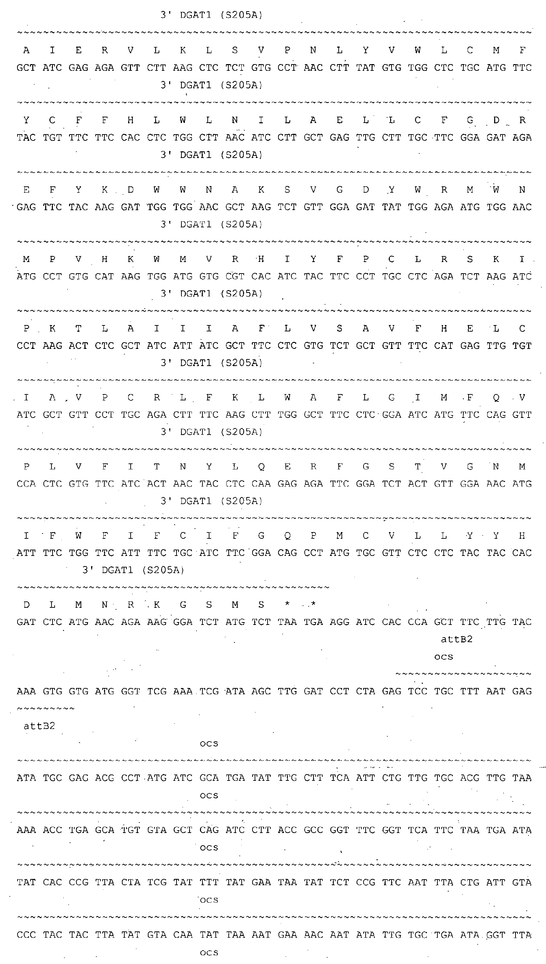

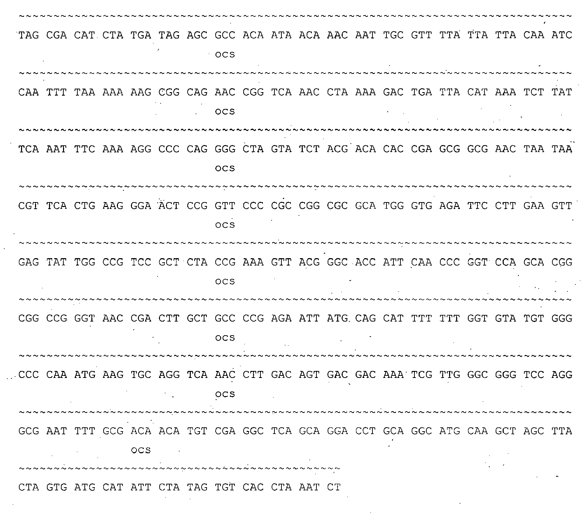

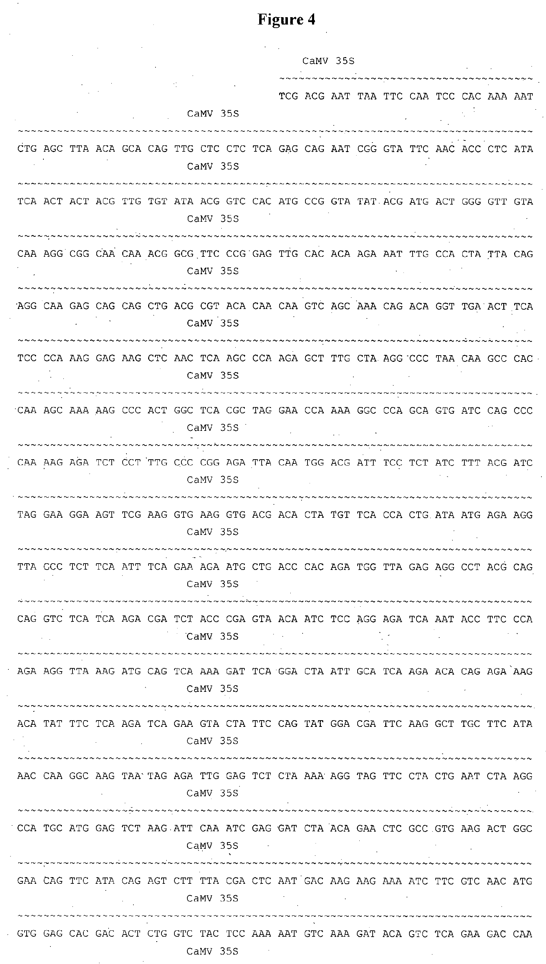

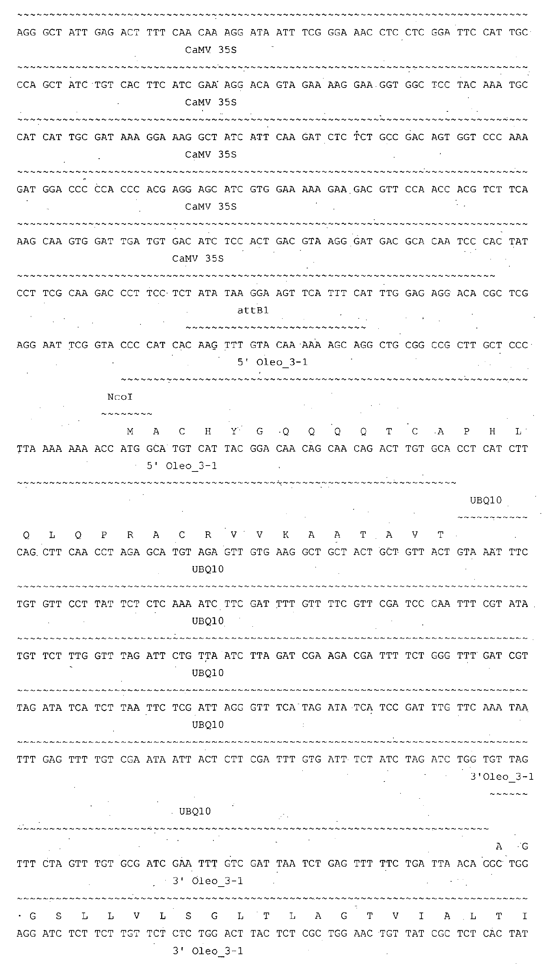

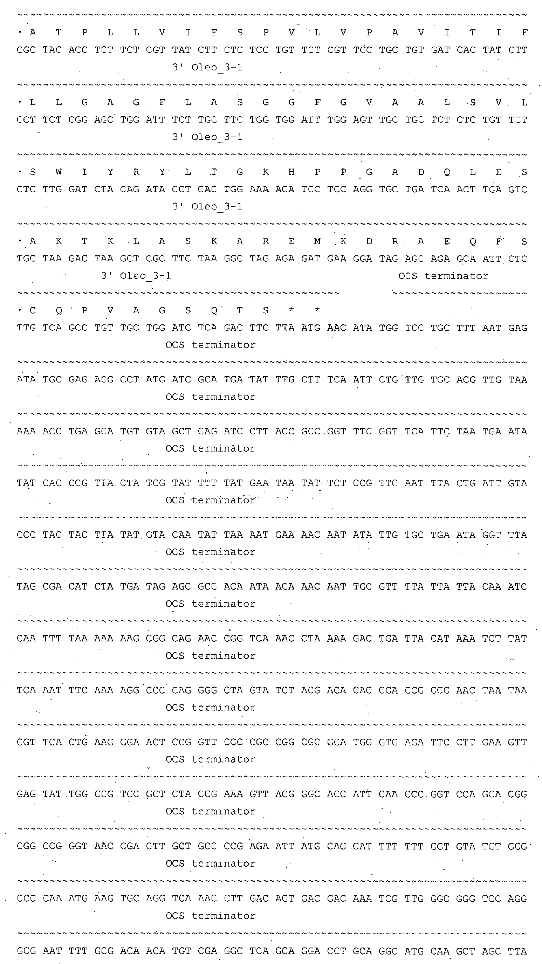

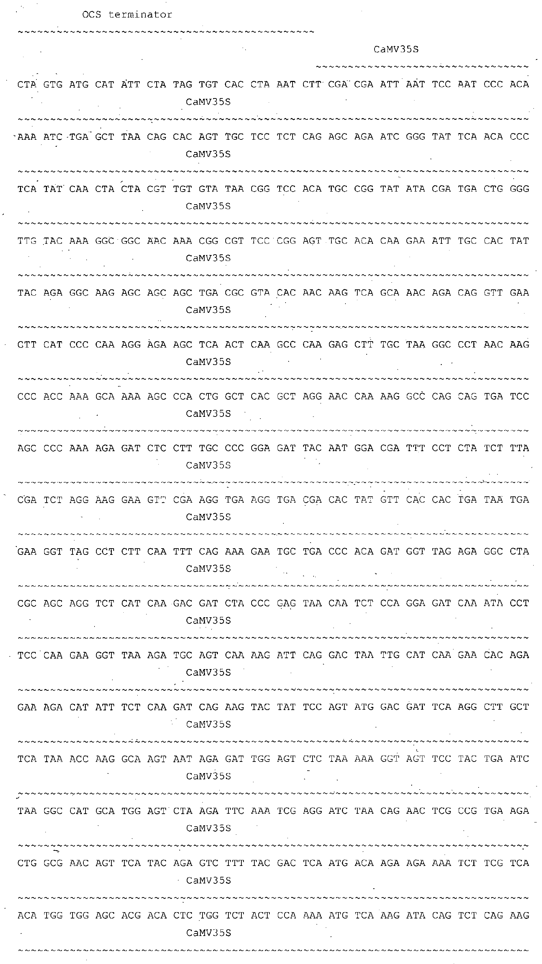

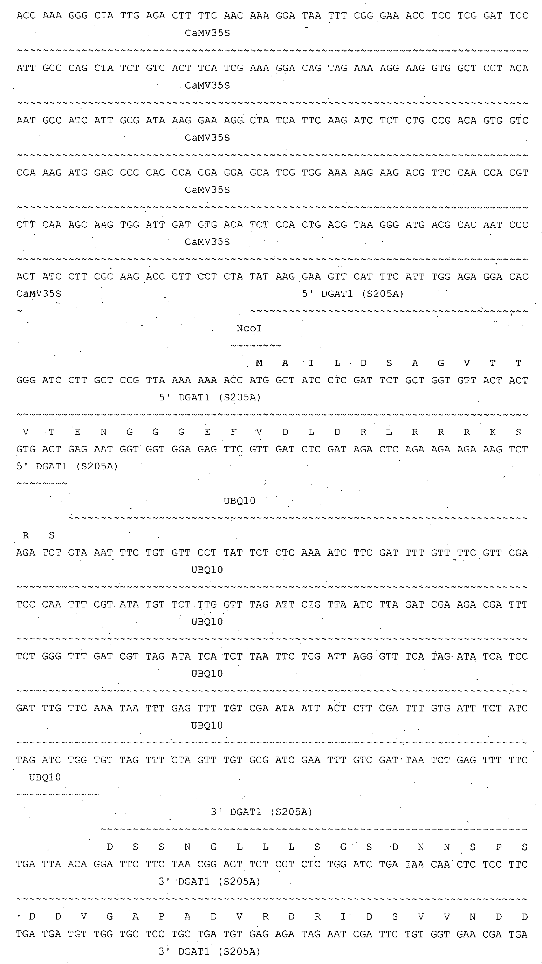

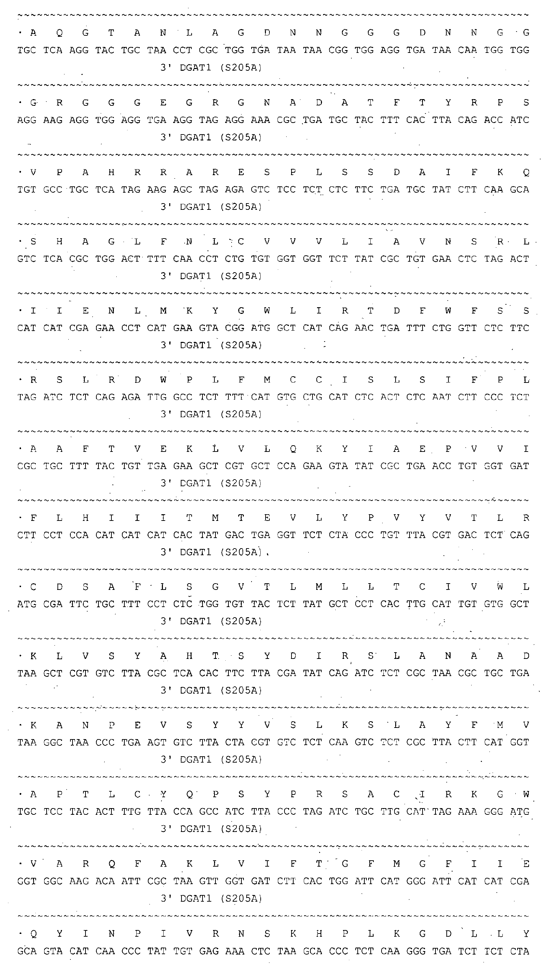

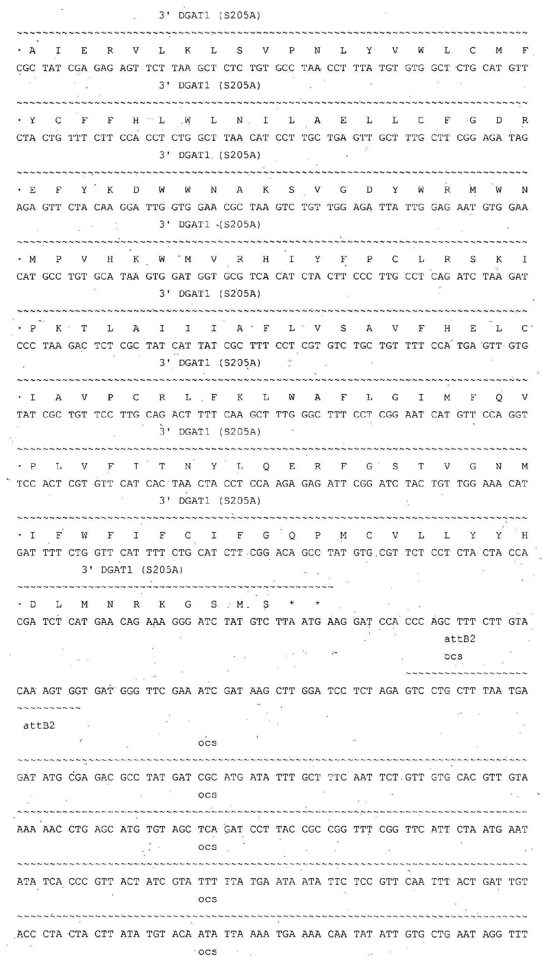

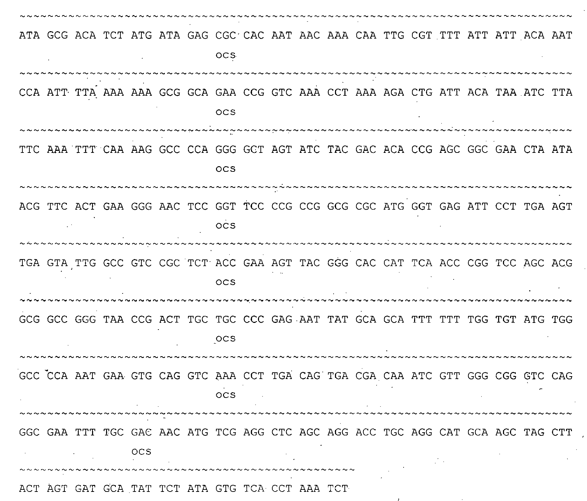

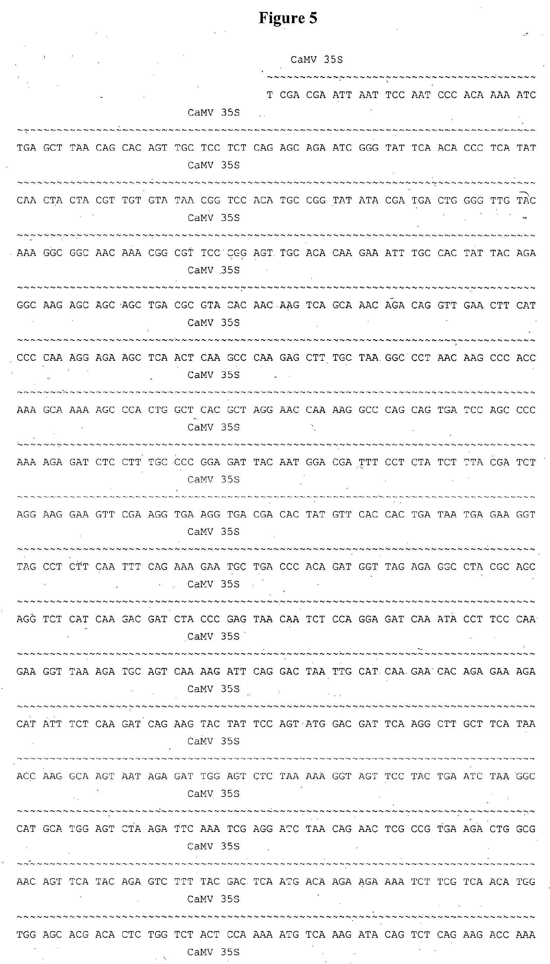

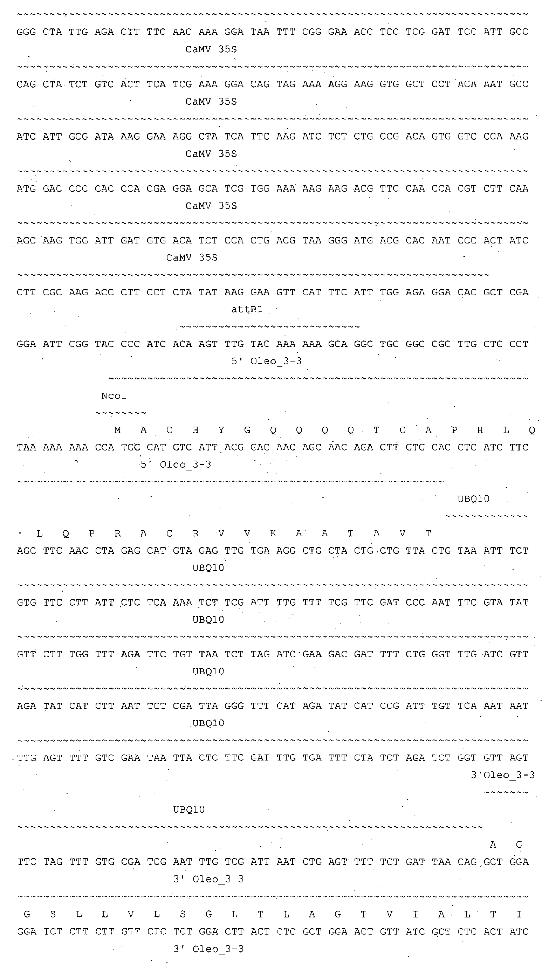

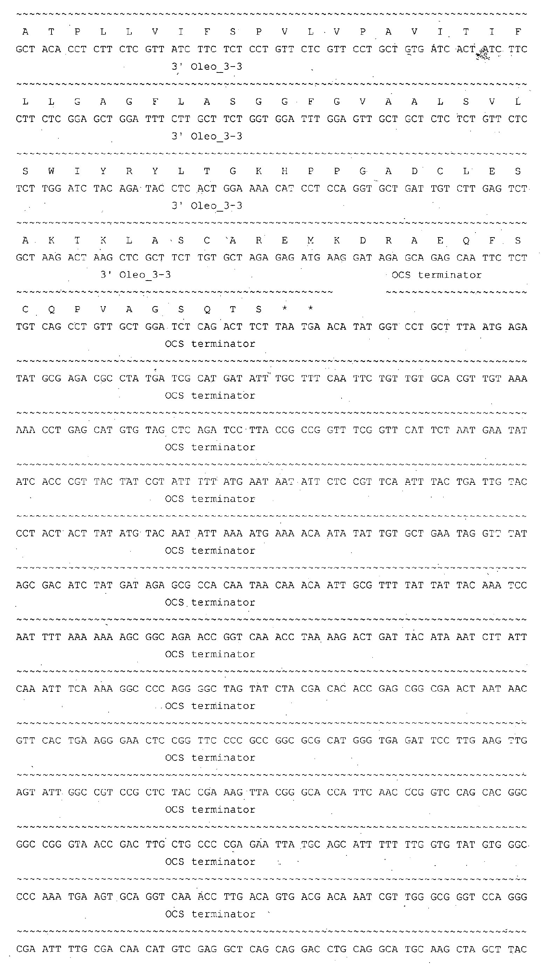

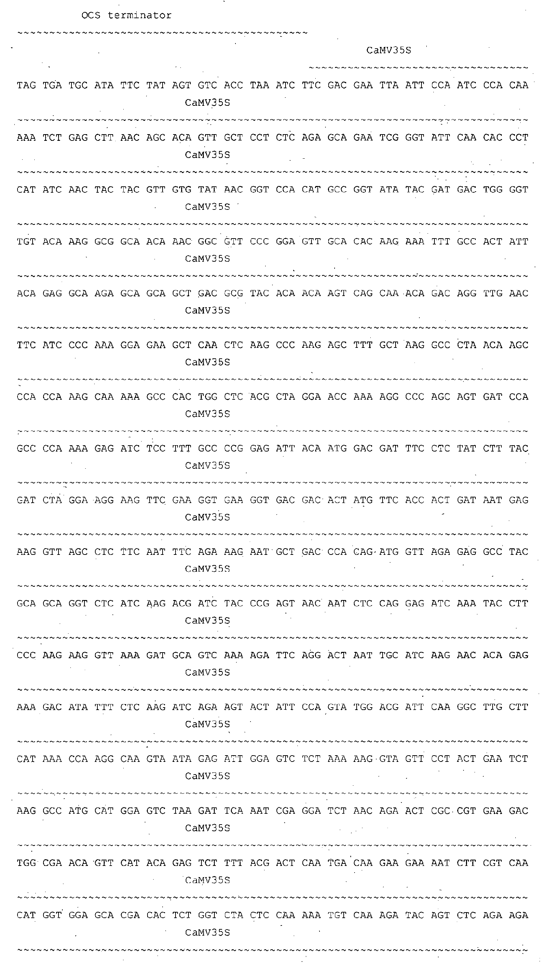

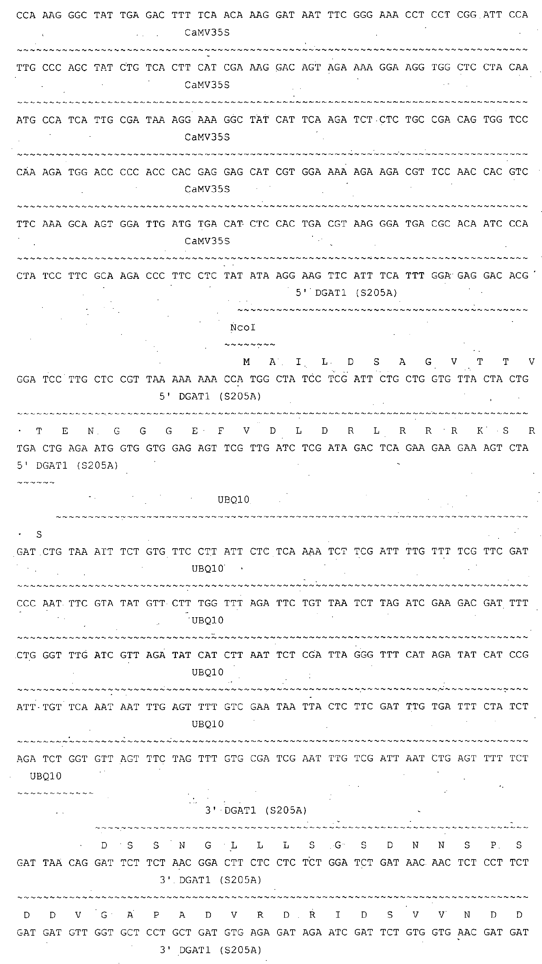

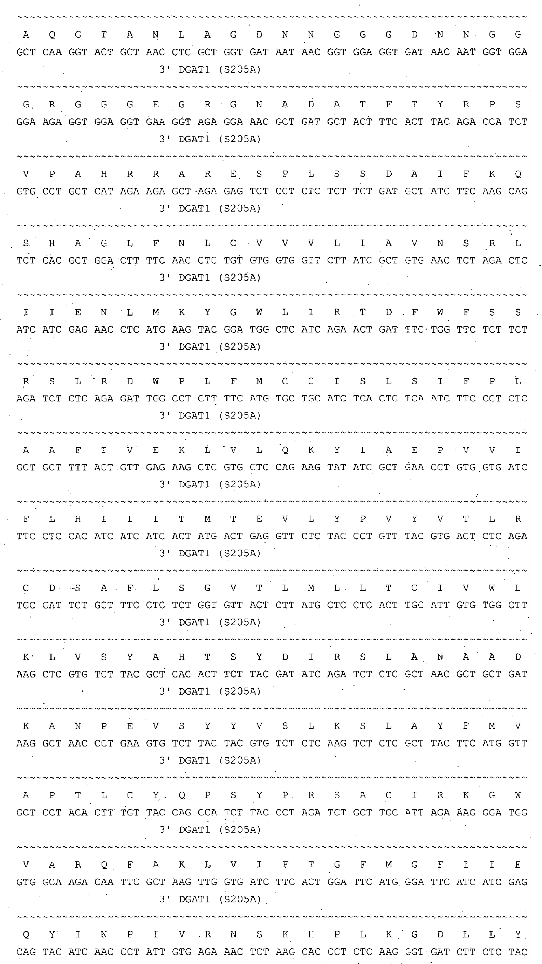

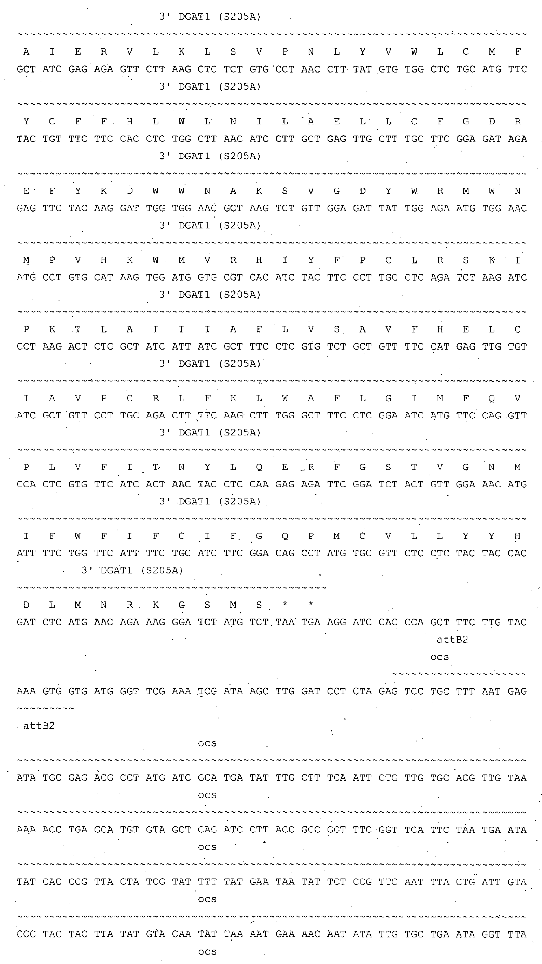

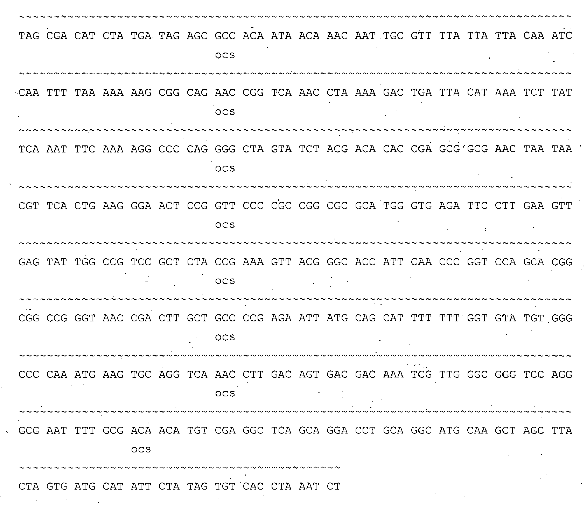

The constructs were designed so could be relatively simply sub cloned from the GENEART provided backbone (pCR4Blunt-TOPO) into pET29b (Novogen) via NcoI/XhoI digestion and ligation. This placed the oleosin coding sequence downstream of the pET29 N-terminal S•tag fusion and upstream of the C-terminal His tag (Figures 1-5 and SEQ ID Nos 1-10). The oleosin and modified oleosin sequences used are summarised in the Summary of Sequences table.

### Expression in E. coli and purification of modified oleosins containing at least one artificially introduced cysteine

Expression of the recombinant sesame seed oleosins (with and without engineered cysteines) in the *E*. *coli* expression system was evaluated by SDS-PAGE/Coomassie brilliant blue staining and SDS-PAGE/immunoblot analysis using antibodies raised against the sesame seed oleosin (described in Example 1).

Expression of recombinant modified oleosin was induced in a freshly inoculated 10mL culture of *E. coli* (BL21 Rosetta-Gami) containing an oleosin (with or without engineered cysteine residues) coding sequence in the pET29 expression vector. The culture was grown at 37°C, 220rpm, until mid log phase (OD₆₀₀0.5 - 0.7); expression was induced by the addition of IPTG to 1 mM final concentration. The induced culture was incubated at 37°C, 220rpm, for a further 2-3 h. Given the properties of modified oleosin the applicants did not attempt to express it in a soluble form but rather chose to extract it from inclusion bodies. Aliquots (1mL) of the culture were transferred to 1.5mL microfuge tubes and the cells pelleted by centrifugation (2655 xg for 5 min at 4°C).

Pelleted cells were resuspended in BugBuster® Reagent (Merck) at 5 mL/g of wet cell pellet, with the addition of DNase to 40 µg/mL and mixed gently on a rotator for 30 min followed by centrifugation at 8000g for 10 min at 4°C. The resultant cell pellet was retreated with BugBuster® and DNase as above. The remaining soluble protein and suspended cell debris was separated from the insoluble inclusion bodies by centrifugation at 8000g for 10 min at 4°C.

Recombinant oleosins were further purified from the inclusion bodies using a procedure adapted from D'Andréa *et al.* (2007). Briefly: the inclusion body preparation was washed by resuspension in 200 mM sodium carbonate buffer pH 11 (5 mL per gram of original cell pellet) and re-pelleted by centrifugation at 8000 xg for 10 min at 4°C. The washed inclusion body pellet was again re-suspended in 5 mL 200 mM sodium carbonate buffer per gram of pellet and added to 9 volumes of freshly prepared chloroform:methanol mix (5:4 v/v) giving a final ratio of 5:4:1 1 (chloroform:methanol:buffer). The suspension was gently mixed for 5 min which formed a milky, single phase mixture; this was centrifuged at 10,000 ×g for 10 min at 4°C, and the supernatant containing the modified oleosin was carefully separated from the pellet and transferred into a new tube. Aliquots of the supernatant were dried down under a stream of nitrogen and the protein re-solubilised in 8M urea and quantified by Qubit™ (Invitrogen).

### EXAMPLE 3: Use of anti-sesame seed oleosin antibodies to bind sesame seed oleosin with artificially introduced cysteines

A dot-blot was used to compare the ability of the anti-sesame seed oleosin antibodies (Abs) described in Example 1 to bind to oleosin without cysteines versus the oleosins containing cysteines (described in Example 2). Dilution series from 12 to 0.25ng of purified Ole-0-0, Ole-1-3 and Ole-3-1 were spotted onto a pre-equilibrated Hybond-P PVDF Transfer membrane. This was incubated with the anti-sesame seed oleosin antibodies at 1:2000 as the primary Ab. The blot was then incubated with the appropriate secondary Ab and developed by chemiluminescence (Figure 7). The results indicate that on an immunoblot, the anti-sesame seed oleosin antibodies are up to an order of magnitude more sensitive to the oleosin without cysteine residues than the oleosins with cysteine residues. As a consequence of the different sensitivities it was necessary to load different quantities of recombinant protein onto the gels for analysis by immunoblotting. Despite the non uniform lane loading it is still possible to compare different oleosins between lanes in terms of their relative distribution between monomeric and oligomeric forms.

### EXAMPLE 4: Creation of artificial oil bodies with E. coli expressed modified oleosins containing at least one artificially introduced cysteine and altering the degree of cross linking

### Preparation of artificial oil bodies

Artificial oil bodies (AOBs) were then prepared by drying down aliquots of the supernatant described in Example 3, calculated to contain either 150µg or 1mg of recombinant oleosin.

The process of generating AOBs involved combining PL, TAG, and the recombinant oleosin/modified oleosin. In the absence of strong chaotropic agents the disruptive force required to dissociate individual recombinant oleosins from the purified fraction involved several alternating cycles of sonicating and cooling. This was achieved by solubilising the 150µg and 1mg oleosin/modified oleosin samples in 20µL chloroform containing 150µg PL (Sigma, Cat#P3644) and mixed with 60µL of purified sesame seed oil (Tzen and Huang 1992) and 940µL of AOB buffer (50mM sodium phosphate buffer pH 8.0, 100mM NaCl). The complete mixture was then sonicated three times for 30sec (Sonics & Materials Vibra∼Cell VC600, 600 W, 20 kHz; ⅛" tapered micro-tip probe, power setting #3).

The applicants also found that the purification procedure could be successfully scaled up and when a 50g cell pellet was used as the starting material it was necessary to substitute the stream of nitrogen with a rotary vacuum evaporator to remove the chloroform and the majority of the methanol. At this point the majority of oleosin/modified oleosin precipitated out of the azeotropic solvent and was separated by centrifugation at 12,000g for 10min.

Inclusion bodies were suspended in 1mL AOB Buffer II (50 mM sodium phosphate, pH 8.0, 100 mM NaCl, 20 mM β-mercaptoethanol, 10 mM DTT and 5% [v/v] sesame oil) and then sonicated 4x. AOBs were concentrated by centrifugation at 12,000 rpm for 10min, this resulted in the formation of a suspension of AOBs overlaying the aqueous fraction. The underlying aqueous fraction was removed by pipette, and the remaining AOBs were washed (to remove soluble proteins and reducing agents) by gentle agitation in 1mL AOB Buffer III (50 mM sodium phosphate, pH 8.0, 100 mM NaCl). After washing, the AOBs were re-concentrated by centrifugation, and the underlying aqueous fraction removed, then re-suspended by vortexing in AOB Buffer IV (50 mM sodium phosphate buffer, pH 8.0, 100 mM NaCl, 1 mM GSSG) and the AOBs stored at 4°C for further analyses.

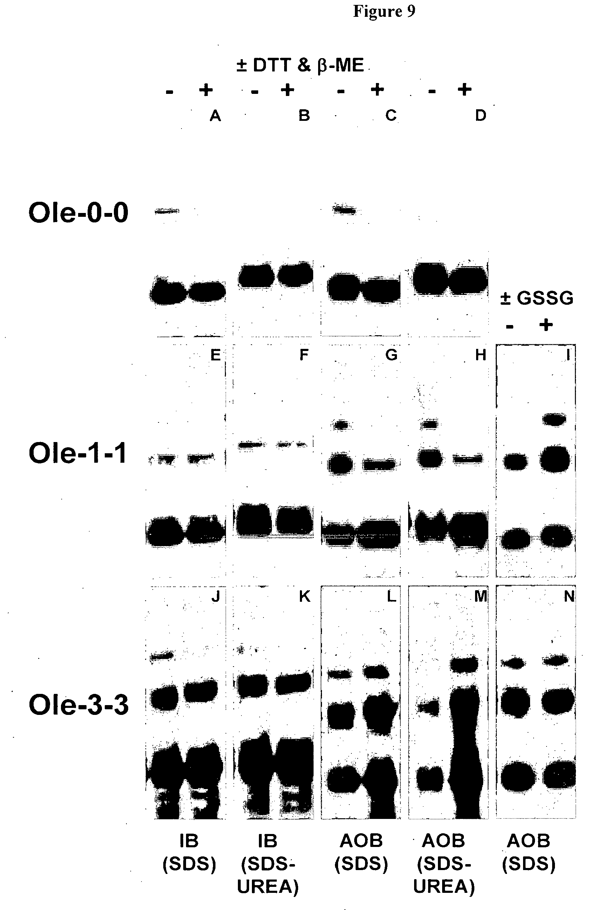

Recombinant Ole-0-0, and all variations of the oleosin-cysteines were successfully expressed and located in *E. coli* inclusion bodies (Figure 9). Ole-0-0 was predominantly present as a monomer (in both inclusion bodies as well as AOBs); this migrated fractionally faster than the 20kDa molecular weight marker (in reducing and non reducing SDS and SDS-UREA PAGE). Also present were two slower migrating immunoreactive bands of approximately 35 and 36 kDa which likely correspond to two forms of dimeric oleosin. While Ole-0-0 is not predicted to contain any cysteine residues the overall intensity and ratio of the two apparent dimers was influenced by the presence of reducing agents β-ME @ 5% of the sample loading buffer and 10mM DTT).

In the inclusion bodies, the predominant form of Ole-1-1 is monomeric. Only one dimeric form appeared to be present and this was not influenced by reducing agents or urea. Ole-1-1 from AOBs (generated in the presence of reducing agent and then in the presence of oxidising agent) showed a large increase in the ratio of dimer to monomer as well as the formation of trimeric, tetrameric and likely pentameric oligomers (the electrophoretic focus of these oligomers was considerably improved in the SDS-UREA gel). Removal of the GSSG and re-introduction of reducing agents to the AOBs resulted in the presence of only monomer and dimer in similar proportions seen in the inclusion bodies. AOBs generated with Ole-1-1 (in the absence of both reducing agents and GSSG ) showed the presence of almost equal portions of monomer and dimer and a small amount of trimer, indicating that the conditions under which the AOBs are formed have some reducing potential. The subsequent addition of GSSG resulted in an increase in the oligomeric portions as well as the appearance of a tetrameric form.

While the monomer was the predominant form of Ole-3-3 in the inclusion bodies, a comparatively high percentage was also present in multiple oligomeric forms. The proportion of oligomers declined to a small extent with the addition of reducing agent and slightly more by the addition of both reducing and chaotropic agents. Oligomeric forms of Ole-3-3 that were larger than a trimer were poorly resolved when the recombinant protein was extracted from AOBs. The creation of large oligomeric forms was promoted by the addition of GSSG and in the absence of reducing and chaotropic agents a portion of these oligomeric forms failed to enter the stacking gel. Combined, these results indicate that on the AOBs, Ole-3-3 was highly cross-linked and the position of the cross-links was more variable compared to the Ole-3-3 recovered from the inclusion bodies. This suggests that, despite considerable pre-existing cross-linking (within the inclusion bodies), on the AOB Ole-3-3 has access to a high number of potential partners for cross-linking. Similarly for Ole-1-3 and Ole-3-1, the number of cross-linked species increased when there was more than one cysteine on one or both hydrophilic regions (Figures 8 and 9).

It could be anticipated that in non-reducing SDS-PAGE, oligomers containing the same number of oleosins but with the disulfide bonds in different positions would migrate differently to each other. Indeed this can be seen in Figure 8 where the data indicates that the position of the oleosin arms, relative to one another are at different positions over the oil body. For example the Ole-1-1 can only form one disulfide bond per arm and this has to form at the same position, where as the presence of three cysteines enables more than one disulfide bond to form but it also allows the disulfide bonds to form with different degrees of hydrophilic arm overlap as well as having multiple oleosins bound to the same arm (Figures 8 and 9).

The addition of SDS and reducing agents (DTT and β-ME) decreased the number of oligomeric complexes (Figure 9). The addition of SDS and urea results in a similar pattern to SDS alone except that the previously resolved multiple dimeric forms migrated as one and the trimeric and tetrameric forms appear to be in higher abundance presumably because they are also migrating as single bands which increases intensity correspondingly (Figure 9). In contrast, the presence of SDS, reducing agent and urea resulted in fewer oligomeric forms of Ole-1-1 and Ole-1-3 but not Ole-3-1 or Ole-3-3 (Figure 9). In the case of Ole-3-1 and Ole-3-3 it appears that the urea does not completely denature the disulfides oleosins and may indeed prevent the complete reduction of the disulfide bonds. It could be that these bonds are formed during the generation of inclusion bodies (would need to see reduced and non reduced inclusion body preps). Furthermore, the presence of the dimeric oleosin formed in the absence of engineered cysteine residues (Figures 8 and 9) indicates that some oligomerisation is due to other types of attraction, e.g, strong hydrophobic bonding that is not fully disrupted by SDS but can be almost completely disrupted by the combination of both SDS and urea (Figure 8 and 9).

The effect of increasing the number of potential cross-linking sites in an oleosin peptide on AOB integrity and emulsion stability can be assessed as follows.

### Quantitative Determination ofAOB integrity

Assessment of AOB stability and integrity using either absorbance (OD₆₀₀), direct counting of AOBs using a hemocytometer, or visual evaluation of coalescence by microscopy proved to be highly variable and amongst other things was influenced by the: degree of pre-sampling agitation; quantity of sample removed; time left under the microscope. To avoid this the applicants devised a simple method to quantify the amount of TAG released from the AOBs into the surrounding media during a variety of treatments as a means of comparing integrity. Essentially equal quantities (based on FAMES-GC/MS estimation of TAG and Bradfords determination of protein) of AOB preparations are made up to a total volume of 200µL using AOB buffer (containing Proteinase K [PNK] when appropriate at a 1:1 ratio of PNK:total proteins in OB or AOB samples in a 250µL GC glass insert tubes and covered with a plastic cap. Following the treatment (elevated temperature or exposure to PNK) 15µL of fish oil (Vitamax®, Australia) is added to the sample and mixed by vortexing followed by centrifugation at 5,200g for 1min. The addition of fish oil followed by vortexing enables any TAG that had leaked from the AOBs to mix with the added fish oil and be floated by brief centrifugation. 4µL of the oil phase is sampled and subjected to fatty acid methyl esterification (FAME) and then analysed by GC-MS (Shimadzu model numbers, fitted with a 50mQC2/BPX70-0.25 GC capillary column (SGE) as described by Browse *et al.* (1986). In the absence of added fish oil the quantity of TAG that had leaked from the AOBs was too small to form a samplable visible layer even after centrifugation, in such a case the maximum volume would have been 6µL. The very different lipid profiles of fish oil and sesame oil enabled us to easily distinguish the leaked TAG from the added TAG.

Using the internal C15:0 and C17:0 standards the applicants can calculate the absolute amounts of C18:2 (the major lipid in sesame seed oil) recovered after treatment.

### Determination of AOB integrity and emulsion stability at elevated temperature

Oil in water emulsions are less stable at elevated temperatures; hence, it is of interest to investigate if modified oleosins with varying numbers in introduced cysteines influence AOB integrity at elevated temperature. To achieve this the applicants determine the integrity (using the method described above) of OBs and AOBs (containing different oleosins) in a phosphate buffer (50mM Na-phosphate buffer pH8, 10mM NaCl) at 95°C. AOBs are heated for 2h. Integrity is determined as above.

The effect of higher ratios of crosslinked oleosin:TAG on the stability of AOBs in rumen fluid can be assessed as follows.

### Determination ofAOB integrity in rumen fluid

One of the aims of disulfide was to provide some degree of protection from biohydrogenation by rumen microflora. Assessment of AOB stability with rumen fluid can be assessed as follows. AOBs are added to an equal volume (25µL) of rumen fluid. Samples are incubated at 39°C for 0, 15, 30, 60, 120 and 240min, at the end of the incubation an equal volume of loading buffer (Invitrogen) is added, mixed and heated at 70°C for 10min. 15µL of each sample/loading buffer mix is compared by SDS-PAGE/immunoblot. Integrity is determined as above.

### Analysis of AOB integrity in Proteinase K

To investigate the influence of modified oleosin in a controllable and repeatable highly degradative environment integrity is determined (using the method described above) of AOBs (containing different modified oleosins) after incubation in an phosphate buffer (50mM Na-phosphate buffer pH8, 10mM NaCl) containing 1:1 (g/g protein) Proteinase K (Invitrogen) at 37°C for 4h. While the maximum activity of Proteinase K is achieved below 65°C the lower temperature is used in order to reduce the influence of temperature on AOB instability. Integrity is determined as above.

### EXAMPLE 5: Design and in planta expression of modied oleosin containing one or more artificially introduced cysteines

### Construct design for expression in planta

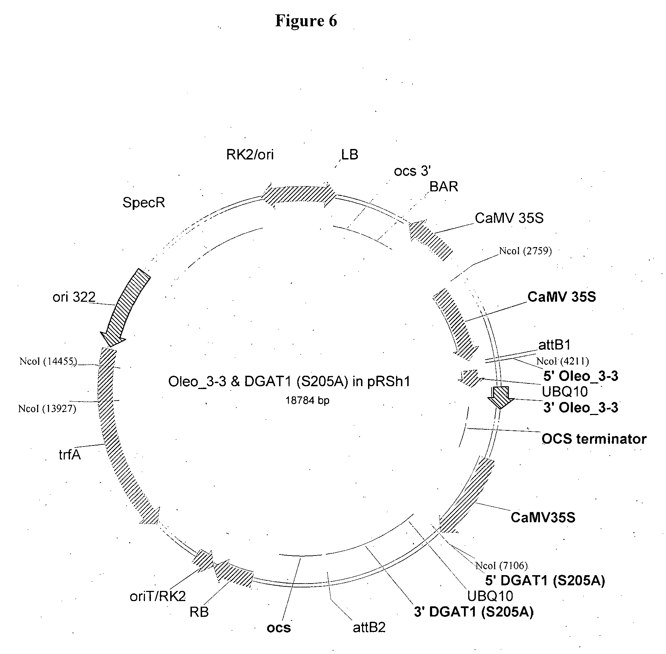

The applicants synthesised individual coding sequences for the sesame seed oleosin (based on GenBank clone AF091840) with different numbers of cysteines in the N- and C-terminal arms. The coding sequence was flanked by a 5' NotI site and a 3' NdeI site. A separate acceptor cassette was synthesised containing an attL1 site, a NotI site and NdeI site followed by a nos termination sequence, a forward facing CaMV35s promoter, the *Arabidopsis thaliana* DGAT1 (S205A) (SEQ ID NOs 11-20 and Figures 1-5) plus its own UBQ10 intron, an attL2 site. The sesame seed oleosins with different numbers of cysteines were individually transferred to the acceptor cassette via the NotI and NdeI sites. Each of these completed cassettes were then transferred to a plant binary vector pRSh1, Figure 6 (Winichayakul et al., 2008) via the LR recombination reaction. This placed the oleosin downstream of a CaMV35S promoter (already contained within pRSh1) and placed a nos terminator (already contained within pRSh1) downstream of the Arabidopsis DGAT1 (S205A) (Figures 1-5). The nucleotide sequences encoding the sesame seed oleosins (with cysteines) and DGAT1 were optimised for expression in *Arabidopsis thaliana,* this included optimisation of codon frequency, GC content, removal of cryptic splice sites, removal of mRNA instability sequences, removal of potential polyadenylation recognition sites, and addition of tetranucleotide stop codon (Brown et al, 1990; Beelman and Parker, 1995; Rose, 2004; Rose and Beliakoff, 2000; Norris et al., 1993).

It should be noted that the oleosin sequence used is for example only. Any oleosin or steroleosin or caoleosin sequences could be engineered to contain cross-linking regions. The coding sequences of the complete ORFs (after splicing) were checked against repeat of the original oleosin translated sequence and found to be identical over the oleosin coding regions.

### Transformation of Arabidopsis thaliana with sesame seed oleosins containing cysteines

Transformation of *Arabidopsis thaliana* var Columbia (with constructs described above), analyses of T2 seeds for modified oleosin, immunoblot analysis of *Arabidopsis thaliana* oil bodies containing sesame seed oleosin with different numbers of cysteines was performed as described previously (Scott et al., 2007).

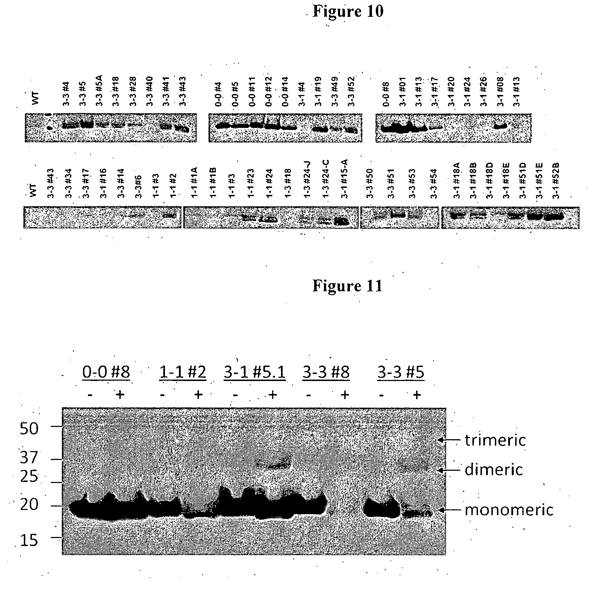

Both the floral-dip (Clough, 1998) and floral-drop methods (Martinez-Trujillo, 2004) were used in the transformation of Arabidopsis by *Agrobacterium tumefaciens* GV3101 containing the binary constructs. T1 seed was collected from the treated plants, germinated and selected by spraying at 14 d and 21 d post-germination with Basta®. Basta® resistant T1 plants (71, 62 and 23 transformants containing the single sesame seed oleosin, and modified oldeosin constructs respectively) were transplanted, allowed to self-fertilise, set seed and the T2 seed was collected. Equal quantities of seed extract from Basta® resistant Arabidopsis plants were analysed by SDS-PAGE/immunoblot with the anti-sesame seed oleosin antibodies; recombinant sesame seed oleosin and modified oldeosin of the appropriate size was observed in the majority of samples (Figure 10). Southern blot analysis was performed on selected T2 lines to determine the number of insertion sites.

### EXAMPLE 6: Extraction and purificiation oil bodies with modified oleosins containing at least one artificially introduced cysteine from the seeds of Arabidopsis thaliana

### Crude Oil Body Preparations from Arabidopsis thaliana seeds

Crude OB preparations were prepared, from seed of plants produced as described in Example 5, by either grinding 200mg seed with a mortar and pestle containing a spatula tip of sand and 750µL Extraction Buffer (10mM phosphate buffer, pH 7.5 containing 600mM sucrose) or by homogenising 25mg of seed in 300µL Extraction Buffer using a Wiggenhauser D-130 Homogenizer. A further 750µL Extraction Buffer was added and the slurry in the mortar and transferred to a 2mL microfuge tube whereas the homogenizer tip was rinsed in 1mL Extraction Buffer and this volume was added to the homogenised seed. Samples were then centrifuged at 20, 000 ×*g* for 5min; this left a pellet and aqueous supernatant which was overlaid by an immiscible oily layer containing both intact and disrupted oil bodies as well as free TAG. The upper oil layer was gently pushed to the side of the tube, and the aqueous layer and pelleted material discarded. The oil layer was then re-suspended from the side of the tube by vortexing in Extraction Buffer and placed in a fresh 2mL microfuge tube. The final volume was made up to 0.5mL with Extraction Buffer.

### Purified Oil Body preparations from Arabidopsis thaliana seeds and cross linking cysteine residues between the engineered oleosins

25 mg of *Arabidopsis* seed (of plants transformed as described in Example 5) was ground in 300 µl extraction buffer (10 mM Phosphate buffer, pH 7.5 containing 600 mM sucrose) using a Wiggenhauser D-130 Homogenizer. Seed was ground until crushed and the sample appeared "creamy" and frothy as starch was released from the seeds. The homogenizer tip was rinsed in 1 ml buffer and this volume was added to the crushed seed. Samples were prepared up to this point in lots of 4, then centrifuged 14,000rpm for 5 mins. A thin gel loading tip was used to gently push the oil layer to the side of the tube, and the aqueous layer removed to a fresh tube. The oil layer was resuspended from the side of the tube using extraction buffer and placed in a fresh 2 ml tube. The final volume was made up to 0.5 ml (as read on the side of the tube) with extraction buffer, samples were divided into two and oxidising agent (3mM GSSG) was added to one tube and incubated at room temperature for 10 min. Oil body preparations were then added to an equal volume of 2 x gel loading buffer and boiled for 5min before loading on to a gel.

Samples were run either on pre-cast NuPAGE Novex 4-12% Bis-Tris Midi Gels(Invitrogen) on a Criterion gel rig system (Bio-Rad), or NuPAGE® Novex 12% Bis-Tris gradient Gel 1.0 mm, 15 well, cat# NP0343BOX, with NuPAGE® MES SDS Running Buffer (for Bis-Tris Gels only) (20X), cat# NP0002-02, or on hand-cast Tris-HCl gels. Gels were stained by SafeStain (Invitrogen) to show total protein loaded or blotted onto Nitrocellulose membrane using the iBlot system (Invitrogen). In each case, the negative control was a sample extracted from wild type Columbia seed and the positive control was the same extraction method (although grinding was by mortar and pestle) performed on wild type sesame seed. 10µl of each sample and the negative control were loaded onto the gel, and 5µl was used for the positive control.

Following blotting, the membrane was blocked in a solution of 12.5% skim milk powder in TBST (50 mM Tris pH 7.4, 100 mM NaCl, 0.2 % Tween) for at least 1.5 hours. The membrane was then washed in TBST 3 x 5 mins before incubating with primary antibody (anti-sesame) at 1/1000 in TBST for 1 hour at room temperature. Following 3 further TBST washes, incubation with secondary antibody (anti-rabbit) at 1/5000 was carried out for 1 hour at room temperature. The membrane underwent 3 further washes then the signal was developed using standard chemiluminesence protocol.

Figure 11 shows the accumulation of sesame seed oleosin units on the oil bodies under the control of the CaMV35S promoter. It can be seen that recombinant oleosin and polyoleosin was found to accumulate in the seeds of *Arabidopsis thaliana* and was correctly targeted to the oil bodies (Figure 11). In addition, it can be seen that in the presence of oxidising agent for 10 minutes the recombinant oleosins containing cysteines formed cross-links as evidenced by the appearance of oligomers and corresponding disappearance of the monomeric forms in these samples and not in the wild type or non oxidised transgenic oil bodies.

The effect of increasing the number of potential cross-linking sites in an oleosin peptide on *in planta* OB integrity and emulsion stability can be assessed as follows.

### Quantitative Determination of OB integrity

Assessment of OB stability and integrity using either absorbance (OD₆₀₀), direct counting of AOBs using a hemocytometer, or visual evaluation of coalescence by microscopy proved to be highly variable and amongst other things was influenced by the: degree of pre-sampling agitation; quantity of sample removed; time left under the microscope. To avoid this the applicants devised a simple method to quantify the amount of TAG released from the OBs into the surrounding media during a variety of treatments as a means of comparing integrity. Essentially equal quantities (based on FAMES-GC/MS estimation of TAG and Bradfords determination of protein) of OB preparations are made up to a total volume of 200µL using AOB buffer (containing Proteinase K [PNK] when appropriate at a 1:1 ratio of PNK:total proteins in OB samples in a 250µL GC glass insert tubes and covered with a plastic cap. Following the treatment (elevated temperature or exposure to PNK) 15µL of fish oil (Vitamax®, Australia) is added to the sample and mixed by vortexing followed by centrifugation at 5,200g for 1min. The addition of fish oil followed by vortexing enables any TAG that had leaked from the OBs to mix with the added fish oil and be floated by brief centrifugation. 4µL of the oil phase is sampled and subjected to fatty acid methyl esterification (FAME) and then analysed by GC-MS (Shimadzu model numbers, fitted with a 50mQC2/BPX70-0.25 GC capillary column (SGE) as described by Browse *et al.* (1986). In the absence of added fish oil the quantity of TAG that had leaked from the OBs was too small to form a samplable visible layer even after centrifugation, in such a case the maximum volume would have been 6µL. The very different lipid profiles of fish oil and sesame oil enabled us to easily distinguish the leaked TAG from the added TAG.

Using the internal C15:0 and C17:0 standards the applicants can calculate the absolute amounts of C 18:2 (the major lipid in sesame seed oil) recovered after treatment.

### Determination of OB integrity and emulsion stability at elevated temperature

Oil in water emulsions are less stable at elevated temperatures; hence, it is of interest to investigate if modified oleosins with varying numbers in introduced cysteines influence OB and AOB integrity at elevated temperature. To achieve this the applicants determine the integrity (using the method described above) of OBs (containing different oleosins) in an phosphate buffer (50mM Na-phosphate buffer pH8, 100m NaCl) at 95°C. AOBs are heated for 2h. Integrity is determined as above.

The effect of higher ratios of crosslinked oleosin:TAG increase the stability of OBs in rumen fluid can be assessed as follows:

### Determination of OB integrity in rumen fluid.

One of the aims of disulfide was to provide some degree of protection from biohydrogenation by rumen microflora. Assessment of OB stability with rumen fluid can be assessed as follows. OBs are added to an equal volume (25µL) of rumen fluid. Samples are incubated at 39°C for 0, 15, 30, 60, 120 and 240min, at the end of the incubation an equal volume of loading buffer (Invitrogen) is added, mixed and heated at 70°C for 10min. 15µL of each sample/loading buffer mix is compared by SDS-PAGE/immunoblot. Integrity is determined as above.

### Analysis of OB integrity in Proteinase K

To investigate the influence of modified oleosin in a controllable and repeatable highly degradative environment integrity is determined (using the method described above) of AOBs (containing different modified oleosins) after incubation in an phosphate buffer (50mM Na-phosphate buffer pH8, 100ms NaCl) containing 1:1 (g/g protein) Proteinase K (Invitrogen) at 37°C for 4h. While the maximum activity of Proteinase K is achieved below 65°C the lower temperature is used in order to reduce the influence of temperature on OB instability. Integrity is determined as above.

### EXAMPLE 7: Production of oil bodies in the leaves of Arabidopsis thaliana

In order to produce oil bodies in vegetative tissue, it is necessary to produce triacyclglycerol in such tissue (e.g. leaves).

### Production of triacylglycerol in the vegetative portions of the plant

In most plants (including *Lolium perenne*) the majority of leaf lipids are attached to a glycerol backbone and exist as diacylglycerols. These are incorporated into lipid bi-layers where they function as membranes of multiple sub-cellular organelles or the as the membrane of the cell itself. The majority of lipid bilayer in the leaf is the chloroplast thylakoid membrane. A smaller amount of leaf lipid exists as epicuticular waxes and an even smaller percentage is present in the form of triacylglycerol (TAG).

Most plants synthesise and store TAG in developing embryos and pollen cells where it is subsequently utilised to provide catabolizable energy during germination and pollen tube growth. Dicotyledonous plants can accumulate up to approximately 60% of their seed weight as TAG. Ordinarily, this level is considerably lower in the monocotyledonous seeds where the main form of energy storage is carbohydrates (e.g., starch)The only committed step in TAG biosynthesis is the last one, i.e., the addition of a third fatty acid to an existing diacylglycerol, thus generating TAG. In plants this step is performed by one of three enzymes including: acyl CoA:diacylglycerol acyltransferase (DGAT1), an unrelated acyl CoA:diacylglycerol acyl transferase (DGAT2), and phospholipid:diacylglycerol acyltransferase (Zou *et al.,* 1999; Bouvier-Navé *et al.,* 2000; Dahlqvist et al., 2000; Lardizabal et al., 2001). Over expression of the transcribed region of any of these genes in the vegetative portions of plants leads to the formation of TAG droplets in the cytoplasm of leaf cells, as demonstrated by the over expression of: Arabidopsis DGAT1 in tobacco by Bouvier-Navé et al., (2000); Tung tree DGAT2 in yeast and tobacco by Shockey et al., (2006); Arabidopsis PDAT in Arabidopsis by Stahl et al., (2004). Over expression of Arabidopsis DGAT1 in some cases was demonstrated to increase the total lipid level but not necessarily by the accumulation of TAG, e.g. in *Lotus japonicus* hairy roots (Bryan et al., 2004) and in *Lolium perenne* leaves (Cookson et al., 2009).

To demonstrate the accumulation of TAG in the leaves of these plants you can compare the total quantity of lipid extract from leaves of these plants with those of untransformed plants or plants transformed with the empty binary vector. Ensuring the plants are grown under the same environmental conditions and that the leaves sampled are physiologically equivalent. With the appropriate internal standards the quantification of the total lipid extract can be achieved using FAMES GC-MS analysis (as described by Winichayakul et al, 2008 Delivery of grasses with high levels of unsaturated, protected fatty acids. Proceedings of the New Zealand Grassland Association, 70:211-216.). Alternatively, the total lipids can be extracted using the Folsch method (Folsch et al., 1957 J. Folsch, M. Lees and G.A. Slone-Stanley, A simple method for the determination of total lipid extraction and purification, Journal of Biological Chemistry 226 (1957), pp. 497-507.) and quantified using appropriate internal standards with a GC-MS fitted with a Restek (Restek Corp., Bellefonte, PA) RTX65TG column.

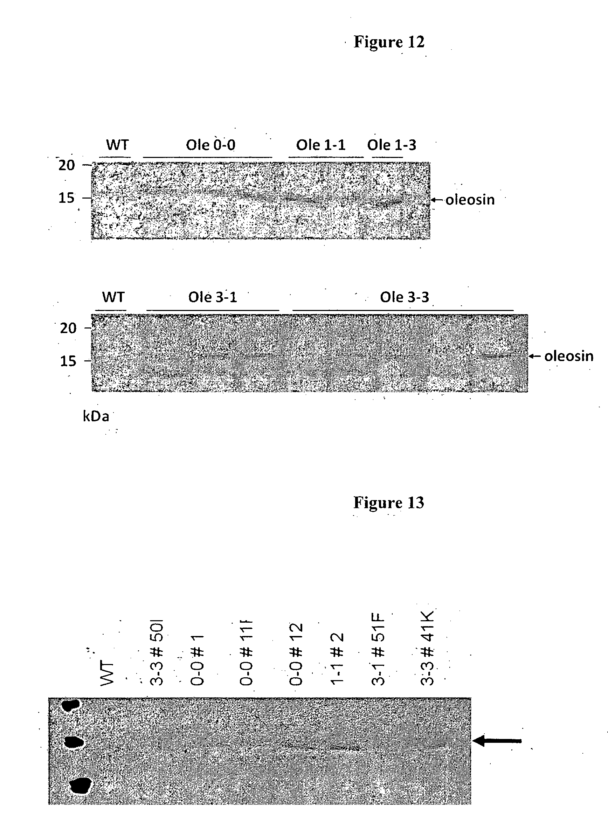

Leaves were sampled from plants over expressing the *A. thaliana* DGAT1 (S205A) and the sesame seed oleosin construct (either Oleo_0-0, or Oleo_1-1, or Oleo_1-3, or Oleo_3-1, or Oleo_3-3, SEQ ID NOs 11-20, Figures 1-5) and analysed by SDS-PAGE/immunoblot using the polyclonal anti-sesame seed oleosin antisera. It can be seen that recombinant oleosin was found to accumulate in the leaves of *Arabidopsis thaliana* leaves (Figure 12).

The simultaneous expression and accumulation of oleosin/modified oleosin protein in the same cell (for example leaf cell) will result in the production of triglyceride oil bodies encapsulated by a phospholipid monolayer embedded with oleosin; this has been demonstrated with un-modified oleosin in yeast (Ting et al., 1997) and seeds (Abell et al., 2004).

### Oil body preparations from the leaves of transgenic Arabidopsis thaliana

Oil bodies can be extracted from the leaves of transgenic Arabidopsis thaliana expressing DGAT1 (S205A) and the sesame seed oleosin construct (either Oleo_0-0, or Oleo_1-1, or Oleo_1-3, or Oleo_3-1, or Oleo_3-3, SEQ ID NOs 11-20, Figures 1-5).

The effect of increasing the number of potential cross-linking sites in an oleosin peptide on the OBs of such plants can be assessed by measuring OB integrity and emulsion stability can as described in Example 6.

### Design and construction of oleosins containing more than three cysteine residues in each hydrophilic arms

The ole-3,3 lines had substantial levels of elevated lipid levels in the form of TAGs when co-expressed with DGAT1 (S205A) while the lines containing ole-0,0 did not have elevated lipid levels above the DGAT1 over expressing control. The ole-1,1, ole-1,3 and ole-3,1 showed there was a correlation between the level of lipid accumulation in the leaf and the increase in the number of cysteines engineered into each arm (Table 3).

**Table 3. Fatty acid composition (as %Dry Weight) of Arabidopsis leaves expressing either vector control, DGAT1 (S205A) alone, or DGAT1 (S205A) and different forms of oleosin (containing either no additional cysteines or up to 3 additional cysteines in each hydrophilic arm).**

| **Fatty acid profile** | **Vector control** | **DGAT1 ALONE DGAT1SA #2** | **DGAT1 +OLE 0-0 (#11)** | **DGAT1 +OLE 1-1 (#9)** | **DGAT1 +OLE 1-3 (#5)** | **DGAT1 +OLE 3-1 (#18)** | **DGAT1 +OLE 3-3 (#47)** |
|---|---|---|---|---|---|---|---|
| C16:0 | 0.55±0.035 | 0.55±0.001 | 0.54±0.014 | 0.57±0.001 | 0.68±0.042* | 0.62±0.084 | 0.95±0.049* |
| C16:1 | 0.085±0.007 | 0.105±0.007 | 0.11±0.001 | 0.13±0.014 | 0.1±0.001 | 0.135±0.021 | 0.11±0.001 |
| C16:3 | 0.34±0.021 | 0.41±0.028 | 0.42±0.007 | 0.48±0.028 | 0.51±0.035 | 0.55+0.071* | 0.62±0.049* |
| C18:1 | 0.095±0.007 | 0.075±0.007 | 0.1±0.001 | 0.185±0.007 * | 0.345±0.007 * | 0.2±0.014* | 0.61±0.014* |
| C18:2 | 0.55±0.014 | 0.46±0.035 | 0.56±0.014 | 0.77±0.049* | 0.97±0.007* | 0.79±0.113* | 1.82±0.113* |
| C18:3 | 1.67±0.056 | 1.91±0.028 | 1.78±0.014 | 1.68±0.028 | 1.74±0.014 | 1.9±0.28 | 2.29±0.056* |
| C20:0 | Not detected | Not detected | Not detected | Not detected | Not detected | Not detected | 0.054±0.003 |

The correlation between the increase in total lipid (shown to be TAG) and the number of cysteines engineered into the hydrophilic domains indicated that the number of cysteines may be a way to influence the level of TAG desired. Consequently new constructs containing more than 3 cysteines per hydrophilic arm were designed. While it is not possible to put an infinite number of cysteines/hydrophilic arm; the limitations include:
- Length of the arms - if additional residues were added to make space for the cysteines then eventually the degree of hydrophobic domain interaction would be reduced since their ability to come into contact would be limited by their freedom to move on the OB.
- Maintaining the proportion of +, - and amphipahthic residues - if the balance of these residues and distribution of these residues is altered dramatically it is likely that the hydrophilic arms would not actually interact with the surface of the OB and as such would not provide any protection against lipases or coalescence.
- Sulfur availability - increasing the number of cysteines per oleosin molecule may place the plant under nutritional stress if sulphur is limiting.

The original cysteine-oleosin was engineered to carry 3 relatively evenly spaced unpaired cysteines in each arm by replacing amino acids and predominantly those that could be predicted to be neutral or charged but not hydrophobic.

The oleosin presumably needs to have a certain level of negative charge and in the C-terminus this appears to be achieved by K (Lys), hence continuing the strategy of swapping charged or neutral residues with additional cysteines may result in poor stability in terms of preventing coalescence. Furthermore, in the N-terminal hydrophilic region there appears to be too few residues left between the engineered cysteines to enable further substitution of residues whilst maintaining the spacing and oscillation between positive and negatively charged amino acids. Hence, for both N- and C-termini added additional residues (cysteines) rather than substitute existing residues with cysteines. Alternatively, an oleosin with longer hydrophilic arms could have been used.

Two additional constructs (Ole-5, 6 and Ole-6,7) were also designed. These are not purposely unbalanced in terms of cysteine residues per arm but organised to attempt to give typically 4-5 residues between each cysteine. In fact to increase the cysteines to 6 in the N-terminal arm it was necessary to generate additional residues (as opposed to substitution of existing residues); this as achieved by replicate the first 6 residues from the Ole-3,3.

Rather than have completely new nucleotide sequences designed the triplet TGT to code for cysteine was added (where appropriate) to generate Ole_5,6. For additional glutamine residues the codon triplet GGA was used. For the additional N-terminal 6 residues on Ole_6-7 the N-terminus of Ole_3,3 was replicated and fused in frame.

Sublconing strategy was designed to be identical to initial cysteine oleosins, i.e., subcloned into oleoacceptor by NotI/NdeI. This is then recombined by LR reaction into pRSH1 (Winichayakul et al., 2008). Essentially places both Arabidopsis DGAT1 (S205A) and oleosin under their own CaMV35s promoters and OCS terminators. Both DGA1 and oleosin clones contain a UBQ10 intron.

NetGene2 was used to predict the splicing pattern of Ole_5,6 and Ole_6,7. Both were predicted to have only one donor and acceptor site on the direct strand (both were predicted to have a very high probability of recognition) and no sites on the complementary strand.

The data indicates that the oleosins containing 1,3 or 3,1 cysteines do accumulate detectable levels of TAG but this is certainly less than the 3,3 cysteine oleosins (the 1,1 accumulated trace amounts while the 0, 0 did not). This suggests even more strongly that the 5,6 and 6,7 oleosins are likely to accumulate even more TAG than the 3,3 construct. The first data from the 5,6 and 6,7 constructs will be available soon.

### Transformation of oleosins containing engineered cysteines and DGAT1 into wild type Arabidopsis thaliana

Five disulfide-oleosin/DGAT1 (S205A) gene constructs and one control (construct containing DGAT1 (S205A) but not oleosin) were been transferred to the plant binary vector pRSh1 (Winichayakul et al., 2008) and transformed into wild type Arabidopsis thaliana using Agrobacterium-mediated transformation.

A modification of the traditional floral dip method was followed since it has been reported that floral dipping tends to damage developing siliques due to the presence of detergent in the inoculums (Martinez-Trujillo et al., 2004). Therefore, a drop by drop inoculation to every flower was carried out using a micropipette. The inoculation was repeated after one week to introduce the inoculum to the newly developed flowers. Seeds were collected when the siliques have dried up, then cleaned and planted for screening of transformants.

Screening for transformants was performed by BASTA selection and homozygous transformants were selected using segregation ratio analysis for BASTA resistance.

### Transformation of oleosins containing engineered cysteines and DGAT1 into wild type Trifolium repens

Transformation into *Trifolium repens* (white clover) was performed according to the procedure of Voisey *et al.,* (1994).

Seeds were weighed to provide approximately 400 - 500 cotyledons (ie. 200 - 250 seeds) for dissection (0.06gm = 100 seeds). In a centrifuge tube, seeds were rinsed with 70% ethanol for 1 minute. Surface sterilised in bleach (5% available chlorine) by shaking on a circular mixer for 15 minutes followed by four washes in sterile water. Seeds were imbibed overnight at 4degC.

The same constructs used to transform Arabidopsis (abover) were maintained in Agrobacterium strain GV3101 and inoculated into 25 mL of MGL broth (Table 4) containing spectinomycin at a concentration of 100mg/L. Cultures were grown overnight (16 hours) on a rotary shaker (200rpm) at 28oC. Bacterial cultures were harvested by centrifugation (3000xg, 10 minutes). The supernatant was removed and the cells resuspended in a 5mL solution of 10mM MgSO4.

Cotyledons were dissected from seeds using a dissecting microscope. First, the seed coat and endosperm were removed. Cotyledons were separated from the radical with the scalpel by placing the blade between the cotyledons and slicing through the remaining stalk. Cotyledonary explants were harvested onto a sterile filter disk on CR7 media.

For transformation, a 3ul aliquot of Agrobacterium suspension was dispensed to each dissected cotyledon. Plates were sealed and cultured at 25degC under a 16 hour photoperiod. Following a 72 hour period of co-cultivation, transformed cotyledons were transferred to plates containing CR7 medium supplemented with ammonium glufosinate (2.5mg/L) and timentin (300mg/L) and returned to the culture room.

Following the regeneration of shoots, explants were transferred to CR5 medium supplemented with ammonium glufosinate (2.5mg/L) and timentin (300mg/L). Regenerating shoots are subcultured three weekly to fresh CR5 media containing selection.

As root formation occurs, plantlets were transferred into tubs containing CR0 medium containing ammonium glufosinate selection. Large clumps of regenerants were divided to individual plantlets at this stage. Whole, rooted plants growing under selection were then potted into sterile peat plugs. Once established in peat plugs plants were then transfer to the greenhouse.

**Table 4. Media compositions used for Trifolium repens transformation.**

| | | | |
|---|---|---|---|
| A. | CR#0 | | |
| | | | |
| | MS salts | | |
| | B5 vitamins | | |
| | sucrose | | 30 g/L |
| | pH5.8 | | (KOH) |
| | agar | | 8.0 g/L |
| | | | |
| | CR#5 | | |
| | | | |
| | MS salts | | |
| | B5 vitamins | | |
| | sucrose | | 30 g/L |
| | BA | | 0.1mg/L |
| | NAA | | 0.05mg/L |
| | pH 5.8 | | (KOH) |
| | agar | | 8.0g/L |
| | | | |
| B. | CR#7 | | |
| | | | |
| | MS salts | | |
| | B5 vitamins | | |
| | sucrose | | 30 g/L |
| | BA | | 1.0mg/L |
| | NAA | | 0.05mg/L |
| | pH 5.8 | | (KOH) |
| | agar | | 8.0g/L |
| | | | |
| C. | MGL | | |
| | | | |
| | Mannitol | | 5.0g/L |
| | L glutamic acid | 1.0g/L | |
| | KH2PO4 | | 250mg/L |
| | | | |
| | MgSO4 | | 100mg/L |
| | NaCl | | 100mg/L |
| | Biotin | | 100mg/L |
| | | | |
| | Bactotryptone | 5.0g/L | |
| | Yeast extract | 2.5g/L | |
| | pH7.0 | | (NaOH) |

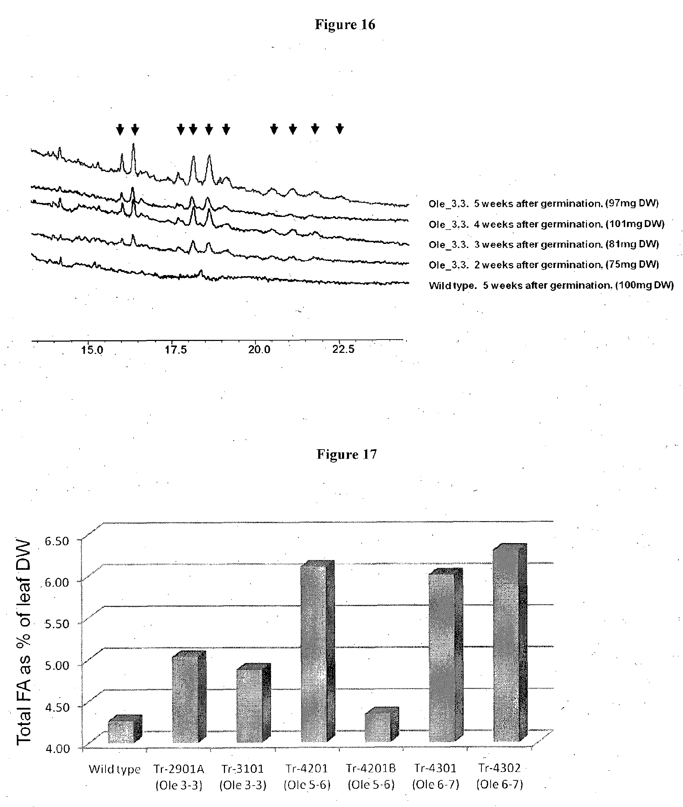

FAMES GC/MS results showed the transgeneic *Trifolium repens* (containing DGAT1 (S205A) and either Ole_3,3 or Ole_5,6 or Ole 6,7) had elevated total leaf lipid profiles compared to wild type (Figure 17). There was a general correlation between the highest level of leaf lipid and the highest number of cysteines engineered into the oleosin.

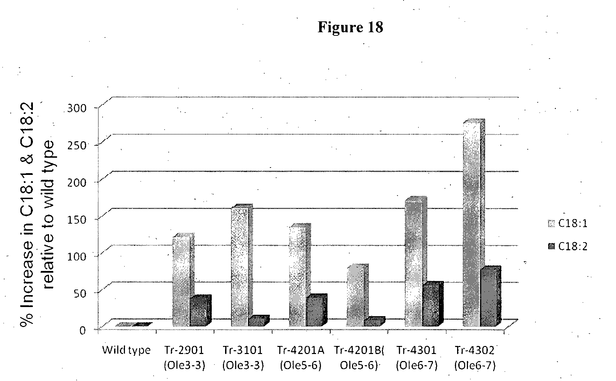

FAMES GC/MS results showed the transgeneic *Trifolium repens* (containing DGAT1 (S205A) and either Ole_3,3 or Ole_5,6 or Ole 6,7) had elevated C18:1 and C18:2 leaf lipid profiles compared to wild type as also seen in Arabidopsis (Figure 18). The highest level of leaf C18:1 and C18:2 was found in plants transformed with the oleosin containing the highest number of engineered cysteines.

### Determination of oil body assembly in leaves (and seeds)

Further screening was conducted using immunoblot analysis (with an anti-sesame seed oleosin antibody, Scott et al., 2007) to determine the lines overexpressing the oleosin protein. Using this method, either oil bodies (OBs) were extracted from T2 seeds of putative transformants using sucrose density gradient or total protein was extracted from leaves in a denaturing/reducing buffer and proteins were separated in SDS-PAGE, transferred to nitrocellulose membrane, and challenged with an antibody raised against the sesame oleosin (Scott et al., 2007).

Crude oil body (OB) was extracted from 25 mg of seeds in 500 µL OB buffer (10 mM Sodium phosphate, pH 7.5 containing 600 mM sucrose). After centrifugation at 13,000 x g, the aqueous layer was carefully suck out and the fat pad layer was resuspended in 200 µL of OB buffer without disturbing the pellet at the bottom. 20 µL of each OB extract was added with 4x loading dye and 10x reducing agent, heated up to 70°C for 5 min and loaded onto 4-12% polyacrylamide gel for immunoblot analysis. The blot was incubated in α-sesame oleosin antibody (1°Ab) at 1:750 dilution for one hour, and another one hour in secondary antibody (1:10,000).

Oleosin is naturally expressed in seeds and not in the leaves. However, since we have co-expressed DGAT1 with oleosin both under the control of CaMV35S promoters it could be anticipated that this would enable detectable levels of oleosin to accumulate in the leaves. Leaves from transformed lines with high expression of recombinant oleosin in the seeds (identified by immunoblot analysis) were analyzed by immunoblot using antibodies raised against the sesame oleosin.

Table 5 below summarises the number of putative transformants generated and the number of plants expressing recombinant oleosin in the seed and leaf.

**Table 5**

| Gene construct ID | Number of putative transformants (based on BASTA resistance) | Number of lines seeds were analysed by immunoblot (anti sesame seed oleosin antibody) | Number of lines with a positive immunoreactive band at the appropriate size in the seed extract | Number of lines with a positive immunore active band at the appropriate size in the leaf extract |
|---|---|---|---|---|
| pRSh1-DGAT1(S2 05A) control | 8 | N/A | N/A | |
| pRSh1-DGAT1(S2 05A)-Ole_0-0 | 14 | 8 | 7 | 3 |
| pRSh1-DGAT1(S2 05A)-Ole_1-1 | 22 | 2 | 1 | 1 |
| pRSh1-DGAT1(S2 05A)-Ole_1-3 | 20 | 0 | 0 | 1 |
| pRSh1-DGAT1(S2 05A)-Ole_3-1 | 23 | 8 | 4 | 2 |
| pRSh1-DGAT1(S2 05A)-Ole_3-3 | 54 | 22 | 16 | 5 |

It should be noted the level of recombinant oleosin that accumulated in the leaves was considerably lower than in the seeds. However, the proportion of individual lines accumulating detectable levels in both the leaves was much greater than when oleosin was expressed alone (Roberts Lab, unpublished data) indicating that the co-expression of both DGAT1 and oleosin in the leaf has lead to the accumulation of higher levels of oleosin.

### Analysis of leaves from transgenic plants co-expressing DGAT1 (S205A) and disulfide oleosins

The seeds from homozygous lines over expressing the oleosin protein in the seeds were germinated to allow growth of 2, 3, 4 or 5 weeks. Sufficient leaf material was harvested for FAMES GC-MS, as well as by GC-MS using a RTX 65-TG Restek column which enable the separation and identification of free fatty acids, diacylglycerides, wax esters, sterol esters and triacylglycerides without derivatization.

### Preparation of material for FAMES-GC/MS analysis

10 mg of freeze-dried leaf powder was placed in a 13 x 100 mm screw-cap tube, 10 µL of non methylated internal standard (C15:0 FA, 4 mg/mL dissolved in heptane) was added, To this mixture, 1 mL of the methanolic HCl reagent (1 mL of 3 M solution diluted to 1 M using dry methanol which had been treated with 5% 2,2-dimeethoxypropane as a water scavenger_. The tube was then flushed with N2 gas then sealed immediately with Teflon-lined cap, and heated at 80 °C for 1 h. After the tubes had cooled to room temperature, 10 µL pre-methylated standard (4 mg/mL of C17:0 dissolved in heptane) was added. To this mixture, 0.6 mL heptane and 1.0 mL of 0.9% (w/v) NaCl was added, and mixed thoroughly by vortexing. Following centrifugation at 500 rpm for 1 min at room temperature, 100 µL of the top layer (containing heptanes) was collected and transferred to a flat-bottom glass insert fitted into a brown vial for GC/MS analysis.

### FAMES GC/MS Analysis

The FAMES GC/MS was analysed using the SGE capillary column BPX70 (50m x 0.22mm x 0.25 µm). The condition of GC-MS was as follows: the temperature was programmed from 80 °C to 150 °C at 15 °C /min and then to 250 °C at 8 °C /min and held isothermal for 10 min. Samples were injected in a split mode; total flow of 28.4 mL/min; column flow of 0.82 mL/min; and a purge flow of 3.0 mL/min. The pressure was kept at 150 kPa; ion source temperature was 200 °C and an interface temperature was kept at 260 °C. The target compounds were acquired by mass spectrometry in a scan mode starting at 50 m/z and ending at 350 m/z.

### TAG and polar lipid extraction

TAG was extracted using a modified method of Ruiz-López et al., (2003). Briefly, for each TAG analysis, betweeen34-80 mg of freeze-dried leaf powder was placed into tared 13-mm screw cap tube and weighed, 2.4 mL of 0.17 M NaCl in MeOH was added and mixed by vortexing. Following the addition of 4.8 mL heptane and 10 µL of internal standard (C14:0, 10 µg.µL-1), the suspension was mixed gently and incubated without shaking in 80 °C water bath for 2 h. After cooling to room temperature, the upper phase (containing lipids) was transferred to fresh screw-cap tube and evaporated to dryness under stream of N gas. Finally, the dried powder were resuspended in 100 µL heptanes, mixed thoroughly then transferred to a flat-bottom glass insert fitted into a brown glass vial for TAG analysis.

### TAG GC-MS analysis

TAG analysis was performed on a Hewlett Packard (HP) GC and Shimadzu Scientific Instruments Inc. MS (QP2010). All analyses were performed with a RESTEK capillary column MXT-65TG (65% diphenyl - 35% dimethyl polysiloxane, 30.0 m x 0.10 µm thickness x 0.25 mm diameter) in Electron Impact (EI) ionization mode. Helium was used as the carrier gas. All samples were injected in splitless mode, in 1.0 µl aliquots, and a column flow of 1.2 mL.min-1. The gas chromatograph was programmed from 200 to 370 °C at 15 °C.min-1 and kept isothermal at 370 °C for 15 min. The sample injector port temperature was maintained at 350 °C, column oven temperature at 200 °C, with a pressure of 131.1 kPa and a purge flow of 3.0 mL.min-1. The mass spectrometric conditions were as follows: ion source temperature was held at 260 °C during the GC-MS runs, the mass spectra were obtained at ionization voltage of 70 eV at an emission current of 60 µA and an interface temperature of 350 °C. Acquisition mode was by scanning at a speed of 5000, 0.25 sec per scan. Chromatograph peaks with mass to charge ratio of 45 m/z to 1090m/z were collected starting at 9 min and ending at 25 min.

### EXAMPLE 8: Further oleosins, caloleosins and steroleosins engineered to contain additional cysteine residues in the N- and C- terminal hydrophilic arms

The applicants have used the same strategy as for sesame seed oleosin, accession number AAD42942, (i,e., substituting charged residues predicted to be on the surface of OBs with cysteines) to engineer cysteines into the N- and C- terminal hydrophilic arms of oleosins caoleosins and steroleosins. In some cases it has been possible to substitute only negatively charged amino acids (Glutamic acid and Aspartic acid) that are relatively evenly spaced. In the case of the sesame oleosin AAD42942 it was necessary to sometimes compromise on the charge substitution. It should be noted in the examples below that two caleosins (AAB71227 and AAF13743) contain two endogenous cysteines in their C-terminal arm. These are left unaltered in the engineering.

To determine the position of the amino acid substitution each protein was aligned with the sesame oleosin (AAD42942) in the original form as well as the forms containing 1 or 3 cysteines per hydrophilic arm (i.e., ole_0,0; ole_1,1; ole_3,1; ole_1,3; ole_3,3). The potential glutamic acids and aspartic acids in N-terminus or C-terminus of each of the hydrophilic arms (determined by hydrophobicity plots) were then highlighted with grey boxes, as were the relevant lysine, arginine and glutamine residues (which were all successfully altered in the sesame oleosin (AAD42942). The mutation of these residues to cysteine were then considered along with their spacing with each other. The final substitutions are then shown with the original peptide sequence and the engineered sequence only. In this case only 3 cysteines were engineered into each arm, however, the number could have been greater or fewer. An alternative approach would have been to work with each protein in isolation and simply begin by identifying the hydrophilic regions by hydrophobicity plot then begin the process of substitution with the most appropriate charged amino acid.

Table 6 below shows additional oleosin and caoleosins that the applicants have modifiedto introduce cysteines in the hydrophilic portions.

**Table 6.**

| **Protein Type** | **Plant Source** | **Accession Number** | **SEQ ID NO** |
|---|---|---|---|
| oleosin | *Brassica oleraceae* (pollen oleosin) | CAA65272.1 | 90 |
| oleosin | Maize | NP_001147032.1 | 91 |
| oleosin | Rice | AAL40177.1 | 92 |
| caoleosin | Sesame | AAF13743 | 93 |
| caoleosin | Soybean | AAB71227 | 94 |
| caoleosin | Maize | NP_001151906 | 95 |
| steroleosin | Sesame | AAL13315 | 96 |
| steroleosin | Brassica napus | ACG69522 | 97 |
| steroleosin | Maize | NP_001152614.1 | 98 |

**Table 7 below references the SEQ ID NO in the modified oleosins**

| **Protein Type** | **Plant Source** | **Accession Number** | **SEQ ID NO** |
|---|---|---|---|
| oleosin | *Brassica oleraceae* (pollen oleosin) | X96409 | 99 |
| oleosin | Maize | NP_001147032.1 | 100 |
| oleosin | Rice | AAL40177.1 | 101 |
| caoleosin | Sesame | AAF13743 | 102 |
| caoleosin | Soybean | AAB71227 | 103 |
| caoleosin | Maize | NP_001151906 | 104 |
| steroleosin | Sesame | AAL13315 | 105 |
| steroleosin | Brassica napus | ACG69522 | 106 |
| steroleosin | Maize | NP_001152614.1 | 107 |

The modified sequence can be expressed as described in the examples above to produce oil bodies, emulsions, transgenic host cells, plants etc, and to test the properties of each.

It is not the intention to limit the scope of the invention to the abovementioned examples only.

### REFERENCES

Abell et al., (2004). Plant J., 37: 461-70.
Altschul et al., (1997) Nucleic Acids Res. 25: 3389-3402,
Andrianov et al., (2010). Plant Biotechnol J. 8(3):277-87.
Ausubel et al., (1987) Current Protocols in Molecular Biology, Greene Publishing Bairoch and Bucher (1994), Nucleic Acids Res. 22, 3583
Bao et al, (2000) Plant J. 22(1):39-50.
Birch (1997) Ann Rev Plant Phys Plant Mol Biol, 48, 297
Bolton and McCarthy (1962) PNAS 84:1390
Bowie et al., (1990). Science 247, 1306
Bouvier-Nave et al., (2000) Eur. J. Biochem. 267, 85-96.
Bryan et al., (2007) Modification of fatty acid biosynthesis. United States Patent 20070118927.
Capuano et al., (2007). Biotechnol Adv. 25:203-206.
Notredame et al., (2000). J. Mol. Biol. 302: 205-217
Chen et al., (1999). Plant Cell Physiol., 40:1079-1086.Chiang et al., (2005). J Agric Food Chem 53:4799-804.
Chiang et al., (2007). Protein Expr Purif. 52:14-8.
Cookson et al., (2009). Improvements in and relating to oil production. PCT/NZ2008/000085 WO/2008/130248
Dahlqvist et al., (2000). Proc Natl Acad Sci USA. 97, 6487-6492.
Deckers et al., (2003). United States patent US 6582710
Demeyer and Doreau, (1999). Proc Nutr Soc. 58(3):593-607.
Deutscher (1990) Ed, Methods in Enzymology, Vol. 182, Guide to Protein Purification
Draper et al., 1988, Plant Genetic Transformation and Gene Expression. A Laboratory Manual. Blackwell Sci. Pub. Oxford, p. 365
Falquet et al., 2002, Nucleic Acids Res. 30, 235
Feng and Doolittle, 1987, J. Mol. Evol. 25, 351
Firkins et al., (2006). J Dairy Sci. 89 Suppl 1 :E31-51. Review. Greenspan.
Frandsen et al., (2001). Physiologia Plantarum, 112:301-307.
Frohman (1993). Methods Enzymol. 218: 340-56
Galun and Breiman (1997). Transgenic Plants. Imperial College Press, London
Gelvin et al., 1993, Plant Molecular Biol. Manual. Kluwer Acad. Pub. Dordrecht
Giesen et al., Nucleic Acids Res. 1998 Nov 1;26(21):5004-6
Halford & Hardie (1998). Plant Mol Biol. 37:735-48. Review
Harada et al., (2002). OLEOSIN/PHOSPHOLIPID COMPLEX AND PROCESS FOR PRODUCING THE SAME. World Patent WO/2002/026788.
Hellens et al., (2000). Plant Mol Biol 42: 819-32
Hellens et al., (2005). Plant Meth 1: 13
Herrera-Estrella et al., (1993). Nature 303, 209
Hofmann et al., (1999). Nucleic Acids Res. 27, 215
Hou et al., (2003). J Dairy Sci; 86: 424-8.
Huang (1992). Ann. Rev. Plant Physiol. Plant Mol. Biol. 43:177-200.
Huang, X. (1994) Computer Applications in the Biosciences 10, 227-235
Jeanmougin et al., (1998) Trends Biochem. Sci. 23, 403-5.
Jenkins and Bridges (2007). Eur. J. Lipid Sci. Technol. 109:778-789.
Jenkins and McGuire (2006). J Dairy Sci. 89(4):1302-10. Review.
Kaup et al., (2002) Plant Physiol. 129(4):1616-26.
Kyte and Doolitle (1982) J. Mol. Biol. 157:105-132
Lanfranco L. (2003). Riv Biol. 96(1):31-54.
Lardizabal et al., (2001). J.B.C. 276, 38862-38869.
Leprince et al., (1998). Planta 204 109-119.
Lin and Tzen. (2004). Plant Physiology and Biochemistry. 42:601-608.
Lock and Bauman (2004). Lipids. 39(12):1197-206. Review.
Loer and Herman (1993). Plant Physiol. 101(3):993-998.
Mayer and Fowler (1985). J Cell Biol. 100(3):965-73.
Mekhedov et al., (2000). Plant Physiol. 122(2):389-402).
Mullis et al., Eds. 1994 The Polymerase Chain Reaction, Birkhauser
Murphy (1993). Prog. Lipid Res. 32:247-280.
Needleman and Wunsch, (1970) J. Mol. Biol. 48, 443-453
Nielsen et al., Science. (1991) 254(5037):1497-500
Ohlrogge and Jaworski (1997). Annu Rev Plant Physiol Plant Mol Biol. 48:109-136.
Papapostolou and Howorka (2009). Mol Biosyst. 5(7):723-32. Review.
Peng (2004). Development and applications of artificial sesame oil body. Ph.D. Dissertation. National ChunHsing University Graduate Institute of Biotechnology. Taichung, Taiwan.
Peng et al., (2006) . Stability enhancement of native and artificial oil bodies by genipin crosslink. Taiwan patent I250466.
Peng et al., (2004). J Biotechnol 2004; 111: 51-7.
Potrykus and Spangenburg (1995). Gene Transfer to Plants. Springer-Verlag, Berlin
Roberts et al., (2008). The Open Biotechnology Journal 2:13-21.
Roux et al., (2004). J Agric Food Chem. 52(16):5245-9.
Scott et al., (2007). Polyoleosins. WO2007045019.
Saha et al., (2006). Plant Physiol. 141(4):1533-43.
Sambrook et al., Eds, 1987, Molecular Cloning, A Laboratory Manual, 2nd Ed. Cold Spring Harbor Press
Sarmiento et al., (1997). Plant J. 11(4):783-96.
Schrott (1995) In: Gene Transfer to Plants (Potrykus, T., Spangenberg. Eds) Springer Verlag. Berline, pp. 325-336
Shimada et al., (2008). Plant J. 55(5):798-809.
Shockey et al., (2006). Plant Cell., 18,2294-2313.
Siloto et al., (2006). Plant Cell. 18(8): 1961-74.
Slack et al., (1980). Biochem J. 190(3):551-561.
Slocombe et al., (2009). Plant Biotechnol J. 7(7):694-703.
Stahl et al., (2004). Plant Physiology, 135: 1324-1335.
Stewart et al., (1969), In: Solid-Phase Peptide Synthesis, WH Freeman Co, San Francisco California
Thompson et al., (1994) Nucleic Acids Research, 22:4673-4680
Ting et al., (1997). J Biol Chem., 272: 3699-3706.
Tadege et al., (2005). Trends Plant Sci. 10(5):229-35.
Triglia et al., 1998, Nucleic Acids Res 16, 8186
Tzen et al., (1992). J. Biol. Chem. 267: 15626-34
Tzen et al., (2003). Adv Plant Physiol., 6: 93-104.
Tzen et al., (1997). J Biochem. 121(4):762-8.
Voisey et al., (1994). Plant Cell Reports 13: 309 314.
Winichayakul et al., (2008). Proc. NZGA, 70:211-216
Xu et al., (2005). Plant Cell. 17(11):3094-110.
Zou et al., (1999). Plant J. 19, 645-653.
Zou et al., (2008). Plant Biotech. J. 6(8):799-818.

**SUMMARY OF SEQUENCE LISTING**

| **SEQ ID NO:** | **Type** | **SPECIES** | **COMMENTS** |
|---|---|---|---|
| 1 | polynucleotide | artificial | Oleosin disulfide 0,0 nucleotide sequence, as cloned into pET29b using NdeI and XhoI restriction sites (adds N-terminal S•tag thrombin cleavage site and C-terminal His tag). |
| 2 | polynucleotide | artificial | Oleosin disulfide 1,1 nucleotide sequence, as cloned into pET29b using Ndel and XhoI restriction sites (adds N-terminal S•tag thrombin cleavage site and C-terminal His tag). |
| 3 | polynucleotide | artificial | Oleosin disulfide 1,3 nucleotide sequence, as cloned into pET29b using NdeI and XhoI restriction sites (adds N-terminal S•tag thrombin cleavage site and C-terminal His tag). |
| 4 | polynucleotide | artificial | Oleosin disulfide 3,1 nucleotide sequence, as cloned into pET29b using NdeI and XhoI restriction sites (adds N-terminal S•tag thrombin cleavage site and C-terminal His tag). |
| 5 | polynucleotide | artificial | Oleosin disulfide 3,3 nucleotide sequence, as cloned into pET29b using Ndel and Xhol restriction sites (adds N-terminal S•tag thrombin cleavage site and C-terminal His tag). |
| 6 | Polypeptide | Artificial | Oleosin disulfide 0,0 peptide sequence, as cloned into pET29b using NdeI and Xhol restriction sites (adds N-terminal S•tag thrombin cleavage site and C-terminal His tag). |
| 7 | Polypeptide | Artificial | Oleosin disulfide 1,1 peptide sequence, as cloned into pET29b using NdeI and XhoI restriction sites (adds N-terminal S•tag thrombin cleavage site and C-terminal His tag). |
| 8 | Polypeptide | Artificial | Oleosin disulfide 1,3 peptide sequence, as cloned into pET29b using NdeI and XhoI restriction sites (adds N-terminal S•tag thrombin cleavage site and C-terminal His tag). |
| 9 | Polypeptide | Artificial | Oleosin disulfide 3,1 peptide sequence, as cloned into pET29b using NdeI and XhoI restriction sites (adds N-terminal S•tag thrombin cleavage site and C-terminal His tag). |
| 10 | Polypeptide | Artificial | Oleosin disulfide 3,3 peptide sequence, as cloned into pET29b using NdeI and XhoI restriction sites (adds N-terminal S•tag thrombin cleavage site and C-terminal His tag). |
| 11 | Polynucleotide | Artificial | (Nucleotide sequence of Oleosin disulfide 0,0 including Kozac sequence and UBQ10 intron, as transformed into *Arabidopsis thaliana* under the control of the CaMV35s promoter.) |
| 12 | Polynucleotide | Artificial | Nucleotide sequence of Oleosin disulfide 1,1 including Kozac sequence and UBQ10 intron, as transformed into *Arabidopsis thaliana* under the control of the CaMV35s promoter. |
| 13 | Polynucleotide | Artificial | (Nucleotide sequence of Oleosin disulfide 1,3 including Kozac sequence and UBQ10 intron, as transformed into *Arabidopsis thaliana* under the control of the CaMV35s promoter.) |
| 14 | Polynucleotide | Artificial | Nucleotide sequence of Oleosin disulfide 3,1 including Kozac sequence and UBQ10 intron, as transformed into *Arabidopsis thaliana* under the control of the CaMV35s promoter. |
| 15 | Polynucleotide | Artificial | Nucleotide sequence of Oleosin disulfide 3,3 including Kozac sequence and UBQ10 intron, as transformed into *Arabidopsis thaliana* under the control of the CaMV35s promoter. |
| 16 | Polypeptide | Artificial | Peptide sequence of Oleosin disulfide 0,0, as transformed into *Arabidopsis thaliana* under the control of the CaMV35s promoter. |
| 17 | Polypeptide | Artificial | Peptide sequence of Oleosin disulfide 1,1, as transformed into *Arabidopsis thaliana* under the control of the CaMV35s promoter.) |
| 18 | Polypeptide | Artificial | Peptide sequence of Oleosin disulfide 1,3, as transformed into *Arabidopsis thaliana* under the control of the CaMV35s promoter. |
| 19 | Polypeptide | Artificial | Peptide sequence of Oleosin disulfide 3,1, as transformed into *Arabidopsis thaliana* under the control of the CaMV35s promoter. |
| 20 | Polypeptide | Artificial | Peptide sequence of Oleosin disulfide 3,3, as transformed into *Arabidopsis thaliana* under the control of the CaMV35s promoter |
| 21 | Polynucleotide | Artificial | Nucleotide sequence of Oleosin disulfide 5,6 including Kozac sequence and UBQ10 intron, as transformed into *Arabidopsis thaliana* under the control of the CaMV35s promoter. |
| 22 | polynucleotide | Artificial | Nucleotide sequence of Oleosin disulfide 6,7 including Kozac sequence and UBQ10 intron, as transformed into *Arabidopsis thaliana* under the control of the CaMV35s promoter. |
| 23 | Polypeptide | Artificial | Peptide sequence of Oleosin disulfide 5,6, as transformed into *Arabidopsis thaliana* under the control of the CaMV35s promoter |
| 24 | Polypeptide | Artificial | Peptide sequence of Oleosin disulfide 6,7, as transformed into *Arabidopsis thaliana* under the control of the CaMV35s promoter |
| 25 | Polynucleotide | Artificial | Oleoacceptor (contains OCS terminator, CAMV35S promoter, DGAT1 (S205A) from Arabidopsis and UBQ10 intron) |
| 26 | Polynucleotide | Artificial | Oleosin_0,0 and DGAT1 (S205A) in pRSH1 |
| 27 | Polynucleotide | Artificial | Oleosin_1,1 and DGAT1 (S205A) in pRSH1 |
| 28 | Polynucleotide | Artificial | Oleosin_1,3 and DGAT1 (S205A) in pRSH1 |
| 29 | Polynucleotide | Artificial | Oleosin_3,1 and DGAT1 (S205A) in pRSH1 |
| 30 | Polynucleotide | Artificial | Oleosin_3,3 and DGAT1 (S205A) in pRSH1 |
| 31 | Polynucleotide | Artificial | Oleosin_5,6 and DGAT1 (S205A) in pRSH1 |
| 32 | Polynucleotide | Artificial | Oleosin_6,7 and DGAT1 (S205A) in pRSH1 |
| 33 | Polypeptide | Artificial | DGAT1 (S205A) |
| 34 | Polynucleotide | S. *indicum* | Oleosin - AF302807 |
| 35 | Polypeptide | S. *indicum* | Oleosin - AAG23840 |
| 36 | Polynucleotide | S. *indicum* | Oleosin - U97700 |
| 37 | Polypeptide | S. *indicum* | Oleosin - AAB58402 |
| 38 | Polynucleotide | *A. thaliana* | Oleosin - X62353 |
| 39 | Polypeptide | *A. thaliana* | Oleosin - CAA44225 |
| 40 | Polynucleotide | *A. thaliana* | Oleosin - BT023738 |
| 41 | Polypeptide | *A. thaliana* | Oleosin - AAZ23930 |
| 42 | Polynucleotide | *H. annuus* | Oleosin - X62352.1 |
| 43 | Polypeptide | *H. annuus* | Oleosin - CAA44224.1 |
| 44 | Polynucleotide | *B. napus* | Oleosin - X82020.1 |
| 45 | Polypeptide | *B. napus* | Oleosin - CAA57545.1 |
| 46 | Polynucleotide | *Z. mays* | Oleosin - NM_001153560.1 |
| 47 | Polypeptide | *Z*. *mays* | Oleosin - NP_001147032.1 |
| 48 | Polynucleotide | *O*. *sativa* | Oleosin - L76464 |
| 49 | Polypeptide | *O. sativa* | Oleosin - AAL40177.1 |
| 50 | Polynucleotide | *B. oleracea* | Oleosin - AF117126.1 |
| 51 | Polypeptide | *B. oleracea* | Oleosin - AAD24547.1 |
| 52 | Polynucleotide | *C*. *arabica* | Oleosin - AY928084.1 |
| 53 | Polypeptide | *C*. *arabica* | Oleosin - AAY14574.1 |
| 54 | Polynucleotide | *S*. *indicum* | Steroleosin - AF421889 |
| 55 | Polypeptide | *S*. *indicum* | Steroleosin - AAL13315 |
| 56 | Polynucleotide | *B. napus* | Steroleosin - EU678274 |
| 57 | Polypeptide | *B. napus* | Steroleosin - ACG69522 |
| 58 | Polynucleotide | *Z*. *mays* | Steroleosin - NM_001159142.1 |
| 59 | Polypeptide | *Z*. *mays* | Steroleosin - NP_001152614.1 |
| 60 | Polynucleotide | *B. napus* | Steroleosin - EF143915.1 |
| 61 | Polypeptide | *B. napus* | Steroleosin - ABM30178.1 |
| 62 | Polynucleotide | *S*. *indicum* | Caleosin - AF109921 |
| 63 | Polypeptide | *S*. *indicum* | Caleosin - AAF13743 |
| 64 | Polynucleotide | *G. max* | Caleosin - AF004809 |
| 65 | Polypeptide | *G*. *max* | Caleosin - AAB71227 |
| 66 | Polynucleotide | *Z*. *mays* | Caleosin - NM_001158434.1 |
| 67 | Polypeptide | *Z*. *mays* | Caleosin - NP_001151906 |
| 68 | Polynucleotide | *B. napus* | Caleosin - AY966447.1 |
| 69 | Polypeptide | *B. napus* | Caleosin - AAY40837.1 |
| 70 | Polynucleotide | C. *revoluta* | Caleosin - FJ455154.1 |
| 71 | Polypeptide | C. *revoluta* | Caleosin - ACJ70083.1 |
| 72 | Polynucleotide | *C*. *sativus* | Caleosin - EU232173.1 |
| 73 | Polypeptide | *C*. *sativus* | Caleosin - ABY56103.1 |
| 74 | Polynucleotide | *A. thaliana* | DGAT1 - NM_127503 |
| 75 | Polypeptide | *A. thaliana* | DGAT1 - NP_179535 |
| 76 | Polynucleotide | *T. majus* | DGAT1 - AY084052 |
| 77 | Polypeptide | *T. majus* | DGAT1 - AAM03340 |
| 78 | Polynucleotide | *Z*. *mays* | DGAT1 - EU039830.1 |
| 79 | Polypeptide | *Z*. *mays* | DGAT1 - ABV91586.1 |
| 80 | Polynucleotide | *A. thaliana* | DGAT2 - NM_115011 |
| 81 | Polypeptide | *A. thaliana* | DGAT2 - NP_566952.1 |
| 82 | Polynucleotide | *B. napus* | DGAT2 - FJ858270 |
| 83 | Polypeptide | *B. napus* | DGAT2 - AC090187.1 |
| 84 | Polynucleotide | *A*. *hypogaea* | DGAT3 (soluble DGAT) - AY875644 |
| 85 | Polypeptide | *A. hypogaea* | DGAT3 (soluble DGAT) - AAX62735.1 |
| 86 | Polynucleotide | *A. thaliana* | FDAT - NM_121367 |
| 87 | Polypeptide | *A. thaliana* | PDAT - NP_196868.1 |
| 88 | Polynucleotide | *R. communis* | PDAT - XM_002521304 |
| 89 | Polypeptide | *R*. *communis* | PDAT - XP_002521350 |
| 90 | Polypeptide | *B. oleraceae* | Oleosin - CAA65272.1 |
| 91 | Polypeptide | *Z*. *mays* | Oleosin - NP_001147032.1 |
| 92 | Polypeptide | *O*. *sativa* | Oleosin - AAL40177.1 |
| 93 | Polypeptide | *S*. *indicum* | Caleosin - AAF13743 |
| 94 | Polypeptide | *G. Max* | Caleosin - AAB71227 |
| 95 | Polypeptide | *Z*. *mays* | Caleosin - NP_001151906 |
| 96 | Polypeptide | *S*. *indicum* | Steroleosin - AAL13315 |
| 97 | Polypeptide | Brassica napus | steroleosin ACG69522 |
| 98 | Polypeptide | *Z*. *mays* | steroleosin NP_001152614.1 |
| 99 | Polypeptide | *Brassica oleraceae* | Modified pollen oleosin - CAA65272.1 |
| 100 | Polypeptide | *Zea mays* | Modified oleosin - NP_001147032.1 |
| 101 | Polypeptide | *Oryza sativa* | Modified oleosin - AAL40177.1 |
| 102 | Polypeptide | *S*. *indicum* | Modified caoleosin - AAF13743 |
| 103 | Polypeptide | *G. soja* | Modified caoleosin - AAB71227 |
| 104 | Polypeptide | *Z*. *mays* | Modified caoleosin - NP_001151906 |
| 105 | Polypeptide | *S*. *indicum* | Modified steroleosin - AAL13315 |
| 106 | Polypeptide | *Brassica napus* | Modified steroleosin - ACG69522 |
| 107 | Polypeptide | *Z*. *mays* | Modified steroleosin - NP_001152614.1 |

## Claims

1. A polynucleotide encoding a modified oleosin including at least one artificially introduced cysteine, wherein the cysteine is introduced into at least one of:
a) the N-terminal hydrophilic region of the oleosin, and
b) the C-terminal hydrophilic region of the oleosin.

2. The polynucleotide of claim 1 in which the polynucleotide encodes a fusion protein including the modified oleosin fused to a protein of interest.

3. A genetic construct, expression construct, host cell, plant cell or plant, wherein the construct, cell or plant comprises the polynucleotide of claim 1 or 2.

4. The host cell, plant cell or plant of claim 3 that is also genetically modified to express a triacylglycerol (TAG) synthesising enzyme.

5. A modified oleosin that:
a) is encoded by the polynucleotide of claim 1, or
b) includes at least one artificially introduced cysteine, wherein the cysteine is introduced into at least one of:
a) the N-terminal hydrophilic region of the oleosin, and
b) the C-terminal hydrophilic region of the oleosin.

6. A fusion protein comprising the modified oleosin of claim 5 and a protein of interest.

7. An oil body, emulsion, composition, or composition with a carrier, wherein the oil body, emulsion or composition comprises at least one of:
i) a modified oleosin of claim 5, and
ii) a fusion protein of claim 6.

8. An emulsion, composition, composition with carrier, plant or part thereof, vegetative tissue of a plant, seed of a plant, animal feed, animal feed comprising a plant or a tissue thereof, wherein the composition, plant, plant part, tissue, seed or animal feed comprises an oil body of claim 7.

9. A method for producing an oil body, the method comprising the step of combining:
a) at least two of the modified oleosins of claim 5,
b) triacylglycerol, and
c) phospholipid.

10. The method of claim 9 in which the components of a), b) and c) are combined as specified in at least one of:
i) within a host cell;
ii) within a host cell in which the modified oleosins are expressed;
iii) within the host cell of i) or ii) that is also genetically modified to express a triacyglycerol (TAG) synthesising enzyme;
iv) within the host cell of any one of i) - iii) that forms part of an organism;
v) within the host cell of any one of i) - iii) that forms part of a plant;
vi) within the host cell of any one of i) - iii) that forms part of a plant that accumulates about 50% to about 400% more lipid than does a suitable control plant; and
vii) *in vitro*;
wherein the method optionally includes the additional step of purifying the oil bodies from the cell or organism.

11. A method of producing a plant that accumulates more oil than a suitable control plant, the method comprising providing a plant transformed with a polynucleotide of claim 1, wherein the plant expresses a modified oleosin encoded by the polynucleotide.

12. The method of claim of claim 11 wherein the plant is also transformed with a polynucleotide encoding a TAG synthesising enzyme to express the TAG synthesising enzyme and thus synthesise TAG.

13. The method of claim 11 or 12 wherein the plant accumulates about 50% to about 400% more lipid than does a suitable control plant.

14. The method of any one of claims 11 to 13 wherein the plant is processed into at least one of:
i) an animal feed; and
ii) a biofuel feed stock.

15. A method for producing an oil body in a host cell, the method comprising:
a) introducing into a host cell at least one polynucleotide of claim 1 and a nucleic acid molecule encoding a TAG synthesizing enzyme ; and
b) culturing the host cell in order to express the modified oleosin and the TAG synthesizing enzyme,
wherein the method optionally comprises at least one of the additional steps:
i) processing the host cell into an oil fraction; and
ii) processing the oil into at least one of:
A) a fuel,
B) an oleochemical;
C) a nutritional oil;
D) a cosmetic oil;
E) a polyunsaturated fatty acid (PUFA); and
F) a combination of any one of A) to E).

## Patentansprüche

1. Polynucleotid, das für ein modifiziertes Oleosin kodiert, das zumindest ein künstlich eingeführtes Cystein umfasst, worin das Cystein in zumindest eine aus:
a) der N-terminalen hydrophilen Region des Oleosins und
b) der C-terminalen hydrophilen Region des Oleosins
eingeführt ist.

2. Polynucleotid nach Anspruch 1, worin das Polynucleotid für ein Fusionsprotein kodiert, das das modifizierte Oleosin umfasst, das an ein Protein von Interesse fusioniert ist.

3. Genetisches Konstrukt, Expressionskonstrukt, Wirtszelle, Pflanzenzelle oder Pflanze, worin das Konstrukt, die Zelle oder die Pflanze das Polynucleotid nach Anspruch 1 oder 2 umfasst.

4. Wirtszelle, Pflanzenzelle oder Pflanze nach Anspruch 3, die ebenfalls genetisch modifiziert ist, um ein Triacylglycerin (TAG) synthetisierendes Enzym zu exprimieren.

5. Modifiziertes Oleosin, das:
a) durch das Polynucleotid nach Anspruch 1 kodiert wird oder
b) zumindest ein künstlich eingeführtes Cystein umfasst, worin das Cystein in zumindest eine aus:
a) der N-terminalen hydrophilen Region des Oleosins und
b) der C-terminalen hydrophilen Region des Oleosins
eingeführt ist.

6. Fusionsprotein, umfassend das modifizierte Oleosin nach Anspruch 5 und ein Protein von Interesse.

7. Ölkörper, Emulsion, Zusammensetzung oder Zusammensetzung mit einem Träger, worin der Ölkörper, die Emulsion oder die Zusammensetzung zumindest eines aus:
i) einem modifizierten Oleosin nach Anspruch 5 und
ii) einem Fusionsprotein nach Anspruch 6
umfasst.

8. Emulsion, Zusammensetzung, Zusammensetzung mit einem Träger, Pflanze oder Teil davon, vegetatives Gewebe einer Pflanze, Samen einer Pflanze, Tierfutter, Tierfutter umfassend eine Pflanze oder ein Gewebe davon, worin die Zusammensetzung, die Pflanze, der Pflanzenteil, das Gewebe, der Samen oder das Tierfutter einen Ölkörper nach Anspruch 7 umfasst.

9. Verfahren zur Herstellung eines Ölkörpers, wobei das Verfahren den Schritt des Kombinierens von:
a) zumindest zwei der modifizierten Oleosine nach Anspruch 5,
b) Triacylglycerin und
c) Phospholipid
umfasst.

10. Verfahren nach Anspruch 9, worin die Komponenten von a), b) und c) wie spezifiziert in zumindest einem aus:
i) innerhalb einer Wirtszelle;
ii) innerhalb einer Wirtszelle, in der die modifizierten Oleosine exprimiert werden;
iii) innerhalb der Wirtszelle von i) oder ii), die ebenfalls genetisch modifiziert ist, um ein Triacylglycerin (TAG) synthetisierendes Enzym zu exprimieren.
iv) innerhalb der Wirtszelle von einem beliebigen von i)-iii), die einen Teil eines Organismus bildet;
v) innerhalb der Wirtszelle von einem beliebigen von i)-iii), die einen Teil einer Pflanze bildet;
vi) innerhalb der Wirtszelle von einem beliebigen von i)-iii), die einen Teil einer Pflanze bildet, die etwa 50 % bis etwa 400 % mehr Lipid als eine geeignete Kontrollpflanze ansammelt; und
vii) in vitro;
kombiniert werden, worin das Verfahren gegebenenfalls den zusätzlichen Schritt der Reinigung der Ölkörper von der Zelle oder dem Organismus umfasst.

11. Verfahren zur Herstellung einer Pflanze, die mehr Öl als eine geeignete Kontrollpflanze ansammelt, wobei das Verfahren das Bereitstellen einer mit einem Polynucleotid nach Anspruch 1 transformierten Pflanze umfasst, worin die Pflanze ein modifiziertes Oleosin, für das das Polynucleotid kodiert, exprimiert.

12. Verfahren nach Anspruch 11, worin die Pflanze ebenfalls mit einem Polynucleotid, das für ein TAG-synthetisierendes Enzym kodiert, transformiert wird, um das TAG-synthetisierende Enzym zu exprimieren und somit TAG zu synthetisieren.

13. Verfahren nach Anspruch 11 oder 12, worin die Pflanze etwa 50 % bis etwa 400 % mehr Lipid als eine geeignete Kontrollpflanze ansammelt.

14. Verfahren nach einem der Ansprüche 11 bis 13, worin die Pflanze in zumindest eines von:
i) einem Tierfutter und
ii) einen Biokraftstoffzuführungsbestand
verarbeitet wird.

15. Verfahren zur Herstellung eines Ölkörpers in einer Wirtszelle, wobei das Verfahren Folgendes umfasst:
a) das Einführen von zumindest einem Polynucleotid nach Anspruch 1 und einem Nucleinsäuremolekül, das für ein TAG-synthetisierendes Enzym kodiert, in eine Wirtszelle; und
b) das Kultivieren der Wirtszelle, um das modifizierte Oleosin und das TAG-synthetisierende Enzym zu exprimieren, worin das Verfahren gegebenenfalls zumindest einen der folgenden zusätzlichen Schritte umfasst:
i) das Verarbeiten der Wirtszelle in eine Ölfraktion; und
ii) das Verarbeiten des Öls in zumindest eines aus:
A) einem Kraftstoff,
B) einer oleochemischen Substanz;
C) einem Speiseöl;
D) einem kosmetischen Öl;
E) einer mehrfach ungesättigten Fettsäure (PUFA); und
F) einer Kombination von einem beliebigen von A) bis E).

## Revendications

1. Polynucléotide codant une oléosine modifiée comprenant au moins une cystéine introduite artificiellement, dans lequel la cystéine est introduite dans au moins une parmi
a) la région hydrophile N-terminale de l'oléosine, et
b) la région hydrophile C-terminale de l'oléosine.

2. Polynucléotide selon la revendication 1, dans lequel le polynucléotide comprend une protéine de fusion incluant l'oléosine modifiée fusionnée à une protéine d'intérêt.

3. Construction génétique, construction d'expression, cellule hôte, cellule végétale ou plante, la construction, la cellule ou la plante comprenant le polynucléotide de la revendication 1 ou 2.

4. Cellule hôte, cellule végétale ou plante selon la revendication 3, qui est également modifiée pour exprimer une enzyme synthétisant le triacylglycérol (TAG).

5. Oléosine modifiée qui :
a) est codée par le polynucléotide de la revendication 1,
b) comprend au moins une cystéine introduite artificiellement, dans lequel la cystéine est introduite dans au moins une parmi
a) la région hydrophile N-terminale de l'oléosine, et
b) la région hydrophile C-terminale de l'oléosine.

6. Protéine de fusion comprenant l'oléosine modifiée selon la revendication 5 et une protéine d'intérêt.

7. Corps huileux, émulsion, composition ou composition avec un support, le corps huileux, l'émulsion ou la composition comprenant au moins un élément parmi
i) une oléosine modifiée selon la revendication 5, et
ii) une protéine de fusion selon la revendication 6.

8. Emulsion, composition, composition avec un support, plante ou partie de celle-ci, tissu végétal d'une plante, semence d'une plante, alimentation animale, alimentation animale comprenant une plante ou un tissu de celle-ci, la composition, la plante, la partie de plante, le tissu, la semence ou l'alimentation animale comprenant un corps huileux selon la revendication 7.

9. Procédé de production d'un corps huileux, le procédé comprenant l'étape consistant à combiner :
a) au moins deux des oléosines modifiées selon la revendication 5,
b) du triacylglycérol, et
c) un phospholipide.

10. Procédé selon la revendication 9, dans lequel les composants d'a), b), et c) sont combinés comme spécifié dans au moins une parmi :
i) une cellule hôte ;
ii) une cellule hôte dans laquelle les oléosines modifiées sont exprimées ;
iii) la cellule hôte de i) ou ii) qui est également modifiée pour exprimer une enzyme synthétisant le triacylglycérol (TAG) ;
iv) la cellule hôte selon l'une quelconque des possibilités i) à iii) qui forme une partie d'un organisme ;
V) la cellule hôte de l'une quelconque des possibilités i) à iii) qui forme une partie d'une plante ;
VI) la cellule hôte de l'une quelconque des possibilités i) à iii) qui forme une partie d'une plante qui accumule d'environ 50 % à environ 400 % de lipide en plus qu'une plante témoin adaptée ; et
vii) *in vitro* ;
dans lequel le procédé comprend facultativement l'étape supplémentaire consistant à purifier les corps huileux de la cellule ou de l'organisme.

11. Procédé de production d'une plante qui accumule plus d'huile qu'une plante témoin adaptée, le procédé comprenant l'étape consistant à fournir une plante transformée à l'aide d'un polynucléotide selon la revendication 1, dans lequel la plante exprime une oléosine modifiée codée par le polynucléotide.

12. Procédé selon la revendication 11, dans lequel la plante est également transformée à l'aide d'un polynucléotide codant une enzyme synthétisant le TAG pour exprimer l'enzyme synthétisant le TAG, et synthétise ainsi le TAG.

13. Procédé selon la revendication 11 ou 12, dans lequel la plante accumule d'environ 50 % à environ 400 % de lipide en plus qu'une plante témoin adaptée.

14. Procédé selon l'une quelconque des revendications 11 à 13, dans lequel la plante est transformée en au moins une parmi :
i) une alimentation animale ; et
ii) une matière première de biocarburant.

15. Procédé de production d'un corps huileux dans une cellule hôte, le procédé comprenant les étapes consistant à :
a) introduire dans une cellule hôte au moins un polynucléotide selon la revendication 1 et une molécule d'acide nucléique codant une enzyme synthétisant le TAG ; et
b) mettre en culture la cellule hôte pour exprimer l'oléosine modifiée et l'enzyme synthétisant le TAG, dans lequel le procédé comporte facultativement au moins l'une des étapes supplémentaires suivantes :
i) transformer la cellule hôte en une fraction huileuse ; et
ii) transformer l'huile en au moins un élément parmi :
A) un carburant ;
B) un produit oléochimique ;
C) une huile nutritive ;
D) une huile cosmétique ;
E) un acide gras polyinsaturé (PUFA) ; et
F) une combinaison de plusieurs parmi A) à E).
